(19) Europäisches Patentamt
European Patent Office
Office européen des brevets

(11) EP 4 015 004 A1

(12) EUROPEAN PATENT APPLICATION

(43) Date of publication:
**22.06.2022 Bulletin 2022/25**

(21) Application number: **20215263.3**

(22) Date of filing: **18.12.2020**

(51) International Patent Classification (IPC):
***A61K 47/64*** *(2017.01)* ***A61K 49/00*** *(2006.01)*
***A61P 35/00*** *(2006.01)*

(52) Cooperative Patent Classification (CPC):
**A61P 35/00; A61K 47/641; A61K 47/645; A61K 49/001; A61K 49/0032; A61K 49/0041; A61K 49/0056**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Phi Pharma SA**
**1950 Sion (CH)**

(72) Inventors:
- **Bonny, Christoph**
  **1580 Avenches (CH)**
- **Otten, Luc**
  **1263 Crassier (CH)**
- **Peer, Andreas**
  **1971 Grimisuat (CH)**
- **Mathieu, Marc**
  **1000 Lausanne (CH)**
- **Zorzi, Alessandro**
  **1950 Sion (CH)**
- **Zoete, Vincent**
  **1110 Morges (CH)**
- **Danilo, Maxime**
  **74500 Evian-les-Bains (FR)**
- **Galibert, Laurent**
  **01280 Prévessin (FR)**

(74) Representative: **Vossius & Partner**
**Patentanwälte Rechtsanwälte mbB**
**Siebertstrasse 3**
**81675 München (DE)**

# (54) PROTEOGLYCAN SPECIFIC BRANCHED PEPTIDES

(57) The present invention relates to oligomeric compounds comprising at least two peptide moieties that bind specifically to proteoglycans, their payload conjugates and pharmaceutical compositions containing the same. Instant compounds, payload conjugates and pharmaceutical compositions are particularly useful in the treatment of cancer and in diagnostics.

EP 4 015 004 A1

SEQUENCE LISTING

<110> Phi Pharma

<120> Oligomeric compounds binding specifically to proteoglycans

<130> AD2400 EP S3

<160> 91

<170> BiSSAP 1.3.6

<210> 1
<211> 4
<212> PRT
<213> Artificial Sequence

<220>
<223> Linker1

<220>
<221> VARIANT
<222> 1
<223> Xaa at position 1 is D-His

<400> 1
Xaa Ser Lys Cys
1

<210> 2
<211> 6
<212> PRT
<213> Artificial Sequence

<220>
<223> Linker2

<220>
<221> VARIANT
<222> 1
<223> Xaa at position 1 is D-His

<400> 2
Xaa Ser Lys Ser Lys Cys
1               5

<210> 3
<211> 7
<212> PRT
<213> Artificial Sequence

<220>
<223> Linker3

<220>

<221> VARIANT
<222> 1
<223> Xaa at position 1 is D-His

<220>
<221> VARIANT
<222> 4
<223> Xaa at position 4 is beta-alanine

<400> 3
Xaa Ser Lys Xaa Ser Lys Cys
1 5

<210> 4
<211> 7
<212> PRT
<213> Artificial Sequence

<220>
<223> Linker4

<220>
<221> VARIANT
<222> 1
<223> Xaa at position 1 is D-His

<220>
<221> VARIANT
<222> 5
<223> Xaa at position 5 is beta-alanine

<400> 4
Xaa Ser Lys Ser Xaa Lys Cys
1 5

<210> 5
<211> 6
<212> PRT
<213> Artificial Sequence

<220>
<223> Linker5

<220>
<221> VARIANT
<222> 1
<223> Xaa at position 1 is D-His

<220>
<221> VARIANT
<222> 5
<223> Lys at position 5 is connected to Cys at position 6 via a PEG1, PEG5, PEG16 or PEG36 linker

<400> 5
Xaa Ser Lys Ser Lys Cys

1 5

<210> 6
<211> 4
<212> PRT
<213> Artificial Sequence

<220>
<223> Linker6

<220>
<221> VARIANT
<222> 1
<223> Xaa at position 1 is D-His

<220>
<221> VARIANT
<222> 3
<223> Lys at position 3 is connected to Cys at position 4 via a PEG1, PEG5, PEG16 or PEG36 linker

<400> 6
Xaa Ser Lys Cys
1

<210> 7
<211> 5
<212> PRT
<213> Artificial Sequence

<220>
<223> Linker7

<220>
<221> VARIANT
<222> 1
<223> Xaa at position 1 is D-His

<220>
<221> VARIANT
<222> 3
<223> Lys at position 3 is connected to Lys at position 4 via a PEG1, PEG3 or PEG5 linker

<400> 7
Xaa Ser Lys Lys Cys
1 5

<210> 8
<211> 3
<212> PRT
<213> Artificial Sequence

<220>
<223> Linker8

<220>
<221> VARIANT
<222> 1
<223> Xaa at position 1 is D-His, wherein D-His is connected to L-Lys at position 2 via a PEG2 or PEG5 linker

<400> 8
Xaa Lys Cys
1

<210> 9
<211> 6
<212> PRT
<213> Artificial Sequence

<220>
<223> Linker9

<220>
<221> VARIANT
<222> 1
<223> Xaa at position 1 is D-His

<220>
<221> VARIANT
<222> 3
<223> Xaa at position 3 is an amino acid comprising two primary amine groups

<220>
<221> VARIANT
<222> 5
<223> Xaa at position 5 is an amino acid comprising two primary amine groups

<400> 9
Xaa Ser Xaa Ser Xaa Cys
1               5

<210> 10
<211> 4
<212> PRT
<213> Artificial Sequence

<220>
<223> Linker10

<220>
<221> VARIANT
<222> 1
<223> Xaa at position 1 is D-His

<220>
<221> VARIANT
<222> 3

<223> Xaa at position 3 is an amino acid comprising two primary amine groups

<400> 10
Xaa Ser Xaa Cys
1

<210> 11
<211> 6
<212> PRT
<213> Artificial Sequence

<220>
<223> Linker11

<220>
<221> VARIANT
<222> 1
<223> Xaa at position 1 is D-His

<400> 11
Xaa Ser Lys Gly Lys Cys
1               5

<210> 12
<211> 6
<212> PRT
<213> Artificial Sequence

<220>
<223> Linker12

<220>
<221> VARIANT
<222> 1
<223> Xaa at position 1 is D-His

<400> 12
Xaa Gly Lys Ser Lys Cys
1               5

<210> 13
<211> 6
<212> PRT
<213> Artificial Sequence

<220>
<223> Linker13

<220>
<221> VARIANT
<222> 1
<223> Xaa at position 1 is D-His

<400> 13

Xaa Gly Lys Gly Lys Cys
1 5

<210> 14
<211> 4
<212> PRT
<213> Artificial Sequence

<220>
<223> Linker14

<220>
<221> VARIANT
<222> 1
<223> Xaa at position 1 is D-His

<400> 14
Xaa Gly Lys Cys
1

<210> 15
<211> 6
<212> PRT
<213> Artificial Sequence

<220>
<223> Linker15

<220>
<221> VARIANT
<222> 1
<223> Xaa at position 1 is D-His

<220>
<221> VARIANT
<222> 3
<223> Xaa at position 3 is an amino acid comprising two primary amine groups

<220>
<221> VARIANT
<222> 5
<223> Xaa at position 5 is an amino acid comprising two primary amine groups

<400> 15
Xaa Ser Xaa Gly Xaa Cys
1 5

<210> 16
<211> 6
<212> PRT
<213> Artificial Sequence

<220>

<223> Linker16

<220>
<221> VARIANT
<222> 1
<223> Xaa at position 1 is D-His

<220>
<221> VARIANT
<222> 3
<223> Xaa at position 3 is an amino acid comprising two primary amine groups

<220>
<221> VARIANT
<222> 5
<223> Xaa at position 5 is an amino acid comprising two primary amine groups

<400> 16
Xaa Gly Xaa Ser Xaa Cys
1               5

<210> 17
<211> 6
<212> PRT
<213> Artificial Sequence

<220>
<223> Linker17

<220>
<221> VARIANT
<222> 1
<223> Xaa at position 1 is D-His

<220>
<221> VARIANT
<222> 3
<223> Xaa at position 3 is an amino acid comprising two primary amine groups

<220>
<221> VARIANT
<222> 5
<223> Xaa at position 5 is an amino acid comprising two primary amine groups

<400> 17
Xaa Gly Xaa Gly Xaa Cys
1               5

<210> 18
<211> 4
<212> PRT
<213> Artificial Sequence

<220>
<223> Linker18

<220>
<221> VARIANT
<222> 1
<223> Xaa at position 1 is D-His

<220>
<221> VARIANT
<222> 3
<223> Xaa at position 3 is an amino acid comprising two primary amine groups

<400> 18
Xaa Gly Xaa Cys
1

<210> 19
<211> 4
<212> PRT
<213> Artificial Sequence

<220>
<223> Linker19

<220>
<221> VARIANT
<222> 1
<223> Xaa at position 1 is D-His

<220>
<221> VARIANT
<222> 4
<223> Xaa at position 4 is L-propargylglycine

<400> 19
Xaa Ser Lys Xaa
1

<210> 20
<211> 6
<212> PRT
<213> Artificial Sequence

<220>
<223> Linker20

<220>
<221> VARIANT
<222> 1
<223> Xaa at position 1 is D-His

<220>
<221> VARIANT

<222> 6
<223> Xaa at position 6 is L-propargylglycine

<400> 20
Xaa Ser Lys Ser Lys Xaa
1               5

<210> 21
<211> 4
<212> PRT
<213> Artificial Sequence

<220>
<223> Linker21

<220>
<221> VARIANT
<222> 1
<223> Xaa at position 1 is D-His

<220>
<221> VARIANT
<222> 4
<223> Xaa at position 4 is L-biocytin

<400> 21
Xaa Ser Lys Xaa
1

<210> 22
<211> 6
<212> PRT
<213> Artificial Sequence

<220>
<223> Linker22

<220>
<221> VARIANT
<222> 1
<223> Xaa at position 1 is D-His

<220>
<221> VARIANT
<222> 6
<223> Xaa at position 6 is L-biocytin

<400> 22
Xaa Ser Lys Ser Lys Xaa
1               5

<210> 23
<211> 3
<212> PRT
<213> Artificial Sequence

<220>
<223> Linker23

<220>
<221> VARIANT
<222> 1
<223> Xaa at position 1 is D-His

<400> 23
Xaa Lys Cys
1

<210> 24
<211> 5
<212> PRT
<213> Artificial Sequence

<220>
<223> Linker24

<220>
<221> VARIANT
<222> 1
<223> Xaa at position 1 is D-His

<400> 24
Xaa Lys Ser Lys Cys
1 5

<210> 25
<211> 3
<212> PRT
<213> Artificial Sequence

<220>
<223> Linker25

<220>
<221> VARIANT
<222> 1
<223> Xaa at position 1 is D-His

<220>
<221> VARIANT
<222> 2
<223> Lys at position 2 is connected to Cys at position 3 via a PEG1 linker

<400> 25
Xaa Lys Cys
1

<210> 26
<211> 5
<212> PRT

<213> Artificial Sequence

<220>
<223> Linker26

<220>
<221> VARIANT
<222> 1
<223> Xaa at position 1 is D-His

<220>
<221> VARIANT
<222> 4
<223> Lys at position 4 is connected to Cys at position 5 via a PEG1 linker

<400> 26
Xaa Lys Ser Lys Cys
1               5

<210> 27
<211> 5
<212> PRT
<213> Artificial Sequence

<220>
<223> Linker27

<220>
<221> VARIANT
<222> 1
<223> Xaa at position 1 is D-His

<400> 27
Xaa Ser Ser Lys Cys
1               5

<210> 28
<211> 7
<212> PRT
<213> Artificial Sequence

<220>
<223> Linker28

<220>
<221> VARIANT
<222> 1
<223> Xaa at position 1 is D-His

<400> 28
Xaa Ser Ser Lys Ser Lys Cys
1               5

<210> 29

<211> 6
<212> PRT
<213> Artificial Sequence

<220>
<223> Linker29

<220>
<221> VARIANT
<222> 1
<223> Xaa at position 1 is D-His

<400> 29
Xaa Ser Lys Ser Ser Cys
1 5

<210> 30
<211> 8
<212> PRT
<213> Artificial Sequence

<220>
<223> Linker 30

<220>
<221> VARIANT
<222> 1
<223> Xaa at position 1 is D-His

<400> 30
Xaa Ser Lys Ser Lys Ser Ser Cys
1 5

<210> 31
<211> 3
<212> PRT
<213> Artificial Sequence

<220>
<223> Linker31

<220>
<221> VARIANT
<222> 1
<223> Xaa at position 1 is D-His

<220>
<221> VARIANT
<222> 3
<223> L-Lys at position 3 is connected to L-Ser-D-His via the primary amine group in the L-Lys side chain

<400> 31
Xaa Ser Lys
1

<210> 32
<211> 3
<212> PRT
<213> Artificial Sequence

<220>
<223> Linker32

<220>
<221> VARIANT
<222> 1
<223> Xaa at position 1 is D-His

<220>
<221> VARIANT
<222> 3
<223> L-Lys at position 3 is connected to L-Ser-L-Lys[ε-N-L-Ser-D-His]-L-Ser-D-His via the primary amine group in the L-Lys side chain

<400> 32
Xaa Ser Lys
1

<210> 33
<211> 5
<212> PRT
<213> Artificial Sequence

<220>
<223> Linker33

<220>
<221> VARIANT
<222> 1
<223> Xaa at position 1 is D-His

<220>
<221> VARIANT
<222> 3
<223> L-Lys at position 3 is connected to L-Ser-D-His via the primary amine group in the L-Lys side chain

<220>
<221> VARIANT
<222> 5
<223> L-Lys at position 5 is connected to L-Ser-D-His via the primary amine group in the L-Lys side chain

<400> 33
Xaa Ser Lys Ser Lys
1               5

<210> 34
<211> 5

<212> PRT
<213> Artificial Sequence

<220>
<223> Linker34

<220>
<221> VARIANT
<222> 1
<223> Xaa at position 1 is D-His

<220>
<221> VARIANT
<222> 3
<223> L-Lys at position 3 is connected to L-Ser-L-Lys[ε-N-L-Ser-D-His]-L-Ser-D-His via the primary amine group in the L-Lys side chain

<220>
<221> VARIANT
<222> 5
<223> L-Lys at position 5 is connected to L-Ser-D-His via the primary amine group in the L-Lys side chain

<400> 34
Xaa Ser Lys Ser Lys
1               5

<210> 35
<211> 5
<212> PRT
<213> Artificial Sequence

<220>
<223> Linker35

<220>
<221> VARIANT
<222> 1
<223> Xaa at position 1 is D-His

<220>
<221> VARIANT
<222> 3
<223> L-Lys at position 3 is connected to L-Ser-D-His via the primary amine group in the L-Lys side chain

<220>
<221> VARIANT
<222> 5
<223> L-Lys at position 5 is connected to L-Ser-L-Lys[ε-N-L-Ser-D-His]-L-Ser-D-His via the primary amine group in the L-Lys side chain

<400> 35
Xaa Ser Lys Ser Lys

1 5

<210> 36
<211> 5
<212> PRT
<213> Artificial Sequence

<220>
<223> Linker36

<220>
<221> VARIANT
<222> 1
<223> Xaa at position 1 is D-His

<220>
<221> VARIANT
<222> 3
<223> L-Lys at position 3 is connected to L-Ser-L-Lys[ε-N-L-Ser-D-His]-L-Ser-D-His via the primary amine group in the L-Lys side chain

<220>
<221> VARIANT
<222> 5
<223> L-Lys at position 5 is connected to L-Ser-L-Lys[ε-N-L-Ser-D-His]-L-Ser-D-His via the primary amine group in the L-Lys side chain

<400> 36
Xaa Ser Lys Ser Lys
1 5

<210> 37
<211> 9
<212> PRT
<213> Artificial Sequence

<220>
<223> Artificial binding peptide 1

<220>
<221> VARIANT
<222> 4
<223> Xaa at position 4 is D-Val

<220>
<221> VARIANT
<222> 7
<223> Xaa at position 7 is L-Ornithine

<400> 37
Arg Tyr Phe Xaa Arg Ile Xaa Tyr Arg
1 5

<210> 38

<211> 9
<212> PRT
<213> Artificial Sequence

<220>
<223> Artificial binding peptide 2

<220>
<221> VARIANT
<222> 4
<223> Xaa at position 4 is D-Val

<400> 38
Arg Arg Phe Xaa Tyr Ile Tyr Arg Arg
1               5

<210> 39
<211> 9
<212> PRT
<213> Artificial Sequence

<220>
<223> Artificial binding peptide 3

<220>
<221> VARIANT
<222> 4
<223> Xaa at position 4 is D-Val

<400> 39
Arg Tyr Phe Xaa Arg Ile Tyr Arg Arg
1               5

<210> 40
<211> 9
<212> PRT
<213> Artificial Sequence

<220>
<223> Artificial binding peptide 4

<220>
<221> VARIANT
<222> 4
<223> Xaa at position 4 is D-Val

<220>
<221> VARIANT
<222> 5
<223> Xaa at position 5 is D-Arg

<220>
<221> VARIANT
<222> 7
<223> Xaa at position 7 is L-Ornithine

<400> 40
Arg Tyr Phe Xaa Xaa Ile Xaa Tyr Arg
1               5

<210> 41
<211> 9
<212> PRT
<213> Artificial Sequence

<220>
<223> Artificial binding peptide 5

<220>
<221> VARIANT
<222> 4
<223> Xaa at position 4 is D-Val

<220>
<221> VARIANT
<222> 6
<223> Xaa at position 6 is D-Ile

<220>
<221> VARIANT
<222> 7
<223> Xaa at position 7 is L-Ornithine

<400> 41
Arg Tyr Phe Xaa Arg Xaa Xaa Tyr Arg
1               5

<210> 42
<211> 9
<212> PRT
<213> Artificial Sequence

<220>
<223> Artificial binding peptide 6

<220>
<221> VARIANT
<222> 4
<223> Xaa at position 4 is D-Val

<220>
<221> VARIANT
<222> 5
<223> Xaa at position 5 is D-Arg

<220>
<221> VARIANT
<222> 6
<223> Xaa at position 6 is D-Ile

<220>

<221> VARIANT
<222> 7
<223> Xaa at position 7 is L-Ornithine

<400> 42
Arg Tyr Phe Xaa Xaa Xaa Xaa Tyr Arg
1               5

<210> 43
<211> 9
<212> PRT
<213> Artificial Sequence

<220>
<223> Artificial binding peptide 7

<220>
<221> VARIANT
<222> 4
<223> Xaa at position 4 is D-Val

<400> 43
Arg Arg Phe Xaa Tyr Ile Tyr Lys Arg
1               5

<210> 44
<211> 9
<212> PRT
<213> Artificial Sequence

<220>
<223> Artificial binding peptide 8

<220>
<221> VARIANT
<222> 4
<223> Xaa at position 4 is D-Gln

<400> 44
Arg Arg Phe Xaa Tyr Ile Tyr Lys Arg
1               5

<210> 45
<211> 9
<212> PRT
<213> Artificial Sequence

<220>
<223> Artificial binding peptide 9

<220>
<221> VARIANT
<222> 4
<223> Xaa at position 4 is D-Val

<400> 45
Arg Tyr Phe Xaa Arg Ile Tyr Lys Arg
1 5

<210> 46
<211> 9
<212> PRT
<213> Artificial Sequence

<220>
<223> Artificial binding peptide 10

<220>
<221> VARIANT
<222> 4
<223> Xaa at position 4 is D-Val

<400> 46
Arg Tyr Phe Xaa Arg Ile Tyr Arg Lys
1 5

<210> 47
<211> 9
<212> PRT
<213> Artificial Sequence

<220>
<223> Artificial binding peptide 11

<220>
<221> VARIANT
<222> 4
<223> Xaa at position 4 is D-Ala

<400> 47
Arg Arg Phe Xaa Tyr Ile Tyr Lys Arg
1 5

<210> 48
<211> 9
<212> PRT
<213> Artificial Sequence

<220>
<223> Artificial binding peptide 12

<220>
<221> VARIANT
<222> 4
<223> Xaa at position 4 is D-Pro

<400> 48
Arg Arg Phe Xaa Tyr Ile Tyr Lys Arg
1 5

<210> 49
<211> 9
<212> PRT
<213> Artificial Sequence

<220>
<223> Artificial binding peptide 13

<220>
<221> VARIANT
<222> 4
<223> Xaa at position 4 is D-Val

<400> 49
Arg Tyr Phe Xaa Arg Ile Lys Tyr Arg
1               5

<210> 50
<211> 9
<212> PRT
<213> Artificial Sequence

<220>
<223> Artificial binding peptide 14

<220>
<221> VARIANT
<222> 4
<223> Xaa at position 4 is D-Gln

<400> 50
Arg Tyr Phe Xaa Arg Ile Lys Tyr Arg
1               5

<210> 51
<211> 8
<212> PRT
<213> Artificial Sequence

<220>
<223> Artificial binding peptide 15

<220>
<221> VARIANT
<222> 3
<223> Xaa at position 3 is D-Val

<220>
<221> VARIANT
<222> 6
<223> Xaa at position 6 is L-Ornithine

<400> 51
Tyr Phe Xaa Arg Ile Xaa Tyr Arg
1               5

<210> 52
<211> 9
<212> PRT
<213> Artificial Sequence

<220>
<223> Artificial binding peptide 16

<220>
<221> VARIANT
<222> 1
<223> Xaa at position 1 is L-2-amino-4-guanidinobutyric acid

<220>
<221> VARIANT
<222> 4
<223> Xaa at position 4 is D-Val

<220>
<221> VARIANT
<222> 5
<223> Xaa at position 5 is L-2-amino-4-guanidinobutyric acid

<220>
<221> VARIANT
<222> 9
<223> Xaa at position 9 is L-2-amino-4-guanidinobutyric acid

<400> 52
Xaa Tyr Phe Xaa Xaa Ile Lys Tyr Xaa
1               5

<210> 53
<211> 8
<212> PRT
<213> Artificial Sequence

<220>
<223> Artificial binding peptide 17

<220>
<221> VARIANT
<222> 3
<223> Xaa at position 3 is D-Val

<400> 53
Tyr Phe Xaa Arg Ile Lys Tyr Arg
1               5

<210> 54
<211> 9
<212> PRT
<213> Artificial Sequence

<220>
<223> Artificial binding peptide 18

<220>
<221> VARIANT
<222> 1
<223> Xaa at position 1 is D-Arg

<220>
<221> VARIANT
<222> 4
<223> Xaa at position 4 is D-Val

<400> 54
Xaa Tyr Phe Xaa Arg Ile Lys Tyr Arg
1               5

<210> 55
<211> 9
<212> PRT
<213> Artificial Sequence

<220>
<223> Artificial binding peptide 19

<220>
<221> VARIANT
<222> 4
<223> Xaa at position 4 is D-Val

<220>
<221> VARIANT
<222> 5
<223> Xaa at position 5 is D-Arg

<400> 55
Arg Tyr Phe Xaa Xaa Ile Lys Tyr Arg
1               5

<210> 56
<211> 9
<212> PRT
<213> Artificial Sequence

<220>
<223> Artificial binding peptide 20

<220>
<221> VARIANT
<222> 1
<223> Xaa at position 1 is D-Arg

<220>
<221> VARIANT
<222> 2
<223> Xaa at position 2 is D-Arg

<220>
<221> VARIANT
<222> 4
<223> Xaa at position 2 is D-Val

<400> 56
Xaa Xaa Phe Xaa Tyr Ile Tyr Arg Arg
1 5

<210> 57
<211> 9
<212> PRT
<213> Artificial Sequence

<220>
<223> Artificial binding peptide 21

<220>
<221> VARIANT
<222> 4
<223> Xaa at position 4 is D-Gln

<400> 57
Arg Ser Thr Xaa Arg Tyr Arg Val Arg
1 5

<210> 58
<211> 9
<212> PRT
<213> Artificial Sequence

<220>
<223> Artificial binding peptide 22

<220>
<221> VARIANT
<222> 4
<223> Xaa at position 4 is D-Val

<400> 58
Arg Tyr Phe Xaa Arg Ile Lys Tyr Arg
1 5

<210> 59
<211> 9
<212> PRT
<213> Artificial Sequence

<220>
<223> Artificial binding peptide 23

<220>
<221> VARIANT
<222> 4

<223> Xaa at position 4 is D-Val

<400> 59
Arg Tyr Phe Xaa Arg Ile Lys Ala Arg
1 5

<210> 60
<211> 9
<212> PRT
<213> Artificial Sequence

<220>
<223> Artificial binding peptide 24

<220>
<221> VARIANT
<222> 4
<223> Xaa at position 4 is D-Val

<400> 60
Arg Ala Ala Xaa Arg Ala Lys Tyr Arg
1 5

<210> 61
<211> 9
<212> PRT
<213> Artificial Sequence

<220>
<223> Artificial binding peptide 25

<220>
<221> VARIANT
<222> 4
<223> Xaa at position 4 is D-Val

<400> 61
Arg Ala Ala Xaa Arg Ile Lys Tyr Arg
1 5

<210> 62
<211> 9
<212> PRT
<213> Artificial Sequence

<220>
<223> Artificial binding peptide 26

<220>
<221> VARIANT
<222> 4
<223> Xaa at position 4 is D-Gln

<400> 62
Arg Ser Thr Xaa Arg Tyr Arg Val Arg

1 5

<210> 63
<211> 9
<212> PRT
<213> Artificial Sequence

<220>
<223> Artificial binding peptide 27

<220>
<221> VARIANT
<222> 4
<223> Xaa at position 4 is D-Gln

<400> 63
Arg Ser Thr Xaa Arg Tyr Lys Val Arg
1 5

<210> 64
<211> 9
<212> PRT
<213> Artificial Sequence

<220>
<223> Artificial binding peptide 28

<220>
<221> VARIANT
<222> 4
<223> Xaa at position 4 is D-Gly

<400> 64
Arg Gly Gly Xaa Arg Gly Lys Gly Arg
1 5

<210> 65
<211> 9
<212> PRT
<213> Artificial Sequence

<220>
<223> Artificial binding peptide 29

<220>
<221> VARIANT
<222> 4
<223> Xaa at position 4 is D-His

<400> 65
Arg His His Xaa Arg His Lys His Arg
1 5

<210> 66
<211> 9

<212> PRT
<213> Artificial Sequence

<220>
<223> Artificial binding peptide 30

<220>
<221> VARIANT
<222> 4
<223> Xaa at position 4 is D-Val

<400> 66
Arg Val Val Xaa Arg Val Lys Val Arg
1               5

<210> 67
<211> 9
<212> PRT
<213> Artificial Sequence

<220>
<223> Artificial binding peptide 31

<400> 67
Arg Leu Leu Ile Arg Leu Lys Leu Arg
1               5

<210> 68
<211> 9
<212> PRT
<213> Artificial Sequence

<220>
<223> Artificial binding peptide 32

<220>
<221> VARIANT
<222> 4
<223> Xaa at position 4 is D-Met

<400> 68
Arg Met Met Xaa Arg Met Lys Met Arg
1               5

<210> 69
<211> 9
<212> PRT
<213> Artificial Sequence

<220>
<223> Artificial binding peptide 33

<220>
<221> VARIANT

<222> 4
<223> Xaa at position 4 is D-Ile

<400> 69
Arg Ile Ile Xaa Arg Ile Lys Ile Arg
1               5

<210> 70
<211> 9
<212> PRT
<213> Artificial Sequence

<220>
<223> Artificial binding peptide 34

<220>
<221> VARIANT
<222> 6
<223> Xaa at position 6 is D-Ile

<400> 70
Arg Tyr Phe Val Arg Xaa Lys Tyr Arg
1               5

<210> 71
<211> 9
<212> PRT
<213> Artificial Sequence

<220>
<223> Artificial binding peptide 35

<220>
<221> VARIANT
<222> 4
<223> Xaa at position 4 is D-Val

<400> 71
Arg Tyr Lys Xaa Phe Ile Lys Tyr Arg
1               5

<210> 72
<211> 9
<212> PRT
<213> Artificial Sequence

<220>
<223> Artificial binding peptide 36

<220>
<221> VARIANT
<222> 4
<223> Xaa at position 4 is D-Val

<400> 72

Arg Tyr Lys Xaa Ala Ile Lys Tyr Arg
1 5

<210> 73
<211> 9
<212> PRT
<213> Artificial Sequence

<220>
<223> Artificial binding peptide 37

<220>
<221> VARIANT
<222> 4
<223> Xaa at position 4 is D-Val

<400> 73
Arg Tyr Lys Xaa Arg Ile Ala Tyr Arg
1 5

<210> 74
<211> 9
<212> PRT
<213> Artificial Sequence

<220>
<223> Artificial binding peptide 38

<220>
<221> VARIANT
<222> 4
<223> Xaa at position 4 is D-Val

<400> 74
Arg Tyr Lys Xaa Arg Ile Phe Tyr Arg
1 5

<210> 75
<211> 9
<212> PRT
<213> Artificial Sequence

<220>
<223> Artificial binding peptide 39

<220>
<221> VARIANT
<222> 4
<223> Xaa at position 4 is D-Val

<400> 75
Arg Tyr Lys Xaa Lys Phe Ile Tyr Arg
1 5

<210> 76

<211> 9
<212> PRT
<213> Artificial Sequence

<220>
<223> Artificial binding peptide 40

<220>
<221> VARIANT
<222> 4
<223> Xaa at position 4 is D-Val

<400> 76
Arg Tyr Lys Xaa Phe Ile Arg Tyr Arg
1               5

<210> 77
<211> 9
<212> PRT
<213> Artificial Sequence

<220>
<223> Artificial binding peptide 41

<220>
<221> VARIANT
<222> 4
<223> Xaa at position 4 is D-Val

<400> 77
Arg Tyr Lys Xaa Arg Phe Ile Tyr Arg
1               5

<210> 78
<211> 9
<212> PRT
<213> Artificial Sequence

<220>
<223> Artificial binding peptide 42

<220>
<221> VARIANT
<222> 4
<223> Xaa at position 4 is D-Ile

<400> 78
Arg Met Lys Xaa Val Met Lys Phe Arg
1               5

<210> 79
<211> 9
<212> PRT
<213> Artificial Sequence

<220>
<223> Artificial binding peptide 43

<220>
<221> VARIANT
<222> 4
<223> Xaa at position 4 is D-Phe

<400> 79
Arg Phe Lys Xaa Phe Phe Lys Phe Arg
1               5

<210> 80
<211> 7
<212> PRT
<213> Artificial Sequence

<220>
<223> Artificial binding peptide 44

<220>
<221> VARIANT
<222> 4
<223> Xaa at position 4 is D-Val

<400> 80
Arg Tyr Lys Xaa Arg Ile Lys
1               5

<210> 81
<211> 9
<212> PRT
<213> Artificial Sequence

<220>
<223> Artificial binding peptide 45

<220>
<221> VARIANT
<222> 4
<223> Xaa at position 4 is D-Val

<400> 81
Arg Tyr Lys Xaa Arg Ile Lys Tyr Phe
1               5

<210> 82
<211> 9
<212> PRT
<213> Artificial Sequence

<220>
<223> Artificial binding peptide 46

<220>
<221> VARIANT
<222> 4
<223> Xaa at position 4 is D-Val

<400> 82
Arg Tyr Lys Xaa Arg Ile Lys Tyr Ala
1               5

<210> 83
<211> 9
<212> PRT
<213> Artificial Sequence

<220>
<223> Artificial binding peptide 47

<220>
<221> VARIANT
<222> 4
<223> Xaa at position 4 is D-Val

<400> 83
Arg Tyr Lys Xaa Arg Ile Lys His His
1               5

<210> 84
<211> 7
<212> PRT
<213> Artificial Sequence

<220>
<223> Artificial binding peptide 45

<220>
<221> VARIANT
<222> 4
<223> Xaa at position 4 is D-Ile

<400> 84
Arg Met Lys Xaa Arg Val Lys
1               5

<210> 85
<211> 9
<212> PRT
<213> Artificial Sequence

<220>
<223> Artificial binding peptide 46

<220>
<221> VARIANT
<222> 4
<223> Xaa at position 4 is D-Ile

<400> 85
Arg Met Lys Xaa Arg Val Lys Phe Met
1 5

<210> 86
<211> 9
<212> PRT
<213> Artificial Sequence

<220>
<223> Artificial binding peptide 47

<400> 86
Arg Leu Lys Ile Arg Leu Lys Leu Leu
1 5

<210> 87
<211> 9
<212> PRT
<213> Artificial Sequence

<220>
<223> Artificial binding peptide 48

<220>
<221> VARIANT
<222> 4
<223> Xaa at position 4 is D-Val

<220>
<221> VARIANT
<222> 9
<223> Xaa at position 9 is D-Arg

<400> 87
Arg Tyr Lys Xaa Arg Ile Lys Tyr Xaa
1 5

<210> 88
<211> 261
<212> PRT
<213> Homo sapiens

<220>
<223> bcl-2-binding component 3 isoform 1

<400> 88
Met Lys Phe Gly Met Gly Ser Ala Gln Ala Cys Pro Cys Gln Val Pro
1 5 10 15
Arg Ala Ala Ser Thr Thr Trp Val Pro Cys Gln Ile Cys Gly Pro Arg
20 25 30
Glu Arg His Gly Pro Arg Thr Pro Gly Gly Gln Leu Pro Gly Ala Arg
35 40 45
Arg Gly Pro Gly Pro Arg Arg Pro Ala Pro Leu Pro Ala Arg Pro Pro

```
    50                  55                  60
Gly Ala Leu Gly Ser Val Leu Arg Pro Leu Arg Ala Arg Pro Gly Cys
65                  70                  75                  80
Arg Pro Arg Arg Pro His Pro Ala Ala Arg Cys Leu Pro Leu Arg Pro
                85                  90                  95
His Arg Pro Thr Arg Arg His Arg Arg Pro Gly Gly Phe Pro Leu Ala
            100                 105                 110
Trp Gly Ser Pro Gln Pro Ala Pro Arg Pro Ala Pro Gly Arg Ser Ser
        115                 120                 125
Ala Leu Ala Leu Ala Gly Gly Ala Ala Pro Gly Val Ala Arg Ala Gln
    130                 135                 140
Arg Pro Gly Gly Ser Gly Gly Arg Ser His Pro Gly Gly Pro Gly Ser
145                 150                 155                 160
Pro Arg Gly Gly Gly Thr Val Gly Pro Gly Asp Arg Gly Pro Ala Ala
                165                 170                 175
Ala Asp Gly Gly Arg Pro Gln Arg Thr Val Arg Ala Ala Glu Thr Arg
            180                 185                 190
Gly Ala Ala Ala Ala Pro Pro Leu Thr Leu Glu Gly Pro Val Gln Ser
        195                 200                 205
His His Gly Thr Pro Ala Leu Thr Gln Gly Pro Gln Ser Pro Arg Asp
    210                 215                 220
Gly Ala Gln Leu Gly Ala Cys Thr Arg Pro Val Asp Val Arg Asp Ser
225                 230                 235                 240
Gly Gly Arg Pro Leu Pro Pro Pro Asp Thr Leu Ala Ser Ala Gly Asp
                245                 250                 255
Phe Leu Cys Thr Met
            260
```

<210> 89
<211> 131
<212> PRT
<213> Homo sapiens

<220>
<223> bcl-2-binding component 3 isoform 2

<400> 89

```
Met Lys Phe Gly Met Gly Ser Ala Gln Ala Cys Pro Cys Gln Val Pro
1               5                   10                  15
Arg Ala Ala Ser Thr Thr Trp Val Pro Cys Gln Ile Cys Gly Pro Gln
            20                  25                  30
Pro Ser Leu Ser Leu Ala Glu Gln His Leu Glu Ser Pro Val Pro Ser
        35                  40                  45
Ala Pro Gly Ala Leu Ala Gly Gly Pro Thr Gln Ala Ala Pro Gly Val
    50                  55                  60
Arg Gly Glu Glu Glu Gln Trp Ala Arg Glu Ile Gly Ala Gln Leu Arg
65                  70                  75                  80
Arg Met Ala Asp Asp Leu Asn Ala Gln Tyr Glu Arg Arg Arg Gln Glu
                85                  90                  95
Glu Gln Gln Arg His Arg Pro Ser Pro Trp Arg Val Leu Tyr Asn Leu
            100                 105                 110
Ile Met Gly Leu Leu Pro Leu Pro Arg Gly His Arg Ala Pro Glu Met
        115                 120                 125
Glu Pro Asn
    130
```

<210> 90

<211> 101
<212> PRT
<213> Homo sapiens

<220>
<223> bcl-2-binding component 3 isoform 3

<400> 90
```
Met Lys Phe Gly Met Gly Ser Ala Gln Ala Cys Pro Cys Gln Val Pro
1               5                   10                  15
Arg Ala Ala Ser Thr Thr Trp Val Pro Cys Gln Ile Cys Glu Thr Arg
            20                  25                  30
Gly Ala Ala Ala Ala Pro Pro Leu Thr Leu Glu Gly Pro Val Gln Ser
        35                  40                  45
His His Gly Thr Pro Ala Leu Thr Gln Gly Pro Gln Ser Pro Arg Asp
    50                  55                  60
Gly Ala Gln Leu Gly Ala Cys Thr Arg Pro Val Asp Val Arg Asp Ser
65                  70                  75                  80
Gly Gly Arg Pro Leu Pro Pro Pro Asp Thr Leu Ala Ser Ala Gly Asp
                85                  90                  95
Phe Leu Cys Thr Met
            100
```

<210> 91
<211> 193
<212> PRT
<213> Homo sapiens

<220>
<223> bcl-2-binding component 3 isoform 4

<400> 91
```
Met Ala Arg Ala Arg Gln Glu Gly Ser Ser Pro Glu Pro Val Glu Gly
1               5                   10                  15
Leu Ala Arg Asp Gly Pro Arg Pro Phe Pro Leu Gly Arg Leu Val Pro
            20                  25                  30
Ser Ala Val Ser Cys Gly Leu Cys Glu Pro Gly Leu Ala Ala Ala Pro
        35                  40                  45
Ala Ala Pro Thr Leu Leu Pro Ala Ala Tyr Leu Cys Ala Pro Thr Ala
    50                  55                  60
Pro Pro Ala Val Thr Ala Ala Leu Gly Gly Ser Arg Trp Pro Gly Gly
65                  70                  75                  80
Pro Arg Ser Arg Pro Arg Gly Pro Arg Pro Asp Gly Pro Gln Pro Ser
                85                  90                  95
Leu Ser Leu Ala Glu Gln His Leu Glu Ser Pro Val Pro Ser Ala Pro
            100                 105                 110
Gly Ala Leu Ala Gly Gly Pro Thr Gln Ala Ala Pro Gly Val Arg Gly
        115                 120                 125
Glu Glu Glu Gln Trp Ala Arg Glu Ile Gly Ala Gln Leu Arg Arg Met
    130                 135                 140
Ala Asp Asp Leu Asn Ala Gln Tyr Glu Arg Arg Arg Gln Glu Glu Gln
145                 150                 155                 160
Gln Arg His Arg Pro Ser Pro Trp Arg Val Leu Tyr Asn Leu Ile Met
                165                 170                 175
Gly Leu Leu Pro Leu Pro Arg Gly His Arg Ala Pro Glu Met Glu Pro
            180                 185                 190
```

Asn

## Description

### Field of the invention

The present invention relates to oligomeric compounds comprising at least two peptide moieties, which may act as transporter molecules specifically recognizing proteoglycans, in particular, chondroitin-4-sulfate (C4S), chondroitin-6-sulfate (C6S) and/or keratan sulfate (KS). The compounds of the invention may be conjugated or otherwise linked to a payload, such as a biologically active moiety (BAM) or an imaging agent. Thus, the payload conjugates of the invention allow the specific targeting and delivery of a payload, which may be, for example, an anti-neoplastic agent or an imaging agent, to a proteoglycan synthesizing cell or tissue and, in particular, into the cytoplasm and/or nuclei of such cells *in vitro* and *in vivo.*

### Background of the invention

Cellular membranes are generally impermeable to macromolecules, including proteins and nucleic acids. Moreover, even smaller molecules may enter living cells only at very low rates and in the presence of high, potentially toxic extracellular concentrations. The lack of means for specifically targeting and delivering a compound of interest into specific cells or tissues has been an obstacle to the therapeutic, prophylactic and diagnostic or experimental use of a potentially large number of biologically active molecules having intracellular sites of action.

Over the past decade various means for intracellular delivery of compounds have been investigated in an attempt to facilitate efficient transfer of a substance of interest from the external medium into tissues or cells. The most common delivery constructs have been based on antibodies (or antibody fragments) or on viral and bacterial peptides discovered to have membrane binding and transport activity. For example, transporter constructs have been investigated based on herpes viral VP22 protein; polypeptides comprising the human immunodeficiency virus (HIV) TAT protein, and polypeptides comprising a homeodomain of an Antennapedia protein (Antp HD), as well as functional fragments and modifications thereof.

The majority of the viral and bacterial peptides investigated in the delivery constructs (also termed cell-penetrating peptides (CPPs)) comprise cationic peptides rich in basic residues such as lysine and/or arginine, or peptides comprising alpha helix enhancing amino acids. CPPs have been used to transfect cells *in vitro* in some experimental animal models, but have demonstrated limited success in clinical trials. It has been postulated that the lack of success in the clinic may arise from their lack of specificity for any particular cell type or tissue, as well as the inherent instability of these peptides *in vivo* (often exhibiting half-lives on the order of several minutes). To circumvent the lack of *in vivo* stability, several stabilized CPPs have been developed which have been chemically modified or which contain non-natural amino acids, including "D" amino-acids. For example, a full "D"-retro-inverso form ("D-TAT") of the archetypal "TAT" peptide has reached clinical Phase 2, but the potentially extremely long persistence of this peptide has limited its uses to topical administration, e.g., to the ear or intraocular for treatment of inflammation of the eye. The systemic administration of such stabilized peptides is contraindicated by virtue of their potential toxicity.

By replacing only specific positions in the "D-TAT" peptide by L-amino acids, peptides have been obtained with an intermediate half-live potentially more suitable for clinical development. The transporter constructs disclosed in the applications WO 2010/072406, WO 2010/072228 or WO2010/072275, comprising amino acid with both L- and D-amino acids are stable enough to prevent degradation by proteases prior to transport of the cargo moiety to its target site. Moreover, these transporter constructs appear not to permanently persist in the cell and are to some extent subject to protease degradation. Nevertheless, while effective trans-membrane transporter activity has been demonstrated, it has been found that upon uptake, the cargo moiety of the cargo-transporter construct is not readily cleaved from the transporter moiety, which is generally a prerequisite for the cargo moiety to be biologically active. Moreover, it has been found that the degradation of the cargo-transporter construct is slow, such that is exhibits the tendency to accumulate in the target cell. Therefore, even if the attached cargo moiety is eventually released or is metabolized, the transporter construct may remain in the cell for a prolonged time and participate in further inter- and intracellular processes leading to unknown and unwanted side effects.

In more recent approaches, peptides binding specifically to certain types of proteoglycans have been identified. Furthermore, it has been demonstrated that these peptides can be internalized by cells upon binding to proteoglycans on the cell surface and thus can act as transporter molecules for delivering cargo moieties, such as drug molecules, to proteoglycan synthesizing cells. In particular, WO 2014/072411 discloses peptides binding specifically to the proteoglycan chondroitin-4-sulfate, WO 2015/162285 discloses peptides binding specifically to the proteoglycan chondroitin-6-sulfate and WO 2015/162287 discloses peptides binding specifically to the proteoglycan keratan sulfate. The peptides disclosed in these documents can be used for the targeted delivery of cargo moieties to cell types that synthesize a specific proteoglycan and may thus allow for a more precise targeting of these specific cell types than in previous approaches.

Despite the recent improvements in the targeted delivery of payloads to cells *in vitro* and *in vivo,* there is still a need to develop improved compounds for the intracellular delivery of desired cargo moieties. In particular, there is a need for targeting compounds that bind proteoglycans with a higher affinity than compounds known in the art to enable and/or improve the use of such compounds in therapy and/or diagnostics.

## Summary of the invention

The inventors have surprisingly discovered that oligomerization of peptides binding to a specific type of proteoglycan results in oligomeric compounds with increased affinity for said specific type of proteoglycan in comparison to the monomeric counterparts. It has been shown by the inventors that the oligomeric compounds of the invention can be used to target cells synthesizing specific types of proteoglycans, in particular, chondroitin-4-sulfate, chondroitin-6-sulfate and/or keratan sulfate, *in vivo* and *in vitro.* Furthermore, the inventors surprisingly found that oligomerization of peptides does not interfere with the transport of the resulting oligomeric compounds into the cytoplasm and/or nucleus of cells synthesizing proteoglycans that are specifically recognized by the oligomeric compound of the invention.
The present invention will be summarized in the following embodiments.
In one embodiment, the present invention relates to a compound having the formula (I):

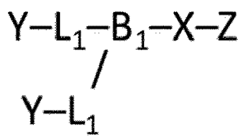

(I)

and all pharmaceutically acceptable salts, polymorphs, and cocrystals thereof, wherein

Z represents a reactive moiety, in particular wherein the reactive moiety is suitable for conjugating a payload to the compound;

X is absent or represents a chemical spacer comprising a single amino acid residue, a dipeptide and/or a moiety comprising 1 to 36 ethylene glycol repeats according to the formula $-NH(CH_2CH_2O)_{1-36}CH_2CO-$;

$B_1$ represents a branching moiety, wherein the branching moiety is derived from an amino acid residue comprising two amino groups and a carboxyl group, wherein $B_1$ is coupled to X through its carboxyl group, and to each $L_1$ through its amino groups;

each $L_1$ independently represents:

i. a single amino acid residue or a dipeptide, preferably wherein the single amino acid residue is L-serine or L-glycine or wherein the dipeptide consists of or comprises L-serine and/or L-glycine, or

ii. a moiety comprising 1 to 36 ethylene glycol repeats according to the formula $-NH(CH_2CH_2O)_{1-36}CH_2CO-$; or

iii. is absent;

and each Y independently represents $P-L_3-$ or a moiety according to formula (II):

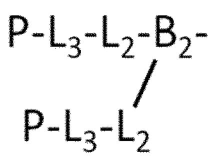

(II)

wherein

$B_2$ represents a branching moiety wherein the branching moiety is derived from an amino acid residue comprising two amino groups and a carboxyl group, wherein $B_2$ is coupled to $L_1$ through its carboxyl group, and to each $L_2$ through its amino groups;

each $L_2$ independently represents:

i. a single amino acid residue or a dipeptide, preferably wherein the single amino acid residue is L-serine or L-glycine or wherein the dipeptide consists of or comprises L-serine and/or L-glycine, or
ii. is absent;

$L_3$ represents a single amino acid residue coupled to $L_2$ through its carboxylic group and coupled to P through its amino-group, and

P represents a peptide moiety, wherein each P independently represents a polypeptide comprising the amino acid sequence $(AA_1)$-$(AA_2)$-$(AA_3)$-$(AA_4)$-$(AA_5)$-$(AA_6)$-$(AA_7)$-$(AA_8)$-$(AA_9)$,

wherein $(AA_1)$ represents a single amino acid residue with a positively charged amino acid side chain, or wherein $(AA_1)$ is absent;

wherein $(AA_5)$ and $(AA_7)$ each represent a single amino acid residue comprising a positively charged amino acid side chain, a hydrophobic amino acid side chain or an aromatic amino acid side chain;

wherein $(AA_2)$, $(AA_3)$, $(AA_4)$ and $(AA_6)$ each represent a single amino acid residue;

wherein $(AA_8)$ represents a single amino acid residue, or wherein $(AA_8)$ is absent;

wherein $(AA_9)$ represents a single amino acid residue comprising a positively charged amino acid side chain, a hydrophobic amino acid side chain or an aromatic amino acid side chain, or wherein $(AA_9)$ is absent; and

wherein $(AA_8)$ and $(AA_9)$ are either both present or both absent, wherein when $(AA_8)$ and $(AA_9)$ are both absent, $(AA_1)$, $(AA_3)$, $(AA_5)$ and $(AA_7)$ represent a single amino acid residue comprising a positively charged amino acid side chain;

wherein P comprises at least 3 amino acid residues comprising a positively charged amino acid side chain; and

wherein each P is coupled to an $\alpha$-amino group of a $L_3$ through the $\alpha$-carboxyl group of residue $(AA_7)$ and/or $(AA_9)$.

In a further embodiment, the present invention relates to the compound according to formula (I), wherein when Y represents

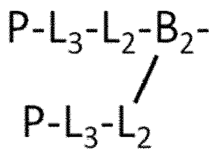

(II)

$L_1$ is not absent.

In a preferred embodiment, $L_3$ is a single amino acid residue, preferably a D-amino acid residue, more preferably a D-amino acid comprising an aromatic or a positively charged amino acid side chain, most preferably a D-Histidine residue.

In again a further embodiment, the present invention relates to the compound according to formula (I), wherein the branching moiety $B_1$ and/or $B_2$ is derived from L-lysine, L-ornithine, $\alpha$-aminoglycine, $\alpha,\gamma$-diaminopropionic acid, $\alpha,\gamma$-diaminobutyric acid, or an amino acid according to formula:

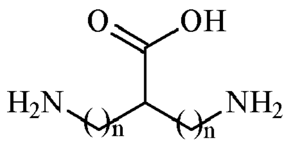

(diAA) wherein n is selected from 1, 2, 3, and 4.

In a particular embodiment, the present invention relates to the compound according to formula (I), wherein the branching moiety $B_1$ and/or $B_2$ is derived from L-lysine or L-ornithine; wherein when $L_1$ and/or $L_2$ are coupled to an amino group comprised in the amino acid side chain of $B_1$ and/or $B_2$, respectively, $L_1$ and/or $L_2$ are not absent.

In a further embodiment, the present invention relates to the compound according to formula (I), wherein the reactive moiety Z is an amino acid residue, in particular wherein the amino acid is selected from a group consisting of: L-cysteine, L-C-propargylglycine, L-biocytin, and $NH_2CH_2CONHSO_2(CH_2)_3COOH$, or wherein the reactive moiety Z is L-cysteamine or 4-aminobutanethiol.

In a further embodiment, the present invention relates to the compound according to formula (I), wherein the single amino acid comprised in the chemical spacer X is L-serine, or, wherein the dipeptide comprised in the chemical spacer X comprises or consists of L-serine.

In a yet further embodiment, the present invention relates to the compound according to formula (I), wherein the compound comprises the structure:

(D-His)-(L-Ser)-(L-Lys)-(L-Cys) (SEQ ID NO:1);
(D-His)-(L-Ser)-(L-Lys)-(L-Ser)-(L-Lys)-(L-Cys) (SEQ ID NO:2);
(D-His)-(L-Ser)-(L-Lys)-(βAla)-(L-Ser)-(L-Lys)-(L-Cys) (SEQ ID NO:3);
(D-His)-(L-Ser)-(L-Lys)-(L-Ser)-((3Ala)-(L-Lys)-(L-Cys) (SEQ ID NO:4);
(D-His)-(L-Ser)-(L-Lys)-(L-Ser)-(L-Lys)-(PEG1/5/16/36)-(L-Cys) (SEQ ID NO:5);
(D-His)-(L-Ser)-(L-Lys)-(PEG1/5/16/36)-(L-Cys) (SEQ ID NO:6);
(D-His)-(L-Ser)-(L-Lys)-(PEG1/3/5)-(L-Lys)-(L-Cys) (SEQ ID NO:7);
(D-His)-(PEG2/5)-(L-Lys)-(L-Cys) (SEQ ID NO:8);
(D-His)-(L-Ser)-(diAA)-(L-Ser)-(diAA)-(L-Cys) (SEQ ID NO:9);
(D-His)-(L-Ser)-(diAA)-(L-Cys) (SEQ ID NO:10);
(D-His)-(L-Ser)-(L-Lys)-(Gly)-(L-Lys)-(L-Cys) (SEQ ID NO:11);
(D-His)-(Gly)-(L-Lys)-(L-Ser)-(L-Lys)-(L-Cys) (SEQ ID NO:12);
(D-His)-(Gly)-(L-Lys)-(Gly)-(L-Lys)-(L-Cys) (SEQ ID NO:13);
(D-His)-(Gly)-(L-Lys)-(L-Cys) (SEQ ID NO:14);
(D-His)-(L-Ser)-(diAA)-(Gly)-(diAA)-(L-Cys) (SEQ ID NO:15);
(D-His)-(Gly)-(diAA)-(L-Ser)-(diAA)-(L-Cys) (SEQ ID NO:16);
(D-His)-(Gly)-(diAA)-(Gly)-(diAA)-(L-Cys) (SEQ ID NO:17);
(D-His)-(Gly)-(diAA)-(L-Cys) (SEQ ID NO:18);
(D-His)-(L-Ser)-(L-Lys)-(L-PAG) (SEQ ID NO:19);
(D-His)-(L-Ser)-(L-Lys)-(L-Ser)-(L-Lys)-(L-PAG) (SEQ ID NO:20);
(D-His)-(L-Ser)-(L-Lys)-(L-Bct) (SEQ ID NO:21);
(D-His)-(L-Ser)-(L-Lys)-(L-Ser)-(L-Lys)-(L-Bct) (SEQ ID NO:22);
(D-His)-(L-Lys)-(L-Cys) (SEQ ID NO:23);
(D-His)-(L-Lys)-(L-Ser)-(L-Lys)-(L-Cys) (SEQ ID NO:24);
(D-His)-(L-Lys)-(PEG1)-(L-Cys) (SEQ ID NO:25);
(D-His)-(L-Lys)-(L-Ser)-(L-Lys)-(PEG1)-(L-Cys) (SEQ ID NO:26);
(D-His)-(L-Ser)-(L-Ser)-(L-Lys)-(L-Cys) (SEQ ID NO:27);
(D-His)-(L-Ser)-(L-Ser)-(L-Lys)-(L-Ser)-(L-Lys)-(L-Cys) (SEQ ID NO:28);
(D-His)-(L-Ser)-(L-Lys)-(L-Ser)-(L-Ser)-(L-Cys) (SEQ ID NO:29); or
(D-His)-(L-Ser)-(L-Lys)-(L-Ser)-(L-Lys)-(L-Ser)-(L-Ser)-(L-Cys) (SEQ ID NO:30),

wherein in the foregoing list (PEGn), with n being an integer or a set of alternative integers separated by slashes, represent a moiety according to the formula-$NH(CH_2CH_2O)_nCH_2CO$-, wherein n is:

1, 5, 16 or 36 for (PEG1/5/16/36), respectively;
1, 3 or 5 for (PEG1/3/5), respectively;
2 or 5 for (PEG2/5), respectively; and
1 for (PEG1),

and wherein diAA represents an amino acid according to formula

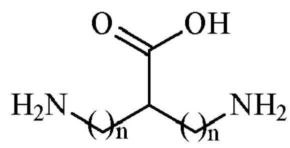

wherein n is selected from 1, 2, 3, and 4.

In again a further embodiment, the present invention relates to the compound according to formula (I), wherein the compound comprises the structure:
(D-His)-(L-Ser)-(L-Lys[ε-N-(L-Ser)-(D-His)])-(L-Cys) (SEQ ID NO:31);

(D-His)-(L-Ser)-(L-Lys[ε-N-(L-Ser)-(L-Lys[ε-N-(L-Ser)-(D-His)]-(L-Ser)-(D-His)])-(L-Cys) (SEQ ID NO:32);

(D-His)-(L-Ser)-(L-Lys[ε-N-(L-Ser)-(D-His)])-(L-Ser)-(L-Lys[ε-N-(L-Ser)-(D-His)])-(L-Cys) (SEQ ID NO:33);

(D-His)-(L-Ser)-(L-Lys[ε-N-(L-Ser)-(L-Lys[ε-N-(L-Ser)-(D-His)])-(L-Ser)-(D-His)])-(L-Ser)-(L-Lys[ε-N-(L-Ser)-(D-His)])-(L-Cys) (SEQ ID NO:34);

(D-His)-(L-Ser)-(L-Lys[ε-N-(L-Ser)-(D-His)])-(L-Ser)-(L-Lys[ε-N-(L-Ser)-(L-Lys[ε-N-(L-Ser)-(D-His)])-(L-Ser)-(D-His)])-(L-Cys) (SEQ ID NO:35);

or

(D-His)-(L-Ser)-(L-Lys[ε-N-(L-Ser)-(L-Lys[ε-N-(L-Ser)-(D-His)])-(L-Ser)-(D-His)])-(L-Ser)-(L-Lys[ε-N-(L-Ser)-(L-Lys[ε-N-(L-Ser)-(D-His)])-(L-Ser)-(D-His)])-(L-Cys) (SEQ ID NO:36).

In yet a further embodiment, the present invention relates to the compound according to formula (I), wherein in at least one, at least two, at least three or all peptide moieties P, at least two of the amino acid residues ($AA_1$), ($AA_5$), ($AA_7$) and ($AA_9$) comprise an amino acid residue with a positively charged amino acid side chain.

In a further embodiment, the present invention relates to the compound according to formula (I), wherein in at least one, at least two, at least three or all peptide moieties P, amino acid residue ($AA_9$) comprises a positively charged amino acid side chain, in particular wherein ($AA_9$) is selected from a group consisting of: L-arginine, L-lysine and L-2-amino-4-guanidinobutyric acid.

In yet a further embodiment, the present invention relates to the compound according to formula (I), wherein in at least one, at least two, at least three or all peptide moieties P, at least one of the amino acid residues ($AA_2$) and/or ($AA_5$) and at least one of the amino acid residues ($AA_7$) and/or ($AA_8$) comprises a positively charged amino acid side chain.

In a particular embodiment, the present invention relates to the compound according to formula (I), wherein the amino acid comprising the positively charged amino acid side chain in position ($AA_2$) and/or ($AA_5$) is selected from a group consisting of: L-arginine, D-arginine and L-2-amino-4-guanidinobutyric acid; and/or wherein the amino acid comprising the positively charged amino acid side chain in position ($AA_7$) and/or ($AA_8$) is selected from a group consisting of: L-arginine, L-lysine and L-ornithine.

In a further particular embodiment, the present invention relates to the compound according to formula (I),

wherein in at least one, at least two, at least three or all peptide moieties P, between 2 and 5 amino acid residues comprise a hydrophobic and/or aromatic amino acid side chain.

In a further particular embodiment, the present invention relates to the compound according to formula (I), wherein in at least one, at least two, at least three or all peptide moieties P, amino acid residue ($AA_3$) is L-phenylalanine and/or amino acid residue ($AA_6$) is L-isoleucine or D-isoleucine.

In again a further particular embodiment, the present invention relates to the compound according to formula (I), wherein at least one, at least two, at least three or all peptide moieties P comprise a sequence independently selected from a group consisting of:

RYFvRIOYR (SEQ ID NO:37);
RRFvYIYRR (SEQ ID NO:38);
RYFvRIYRR (SEQ ID NO:39);
RYFvrIOYR (SEQ ID NO:40);
RYFvRiOYR (SEQ ID NO:41);
RYFvriOYR (SEQ ID NO:42);
RRFvYIYKR (SEQ ID NO:43);
RRFqYIYKR (SEQ ID NO:44);
RYFvRIYKR (SEQ ID NO:45);
RYFvRIYRK (SEQ ID NO:46) ;
RRFaYIYKR (SEQ ID NO:47);
RRFpYIYKR (SEQ ID NO:48);
RYFvRIKYR (SEQ ID NO:49);
RYFqRIKYR (SEQ ID NO:50);
YFvRIOYR (SEQ ID NO:51);
(Agb)YFv(Agb)IKY(Agb) (SEQ ID NO:52);
YFvRIKYR (SEQ ID NO:53);
rYFvRIKYR (SEQ ID NO:54);
RYFvrIKYR (SEQ ID NO:55);
rrFvYIYRR (SEQ ID NO:56);
RSTqRYRVR (SEQ ID NO:57);
RYFvRIKYR (SEQ ID NO:58);
RYFvRIKAR (SEQ ID NO:59);
RAAvRAKYR (SEQ ID NO:60);
RAAvRIKYR (SEQ ID NO:61);
RSTqRYRVR (SEQ ID NO:62);
RSTqRYKVR (SEQ ID NO:63);
RGGgRGKGR (SEQ ID NO:64);
RHHhRHKHR (SEQ ID NO:65);
RVVvRVKVR (SEQ ID NO:66);
RLLlRLKLR (SEQ ID NO:67);
RMMmRMKMR (SEQ ID NO:68);
RIIiRIKIR (SEQ ID NO:69); or
RYFVRiKYR (SEQ ID NO:70);

wherein capital letters represent L-amino acids and wherein small letters represent D-amino acids.

In a further embodiment, the present invention relates to the compound according to formula (I), wherein at least one, at least two, at least three or all peptide moieties P comprise a positively charged amino acid in position ($AA_1$), ($AA_3$) and ($AA_9$), in particular wherein ($AA_1$) and/or ($AA_9$) are L-arginine and/or wherein ($AA_3$) is L-lysine.

In a particular embodiment, the present invention relates to the compound according to formula (I), wherein at least one, at least two, at least three or all peptide moieties P comprise an amino acid residue comprising a positively charged amino acid side chain in positions ($AA_5$) or ($AA_7$), in particular wherein ($AA_5$) or ($AA_7$) are L-arginine and/or L-lysine.

In a further particular embodiment, the present invention relates to the compound according to formula (I), wherein at least one, at least two, at least three or all peptide moieties P comprise between two and five amino acid residues comprising a hydrophobic and/or aromatic amino acid side chain, in particular wherein the amino acid residues comprising the hydrophobic and/or aromatic side chains are located in positions ($AA_2$), ($AA_4$), ($AA_6$), ($AA_8$) and/or one of positions ($AA_5$) or ($AA_7$).

In a yet further particular embodiment, the present invention relates to the compound according to formula (I),

wherein at least one, at least two, at least three or all peptide moieties P comprise a sequence independently selected from a group consisting of:

RYKvFIKYR (SEQ ID NO:71);
RYKvAIKYR (SEQ ID NO:72);
RYKvRIAYR (SEQ ID NO:73);
RYKvRIFYR (SEQ ID NO:74);
RYKvKFIYR (SEQ ID NO:75);
RYKvFIRYR (SEQ ID NO:76);
RYKvRFIYR (SEQ ID NO:77);
RMKiVMKFR (SEQ ID NO:78); or
RFKfFFKFR (SEQ ID NO:79).

wherein capital letters represent L-amino acids and wherein small letters represent D-amino acids.

In a further embodiment, the present invention relates to the compound according to formula (I), wherein at least one, at least two, at least three or all peptide moieties P comprise an amino acid residue comprising a positively charged amino acid side chain in positions ($AA_1$), ($AA_3$), ($AA_5$) and ($AA_7$), in particular wherein ($AA_1$) and/or ($AA_5$) are L-arginine and/or wherein ($AA_3$) and/or ($AA_7$) are L-lysine.

In a particular embodiment, the present invention relates to the compound according to formula (I), wherein at least one, at least two, at least three or all peptide moieties P comprise between two and five amino acid residues comprising a hydrophobic and/or aromatic amino acid side chain, in particular wherein the amino acid residues comprising the hydrophobic and/or aromatic side chains are located in positions ($AA_2$), ($AA_4$) and/or ($AA_6$), and optionally (AA8) and/or ($AA_9$).

In a particular embodiment, the present invention relates to the compound according to formula (I), wherein in at least one, at least two, at least three or all peptide moieties P, amino acid residues ($AA_8$) and ($AA_9$) are both absent or are both amino acids comprising a positively charged amino acid side chain, a hydrophobic amino acid side chain and/or an aromatic amino acid side chain.

In a further particular embodiment, the present invention relates to the compound according to formula (I), wherein at least one, at least two, at least three or all peptide moieties P comprise a sequence independently selected from a group consisting of:

RYKvRIK (SEQ ID NO:80);
RYKvRIKYF (SEQ ID NO:81);
RYKvRIKYA (SEQ ID NO:82);
RYKvRIKHH (SEQ ID NO:83);
RMKiRVK (SEQ ID NO:84);
RMKiRVKFM (SEQ ID NO:85);
RLKIRLKLL (SEQ ID NO:86); or
RYKvRIKYr (SEQ ID NO:87).

wherein capital letters represent L-amino acids and wherein small letters represent D-amino acids.

In a further embodiment, the present invention relates to the compound according to formula (I), wherein at least one, at least two, at least three or all peptide moieties P comprise at least one D-amino acid, in particular wherein amino acid residue ($AA_4$) and/or ($AA_6$) is a D-amino acid.

In a yet further embodiment, the present invention relates to the compound according to formula (I), wherein at least one, at least two, at least three or all peptide moieties P comprise not more than one amino acid residue comprising a negatively charged amino acid side chain, in particular wherein none of the amino acid residues in position ($AA_1$) to ($AA_3$) and ($AA_5$) to ($AA_9$) comprise an amino acid residue comprising a negatively charged amino acid side chain.

In a further embodiment, the present invention relates to the compound according to formula (I), wherein at least one, at least two, at least three or all peptide moieties P are free of amino acid residues comprising a negatively charged amino acid side chain.

In yet a further embodiment, the present invention relates to the compound according to formula (I), wherein at least one, at least two, at least three or all peptide moieties P comprise not more than one proline residue, in particular wherein none of the amino acid residues in position ($AA_1$) to ($AA_3$) and ($AA_5$) to ($AA_9$) comprise a proline residue.

In again a further embodiment, the present invention relates to the compound according to formula (I), wherein at least one, at least two, at least three or all peptide moieties P are free of proline residues.

In yet a further embodiment, the present invention relates to the compound according to formula (I), wherein at least two, at least three or all peptide moieties P have identical amino acid sequences.

In a further embodiment, the present invention relates to the compound according to the invention further conjugated to a payload.

In another embodiment, the invention relates to the payload conjugate according to the invention, wherein the payload is conjugated to the reactive moiety Z.

In a further embodiment, the present invention relates to the payload conjugate according to the invention, wherein the payload is directly conjugated to the reactive moiety Z or wherein the payload is conjugated to the reactive moiety Z via a linker.

In a further embodiment, the present invention relates to the payload conjugate according to the invention, wherein the linker comprises a PEG moiety, a moiety derived from cadaverine or an alkyl moiety.

In a further embodiment, the present invention relates to the payload conjugate according to the invention, wherein the payload is a biologically active molecule (BAM) or an imaging agent.

In a further embodiment, the present invention relates to the payload conjugate according to the invention, wherein the BAM is a mono- or polysaccharide, a cytotoxic agent, an antineoplastic agent, an anti-inflammatory agent, an anti-viral agent, an anti-bacterial agent or an agent for the treatment of protozoan infections.

In a further embodiment, the present invention relates to the payload conjugate according to the invention, wherein the antineoplastic agent is a pro-apoptotic peptide.

In a further embodiment, the present invention relates to the payload conjugate according to the invention, wherein the payload is an imaging agent.

In a further embodiment, the present invention relates to the payload conjugate according to the invention, wherein the imaging agent comprises a radionuclide, a fluorescent dye, a chemiluminescent agent, a bioluminescent agent, a spectrally resolvable inorganic fluorescent semiconductor nanocrystal, a metal nanoparticles, a nanocluster, a paramagnetic metal ion, an enzyme, a colorimetric label, biotin, dioxigenin, a hapten or a protein.

In another embodiment, the present invention relates to a pharmaceutical composition comprising the payload conjugate according to the invention and a pharmaceutically acceptable carrier or excipient.

In another embodiment, the present invention relates to the payload conjugate according to the invention or the pharmaceutical composition according to the invention for use as a medicament.

In another embodiment, the present invention relates to the payload conjugate according to the invention or the pharmaceutical composition according to the invention for use in the treatment of cancer.

In a further embodiment, the present invention relates to the payload conjugate or the pharmaceutical composition for use according to the invention, wherein the payload conjugate comprises PUMA, SN-38, 6-thioguanine or a pro-apoptopic peptide.

In another embodiment, the present invention relates to the payload conjugate according to the invention or the pharmaceutical composition according to the invention for use in the treatment of disorders involving leukocytes, wherein the payload conjugate specifically binds to chondroitin-4-sulfate.

In a further embodiment, the present invention relates to the payload conjugate or the pharmaceutical composition for use according to the invention, wherein the disorder involving leukocytes is selected from a neoplastic disease, an inflammatory disease, an autoimmune disease, an immunodeficiency disease, a viral infection, a bacterial infection, a protozoan infection or a parasitic infection.

In a further embodiment, the present invention relates to the payload conjugate or the pharmaceutical composition for use according to the invention, wherein the disorder involving leukocytes is a neoplastic disease, in particular wherein the neoplastic disease is leukemia.

In a further embodiment, the present invention relates to the payload conjugate or the pharmaceutical composition for use according to the invention, wherein the disorder involving leukocytes is a viral infection, in particular wherein the viral infection is caused by HIV.

In a further embodiment, the present invention relates to the payload conjugate or the pharmaceutical composition for use according to the invention, wherein the disorder involving leukocytes is a protozoan infection, in particular wherein the protozoan infection is caused by leishmaniasis.

In another embodiment, the present invention relates to the payload conjugate according to the invention or the pharmaceutical composition according to the invention for use in the treatment of a heart disease or condition, a disease or condition of the ovary or testis, a disease or condition of the central nervous system, or a C6S accumulation disease, wherein the payload conjugate specifically binds to chondroitin-6-sulfate.

In another embodiment, the present invention relates to the payload conjugate according to the invention or the pharmaceutical composition according to the invention for use in the treatment of a corneal disease or condition, a retina disease or condition, a disease or condition of the central nervous system, or a keratan sulfate accumulation disease, wherein the payload conjugate specifically binds to keratan sulfate.

In a further embodiment, the present invention relates to the payload conjugate or the pharmaceutical composition for use according to the invention, wherein the C6S accumulation disease and/or the keratan sulfate accumulation disease is Morquio syndrome.

In another embodiment, the present invention relates to the payload conjugate according to the invention or the pharmaceutical composition according to the invention for use in diagnostics.

In another embodiment, the present invention relates to the payload conjugate according to the invention or the pharmaceutical composition according to the invention for use in the visualization of cells comprising chondroitin-4-sulfate, chondroitin-6-sulfate and/or keratan sulfate on their cell surface and/or tissues comprising cells comprising chondroitin-4-sulfate, chondroitin-6-sulfate and/or keratan sulfate on their cell surface.

In a further embodiment, the present invention relates to the payload conjugate or the pharmaceutical composition for use according to the invention, wherein the cells comprising chondroitin-4-sulfate are leukocytes; or wherein the cells and/or tissues comprising chondroitin-6-sulfate are cells and/or tissues of the central nervous system, heart, ovary or testis; or wherein the cells and/or tissues comprising keratan sulfate are cells and/or tissues of the eye, in particular the cornea, the central nervous system, or the endometrial lining of the uterus.

In another embodiment, the present invention relates to the payload conjugate according to the invention or the pharmaceutical composition according to the invention for use in the diagnosis of diseases involving cells and/or tissues comprising proteoglycans on the surface.

In a further embodiment, the present invention relates to the payload conjugate or the pharmaceutical composition for use according to the invention, wherein the proteoglycan on the surface of the cells and/or tissues is chondroitin-4-sulfate and/or wherein the cells are leukocytes; or wherein the proteoglycan on the surface of the cells and/or tissues is chondroitin-6-sulfate and/or wherein the cells and/or tissues are cells and/or tissues of the central nervous system, heart, ovary or testis; or wherein the proteoglycan on the surface of the cells and/or tissues is keratan sulfate and/or wherein the cells and/or tissues are cells and/or tissues of the eye, in particular the cornea, the central nervous system, or the endometrial lining of the uterus.

In another embodiment, the present invention relates to the payload conjugate according to the invention or the pharmaceutical composition according to the invention for use in the diagnosis of diseases associated with elevated levels of proteoglycans on malignant cells, in particular wherein the proteoglycans are chondroitin-4-sulfate, chondroitin-6-sulfate and/or keratan sulfate.

In another embodiment, the present invention relates to the payload conjugate according to the invention or the pharmaceutical composition according to the invention for use in the diagnosis of Morquio syndrome.

**Definitions**

The term "chemical derivatives" as used herein refers to the (chemical) modification of amino acids, amino acid side chains and peptide bonds (including those at the N-terminus, the C-terminus, within the backbone of the consensus structure/sequence) as well as modification of any chemical group within the chemical spacer/linker of consensus structures (iii) to (xiv). The term does not intend to refer to any addition, substitution or deletion of amino acids residues in an amino acid or peptide chain. Chemical derivatives from L-amino acids or L-enantiomeric amino acids typically comprise any naturally or non-naturally occurring derivative of these amino acids, including, without being limited thereto, amino acids as defined above comprising post-translational modifications or synthetic modifications, including acetylation (at the N-terminus of the (poly-)peptide sequence, at lysine residues, etc.), deacetylation, alkylation, such as methylation, ethylation, etc. (preferably at lysine or arginine residues within the (poly-)peptide sequence), dealkylation, such as demethylation, deethylation, etc., amidation (preferably at the C-terminus of the (poly-)peptide sequence), formylation, gamma-carboxylation, glutamylation, glycosylation (preferably at asparagine, lysine, hydroxylysine, serine or threonine residues, etc., within the (poly-)peptide sequence), addition of a heme or haem moiety, hydroxylation, iodination, isoprenylation addition of an isoprenoid moiety such as farnesyl or geranylgeraniol, etc.), lipoylation (attachment of lipoate functionality), such as prenylation, formation of a GPI anchor, including myristoylation, farnesylation, geranylgernaylation, etc., oxidation, phosphorylation (e.g. to a serine, tyrosine, threonine or a histidine moiety, etc., within the (poly-)peptide sequence), sulfation (e.g., of tyrosine), selenoylation, sulfation, etc. Chemical derivatives of amino acids also include, without being limited thereto, modified amino acids, which have been modified by introducing a label, including radioactive labels, a dye or fluorescent group, or a chemoluminesent group.

The term "polymorphs" refers to the various crystalline structures of the compounds of the present invention. This may include, but is not limited to, crystal morphologies (and amorphous materials) and all crystal lattice forms. Salts of the present invention can be crystalline and may exist as more than one polymorph.

Solvates, hydrates as well as anhydrous forms of the salt are also encompassed by the invention. The solvent included in the solvates is not particularly limited and can be any pharmaceutically acceptable solvent. Examples include water and $C_{1-4}$ alcohols (such as methanol or ethanol).

"Pharmaceutically acceptable salts" are defined as derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts

or the quaternary ammonium salts of the parent compound formed, for example, from non-toxic inorganic or organic acids. For example, such conventional non-toxic salts include those derived from inorganic acids such as, but not limited to, hydrochloric, hydrobromic, sulfuric, sulfamic, phosphoric, nitric and the like; and the salts prepared from organic acids such as, but not limited to, acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like. The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Organic solvents include, but are not limited to, nonaqueous media like ethers, ethyl acetate, ethanol, isopropanol, or acetonitrile. Lists of suitable salts can be found in Remington's Pharmaceutical Sciences, 18th ed., Mack Publishing Company, Easton, PA, 1990, p. 1445, the disclosure of which is hereby incorporated by reference.

In the context of the present invention, L-amino acids, also known in the art and referenced herein as L-enantiomeric amino acids, are preferably amino acids selected from natively occurring amino acids or their derivatives. Naturally occurring amino acids are recognized as the standard (proteinogenic) amino acids alanine, arginine, asparagine, aspartic acid, cysteine, glutamine, glutamic acid, glycine, histidine, isoleucine, leucine, lysine, methionine, phenyl alanine, proline, serine, threonine, tryptophan, tyrosine, and valine, but also include non-standard amino acids such as ornithine, citrulline, homocysteine, S-adenosyl methionione, hydroxyproline, selenocysteine, pyrrolysine, lanthionine, 2-aminoisobutyric acid, dehydroalanine, and gamma-aminobutyric acid.

Similarly, in the context of the present invention, D-amino acids, also known in the art and referenced herein as D-enantiomeric amino acids, are preferably non-native (non-proteinogenic) "retro-inverso" amino acids, wherein these non-native (non-proteinogenic) "retro-inverso" amino acids are recognize derived from naturally occurring L-amino acids and/or their derivatives as defined above. In this context, the term "retro-inverso" refers to an isomer of a naturally occurring L-amino acid as defined above (and peptides made therefrom) in which the chirality of the naturally occurring L-amino acid residue is inverted in the corresponding D-amino acid. In other words, in the peptide bonds of D-amino acids the positions of carbonyl and amino groups are exchanged, while the position of the side-chain groups at each alpha carbon is preserved. Accordingly, D-amino acids may be inserted into a peptide sequence consisting of or comprising L-amino acids and therefore may be conjugated with L-amino acids as defined herein by methods known in the art or defined herein.

The amino acids abbreviations used in the present disclosure are shown in the following Table of Correspondence (Table 1). As used throughout the present disclosure and as detailed in the following table, upper-case or a capital letter in the 1 letter symbol references a L-amino acid, while a lowercase 1 letter symbol references a D-amino acid.

**Table 1.** Table of correspondence

| **Symbol** | | | **Amino acid** |
|---|---|---|---|
| **1-Letter** | | **3-Letter** | |
| ***L-amino acid*** | ***D-amino acid*** | | |
| A | a | Ala | Alanine |
| R | r | Arg | Arginine |
| N | n | Asn | Asparagine |
| D | d | Asp | Aspartic acid |
| C | c | Cys | Cysteine |
| E | e | Glu | Glutamic acid |
| Q | q | Gln | Glutamine |
| G | g | Gly | Glycine |
| H | h | His | Histidine |
| I | i | Ile | Isoleucine |
| L | l | Leu | Leucine |
| K | k | Lys | Lysine |
| M | m | Met | Methionine |

(continued)

| Symbol | | | |
|---|---|---|---|
| 1-Letter | | 3-Letter | Amino acid |
| *L-amino acid* | *D-amino acid* | | |
| F | f | Phe | Phenylalanine |
| P | p | Pro | Proline |
| S | s | Ser | Serine |
| T | t | Thr | Threonine |
| W | w | Trp | Tryptophan |
| Y | y | Tyr | Tyrosine |
| V | v | Val | Valine |
| X | x | Xaa | Unknown or other |

As used herein, the term "peptides", "polypeptides" and "proteins" have their meaning as generally understood as in the art, *i.e.,* referring to a chain of amino acids. However, the terms shall not be construed as limiting the length of the amino acid chain.

As used herein, the term "positively charged amino acid" refers to amino acids, the side chains of which comprise a positive charge when dissolved in water at pH = 7.0. Preferably, positively charged amino acids are arginine and lysine. However, the term positive amino acids also encompasses non-canonical and/or non-natural amino acids comprising a positive charge, such as, without limitation, L-ornithine, $\alpha$-aminoglycine, $\alpha,\gamma$-diaminopropionic acid, $\alpha,\gamma$-diaminobutyric acid, or an amino acid according to formula

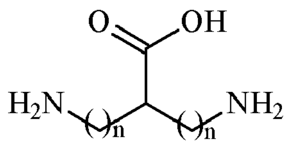

(diAA) wherein n is selected from 1, 2, 3, and 4.

As used herein, the term "negatively charged amino acid" refers to amino acids, the side chains of which comprise a negative charge when dissolved in water at pH = 7.0. Preferably, negatively charged amino acid are aspartic acid and glutamic acid.

As used herein, the term "aromatic amino acid" refers to amino acids, the side chains of which comprise an aromatic ring system. Preferably, aromatic amino acids are phenylalanine, tyrosine, histidine, and tryptophan.

As used herein, the term "hydrophobic amino acid" refers to amino acids bearing a hydrophobic moiety as a side chain. In particular, hydrophobic amino acids include glycine, alanine, valine, leucine, isoleucine, proline, and methionine.

The term "conjugation" as used herein refers to attachment by covalent bonds or by strong non-covalent interactions. Any method normally used by those skilled in the art for the coupling of payloads to a reactive moiety can be used in the present invention. Covalent bonds can be, for example, ester, ether, phosphoester, amide, peptide, imide, carbon-sulfur bonds, carbon-phosphorus bonds, and the like.

The term "payload", as used herein, represents any naturally occurring or synthetically generated molecule, including small-molecular weight molecules or chemical entities that can chemically be synthesized, and larger molecules or biological entities that are produced chemically, enzymatically or by fermentation of host cells.

A "target cell" is a cell that comprises a type of proteoglycan on the cell surface that is specifically bound by the compound of the invention. A "target tissue" is a tissue comprising cells comprising a type of proteoglycan on the cell surface that is specifically bound by the compound of the invention. Within the present invention, compounds are disclosed that specifically bind to a certain type of proteoglycan. That is, compounds are disclosed herein that specifically bind to chondroitin-4-sulfate (C4S), chondroitin-6-sulfate (C6S) and/or keratan sulfate (KS).

The present invention encompasses compounds that specifically bind to proteoglycans, in particular C4S, C6S and/or KS. Accordingly, as used herein, C4S, C6S and/or KS are the target proteoglycans of the compounds of the invention, while other proteoglycans are non-targets. It is well known in the art that the term "specifically binds" designates

the degree to which a compound discriminates between target and non-target molecules, i.e. in the present invention, target and non-target proteoglycans. This is because it is known that no protein/peptide-based binding molecule/compound has absolute specificity, in the sense that it will react with only one specific molecule. That is, where other (non-target) molecules (non-target proteoglycans) are present, a binding compound of the invention can react to some extent with similar motives on these other (non-target) molecules/proteoglycans. However, the affinity of a compound of the invention for a target proteoglycan is significantly greater than its affinity for non-target molecules and/or non-target proteoglycans. This difference in affinity can be used to establish assay conditions, under which a compound of the invention binds almost exclusively to target molecule/proteoglycan. In this respect, the binding (or non-binding) of a compound of the invention to a target molecule/proteoglycan are not understood as absolutes. That is, the compounds of the invention may exhibit some (residual) binding activity for other (non-)targets, but at significantly reduced levels relative to the binding activity for C4S, C6S and/or KS (i.e. the target proteoglycans). In some embodiments, the feature of "specifically binding" a target proteoglycan can indicate that a compound of the invention can discriminate a target from/over a non-target proteoglycan, and may be characterized by a compound of the invention having a binding affinity for the target molecule/proteoglycan at least 10 fold, at least 20 fold, preferably at least 50 fold, and more preferably at least 100 fold better than the affinity for the non-target molecule/proteoglycan. In some embodiments and/or in some assay systems, a compound of the invention may exhibit no detectable or measurable binding to the non-target molecule. In such a case, a compound of the invention specifically binds to a target proteoglycan where it has a binding affinity at least 100 fold greater than the lowest detectable binding of the particular assay. Exemplary assay systems known in the art suitable to determine and compare relative binding affinity and/or binding activity are, but are not limited to, surface plasmon resonance, isothermal titration calorimetry, ELISA and fluorescence polarization .

Binding of the compound according to the invention to proteoglycans, in particular to the target proteoglycans C4S, C6S and/or KS, can be determined by any method known in the art and/or described herein. For example, specific binding can be measured by determining binding for a target molecule, e.g. C4S, C6S and/or KS, compared to binding for a non-target/control molecule, e.g. a molecule of similar structure for which the compounds of the invention do not have binding activity and/or exhibit low binding activity. In certain embodiments, dextrane sulfate may be used as non-target/control molecule. It is most preferred that the comparison of the binding to the target molecule and non-target/control molecule is performed in the same assay system, according to the same protocols and under the same conditions. In this case, specific binding is indicated where the tested compound of the invention having a binding affinity (and/or binding activity as determined by the assay) for the target molecule that is at least 10 fold, at least 20 fold, preferably at least 50 fold, and more preferably at least 100 fold greater than the binding affinity/activity for the non-target/control molecule. As known in the art, such binding assays can also be performed using cells that express the target molecules, i.e., cells that express one or more of C4S, C6S, and KS. Accordingly, specific binding can be assessed by determining the binding of a compound of the invention for a cell known to express C4S, C6S, and/or KS and comparing it to the binding for a cell that does not express a target proteoglycan, preferably that does not any target proteoglycan. Specificity of binding can be determined, for example, with cells that are known to synthesize a specific proteoglycan, for example the cell line HL-60 in case of C4S, and, as a negative control, with cell lines that are known not to synthesize the proteoglycan of interest. Furthermore, mutant variants are known that are incapable of synthesizing proteoglycans and may be used as negative control. The choice of positive cells (i.e. expressing one or more target proteoglycan) and negative/control cells is within the common general knowledge of the skilled person. For example, as described in further detail in the working example sections herein, ELISA plates may be coated with purified proteoglycan in PBS pH 7.4 for 2 hours at 37°C, blocked overnight at 4°C with 200 μl of PBS pH 7.4, Tween 0.05% + BSA 3% + PEG400 2% and washed twice with fresh TSCM buffer pH 6.4 ((Tris 20 mM, NaCl 150 mM, BSA 1%, Tween 0.05%, 2 mM $MgCl_2$, 2mM $CaCl_2$). Binding of the compound according to the invention may then be conducted at serial dilutions in TSCMP (TSCM + 2% (w/v) PEG 600) pH 6.4 for 1 hour at 37°C (100μl per well). Plates may be washed three times with fresh TSCM pH 6.4 (200μl per well) to remove unbound compounds.

In certain embodiments, the compound according to the invention may be coupled to a dye or a fluorophore. In such embodiments, binding of the compound according to the invention to a proteoglycan may be directly determined in an UV/Vis spectrophotometer or a fluorescence scanner.

In certain embodiments, the compound according to the invention may be coupled to a biotin molecule. In such embodiments, binding of the compound according to the invention to a proteoglycan may be indirectly determined with a streptavidin-fluorophore conjugate in a fluorescence scanner. Alternatively, binding of the compound according to the invention to a proteoglycan may be indirectly determined with a streptavidin-horse radish peroxidase (HRP) conjugate. In such embodiments, SAV-HRP may be added at a dilution of 1/8000 (100μl per well) for 20min at room temperature. Following 3 washes with PBS pH 7.4, the substrate 3,3' ,5,5' - tetramethylbenzidine (TMB) may be added for 10min (100μl per well), protected from light. The reaction may stopped by addition of 100μl of 1M $H_2SO_4$ and the absorbance at 450nm may be measured.

A compound according to the invention is defined to bind specifically to a proteoglycan, such as C4S, C6S and/or KS, if it binds to said proteoglycan with a binding affinity below 100 μM, below 50 μM, below 25 μM, below 10

μM, below 5 μM, below 2.5 μM, below 1 μM, below 900 nM, below 800 nM, below 700 nM, below 600 nM, below 500 nM, below 400 nM, below 300 nM, below 200 nM, or below 100 nM.

It is to be understood that a compound according to the invention or even a single peptide moiety may specifically bind to more than one proteoglycan. For example, it is shown in Figure 14 that the peptide with SEQ ID NO:37 binds specifically to C4S and C6S.

As used herein, "radionuclide" refers to a moiety comprising a radioactive isotope of at least one element. Exemplary suitable radiolabels include but are not limited to those described herein. In certain embodiments, a radiolabel is one used in positron emission tomography (PET). In certain embodiments, a radiolabel is one used in single-photon emission computed tomography (SPECT). In certain embodiments, radioisotopes comprise $^{99}$Tc, $^{111}$In, $^{64}$Cu, $^{67}$Ga, $^{186}$Re, $^{188}$Re, $^{1S3}$Sm, $^{177}$Lu, $^{67}$Cu, $^{123}$I, $^{124}$I, $^{125}$I, $^{11}$C, $^{13}$N, $^{15}$O, $^{18}$F, $^{153}$Sm, $^{166}$Ho, $^{177}$Lu, $^{149}$Pm, $^{90}$Y, $^{213}$Bi, $^{103}$Pd, $^{109}$Pd, $^{159}$Gd, $^{140}$La, $^{198}$Au, $^{199}$Au, $^{169}$Yb, $^{175}$Yb, $^{165}$Dy, $^{166}$Dy, $^{67}$Cu, $^{105}$Rh, $^{111}$Ag $^{89}$Zr, $^{225}$AC and $^{192}$Ir.

"Pharmaceutically acceptable" is defined as those compounds, materials, compositions, and/or dosage forms which are, within the scope of sound medical judgment, suitable for use in contact with the tissues of human beings and animals without excessive toxicity, irritation, allergic response, or other problem or complication commensurate with a reasonable benefit/risk ratio. The phrase "pharmaceutically or pharmacologically acceptable" refers to molecular entities and compositions that do not produce adverse, allergic, or other untoward reactions when administered to an animal or a human. As used herein, "pharmaceutically acceptable carrier" includes any and all solvents, dispersion media, antibacterial and antifungal agents, isotonic and absorption delaying agents and the like.

The patients or subjects in the present invention are typically animals, particularly mammals, more particularly humans.

The preferred definitions given in the "Definition"-section apply to all of the embodiments described below unless stated otherwise.

## Description of the Figures

**Fig.1** shows binding affinity measurements of 51-hSC-Mal-Biotin to different GAGs.

**Fig.2** shows binding affinity measurements of $(51\text{-hS})_2$KC-Mal-Biotin, $(51\text{-hS})_2$KS-(51-hS)KC-Mal-Biotin and $[(51\text{-hS})_2\text{KS}]_2$KK(Biotin) to C4S. $(51\text{-hS})_2$KC-Mal-Biotin is shown in squares, $(51\text{-hS})_2$KS-(51-hS)KC-Mal-Biotin is shown in triangles, $[(51\text{-hS})_2\text{KS}]_2$KK(Biotin) is shown in diamonds and VAR2-Biotin is shown in circles.

**Fig.3** shows standardized geometric mean of 680RD fluorescence on CHO-K1 (panel A) and CHO-pgsA-745 (panel B) cells (Alexa Fluor 700 channel). $(51\text{-hS})_2$KC-Mal-Biotin is shown in squares, $(51\text{-hS})_2$KS-(51-hS)KC-Mal-Biotin is shown in triangles, and $[(51\text{-hS})_2\text{KS}]_2$KK(Biotin) is shown in diamonds.

**Fig.4** shows standardized geometric mean of 680RD fluorescence on CHO-K1 (panel A) and CHO-pgsA-745 (panel B) cells (Alexa Fluor 700 channel). $[(51\text{-hS})_2\text{KS}]_2$KC-Mal-680RD is shown in triangles, $[(51\text{-hS})_2\text{KS}]_2$K(PEG1)C-Mal-680RD is shown in squares and $[(51\text{-hS})_2\text{K(PEG1)}]_2$KC-Mal-680RD is shown in diamonds.

**Fig.5** shows standardized geometric mean of 680RD fluorescence on CHO-K1 (panel A) and CHO-pgsA-745 (panel B) cells (Alexa Fluor 700 channel). $[(51\text{-hS})_2\text{KS}]_2$KC-Mal-680RD is shown in triangles and $[(51\text{-hS})_2\text{K(PEG5)}]_2$KC-Mal-680RD is shown in squares.

**Fig.6** shows standardized geometric mean of 680RD fluorescence on HL-60 (panel A) and U937 (panel B) cells (Alexa Fluor 700 channel). $(51\text{-hS})_2$KC-Mal-Biotin is shown in squares, $(51\text{-hS})_2$KS-(51-hS)KC-Mal-Biotin is shown in triangles, $[(51\text{-hS})_2\text{KS}]_2$KK(Biotin) is shown in diamonds and VAR2-Biotin is shown in circles.

**Fig.7** shows standardized geometric mean of 680RD fluorescence on the colorectal cell line HCT116 (Alexa Fluor 700 channel). $(21\text{-hS})_2$KC-Mal-Biotin is shown in squares, (51-hS)2KS-(51-hS)KC-Mal-Biotin is shown in triangles, $[(51\text{-hS})_2\text{KS}]_2$KK(Biotin) is shown in diamonds and VAR2-Biotin is shown in circles.

**Fig.8** shows internalization time course of certain compounds in HCT-116 cells. $[(51\text{-hS})_2\text{KS}]_2$KC-Mal-A488 is shown in squares and VAR2-A488 in triangles.

**Fig.9** shows internalization time course of certain compounds in HL60 (panel A) and K562 (panel B) cells. $[(51\text{-hS})_2\text{KS}]_2$KC-Mal-A488 is shown in squares and VAR2-A488 is shown in triangles.

**Fig.10** shows internalization time course of certain compounds in L-363 (panel A) and H929 (panel B) cells. $[(51\text{-}hS)_2KS]_2KC$-Mal-A488 is shown in squares and VAR2-A488 is shown in triangles.

**Fig.11** shows dependence of internalization of the compound $[(51\text{-}hS)_2KS]_2KC$-Mal-A488 on GAG.

**Fig.12** shows for Group A according to Table 12, fluorescence signal intensity (expressed as Average Radiant Efficiency) in the tumor, muscle and abdominal region at 30 min, 1 h, 3 h, 8 h, 24 h and 72 h after $[(51\text{-}hS)_2KS]_2KC$-Mal-680RD administration; values are plotted as mean and standard deviation (a); fluorescence signal intensity extrapolation of tumor and muscle (b).

**Fig.13** shows for Group B according to Table 12, fluorescence signal intensity (expressed as Average Radiant Efficiency) in the tumor, muscle and abdominal region at 30 min, 1 h, 3 h, 8 h, 24 h and 72 h after $[(51\text{-}hS)_2KS]_2KC$-Mal-680RD administration; values are plotted as mean and standard deviation (a); fluorescence signal intensity extrapolation of tumor and muscle (b).

**Fig.14** shows fluorescence signal intensity (expressed as Average Radiant Efficiency) in the tumors excised 72 hours after $[(51\text{-}hS)_2KS]_2KC$-Mal-680RD administration in group A, B and C.

**Fig.15** shows chemical structures of different azonafides.

## Detailed description of the invention

The compounds of the present invention will be described in the following. It is to be understood that all possible combinations of the following definitions are also envisaged.

In one embodiment, the present invention relates to a compound having the formula (I):

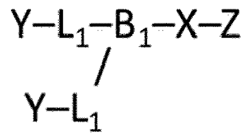

(I)

and all pharmaceutically acceptable salts, polymorphs, and cocrystals thereof.

Z represents a reactive moiety. In particular, the reactive moiety is suitable for conjugating a payload to the compound. In certain embodiments of the present invention, Z is an amino acid residue, in particular wherein the amino acid is selected from a group consisting of: L-cysteine, L-C-propargylglycine, L-biocytin, and $NH_2CH_2CONHSO_2(CH_2)_3COOH$. However, it is to be understood that numerous amino acids (including non-canonical amino acids, non-natural amino acids and amino acid derivatives) are known in the art which may serve as reactive moiety Z in the context of the present invention. In particular, amino acids with reactive groups in their amino acid side chains may be used as reactive moiety Z in the context of the present invention. Preferably, Z is selected from a group consisting of L-cysteine and L-C-propargylglycine.

In certain embodiments of the present invention, the reactive moiety Z is not an amino acid. That is, the reactive moiety Z may be any molecule that can be coupled to X or B1 and comprises a reactive group. In certain embodiments, the reactive moiety Z is couples to a carboxyl group of X or B1. In certain embodiments, the reactive moiety Z is cysteamine or 4-aminobutanethiol.

Preferably, the Z moiety comprises an amino group and is linked to X by a bond formed by its amino group. In certain embodiments of the present invention, wherein X is absent, Z is linked to $B_1$ by a bond formed by its amino group.

X is absent or represents a chemical spacer. Preferably, X comprises a single amino acid residue, a dipeptide and/or a moiety comprising 1 to 36 ethylene glycol repeats. In certain embodiments of the present invention, the chemical spacer X is a single amino acid. Preferably, the single amino acid comprised in the chemical spacer X is L-serine. Accordingly, said L-serine is linked through its carboxyl group to the amino group of Z by forming a peptide bond, and through its amino group to the carboxyl group of $B_1$, by forming a peptide bond.

In certain embodiments of the present invention, the chemical spacer X comprises a dipeptide. Preferably, the dipeptide comprised in the chemical spacer X comprises or consists of L-serine. More preferably, the chemical spacer X is derived from L-seryl-L-serine, L-seryl-glycine or glycyl-L-serine. In the embodiments of the invention wherein the chemical spacer X is a dipeptide, the C-terminal residue of the dipeptide is linked through its carboxyl group to the amino

group of Z by forming a peptide bond, and N-terminal residue of the dipeptide is linked through its amino group to the carboxyl group of $B_1$, by forming a peptide bond.

In embodiments where the reactive moiety Z is not an amino acid, the reactive moiety Z preferably comprises an amine function an is linked to a carboxyl group in the chemical spacer X or the branching moiety B1 via an amide bond.

In certain embodiments of the invention, the chemical spacer X comprises a moiety comprising 1 to 36 ethylene glycol repeats. Preferably, the moiety comprising 1 to 36 ethylene glycol repeats is a moiety according to the formula $-NH(CH_2CH_2O)_{1-30}CH_2CO-$. In certain embodiments, the chemical spacer X comprises a moiety comprising 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 or 36 ethylene glycol repeats. More preferably, the said moiety is selected from the group consisting of $-NHCH_2CH_2OCH_2CO-$, $-NH(CH_2CH_2O)_5CH_2CO-$, $-NH(CH_2CH_2O)_{10}CH_2CO-$, and $-NH(CH_2CH_2O)_{36}CH_2CO-$.

In certain embodiments of the present invention, the chemical spacer X comprises combinations of a single amino acid residue, a dipeptide and/or a moiety comprising 1 to 36 ethylene glycol repeats. It is to be understood that all the combinations of the above described moieties are possible. Preferably, these moieties are connected to each other by peptide bonds. More preferably, the chemical spacer X is linked through a carboxyl group to the amino group of Z by forming a peptide bond, and through an amino group to the carboxyl group of $B_1$, also by forming a peptide bond.

Preferably, the chemical spacer X is L-serine.

$B_1$ represents a branching moiety. The branching moiety is derived from an amino acid residue comprising two amino groups and a carboxyl group. $B_1$ is coupled to X through its carboxyl group, and to each $L_1$ through its amino groups. Preferably, the branching moiety $B_1$ is derived from L-lysine; L-ornithine, α-aminoglycine, α,γ-diaminopropionic acid, α,γ-diaminobutyric acid, or an amino acid according to formula

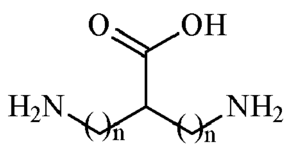

(diAA) wherein n is selected from 1, 2, 3, and 4.

In certain embodiments of the present invention, wherein X is absent, $B_1$ is coupled to Z through its carboxyl group. In certain embodiments of the present invention, wherein $L_1$ is absent, $B_1$ is coupled to Y through one of its amino groups.

In a preferred embodiment of the present invention, $B_1$ is L-lysine. L-lysine as $B_1$ in the compound according to formula (I) is coupled to X through its carboxyl group, to one $L_1$ moiety through its backbone amino group, and to another $L_1$ moiety through its side chain amino group.

In another preferred embodiment of the present invention, $B_1$ is diAA. diAA as $B_1$ in the compound according to formula (I) is coupled to X through its carboxyl group, and to each $L_1$ moiety through one of its amino groups.

Each $L_1$ independently represents:

i. a single amino acid residue or a dipeptide, preferably wherein the single amino acid residue is L-serine or L-glycine or wherein the dipeptide consists of or comprises L-serine and/or L-glycine, or
ii. a moiety comprising 1 to 36 ethylene glycol repeats according to the formula

$$-NH(CH_2CH_2O)_{1-36}CH_2CO-;$$

or
iii. is absent.

In certain embodiments of the present invention, $L_1$ is a single amino acid residue. Preferably, $L_1$ is L-serine or L-glycine, more preferably $L_1$ is L-serine. The single amino acid residue is coupled to one of the amino groups of $B_1$ through its carboxyl group, and to Y moiety through its amino group.

In certain embodiments of the present invention, $L_1$ is a dipeptide. Preferably, the dipeptide consists of or comprises L-serine and/or L-glycine. More preferably, $L_1$ is derived from L-seryl-L-serine, L-seryl-glycine or glycyl-L-serine. In the embodiments of the invention wherein $L_1$ is a dipeptide, the C-terminal residue of the dipeptide is linked through its carboxyl group to the amino group of $B_1$ by forming a peptide bond, and N-terminal residue of the dipeptide is linked through its amino group to Y.

In certain embodiments of the present invention, $L_1$ is or comprises beta-alanine. That is, in certain embodiments, $L_1$ is a single beta-alanine residue. In other embodiments, $L_1$ is derived from beta-alanine-L-serine, L-seryl-beta-alanine.

In certain embodiments of the present invention, $L_1$ is a moiety comprising 1 to 36 ethylene glycol repeats. In

certain embodiments, the $L_1$ is a moiety comprising 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 or 36 ethylene glycol repeats. Preferably, $L_1$ is $-NH(CH_2CH_2O)_2CH_2CO-$ or $-NH(CH_2CH_2O)_5CH_2CO-$.

In certain embodiments of the present invention, $B_1$ is L-lysine and each $L_1$ is L-serine. Thus, in certain embodiments of the present invention, the compound according to formula (I) comprises the moiety:

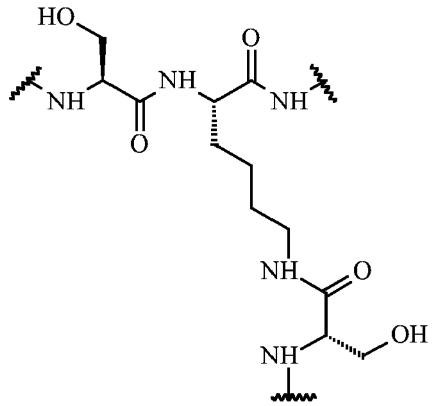

Each Y independently represents $P-L_3-$ or a moiety according to formula (II)

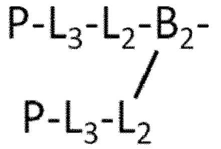

(II)

$B_2$ represents a branching moiety. The branching moiety $B_2$ is derived from an amino acid residue comprising two amino groups and a carboxyl group. $B_2$ is coupled to $L_1$ through its carboxyl group, and to each $L_2$ through its amino groups. Preferably, the branching moiety $B_2$ is derived from L-lysine; L-ornithine, $\alpha$-aminoglycine, $\alpha,\gamma$-diaminopropionic acid, $\alpha,\gamma$-diaminobutyric acid, or an amino acid according to formula

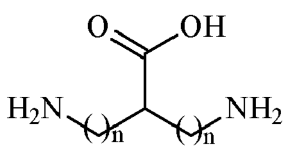

(diAA) wherein n is selected from 1, 2, 3, and 4.

In certain embodiments of the present invention, wherein $L_2$ is absent, $B_2$ is coupled to $L_3$ through one of its amino groups.

In a preferred embodiment of the present invention, $B_2$ is L-lysine. L-lysine as $B_2$ in the compound according to formula (I) is coupled to $L_1$ through its carboxyl group, to one $L_2$ moiety through its backbone amino group, and to another $L_2$ moiety through its side chain amino group.

In another preferred embodiment of the present invention, $B_2$ is diAA. diAA as $B_2$ in the compound according to formula (I) is coupled to $L_1$ through its carboxyl group, and to each $L_2$ moiety through one of its amino groups.

Each $L_2$ independently represents:

i. a single amino acid residue or a dipeptide, preferably wherein the single amino acid residue is L-serine or L-glycine or wherein the dipeptide consists of or comprises L-serine and/or L-glycine, or
ii. is absent.

In certain embodiments of the present invention, $L_2$ is a single amino acid residue. The single amino acid residue is coupled to one of the amino groups of $B_2$ through its carboxyl group, and to $L_3$ through its amino group. Preferably, $L_2$ is L-serine or L-glycine, more preferably $L_2$ is L-serine. The single amino acid residue is coupled to one

of the amino groups of $B_2$ through its carboxyl group, and to Y moiety through its amino group.

In certain embodiments of the present invention, $L_2$ is a dipeptide. Preferably, the dipeptide consists of or comprises L-serine and/or L-glycine. More preferably, $L_2$ is derived from L-seryl-L-serine, L-seryl-glycine or glycyl-L-serine. In the embodiments of the invention wherein $L_2$ is a dipeptide, the C-terminal residue of the dipeptide is linked through its carboxyl group to the amino group of $B_2$ by forming a peptide bond, and N-terminal residue of the dipeptide is linked through its amino group to Y.

$L_3$ represents a single amino acid residue coupled to $L_2$ through its carboxylic group and coupled to P through its amino-group.

In certain embodiments, $L_3$ may be a D-amino acid residue. In certain embodiments, $L_3$ may be an amino acid residue comprising an aromatic or a positively charged amino acid side chain. In certain embodiments, $L_3$ may be a D-amino acid residue comprising an aromatic or a positively charged amino acid side chain. In certain embodiments, $L_3$ may be a L- or D-histidine residue. Preferably, $L_3$ represents a D-histidine residue.

That is, in a preferred embodiment, each Y independently represents P-(D-His)- or a moiety according to formula (IIa)

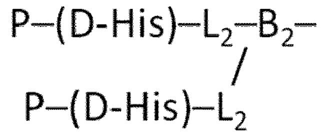

(IIa)

In certain embodiments of the present invention, when at least one Y represents

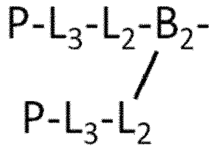

(II)

$L_1$ coupled to $B_2$ is not absent.

In certain embodiments of the present invention, wherein the branching moiety $B_1$ and/or $B_2$ is derived from L-lysine or L-ornithine; when $L_1$ and/or $L_2$ are coupled to an amino group comprised in the amino acid side chain of $B_1$ and/or $B_2$, respectively, $L_1$ and/or $L_2$ are not absent. That is, in certain embodiments, $L_3$ is not directly linked to an amino group in the amino acid side chain of $B_1$ and/or $B_2$.

In certain embodiments, the compound of the present invention comprises the structure:

(D-His)-(L-Ser)-(L-Lys)-(L-Cys) (SEQ ID NO:1);
(D-His)-(L-Ser)-(L-Lys)-(L-Ser)-(L-Lys)-(L-Cys) (SEQ ID NO:2);
(D-His)-(L-Ser)-(L-Lys)-(βAla)-(L-Ser)-(L-Lys)-(L-Cys) (SEQ ID NO:3);
(D-His)-(L-Ser)-(L-Lys)-(L-Ser)-(βAla)-(L-Lys)-(L-Cys) (SEQ ID NO:4);
(D-His)-(L-Ser)-(L-Lys)-(L-Ser)-(L-Lys)-(PEG1)-(L-Cys) (SEQ ID NO:5);
(D-His)-(L-Ser)-(L-Lys)-(PEG1/5/16/36)-(L-Cys) (SEQ ID NO:6);
(D-His)-(L-Ser)-(L-Lys)-(PEG1/3/5)-(L-Lys)-(L-Cys) (SEQ ID NO:7);
(D-His)-(PEG2/5)n-(L-Lys)-(L-Cys) (SEQ ID NO:8);
(D-His)-(L-Ser)-(diAA)-(L-Ser)-(diAA)-(L-Cys) (SEQ ID NO:9);
(D-His)-(L-Ser)-(diAA)-(L-Cys) (SEQ ID NO:10);
(D-His)-(L-Ser)-(L-Lys)-(Gly)-(L-Lys)-(L-Cys) (SEQ ID NO:11);
(D-His)-(Gly)-(L-Lys)-(L-Ser)-(L-Lys)-(L-Cys) (SEQ ID NO:12);
(D-His)-(Gly)-(L-Lys)-(Gly)-(L-Lys)-(L-Cys) (SEQ ID NO:13);
(D-His)-(Gly)-(L-Lys)-(L-Cys) (SEQ ID NO:14);
(D-His)-(L-Ser)-(diAA)-(Gly)-(diAA)-(L-Cys) (SEQ ID NO:15);
(D-His)-(Gly)-(diAA)-(L-Ser)-(diAA)-(L-Cys) (SEQ ID NO:16);
(D-His)-(Gly)-(diAA)-(Gly)-(diAA)-(L-Cys) (SEQ ID NO:17);
(D-His)-(Gly)-(diAA)-(L-Cys) (SEQ ID NO:18);

(D-His)-(L-Ser)-(L-Lys)-(L-PAG) (SEQ ID NO:19);
(D-His)-(L-Ser)-(L-Lys)-(L-Ser)-(L-Lys)-(L-PAG) (SEQ ID NO:20);
(D-His)-(L-Ser)-(L-Lys)-(L-Bct) (SEQ ID NO:21);
(D-His)-(L-Ser)-(L-Lys)-(L-Ser)-(L-Lys)-(L-Bct) (SEQ ID NO:22);
(D-His)-(L-Lys)-(L-Cys) (SEQ ID NO:23);
(D-His)-(L-Lys)-(L-Ser)-(L-Lys)-(L-Cys) (SEQ ID NO:24);
(D-His)-(L-Lys)-(PEG1)-(L-Cys), (SEQ ID NO:25);
(D-His)-(L-Lys)-(L-Ser)-(L-Lys)-(PEG1)-(L-Cys), (SEQ ID NO:26);
(D-His)-(L-Ser)-(L-Ser)-(L-Lys)-(L-Cys) (SEQ ID NO:27);
(D-His)-(L-Ser)-(L-Ser)-(L-Lys)-(L-Ser)-(L-Lys)-(L-Cys) (SEQ ID NO:28);
(D-His)-(L-Ser)-(L-Lys)-(L-Ser)-(L-Ser)-(L-Cys) (SEQ ID NO:29); or
(D-His)-(L-Ser)-(L-Lys)-(L-Ser)-(L-Lys)-(L-Ser)-(L-Ser)-(L-Cys) (SEQ ID NO:30).

Herein, PEG1/5/16/36 represent a moiety according to the formula-$NH(CH_2CH_2O)_nCH_2CO$-, wherein n is 1, 5, 16 or 36, respectively. Further herein, PEG1/3/5 represent a moiety according to the formula -$NH(CH_2CH_2O)_nCH_2CO$-, wherein n is 1, 3 or 5, resepectively. Further herein, PEG2/5 represent a moiety according to the formula -$NH(CH_2CH_2O)_nCH_2CO$-, wherein n is 2 or 5, respectively. Further herein, PEG1 represent a moiety according to the formula-$NH(CH_2CH_2O)_nCH_2CO$-, wherein n is 1. **B**-Ala represents beta-alanine and L-Bct represents L-Biocytin.

Preferably, in certain embodiments, the compound of the present invention comprises the structure:

(D-His)-(L-Ser)-(L-Lys[E-N-(L-Ser)-(D-His)]) (SEQ ID NO:31);

(D-His)-(L-Ser)-(L-Lys[ε-N-(L-Ser)-(L-Lys[ε-N-(L-Ser)-(D-His)])-(L-Ser)-(D-His)]) (SEQ ID NO:32);

(D-His)-(L-Ser)-(L-Lys[E-N-(L-Ser)-(D-His)])-(L-Ser)-(L-Lys[E-N-(L-Ser)-(D-His)]) (SEQ ID NO:33);

(D-His)-(L-Ser)-(L-Lys[**ε**-N-(L-Ser)-(L-Lys[**ε**-N-(L-Ser)-(D-His)])-(L-Ser)-(D-His)])-(L-Ser)-(L-Lys[**ε**-N-(L-Ser)-(D-His)]) (SEQ ID NO:34);

(D-His)-(L-Ser)-(L-Lys[**ε**-N-(L-Ser)-(D-His)])-(L-Ser)-(L-Lys[**ε**-N-(L-Ser)-(L-Lys[**ε**-N-(L-Ser)-(D-His)])-(L-Ser)-(D-His)]) (SEQ ID NO:35);

or

(D-His)-(L-Ser)-(L-Lys[**ε**-N-(L-Ser)-(L-Lys[**ε**-N-(L-Ser)-(D-His)])-(L-Ser)-(D-His)])-(L-Ser)-(L-Lys[**ε**-N-(L-Ser)-(L-Lys[**ε**-N-(L-Ser)-(D-His)])-(L-Ser)-(D-His)]) (SEQ ID NO:36),

wherein the structure further comprises a reactive moiety Z comprised to the free carboxyl group of the L-Lys residue. In certain embodiments, the reactive moiety Z is L-cysteine.

In a particular embodiments, the invention relates to the compounds according to the invention, wherein the compound comprises at least one PEG moiety.

It has been surprisingly shown by the inventors, that compounds comprising a PEG moiety may bind to cell comprising proteoglycans with higher affinity than peptides without a PEG moiety (Figures 3 and 4). In certain embodiments, the PEG moiety may be located between the branching moieties $B_1$ and $B_2$. In certain embodiments, the PEG moiety between the branching moieties $B_1$ and $B_2$ may comprise between 1 and 36 ethylene glycol moieties, preferably between 1 and 12 ethylene glycol moieties, more preferably between 1 and 5 ethylene glycol moieties, most preferably 5 ethylene glycol moieties.

In other embodiments, the PEG moiety may be located between the branching moieties $B_1$ or $B_2$ and the reactive moiety Z. In certain embodiments, the PEG moiety between the branching moieties $B_1$ and $B_2$ may comprise between 1 and 36 ethylene glycol moieties, preferably between 1 and 12 ethylene glycol moieties, more preferably between 1 and 5 ethylene glycol moieties, most preferably 1 ethylene glycol moieties.

In other embodiments, the PEG moiety may replace the moieties $L_1$ and/or $L_2$. In certain embodiments, the PEG moiety replacing the moieties $L_1$ and/or $L_2$ may comprise between 1 and 36 ethylene glycol moieties, preferably between 5 and 12 ethylene glycol moieties.

The synthesis of compounds comprising one or more PEG moieties is known to the person skilled in the art. In particular, compounds comprising a PEG moiety may be synthesized like normal peptides, with the difference that Fmoc-NH-$PEG_{(x)}$-OH is used as a building block instead of an amino acid.

It is to be understood that in embodiments where the compound of the invention comprises two peptide moieties P, the compound preferably comprises the structure set forth in SEQ ID NO:31. In embodiments where the compound of the invention comprises three peptide moieties P, the compound preferably comprises the structure set forth in SEQ ID NO:32 or SEQ ID NO:33, preferably SEQ ID NO:33. In embodiments where the compound of the invention comprises four peptide moieties P, the compound preferably comprises the structure set forth in SEQ ID NO:34 or SEQ ID NO:35, preferably SEQ ID NO:35. In embodiments where the compound of the invention comprises five peptide moieties P, the compound preferably comprises the structure set forth in SEQ ID NO:36.

P represents a peptide moiety. Each P independently represents a polypeptide comprising the amino acid sequence:
$(AA_1)$-$(AA_2)$-$(AA_3)$-$(AA_4)$-$(AA_5)$-$(AA_6)$-$(AA_7)$-$(AA_8)$-$(AA_9)$,

$(AA_1)$ represents a single amino acid residue with a positively charged amino acid side chain, or is absent.

$(AA_5)$ and $(AA_7)$ each represent a single amino acid residue comprising a positively charged amino acid side chain, a hydrophobic amino acid side chain or an aromatic amino acid side chain.

$(AA_2)$, $(AA_3)$, $(AA_4)$ and $(AA_6)$ each represent a single amino acid residue.

$(AA_8)$ represents a single amino acid residue, or is absent.

$(AA_9)$ represents a single amino acid residue comprising a positively charged amino acid side chain, a hydrophobic amino acid side chain or an aromatic amino acid side chain, or is absent.

$(AA_8)$ and $(AA_9)$ are either both present or both absent, wherein when $(AA_8)$ and $(AA_9)$ are both absent, $(AA_1)$, $(AA_3)$, $(AA_5)$ and $(AA_7)$ represent a single amino acid residue comprising a positively charged amino acid side chain.

According to the embodiments of the present invention, each P comprises at least 3 amino acid residues comprising a positively charged amino acid side chain. Preferably, each P comprises 4 amino acid residues comprising a positively charged amino acid side chain.

Further, according to the embodiments of the present invention, each P is coupled to an $\alpha$-amino group of an$L_3$ residue through the $\alpha$-carboxyl group of residue $(AA_7)$ and/or $(AA_9)$.

In certain embodiments of the present invention, in at least one, at least two, at least three or all peptide moieties P comprised in the compound of the invention, at least two of the amino acid residues $(AA_1)$, $(AA_5)$, $(AA_7)$ and $(AA_9)$ comprise an amino acid residue with a positively charged amino acid side chain.

That is, in certain embodiments of the present invention, in at least one, at least two, at least three or all peptide moieties P comprised in the compound of the invention, two of the amino acid residues $(AA_1)$, $(AA_5)$, $(AA_7)$ and $(AA_9)$ comprise an amino acid residue with a positively charged amino acid side chain.

In other embodiments of the present invention, in at least one, at least two, at least three or all peptide moieties P comprised in the compound of the invention, three of the amino acid residues $(AA_1)$, $(AA_5)$, $(AA_7)$ and $(AA_9)$ comprise an amino acid residue with a positively charged amino acid side chain.

In further embodiments of the present invention, in at least one, at least two, at least three or all peptide moieties P comprised in the compound of the invention, all of the amino acid residues $(AA_1)$, $(AA_5)$, $(AA_7)$ and $(AA_9)$ comprise an amino acid residue with a positively charged amino acid side chain.

Amino acids with a positively charged amino acid side chain preferably are selected from a group consisting of: L-arginine, D-arginine, L-lysine, L-ornithine and L-2-amino-4-guanidinobutyric acid.

In certain embodiments of the present invention, at least one, at least two, at least three or all peptide moieties P comprise at least one D-amino acid. In particular, in certain embodiments of the present invention amino acid residue $(AA_4)$ and/or $(AA_6)$ is a D-amino acid.

That is, in certain embodiments of the present invention, at least one, at least two, at least three or all peptide moieties P comprise a D-amino acid, in particular wherein amino acid residue $(AA_4)$ or $(AA_6)$ is a D-amino acid. Preferably, $(AA_4)$ is a D-amino acid,

In other embodiments of the present invention, at least one, at least two, at least three or all peptide moieties

P comprise two D-amino acids, in particular wherein amino acid residues ($AA_4$) and ($AA_6$) are D-amino acids.

In other embodiments of the present invention, at least one, at least two, at least three or all peptide moieties P comprise three or more D-amino acids, in particular wherein amino acid residues ($AA_4$) and ($AA_6$) and at least one of the amino acid residues (AA1), (AA2), (AA5) and/or (AA9) are D-amino acids.

In certain embodiments of the present invention, at least one, at least two, at least three or all peptide moieties P comprise not more than one amino acid residue comprising a negatively charged amino acid side chain. In particular, in certain embodiments of the present invention none of the amino acid residues in position ($AA_1$) to ($AA_3$) and ($AA_5$) to ($AA_9$) comprise an amino acid residue comprising a negatively charged amino acid side chain. In certain embodiments of the present invention, at least one, at least two, at least three or all peptide moieties P are free of amino acid residues comprising a negatively charged amino acid side chain.

That is, the peptides comprised in the compound of the invention is preferably free of amino acids with negatively charged amino acid side chains. If a peptide comprises a negatively charged amino acid side chain, said negatively charged amino acid side chain is preferably comprised in position ($AA_4$). In certain embodiments, the amino acid in position ($AA_4$) may be any L- or D-amino acid.

In certain embodiments of the present invention, at least one, at least two, at least three or all peptide moieties P comprise not more than one proline residue. In particular, none of the amino acid residues in position ($AA_1$) to ($AA_3$) and ($AA_5$) to ($AA_9$) comprise a proline residue. In certain embodiments of the present invention, at least one, at least two, at least three or all peptide moieties P are free of proline residues.

That is, the peptides comprised in the compound of the invention is preferably free of proline residues. If a peptide comprises a proline residue, said proline residue is preferably comprised in position ($AA_4$). In certain embodiments, the amino acid in position ($AA_4$) may be any L- or D-amino acid.

It is preferred that at least one, at least two, at least three, or all peptide moieties P is(are) not moieties that comprise only L- or only D- amino acid residues. That is, it is preferred that at least one, at least two, at least three or all peptide moieties P comprise at least one L- and at least one D- amino acid residue.

In certain embodiments of the present invention, at least two, at least three or all peptide moieties P have identical amino acid sequences. Preferably, all peptide moieties P have identical amino acid sequences.

That is, all peptide moieties comprised in the compound of the invention may have identical amino acid sequences. In certain embodiments, the compound of the invention may comprise two peptide moieties, wherein both peptide moieties have different amino acid sequences. In other embodiments, the compound of the invention may comprise two peptide moieties, wherein both peptide moieties have identical amino acid sequences. In other embodiments, the compound of the invention may comprise three peptide moieties, wherein two or three peptide moieties have identical amino acid sequences. In other embodiments, the compound of the invention may comprise four peptide moieties, wherein two, three or four peptide moieties have identical amino acid sequences.

It is to be understood that the binding specificity for proteoglycans of the compounds disclosed herein is conferred by the peptide moieties comprised in said compounds. Disclosed herein are peptide moieties that bind specifically to proteoglycans, in particular to chondroitin-4-sulfate, chondroitin-6-sulfate and/or keratan sulfate.

In certain embodiments of the present invention, a peptide moiety P comprised in the compound of the invention specifically binds to the proteoglycan chondroitin 4-sulfate (also referred to as C4S). Peptides that bind specifically to C4S are known in the prior art and have been previously disclosed in WO 2014/072411.

<u>That is, the following embodiments apply particularly to peptide moieties that bind specifically to C4S.</u>

In certain embodiments, peptide moieties binding specifically to C4S may comprise a positively charged amino acid side chain in amino acid residue ($AA_9$). In particular, ($AA_9$) is preferably selected from a group consisting of: L-arginine, L-lysine and L-2-amino-4-guanidinobutyric acid. Most preferably ($AA_9$) is L-arginine.

In certain embodiments, peptide moieties binding specifically to C4S may comprise a positively charged amino acid side chain in at least one of the amino acid residues ($AA_2$) and/or ($AA_5$) and in at least one of the amino acid residues ($AA_7$) and/or ($AA_8$).

Preferably, the amino acid comprising the positively charged amino acid side chain in position ($AA_2$) and/or ($AA_5$) is selected from a group consisting of: L-arginine, D-arginine and L-2-amino-4-guanidinobutyric acid; and/or the amino acid comprising the positively charged amino acid side chain in position ($AA_7$) and/or ($AA_8$) is selected from a group consisting of: L-arginine, L-lysine and L-ornithine. In particular, the positively charged amino acid in position ($AA_7$) is L-ornithine or L-lysine and the positively charged amino acid in position ($AA_8$) is L-arginine or L-lysine.

In certain embodiments, peptide moieties binding specifically to C4S may comprise an aromatic or hydrophobic amino acid residue in position ($AA_2$) when ($AA_5$) is a positively charged amino acid. In other embodiments, peptide moieties binding specifically to C4S may comprise an aromatic amino acid residue in position ($AA_5$) when ($AA_2$) is a positively charged amino acid.

In certain embodiments, peptide moieties binding specifically to C4S may comprise an aromatic amino acid

residue in position ($AA_7$) when ($AA_8$) is a positively charged amino acid. In other embodiments, peptide moieties binding specifically to C4S may comprise an aromatic or hydrophobic amino acid residue in position ($AA_8$) when ($AA_7$) is a positively charged amino acid.

In certain embodiments, the aromatic amino acid residue in ($AA_2$), ($AA_5$), ($AA_7$) and/or ($AA_8$) is L-tyrosine.

In certain embodiments of the present invention, in at least one, at least two, at least three or all peptide moieties P that preferably are able to effect specific binding to the proteoglycan C4S, between 2 and 5 amino acid residues comprise a hydrophobic and/or aromatic amino acid side chain. In certain embodiments, amino acid residue ($AA_3$) is L-phenylalanine or another aromatic and/or hydrophobic amino acid residue.

($AA_4$) may be any L- or D-amino acid. However, it is preferred that ($AA_4$) is a hydrophobic D-amino acid. More preferably, ($AA_4$) is selected from the group consisting of: D-valine, D-alanine, D-leucine, D-isoleucine and D-methionine. Most preferably ($AA_4$) is D-valine.

In certain embodiments, amino acid residue ($AA_6$) is a hydrophobic and/or aromatic amino acid. Preferably, ($AA_6$) is L-isoleucine or D-isoleucine.

In certain embodiments, the peptide binding specifically to C4S has the consensus sequence:

a) (P)-(A)-(A)-(Aa)-(P)-(H)-(P)-(A)-(P);
b) (P)-(P)-(A)-(Aa)-(A)-(H)-(A)-(P)-(P);
c) (P)-(A)-(A)-(Aa)-(P)-(H)-(A)-(P)-(P); or
d) (A)-(A)-(Aa)-(P)-(H)-(P)-(A)-(P);

wherein (P) is an L-amino acid with a positively charged amino acid side chain, (A) is an L-amino acid with an aromatic side chain, (Aa) is any L- or D-amino acid, preferably any D-amino acid, more preferably an aromatic D-amino acid, and (H) is an L- or D-amino acid with a hydrophobic amino acid side chain, preferably an L-amino acid with a hydrophobic amino acid side chain.

Alternatively, (P) is a D-amino acid with a positively charged amino acid side chain, (A) is a D-amino acid with an aromatic side chain, (Aa) is any L- or D-amino acid, preferably any D-amino acid, more preferably an aromatic D-amino acid, and (H) may be an L- or D-amino acid with a hydrophobic amino acid side chain, preferably a D-amino acid with a hydrophobic amino acid side chain.

In certain embodiments of the present invention, at least one, at least two, at least three or all peptide moieties P that preferably are able to effect specific binding to the proteoglycan C4S comprise a sequence independently selected from a group consisting of:

RYFvRIOYR (SEQ ID NO:37);
RRFvYIYRR (SEQ ID NO:38);
RYFvRIYRR (SEQ ID NO:39);
RYFvrIOYR (SEQ ID NO:40);
RYFvRiOYR (SEQ ID NO:41);
RYFvriOYR (SEQ ID NO:42);
RRFvYIYKR (SEQ ID NO:43);
RRFqYIYKR (SEQ ID NO:44);
RYFvRIYKR (SEQ ID NO:45);
RYFvRIYRK (SEQ ID NO:46);
RRFaYIYKR (SEQ ID NO:47);
RRFpYIYKR (SEQ ID NO:48);
RYFvRIKYR (SEQ ID NO:49);
RYFqRIKYR (SEQ ID NO:50);
YFvRIOYR (SEQ ID NO:51);
(Agb)YFv(Agb)IKY(Agb) (SEQ ID NO:52);
YFvRIKYR (SEQ ID NO:53);
rYFvRIKYR (SEQ ID NO:54);
RYFvrIKYR (SEQ ID NO:55);
rrFvYIYRR (SEQ ID NO:56);
RSTqRYRVR (SEQ ID NO:57);
RYFvRIKYR (SEQ ID NO:58);
RYFvRIKAR (SEQ ID NO:59);
RAAvRAKYR (SEQ ID NO:60);
RAAvRIKYR (SEQ ID NO:61);
RSTqRYRVR (SEQ ID NO:62);

RSTqRYKVR (SEQ ID NO:63);
RGGgRGKGR (SEQ ID NO:64);
RHHhRHKHR (SEQ ID NO:65);
RVVvRVKVR (SEQ ID NO:66);
RLLlRLKLR (SEQ ID NO:67);
RMMmRMKMR (SEQ ID NO:68);
RIIiRIKIR (SEQ ID NO:69); and
RYFVRiKYR (SEQ ID NO:70);

wherein capital letters represent L-amino acids and wherein small letters represent D-amino acids. Agb represents and L-2-amino-4-guanidinobutyric acid. O represents L-ornithine.

In certain embodiments, the compound of the invention comprises at least one, at least two, at least three or 4 peptide moieties P selected from the group consisting of: RYFvRIOYR (SEQ ID NO:37), RRFvYIYRR (SEQ ID NO:38) and RYFvRIYRR (SEQ ID NO:39).

That is, in one embodiment, the compound of the invention comprises at least one, at least two, at least three or 4 copies of the peptide moiety RYFvRIOYR (SEQ ID NO:37). In one embodiment, the compound of the invention comprises two copies of the peptide moiety RYFvRIOYR (SEQ ID NO:37), wherein the two copies are linked with the structure of SEQ ID NO:31. In one embodiment, the compound of the invention comprises three copies of the peptide moiety RYFvRIOYR (SEQ ID NO:37), wherein the two copies are linked with the structure of SEQ ID NO:32 or SEQ ID NO:33, preferably with the structure of SEQ ID NO:33. In one embodiment, the compound of the invention comprises four copies of the peptide moiety RYFvRIOYR (SEQ ID NO:37), wherein the two copies are linked with the structure of SEQ ID NO:34 or SEQ ID NO:35, preferably with the structure of SEQ ID NO:35.

In one embodiment, the compound of the invention comprises at least one, at least two, at least three or 4 copies of the peptide moiety RRFvYIYRR (SEQ ID NO:38). In one embodiment, the compound of the invention comprises two copies of the peptide moiety RRFvYIYRR (SEQ ID NO:38), wherein the two copies are linked with the structure of SEQ ID NO:31. In one embodiment, the compound of the invention comprises three copies of the peptide moiety RRFvYIYRR (SEQ ID NO:38), wherein the two copies are linked with the structure of SEQ ID NO:32 or SEQ ID NO:33, preferably with the structure of SEQ ID NO:33. In one embodiment, the compound of the invention comprises four copies of the peptide moiety RRFvYIYRR (SEQ ID NO:38), wherein the two copies are linked with the structure of SEQ ID NO:34 or SEQ ID NO:35, preferably with the structure of SEQ ID NO:35.

In one embodiment, the compound of the invention comprises at least one, at least two, at least three or 4 copies of the peptide moiety RYFvRIYRR (SEQ ID NO:39). In one embodiment, the compound of the invention comprises two copies of the peptide moiety RYFvRIYRR (SEQ ID NO:39), wherein the two copies are linked with the structure of SEQ ID NO:31. In one embodiment, the compound of the invention comprises three copies of the peptide moiety RYFvRIYRR (SEQ ID NO:39), wherein the two copies are linked with the structure of SEQ ID NO:32 or SEQ ID NO:33, preferably with the structure of SEQ ID NO:33. In one embodiment, the compound of the invention comprises four copies of the peptide moiety RYFvRIYRR (SEQ ID NO:39), wherein the two copies are linked with the structure of SEQ ID NO:34 or SEQ ID NO:35, preferably with the structure of SEQ ID NO:35.

Further peptides that bind specifically to C4S and may be comprised in the compound of the invention have been disclosed in WO 2014/072411, which is enclosed herein in its entirety.

That is, in certain embodiments of the present invention, the compound of the invention comprises at least one, at least two, at least three or four peptide moieties P that effect specific binding to the proteoglycan C4S, wherein the peptide moieties comprise the consensus sequence

(i) $Arg_1$-$X_2$-$X_3$-$X_4$-$Arg_5$-$X_6$-$X_7$-$X_8$-$Arg_9$ or wherein

(a) $Arg_1$, Args, and $Arg_9$ represent L-arginine; and wherein the remaining amino acids $X_2$ to $X_4$ and $X_6$ to $X_8$ may be any L-or D- amino acid other than L-lysine, D-lysine, L-arginine or D-arginine, with the proviso that either $X_3$ or $X_7$, but not both, represents L-lysine or L-arginine; or wherein

(b) $Arg_1$, Args, and $Arg_9$ represent D-arginine; and wherein the remaining amino acids $X_2$ to $X_4$ and $X_6$ to $X_8$ may be any L-or D- amino acid other than L-lysine, D-lysine, L-arginine or D-arginine, with the proviso that either $X_3$ or $X_7$, but not both, represents D-lysine or D-arginine.

As reflected in the consensus rules above, that the amino acid residues at positions 2, 4, 6, 8 and 3 (where position 7 is defined/selected according to rule (b)) or 7 (where position 3 is defined/selected according to rule (b)) do not contribute to the binding activity of the peptide. Therefore, the targeting moiety of the above peptides, represented by $Arg_1$-$X_2$-$X_3$-$X_4$-$Arg_5$-$X_6$-$X_7$-$X_8$-$Arg_9$ with the proviso that position 3 or 7 (but not both) is a lysine or arginine, can be

seen to be to be identical for all possible peptides encompassed by consensus sequence (i) (with respect to the presentation of the side chains at 1, 5, 9, and 3 or 7, in 3D space). In fact, it is the 3D linear arrangement and/or 3D configuration of the arginine residues at positions 1, 5 and 9, together with the lysine or arginine at position 3 or 7 (but not both) that establishes the binding activity of the targeting moiety sequences and contributes the binding specificity and selectively for C4S relative to other proteoglycans. The remaining amino acid residues, i.e., positions 2, 4, 6, 8 and 3 (where position 7 is defined/selected according to rule (b)) or 7 (where position 3 is defined/selected according to rule (b)) serve primarily as a structural backbone to maintain the proper distance and relative configuration between the arginines at positions 1, 5 and 9, and the lysine or arginine at position 3 or 7 (but not both) of the targeting moiety.

In certain embodiments of the present invention, the peptide moieties P that are preferably able to effect specific binding to the proteoglycan C4S, exhibit a 3D presentation of 3 arginine residues (i.e., $Arg_1$, $Arg_5$, and $Arg_9$) and the one arginine or lysine (i.e., $Arg/Lys_3$ or $Arg/Lys_7$ but not both) equivalent to the peptides and/or targeting moieties of the consensus sequence (i) as defined above. In other words, the invention encompasses molecules comprising or consisting of amino acid residues and also encompasses molecules comprising both amino acid residues and chemical linkers/spacers such that the peptide moieties P exhibit 3 arginines and 1 lysine or arginine in the same or an equivalent relative 3-dimensional confirmation as the arginines at positions 1, 5 and 9, and the lysine or arginine at position 3 or 7 (but not both) as in consensus sequence (i) for C4S binding as above.

It is further understood that all peptides disclosed in WO 2014/072411 to specifically bind to C4S are included herein and may be comprised in the compound of the invention.

In certain embodiments of the present invention, a peptide moiety P comprised in the compound of the invention specifically binds to the proteoglycan chondroitin-6-sulfate (also referred to as C6S). Peptides that bind specifically to C6S are known in the prior art and have been previously disclosed in WO 2015/162285.

That is, the following embodiments apply particularly to peptide moieties that bind specifically to C6S.

In certain embodiments of the present invention, at least one, at least two, at least three or all peptide moieties P that are preferably able to effect specific binding to the proteoglycan C6S, comprise a positively charged amino acid in position ($AA_1$), ($AA_3$) and ($AA_9$). In particular, ($AA_1$) and/or ($AA_9$) are preferably L-arginine and/or ($AA_3$) is preferably L-lysine. In certain embodiments, at least one, at least two, at least three or all peptide moieties P that are preferably able to effect specific binding to the proteoglycan C6S comprise an amino acid residue comprising a positively charged amino acid side chain in positions ($AA_5$) or ($AA_7$). In particular ($AA_5$) or ($AA_7$) are preferably L-arginine and/or L-lysine.

In certain embodiments of the present invention, at least one, at least two, at least three or all peptide moieties P that are preferably able to effect specific binding to the proteoglycan C6S, comprise between two and five amino acid residues comprising a hydrophobic and/or aromatic amino acid side chain. Preferably, at least one, at least two, at least three or all peptide moieties P that are preferably able to effect specific binding to the proteoglycan C6S, comprise five amino acid residues comprising a hydrophobic and/or aromatic amino acid side chain. In particular, the amino acid residues comprising the hydrophobic and/or aromatic side chains are preferably located in positions ($AA_2$), ($AA_4$), ($AA_6$), ($AA_8$) and/or one of positions ($AA_5$) or ($AA_7$).

In certain embodiments of the present invention, at least one, at least two, at least three or all peptide moieties P that are preferably able to effect specific binding to the proteoglycan C6S, comprise a sequence independently selected from a group consisting of:

RYKvFIKYR (SEQ ID NO:71);
RYKvAIKYR (SEQ ID NO:72);
RYKvRIAYR (SEQ ID NO:73);
RYKvRIFYR (SEQ ID NO:74);
RYKvKFIYR (SEQ ID NO:75);
RYKvFIRYR (SEQ ID NO:76);
RYKvRFIYR (SEQ ID NO:77);
RMKiVMKFR (SEQ ID NO:78); and
RFKfFFKFR (SEQ ID NO:79).

wherein capital letters represent L-amino acids and wherein small letters represent D-amino acids.

Further peptides that bind specifically to C6S and may be comprised in the compound of the invention have been disclosed in WO 2015/162285, which is enclosed herein in its entirety.

That is, in certain embodiments of the present invention, the compound of the invention comprises at least one, at least two, at least three or four peptide moieties P that effect specific binding to the proteoglycan C6S, wherein the peptide moieties comprise the consensus sequence

(ii) $Arg_1$-$X_2$-$Lys_3$-$X_4$-$X_5$-$X_6$-$X_7$-$X_8$-$Arg_9$

wherein

(a) $Arg_1$ and $Arg_9$ represent L-arginine; $Lys_3$ represents L-lysine; and wherein the remaining amino acids $X_4$ to $X_8$ may be independently selected from any L-or D-amino acid other than D-arginine, D-lysine, L-arginine or L-lysine, with the proviso that either $X_5$ or $X_7$, but not both, represents L-lysine or L-arginine;
or wherein

(b) $Arg_1$ and $Arg_9$ represent D-arginine; $Lys_3$ represents D-lysine; and wherein the remaining amino acids $X_4$ to $X_8$ may be independently selected from any L-or D-amino acid other than D-arginine, D-lysine, L-arginine or L-lysine, with the proviso that either $X_5$ or $X_7$, but not both, represents D-lysine or L-arginine.

As reflected in consensus sequence (ii), the amino acid residues at positions

- 2, 4, 6, 8 and 7 (where position 5 is defined as the L- or D- lysine or arginine according to the above rules), or
- 2, 4, 6, 8 and 5 (where position 7 is defined as the L- or D- lysine or arginine according to the above rules)

do not contribute to the binding activity of the peptide. Therefore, the targeting moiety of consensus sequence (ii), represented by $Arg_1$-$X_2$-$Lys_3$-$X_4$-$X_5$-$X_6$-$X_7$-$X_8$-$Arg_9$ with the proviso that position $X_5$ or $X_7$ (but not both) is a lysine or arginine can be seen to be to be identical for all possible peptides encompassed by consensus sequence (ii), differentiated only in following conditions (a) or (b), above. It is the 3D linear arrangement and/or 3D configuration of side chains of the arginine residues at positions 1 and 9, of the lysine residue at position 3, and of the lysine or arginine residue at position 5 or 7 (but not both) that establishes the binding activity of the targeting moiety sequences and contributes the binding specificity and selectively for C6S relative to other proteoglycans. The remaining amino acid residues, i.e., at positions 2, 4, 6, 8 and 7 (where position 5 is defined as the L- or D- lysine or arginine according to the above rules) or 5 (where position 7 is defined as the L- or D- lysine or arginine according to the above rules), serve primarily as a structural backbone to maintain the proper distance and relative configuration between the arginines at positions 1 and 5, the lysine at positions 3, and the arginine/lysine at position 5 or 7 (but not both) of the targeting moiety. Thus, provided the 3D spatial orientation of these residues is maintained (in particular, the 3D presentation of the amino acid residue side chains), the peptide, polypeptide and/or compositions of the invention will exhibit specific and selective binding for the proteoglycan C6S.

It is further understood that all peptides disclosed in WO 2015/162285 to specifically bind to C6S are included herein and may be comprised in the compound of the invention.

In certain embodiments, the peptide binding specifically to C4S and/or C6S may have the consensus sequence: e) (X)-(A)-($P_1$)-(Aa)-(X)-(H)-($P_2$)-(A)-(X);
wherein

(i) ($P_1$) is an L-amino acid with a positively charged or aromatic amino acid side chain, in particular wherein ($P_1$) is L-Arg or L-Phe; ($P_2$) is an L-amino acid with a positively charged amino acid side chain, in particular wherein ($P_2$) is L-Lys; (A) is an L-amino acid with an aromatic side chain, in particular wherein (A) is L-Tyr, (Aa) is any L- or D-amino acid, preferably any D-amino acid, more preferably an aromatic D-amino acid or D-Glu; (H) is an L-amino acid with a hydrophobic amino acid side chain, in particular L-Ile; and (X) is an L-amino acid with a positively charged amino acid side chain, in particular wherein (X) is L-ornithine or L-diaminobutyric acid or L-Arg;
or

(ii) ($P_1$) is a D-amino acid with a positively charged or aromatic amino acid side chain, in particular wherein ($P_1$) is D-Arg or D-Phe; ($P_2$) is a D-amino acid with a positively charged amino acid side chain, in particular wherein ($P_2$) is D-Lys; (A) is a D-amino acid with an aromatic side chain, in particular wherein (A) is D-Tyr, (Aa) is any L- or D-amino acid, preferably any D-amino acid, more preferably an aromatic D-amino acid or D-Glu; (H) is an D-amino acid with a hydrophobic amino acid side chain, in particular D-Ile; and (X) is an D-amino acid with a positively charged amino acid side chain, in particular wherein (X) is D-ornithine or D-diaminobutyric acid or D-Arg;

In the above embodiments and throughout the description, it is most preferred that the the peptide(s) that is/are comprised in the compound of the invention, i.e. the peptide component(s) of the compound of the invention, are not individual peptide chains that contain exlusively all D or all L amino acids. That is, it is most preferred that the peptide chain component(s) of the compounds of the invention comprise at least one D- and at least one L- amino acid residue.

In certain embodiments of the present invention, a peptide moiety P comprised in the compound of the invention specifically binds to the proteoglycan keratan sulfate (also referred to as KS). Peptides that bind specifically to KS are known in the prior art and have been previously disclosed in WO 2015/162287.

That is, the following embodiments apply particularly to peptide moieties that bind specifically to KS.

Accordingly, in certain embodiments of the present invention, at least one, at least two, at least three or all peptide moieties P that are preferably able to effect specific binding to the proteoglycan KS comprise an amino acid residue comprising a positively charged amino acid side chain in positions ($AA_1$), ($AA_3$), ($AA_5$) and ($AA_7$). In particular, ($AA_1$) and/or ($AA_5$) are preferably L-arginine and/or ($AA_3$) and/or ($AA_7$) are preferably L-lysine.

In certain embodiments of the present invention, at least one, at least two, at least three or all peptide moieties P that are preferably able to effect specific binding to the proteoglycan KS comprise between two and five amino acid residues comprising a hydrophobic and/or aromatic amino acid side chain. In particular the amino acid residues comprising the hydrophobic and/or aromatic side chains are located in positions ($AA_2$), ($AA_4$) and ($AA_6$), and optionally ($AA_8$) and/or ($AA_9$) when ($AA_8$) and/or ($AA_9$) are present. In particular, the amino acid residues in position ($AA_4$) and ($AA_6$) are preferably hydrophobic amino acids.

In certain embodiments of the present invention, in at least one, at least two, at least three or all peptide moieties P that are preferably able to effect specific binding to the proteoglycan KS, amino acid residues ($AA_8$) and ($AA_9$) are both absent or are both present. When (AA8) and (AA9) are both present, (AA8) and (AA9) may comprise amino acids comprising a positively charged amino acid side chain, a hydrophobic amino acid side chain and/or an aromatic amino acid side chain.

In certain embodiments of the present invention, at least one, at least two, at least three or all peptide moieties P that are preferably able to effect specific binding to the proteoglycan KS, comprise a sequence independently selected from a group consisting of:

RYKvRIK (SEQ ID NO:80);
RYKvRIKYF (SEQ ID NO:81);
RYKvRIKYA (SEQ ID NO:82);
RYKvRIKHH (SEQ ID NO:83);
RMKiRVK (SEQ ID NO:84);
RMKiRVKFM (SEQ ID NO:85);
RLKIRLKLL (SEQ ID NO:86); and
RYKvRIKYr (SEQ ID NO:87).

wherein capital letters represent L-amino acids and wherein small letters represent D-amino acids.

Further peptides that bind specifically to KS and may be comprised in the compound of the invention have been disclosed in WO 2015/162287, which is enclosed herein in its entirety.

That is, in certain embodiments of the present invention, the compound of the invention comprises at least one, at least two, at least three or four peptide moieties P that effect specific binding to the proteoglycan KS, wherein the peptide moieties comprise the consensus sequence
(iii) $Arg_1$-$X_2$-$Lys_3$-$X_4$-Args-$X_6$-$Lys_7$
wherein

(a) $Arg_1$ and $Arg_5$ represent L-arginine; $Lys_3$ and $Lys_7$ represent L-lysine; and wherein the remaining amino acids $X_2$, $X_4$ and $X_6$ may be independently selected from any L-or D- amino acid other than D-arginine, D-lysine; L-arginine or L-lysine;
or wherein

(b) $Arg_1$ and $Arg_5$ represent D-arginine; $Lys_3$ and $Lys_7$ represent D-lysine; and wherein the remaining amino acids $X_2$, $X_4$ and $X_6$ may be independently selected from any L-or D- amino acid other than D-arginine, D-lysine; L-arginine or L-lysine.

As reflected in consensus sequence (iii), the amino acid residues at positions 2, 4, and 6 do not contribute to the binding activity of the peptide. Therefore, the targeting moiety of the above peptides, represented by $Arg_1$-$X_2$-$Lys_3$-$X_4$-$Arg_5$-$X_6$-$Lys_7$, can be seen to be to be identical for all possible peptides encompassed by consensus sequence (iii), differentiated only in following conditions (a) or (b), above. It is the 3D linear arrangement and/or 3D configuration of side chains of the arginine residues at positions 1 and 5 together with those of the lysine residues at positions 3 and 7 that establishes the binding activity of the targeting moiety sequences and contributes the binding specificity and selectively for KS relative to other proteoglycans. The remaining amino acid residues, i.e., at positions 2, 4 and 6, serve primarily as a structural backbone to maintain the proper distance and relative configuration between the arginines at positions 1 and 5, and the lysines at positions 3 and 7 of the targeting moiety.

It is further understood that all peptides disclosed in WO 2015/162287 to specifically bind to KS are included

herein and may be comprised in the compound of the invention.

The compounds of the invention preferably comprise between 2 and 4 peptide moieties P. Preferably, all peptide moieties P comprised in a compound according to the invention comprise peptide moieties that specifically bind to the same proteoglycan. That is, a compound according to the invention may comprise 2, 3 or 4 peptide moieties that specifically bind to C4S, wherein the 2, 3 or 4 peptide moieties may have identical or different amino acid sequences. Alternatively, a compound according to the invention may comprise 2, 3 or 4 peptide moieties that specifically bind to C6S, wherein the 2, 3 or 4 peptide moieties may have identical or different amino acid sequences. Further, a compound according to the invention may comprise 2, 3 or 4 peptide moieties that specifically bind to KS, wherein the 2, 3 or 4 peptide moieties may have identical or different amino acid sequences.

Also encompassed herein are compounds that comprise 2, 3, or 4 peptide moieties, wherein at least one peptide moiety binds to a first proteoglycan and at least one peptide moiety binds to a second proteoglycan.

To describe the compounds of the present invention, the following nomenclature may be used, in particular for compounds wherein branching moiety is lysine, preferably L-lysine K. The peptide moiety can be represented as its identifier, for example 51, 21, or 28, as used herein. One peptide moiety is preferably directly attached to the $L_3$ moiety (herein D-His moiety) comprised in the backbone of the linker. For example, peptide 51 attached to D-His moiety comprised in the backbone of the linker will be denoted as 51-h. As understood herein, the branching moieties, for example lysine, may introduce branching points, i.e. two separate chains of moieties connected by for example peptide bonds may be present, one connected to one amino group, and one to another amino group of the branching moiety. For clarity, in the used herein naming convention one of the said chains will be shown in parenthesis. Preferably, if the branching moiety is an alpha-amino acid, the chain attached to the amino group in the side chain of the branching moiety will preferably be shown in parenthesis. For example, a notation 51-hS(51-hS)KC refers to a situation wherein lysine residue as a branching moiety is attached through its carboxylic group to the amino group of cysteine moiety, and to each of its amino groups a moiety 51-hS (peptide according to identifier 51 attached through its carboxylic group via peptide bond to the amino group of D-Histidine, which is attached through its carboxylic group to an amino group of L-Serine via a peptide bond) is attached through carboxylic group of its L-serine residue via a peptide bond. As both chains attached to the branching moiety are the same, the notation may here be simplified as $(51\text{-hS})_2$KC. In certain embodiments, the compounds of the present invention may comprise three peptide moieties. For example, 51-hS(51-hS)KS-(51-hS)KC, which may be also for simplicity denoted as $(51\text{-hS})_2$KS-(51-hS)KC. Herein, to the first branching moiety, two chains are attached: $(51\text{-hS})_2$KS- to its main chain amino group, and (51-hS) to its side chain amino group. The meaning of $(51\text{-hS})_2$KS moiety is to be understood in analogous way as explained above for $(51\text{-hS})_2$KC. In further certain embodiments of the present invention, four peptide moieties are present in the compounds of the present invention. For example $[(51\text{-hS})_2\text{KS}]_2$KC relates to the situation wherein to both main chain amino group and to side chain amino group of the first branching moiety - the rightmost K in the formula, a $(51\text{-hS})_2$KS moiety, as defined herein, is attached. This reasoning and notation is also valid for other branching moieties, may also be used if the branching moiety is another residue, for example diAA, or Orn. As known to the skilled person, while Orn has a defined main chain amino group and side chain amino group, for diAA moieties as defined herein, there is no difference. Should similar notation be used for other possible branching moieties, it is understood further definitions will be introduced.

The total number of peptide moieties comprised in the molecule according to the invention is preferably 2, 3 or 4.

The following peptide identifiers are used for the peptide moieties:

| | |
|---|---|
| 51: | RYFvRIOYR (SEQ ID NO:37); |
| 21: | RRFvYIYRR (SEQ ID NO:38); |
| 28: | RYFvRIYRR (SEQ ID NO:39); |
| 51v1: | RYFvrIOYR (SEQ ID NO:40); |
| 51v2: | RYFvRiOYR (SEQ ID NO:41); |
| 51v3: | RYFvriOYR (SEQ ID NO:42); |
| 5: | RRFvYIYKR (SEQ ID NO:43); |
| 6: | RRFqYIYKR (SEQ ID NO:44); |
| 17: | RYFvRIYKR (SEQ ID NO:45); |
| 19: | RYFvRIYRK (SEQ ID NO:46); |
| 30: | RRFaYIYKR (SEQ ID NO:47); |
| 32: | RRFpYIYKR (SEQ ID NO:48); |
| 41: | RYFvRIKYR (SEQ ID NO:49); |
| 42: | RYFqRIKYR (SEQ ID NO:50); |
| 50: | YFvRIOYR (SEQ ID NO:51); |
| 52: | AgbYFvAgbIKYAgb (SEQ ID NO:52); |

(continued)

| | |
|---|---|
| 58: | YFvRIKYR (SEQ ID NO:53); |
| 61: | rYFvRIKYR (SEQ ID NO:54); |
| 62: | RYFvrIKYR (SEQ ID NO:55); |
| 2210: | rrFvYIYRR (SEQ ID NO:56). |

In a particular embodiment, the invention relates to the compound according to the invention further conjugated to a payload.

The invention may comprise the compounds according to the invention and may further comprise a payload. The (payload)-(targeting moiety) construct may also be referenced as a payload-conjugate of the invention throughout the disclosure. The payload may be chemically conjugated to the compounds of the invention directly and/or may be linked thereto through a linker group.

As used throughout the disclosure, direct conjugation indicates the conjugation of the payload moiety to any amino acid residue within the compound of the invention or to any suitable chemical group therein, using any chemical coupling known in the art or described herein suitable for the conjugation of the payload moiety to an amino acid residue (*e.g.*, an amino acid side chain) and/or chemical group. Accordingly, direct coupling may result in one or more chemical groups spaced between the payload moiety and the amino acid (*e.g.*, amino acid side chain) or chemical group of the compound of the invention, which groups form as a result of the coupling reaction as is known in the art.

Alternatively, as described herein, the payload moiety may be conjugated to any amino acid residue or chemical group within the compound of the invention indirectly, that is, via a linker group. Therefore, as used throughout this disclosure, indirect conjugation means that the payload is conjugated to a linker group, which linker group is conjugated to an amino acid residue or chemical group within the compound of the invention. The conjugation between the payload and the linker group and between the linker group and an amino acid residue or chemical group of the compound of the invention may be any conjugation method and/or compound suitable for effecting such conjugation as described herein or as is otherwise known in the art.

The direct or indirect conjugation of the payload moiety may be directed to any amino acid residue or chemical linker/spacer group within compounds of the invention. Thus, the payload moiety may be directly or indirectly conjugated to an amino acid residue that is at the an N-terminal end of the compound of the invention. Alternatively or additionally, the payload moiety may be directly or indirectly conjugated to an internal amino acid residue or chemical linker/spacer group within compounds of the invention. As used throughout this disclosure, an internal residue or internal chemical group references an amino acid residue or chemical group of the compound of the invention that is not at the terminus of a peptide chain. As is known in the art, conjugation methods (whether direct or indirect) may require the chemical modification of one or both sites of conjugation (*e.g.*, modification of an amino acid residue, modification of a chemical group within the molecule of the invention and/or modification of the payload moiety). Accordingly, the present invention also encompasses chemical modification of the compounds of the invention.

In a particular embodiment, the invention related to a payload conjugate according to the invention, wherein a payload is conjugated to the reactive moiety Z.

As mentioned above, the payload may be conjugated to any amino acid residue or chemical group comprised in the compound of the invention. However, it is preferred within the present invention that the payload is conjugated to the reactive moiety Z comprised in the compound of the invention. Reactive moiety Z may be any moiety that can be coupled to the chemical spacer X or the branching moiety $B_1$ and at the same time comprises a reactive group that allows for direct or indirect conjugation to a payload.

In certain embodiments, the reactive moiety Z comprises an amino group with which the reactive moiety Z is coupled to a carboxyl group comprised in the chemical spacer X or the branching moiety $B_1$. In certain embodiments, the reactive moiety Z is an amino acid or an amino acid derivative. However, it is to be understood that the reactive moiety Z does not necessarily have to be an amino acid or an amino acid derivative. In particular, the reactive moiety Z does not necessarily have to comprise a carboxyl group.

In embodiments where the reactive moiety Z is an amino acid or an amino acid derivative, the carboxyl group may be free or may be modified in any way known in the art. In certain embodiments, the carboxyl group may be amidated. In certain embodiments, the reactive group for the conjugation of the payload or the linker may be comprised in the amino acid side chain. In certain embodiments, the reactive group for the conjugation of the payload or the linker may be the free carboxyl group of the amino acid. In certain embodiments, the reactive group for the conjugation of the payload or the linker may be the modified carboxyl group of the amino acid.

In one embodiment of the invention, the reactive moiety Z is an amino acid selected from the group consisting of: L-cysteine, L-C-propargylglycine, L-biocytin, and glycine-sulfamylbutyryl ($NH_2CH_2CONHSO_2(CH_2)_3COOH$), or wherein the reactive moiety Z is L-cysteamine or 4-aminobutanethiol.

That is, in certain embodiments, the reactive moiety Z is L-cysteine. L-cysteine comprises a thiol group in its amino acid side chain which can serve as a reactive group for the conjugation of a linker or a payload to the compound of the invention. Without limitation, L-cysteine may be conjugated to thiol-containing linkers or payloads through the formation of a disulfide bond. Alternatively, L-cysteine may be conjugated to linkers or payloads comprising a vinyl group in a thiol-ene reaction to conjugate the compound of the invention to the linker or payload via a thioether bond. Further, L-cysteine may be conjugated to linkers or payloads comprising a maleimide group in a thiol-maleimide click reaction to conjugate the compound of the invention to the linker or payload via a carbon-sulfur bond.

It is to be understood that the reactive moiety Z may be any molecule comprising a thiol group and a chemical group that allows coupling of the reactive moiety Z to the chemical spacer X or the branching moiety $B_1$, in particular wherein the chemical group that allows coupling the reactive moiety Z to the chemical spacer X or the branching moiety $B_1$ is an amine group. That is, in certain embodiments, the reactive moiety Z may be cysteamine or 4-aminobutane-1-thiol. In certain embodiments, the reactive moiety Z may be any amino acid or amino acid derivative comprising a thiol group.

In certain embodiments, the reactive moiety Z is L-C-propargylglycine or any other amino acid or amino acid derivative comprising a terminal or internal alkyne. Several conjugation reactions, in particular click chemistry cycloaddition reactions, are known in the art that take place at terminal or internal alkynes. For example, amino acids or amino acid derivatives comprising a terminal alkyne, such as L-C-propargylglycine, may be conjugated to linkers or payloads comprising a terminal azide in a cycloaddition reaction, such as Huisgen's 1,3-dipolar cycloaddition or Copper-catalyzed azide-alkyne cycloaddition. Further, amino acids or amino acid derivatives comprising an internal alkyne, for example an amino acid derivative comprising DBCO or a derivative thereof, may be conjugated to linkers or payloads comprising a terminal azide in a strain-promoted azide-alkyne cycloaddition. Alternatively, the reactive moiety Z may comprise a terminal azide and may be conjugated to linkers or payloads comprising a terminal or internal alkyne.

Thus, it is to be understood that the reactive moiety Z may be any molecule comprising an internal or terminal alkyne and a chemical group that allows coupling of the reactive moiety Z to the chemical spacer X or the branching moiety $B_1$, in particular wherein the chemical group that allows coupling the reactive moiety Z to the chemical spacer X or the branching moiety $B_1$ is an amine group. In certain embodiments, the reactive moiety Z may be any amino acid or any amino acid derivative comprising an internal or terminal alkyne.

Alternatively, the reactive moiety Z may be any molecule comprising a terminal azide and a chemical group that allows coupling of the reactive moiety Z to the chemical spacer X or the branching moiety $B_1$, in particular wherein the chemical group that allows coupling the reactive moiety Z to the chemical spacer X or the branching moiety $B_1$ is an amine group. In certain embodiments, the reactive moiety Z may be any amino acid or any amino acid derivative comprising a terminal azide.

In certain embodiments, the reactive moiety Z is L-biocytin. L-biocytin, also referred to as $N^{\varepsilon}$-(+)-Biotinyl-L-lysin or biotinyllysin, is an amide formed from the vitamin biotin and the amino acid lysine. The biotin moiety of biocytin may serve as a reactive group for the non-covalent conjugation of a linker or a payload to the compound of the invention. Biotin is known to form strong non-covalent bonds with proteins such as streptavidin, avidin or neutravidin. Thus, compounds of the invention comprising L-biocytin as the reactive moiety Z may be conjugated to linkers or payloads comprising an avidin, streptavidin or neutravidin moiety through the formation of a non-covalent bond between the biotin moiety of L-biocytin and the avidin, streptavidin or neutravidin moiety of the linker or payload.

It is to be understood that the reactive moiety Z may be any molecule comprising a biotin moiety and a chemical group that allows coupling of the reactive moiety Z to the chemical spacer X or the branching moiety $B_1$, in particular wherein the chemical group that allows coupling the reactive moiety Z to the chemical spacer X or the branching moiety $B_1$ is an amine group. In certain embodiments, the reactive moiety Z may be any amino acid or any amino acid derivative comprising a biotin moiety.

In certain embodiments, the invention relates to the payload conjugate according to the invention, wherein the payload is directly conjugated to the reactive moiety Z or wherein the payload is conjugated to the reactive moiety Z via a linker.

The invention encompasses payload conjugates that do not comprise a linker between the payload and any amino acid residue or chemical group within the compound of the invention. Where the payload-conjugate of the invention is lacking the linker, the payload may be conjugated, e.g., chemically conjugated, directly to the reactive moiety Z of the compound of the invention. Non-limiting examples of such chemical conjugation include covalent attachment to the C-terminus of the reactive moiety Z via an ester bond. Where the payload is a peptide or polypeptide, the payload may be directly conjugated to the C-terminus of the reactive moiety Z via a peptide bond. However, it is to be noted that the payload may be conjugated to any suitable reactive group comprised in the reactive moiety Z, preferably the reactive groups disclosed above. That is, without limitation, the payload may comprise a terminal or internal alkene or alkyne, a thiol, a maleimide moiety, a biotin moiety, a streptavidin moiety, an avidin moiety or a neutravidin moiety.

Where a linker is present and/or a linker is used in the indirect linkage of the payload to an amino acid residue or to a chemical group in the reactive moiety Z, such linker may be any linker, *e.g.,* a peptide linker, known in the art or

disclosed herein suitable for linking the payload to the reactive moiety Z comprised in the compound of the invention. The payload may be chemically conjugated to the linker, or, for example, where both the linker, and the payload are peptides or polypeptides, the payload may be linked to the linker via a peptide bond and the linker may also be linked to the reactive moiety Z of the compound of the invention via a peptide bond. Non-limiting examples of linkers include peptide linkers, *e.g.,* comprising one or more residues of glutamic acid, glycine, serine, cysteine and combinations thereof. In certain embodiments, the linker may be a single amino acid that is L- or D-glutamic acid.

It is to be understood that the linker may be any linker known in the art that allows conjugation of a payload to the reactive moiety Z comprised in the compound of the invention. That is, the linker preferably comprises two reactive groups, wherein a first reactive group is suitable for forming a covalent or non-covalent bond with a compatible reactive group in the reactive moiety Z and wherein a second reactive group is suitable for forming a covalent or non-covalent bond with a compatible reactive group in the payload.

In certain embodiments, the linker comprises at least one of the reactive groups selected from the group consisting of:

- maleimide
- 3,4-diphenylthio-maleimide
- 4,5-bis(2-pyridinyl-sulfanyl)-1,2- dihydropyridazine-3,6-dione
- DBCO
- DIBO
- HIPS
- 2-(4-hydroxyphenyl)-5-(methylsulfonyl)-1,3,4-oxadiazole
- APN
- Succinimide
- Succinimidylcarbamates
- Bromoacetamide
- Iodoacetamide

In certain embodiments, the linker may be or comprise biotin-maleimide (N-Biotinoyl-N'-(6-maleimidohexanoyl)hydrazide).

In a particular embodiment, the invention relates to the payload conjugate according to the invention, wherein the linker comprises a PEG moiety, a moiety derived from cadaverine or an alkyl moiety.

In certain embodiments, the linker comprises at least one PEG moiety. The term "PEG" as used herein refers to poly (ethylene glycol), also known as the polyether poly (ethylene oxide) (PEO) or polyoxyethylene (POE). PEG is prepared by polymerization of ethylene oxide and is commercially available over a wide range of molecular weights. The linker of the invention may comprise a PEG moiety of any molecular weight. However, it is to be understood that the term PEG moiety also encompasses a single ethylene glycol moiety. In certain embodiments, the linker may comprise or consist of a PEG moiety that is functionalized at one or both ends. That is, in certain embodiments, the PEG moiety may bind to the reactive group of the reactive moiety Z and/or to the payload via its terminal -OH groups. Alternatively, one or both -OH groups of the PEG moiety may be modified such that they comprise a functional group other than the -OH group. The PEG moiety may then be conjugated to the reactive moiety Z of the compound of the invention and/or to the payload via the functional group that has been introduced into the PEG moiety through the modification. The PEG moiety may be functionalized in any way that allows conjugation of the linker to the reactive moiety Z of the compound of the invention and/or to the payload. In certain embodiments, the PEG moiety may be functionalized with a functional group that is suitable for any of the conjugation reactions discloses herein. That is, the PEG moiety may be functionalized with a molecule comprising a terminal or internal alkene or alkyne, a thiol, a maleimide moiety, a biotin moiety, a streptavidin moiety, an avidin moiety or a neutravidin moiety.

Preferably, as used herein, PEGn, wherein n is an integer or a series of integers separated by slashes ("/") is understood to refer to a moiety according to formula - $NH(CH_2CH_2O),CH_2CO$-, wherein the integer "n" indicates a number of -$(CH_2CH_2O)$-repeating units, or wherein several integers n are separated by slashes, indicates alternate numbers of such repeating units. For example, PEG2/5 is understood to represent a moiety with either 2 or 5 repeating units

In certain embodiments, the PEG moiety may be functionalized with an azide group. In certain embodiments, the linker may comprise the motif $N_3$-$PEG_4$, wherein -$N_3$ is an azide moiety.

Throughout this disclosure, integers may be alternately and equivalently written as subscripts or not. For example, the structure $N_3$-$PEG_4$ may be alternately and equivalently indicated with the term N3-PEG4.

In certain embodiments, the linker may comprise or consist of cadaverine (1,5-diaminopentan). When the linker consists of cadaverine, the first -$NH_2$ group of cadaverine may be conjugated to a carboxyl group comprised in the reactive moiety Z of the compound of the invention. In embodiments where the reactive moiety Z is an amino acid, the first -$NH_2$ group of cadaverine may be bound to the C-terminus of the reactive moiety Z. Alternatively, the first -$NH_2$

group may be bound to any suitable functional group in an amino acid side chain of the reactive moiety Z. The second $-NH_2$ group of cadaverine may be directly conjugated to the payload via any functional group in the payload that is suitable for conjugation with the $-NH_2$ group of cadaverine. Alternatively, the linker may comprise or consist of a cadaverine derivative. That is, one or both $-NH_2$ groups of cadaverine may be modified such that they comprise a functional group that is suitable for conjugation to the reactive moiety Z and/or the payload. Compatible functional groups for the conjugation of the linker to the functional moiety Z and/or the payload have been disclosed herein. Without limitation, said functional groups comprise terminal or internal alkenes or alkynes, thiols, maleimide moieties, biotin moieties, streptavidin moieties, avidin moieties or neutravidin moieties.

In certain embodiments, the linker may comprise an alkyl moiety. The term "alkyl moiety" as used herein encompasses both straight chain alkyl chains and branched alkyl chains. Preferably, the alkyl moiety is an alkyl moiety having from one to fifteen carbon atoms. Also included are methyl, ethyl, propyl, isopropyl, butyl, isobutyl, tert-butyl and the like. Additionally, the alkyl moiety itself may be substituted with one or more substituents. It is to be understood that the alkyl moiety comprised in the linker has to be modified such that it comprises at least one functional group for conjugating the linker to the reactive moiety Z and one functional group for conjugating the linker to the payload. The alkyl moiety may be modified in any way known in the art. The skilled person is aware of methods of modifying an alkyl moiety such that it comprises a functional group that is compatible with a functional group in the reactive moiety Z of the compound of the invention and/or in the payload. In addition, alkyl derivatives are known in the art that comprise functional groups that are compatible with the functional group in the reactive moiety Z of the compound of the invention and/or in the payload. Without limitation, said functional groups comprise terminal or internal alkenes or alkynes, thiols, maleimide moieties, biotin moieties, streptavidin moieties, avidin moieties or neutravidin moieties.

In a particular embodiment, the invention relates to the payload conjugate according to the invention, wherein the linker is a cleavable or self-immolative linker.

In certain embodiments, it is preferred that the payload is released from the compound of the invention after the compound has been taken up by a target cell. To facilitate the release of the payload, the linker may be a cleavable or self-immolative linker.

The term "cleavable linker", as used herein, relates to a linker that is typically susceptible to cleavage under intracellular conditions. Suitable cleavable linkers include, for example, a peptide linker cleavable by an intracellular protease, such as lysosomal protease or an endosomal protease. In exemplary embodiments, the linker can be or comprise a dipeptide linker, such as a valine-citrulline (val-cit), a valine-alanine (val-ala), a phenylalanine-lysine (phe-lys) linker, or maleimidocapronic valine-citruline-p-aminobenzyloxycarbonyl (mc-Val-Cit-PABA) linker. Such linkers are e.g. disclosed in US Patent No. 6214345.

Another linker may comprise sulfosuccinimidyl-4-[N-maleimidomethyl]cyclohexane-1-carboxylate (SMCC). Sulfo-SMCC conjugation may occur via a maleimide group which reacts with sulfhydryls (thiols, -SH) (as found in cystein), while its Sulfo-NHS ester is reactive toward primary amines (as found in Lysine and the protein or peptide N-terminus). Yet another linker may comprise maleimidocaproyl (mc). Other suitable linkers comprise linkers hydrolyzable at a specific pH or a pH range, such as a hydrazone linker. Additional suitable cleavable linkers may include disulfide linkers. The linker may be covalently bound to the compound of the invention to such an extent that the compound of the invention must be degraded intracellularly in order for the drug to be released e.g. the mc linker and the like. Other pH sensitive cleavable linkers that comprise a cyclic benzylidene acetal linker are disclosed in EP3130587.

The term "self immolative linker", as used herein, relates to a linker which degrades spontaneously after an initial reaction has taken place. One example of a self-immolative linker is 4-aminobcnzyl alcohol (PAB), which is often conjugated to a cleavable dipeptide, like valine-citrulline (val-cit), or valine-alanine (val-ala), via the aromatic amine group and via a carbamate group to a primary or secondary amine of a payload. Cleavage of the dipeptide by a suitable protease triggers a 1,6-elimination of PAB and carbon dioxide in PAB and concomitant release of the free toxin. The 4-aminobenzyl moiety may also be coupled directly by alkylation to an amino-, hydroxy- or phenol-group of the payload. Cleavage of the dipeptide results then by 1, 6-elimination to fragmentation of the benzylamine or benzylether bond with concomitant release of the payload.

Other protease triggers for this PAB based self-immolative systems are exemplified but not limited to, by the glycine-proline-leucine-glycine (gly-pro-leu-gly) motive, cleavable by matrix metalloproteases (MMP II) or the alanine-alanine-proline-valine (ala-ala-pro-val) motive, cleavable by elastase.

Another example of self-immolative linker is represented by beta-glycosides of p-hydroxybenzl alcohol with glucuronic acid. Payloads may be conjugated to the benzylic carbon atom by the above-mentioned groups and a target binding moiety via the meta-position of the aromatic ring. Upon cleavage of the glyosidic bond by beta-glucuronidase the payload is liberated by an 1,6-elimination process.

According to one embodiment, the linker is cleavable by at least one agent selected from the group consisting of

- Cysteine protease
- Metallo protease

- Serine protease
- Threonine protease
- Aspartic protease

Cysteine proteases, also known as thiol proteases, are enzymes that degrade proteins. These proteases share a common catalytic mechanism that involves a nucleophilic cysteine thiol in a catalytic triad or dyad. Cysteine proteases are commonly encountered in fruits including the papaya, pineapple, fig and kiwifruit

Metalloproteases are protease enzymes whose catalytic mechanism involves a metal. Most metalloproteases require zinc, but some use cobalt. The metal ion is coordinated to the protein via three ligands. The ligands co-ordinating the metal ion can vary with histidine, glutamate, aspartate, lysine, and arginine. The fourth coordination position is taken up by a labile water molecule. Serine proteases are enzymes that cleave peptide bonds in proteins, in which serine serves as the nucleophilic amino acid at the (enzyme's) active site. They are found ubiquitously in both eukaryotes and prokaryotes. Serine proteases fall into two broad categories based on their structure: chymotrypsin-like (trypsin-like) or subtilisin-like.

Threonine proteases are a family of proteolytic enzymes harboring a threonine (Thr) residue within the active site. The prototype members of this class of enzymes are the catalytic subunits of the proteasome, however the acyltransferases convergently evolved the same active site geometry and mechanism.

Aspartic proteases are a catalytic type of protease enzymes that use an activated water molecule bound to one or more aspartate residues for catalysis of their peptide substrates. In general, they have two highly conserved aspartates in the active site and are optimally active at acidic pH. Nearly all known aspartyl proteases are inhibited by pepstatin.

In particular embodiments the linker is cleavable by at least one agent selected from the group consisting of

- Cathepsin A or B
- Matrix metalloproteinases (MMPs)
- Elastase
- Beta-glucuronidase
- Beta-galactosidase

According to one further embodiment, the linker comprises a motif selected from the group consisting of

- Val-Ala
- Val-Cit
- Val-Lys
- Val-Arg
- Phe-Lys-Gly-Pro-Leu-Gly
- Ala-Ala-Pro-Val
- Beta-glucuronide
- Beta-galactoside

Preferably, the payload is a molecule that can be used in the treatment and/or diagnosis of a medical condition in a subject. That is, in a particular embodiment, the invention relates to the compound according to the invention, wherein the payload is a biologically active molecule (BAM) or an imaging agent.

The compound of the invention has been demonstrated to specifically bind to proteoglycans on the surface of the cells. It is known in the art that the amount and the composition of proteoglycans on the cell surface varies between different cell types and may also vary between healthy and malignant cells. Further, it has been demonstrated by the inventors that, upon binding of the compound of the invention to proteoglycan on the surface of a cell, the compound may be internalized into said cell. Thus, the compound of the invention may be used to deliver payloads to a target cell or tissue or, in certain embodiments, inside a target cell.

In certain embodiments, the payload is a BAM. Without limitation, the compound of the invention may be used to deliver a BAM to a target cell or tissue or, more preferably, inside a target cell. Alternatively, the payload may be an imaging agent. Without limitation, the compound of the invention may be used to deliver the imaging agent to a target cell or tissue such that the target cell or tissue can be visualized by methods known in the art. Further, the compound of the invention may be used to deliver an imaging agent into a target cell such that the target cell can be visualized by methods known in the art.

In certain embodiments, a compound of the invention that specifically binds to C4S may be conjugated to a payload and administered to a subject such that the payload is delivered to cells and/or tissues that comprise C4S on the cell surface. In certain embodiments, the payload is delivered into a cell comprising C4S on the cell surface. In certain

embodiments, the payload is a BAM and the BAM is specifically delivered to a cell and/or tissue that comprises C4S on the cell surface or into a cell that comprises C4S on the cell surface. In certain embodiments, the payload is an imaging agent and the imaging agent is specifically delivered to a cell and/or tissue that comprises C4S on the cell surface or into a cell that comprises C4S on the cell surface.

In certain embodiments, a compound of the invention that specifically binds to C6S may be conjugated to a payload and administered to a subject such that the payload is delivered to cells and/or tissues that comprise C6S on the cell surface. In certain embodiments, the payload is delivered into a cell comprising C6S on the cell surface. In certain embodiments, the payload is a BAM and the BAM is specifically delivered to a cell and/or tissue that comprises C6S on the cell surface or into a cell that comprises C6S on the cell surface. In certain embodiments, the payload is an imaging agent and the imaging agent is specifically delivered to a cell and/or tissue that comprises C6S on the cell surface or into a cell that comprises C6S on the cell surface.

In certain embodiments, a compound of the invention that specifically binds to KS may be conjugated to a payload and administered to a subject such that the payload is delivered to cells and/or tissues that comprise KS on the cell surface. In certain embodiments, the payload is delivered into a cell comprising KS on the cell surface. In certain embodiments, the payload is a BAM and the BAM is specifically delivered to a cell and/or tissue that comprises KS on the cell surface or into a cell that comprises KS on the cell surface. In certain embodiments, the payload is an imaging agent and the imaging agent is specifically delivered to a cell and/or tissue that comprises KS on the cell surface or into a cell that comprises KS on the cell surface.

The skilled person is aware of cell types and tissues that are rich in specific proteoglycans and may thus be targeted with the compound of the invention. Further, the skilled person is aware of methods to determine the composition of proteoglycans on the surface of a cell. For example, and without limitation, mass spectrometry methods may be used for the determination of proteoglycans as described by Kubaski et al., Glycosaminoglycans detection methods: Applications of mass spectrometry, Mol Genet Metab, 2017, 120(1-2), p.67-77.

In a particular embodiment, the invention relates to the payload conjugate according to the invention, wherein the payload is a BAM, in particular wherein the BAM is a mono- or polysaccharide, a cytotoxic agent, an antineoplastic agent, an anti-inflammatory agent, an anti-viral agent, an anti-bacterial agent, an agent for the treatment of protozoan infections or a hormonal agent.

The invention encompasses any BAM expected to exert a therapeutically relevant activity on administration to an organism or on delivery to one or more cells of an organism, whether *in vitro* or *in vivo.* Accordingly, non-limiting examples of BAMs encompassed by the invention include mono- and polysaccharides, cytotoxic agents, antineoplastic agents, anti-inflammatory agents, anti-viral agents, anti-bacterial agents, and agents for the treatment of protozoan infections. The BAM may also be a deoxyribose or ribose.

Examples of anti-inflammatory agents that may be used as BAMs according to the methods of the invention include, but are not limited to, COX-2 inhibitors, prednisone, pazopanib, famotidine, dalfampridine, pegloticase, esomeprazole, aspirin, celecoxib, diclofenac, valdecoxib, rofecoxib, diflunisal, etodolac, fenoprofen, flurbiprofen, ibuprofen, indomethacin, ketoprofen, ketorolac, mefenamic acid, meloxicam, nabumetone, naproxen, oxaprozin, piroxicam, sulindac, tolmetin, lansoprazole, meclofenamate, triamcinolone, methylprednisolone, betamethasone, budesonide, prednisolone, hydrocortisone, dexamethasone and cortisone.

Examples of anti-protozoal agents that may be used as BAMs according to the methods of the invention include, but are not limited to, chloroquine, mefloquine, primaquine, proguanil hydrochloride, proguanil hydrochloride with atovaquone, pyrimethamine, sulfadoxine, quinine, quinoline, doxycycline, clindamycin, artesunate, diloxanide, metronidazole, tinidazole, mepacrine hydrochloride, amphotericin, pentamidine, pyrimethamine, sulfadiazine, azithromycin, atovaquone, trimethoprim-sulphamethoxazole, trimethoprim, dapsone, atovaquone, pentamidine isetionate, amodiaquine, chloroguanide, eflornithine, hydroxychloroquine, iodoquinol, meglumine antimonate, melarsoprol, nifurtimox, paromomycin, sodium stibogluconate, suramin, and tryparsamide.

Examples of hormonal agents that may be used as BAMs according to the methods of the invention include, but are not limited to, afimoxifene, arzoxifene, bazedoxifene, clomifene, fulvestrant, femarelle, lasofoxifene, ospemifene, ormeloxifene, tamoxifen, toremifene, raloxifene, dienogest, mifepristone and ulipristal acetate.

In a particular embodiment, the invention relates to the compound according to the invention, wherein the BAM is a cytotoxic agent or an antineoplastic agent. Preferably the BAM of the "BAM-conjugate" of the invention is a pharmaceutical drug, *e.g.,* selected from cytotoxic or antineoplastic drugs which are suitable as chemotherapy drugs. In general, chemotherapy drugs can be divided into three main categories based on their mechanism of action. They may

(a) stop the synthesis of preDNA molecule building blocks. These agents work in a number of different ways. DNA building blocks are folic acid, heterocyclic bases, and nucleotides, which are made naturally within cells. All of these agents work to block some step in the formation of nucleotides or deoxyribonucleotides (necessary for making DNA). When these steps are blocked, the nucleotides, which are the building blocks of DNA and RNA, cannot be synthesized. Thus the cells cannot replicate because they cannot make DNA without the nucleotides. Examples of drugs

in this class include methotrexate (Abitrexate®), fluorouracil (Adrucil®), hydroxyurea (Hydrea®), and mercaptopurine (Purinethol®), thioguanine, tocoferol, or, more generally, any nucleotide analogue, *e.g.,* 2'-deoxycytidine analogues;

(b) directly damage the DNA in the nucleus of the cell. These agents chemically damage DNA and RNA. They disrupt replication of the DNA and either totally halt replication or cause the manufacture of nonsense DNA or RNA (i.e., the new DNA or RNA does not code for anything useful). Examples of drugs in this class include cisplatin (Platinol®) and antibiotics - daunorubicin (Cerubidine®), doxorubicin (Adriamycin®) belonging to the class of anthracycline antitumor agents and etoposide (VePesid®) or any intercalator; further included are radionuclides (*e.g.,* alpha-radionucleids) commonly used in target cancer treatment (*e.g.,* Bismuth-213); or

(c) effect the synthesis or breakdown of the mitotic spindles. Mitotic spindles serve as molecular railroads with "North and South Poles" in the cell when a cell starts to divide itself into two new cells. These spindles are very important because they help to split the newly copied DNA such that a copy goes to each of the two new cells during cell division. These drugs disrupt the formation of these spindles and therefore interrupt cell division. Examples of drugs in this class of mitotic disrupters include: Vinblastine (Velban®), Vincristine (Oncovin®) and Paclitaxel (Taxol®).

The BAM of the "BAM-conjugate" of the invention may act according to one of the above modes of action. In other terms, each of the classes of anti-tumor drugs, i.e., alkylating agents, nitrosoureas, antimetabolites, plant alkaloids, antitumor antibiotics, and steroid hormones may be used as component the BAM of the inventive payload conjugate molecule. To describe these drug classes in more detail it is emphasized that each anti-cancer drug may also be categorized according to its effect on the cell cycle and cell chemistry as disclosed above. Alkylating agents kill cells by directly attacking DNA.

Alkylating agents may be used, in particular, in the treatment of chronic leukemias, Hodgkin's disease, lymphomas, and certain carcinomas of the lung, breast, prostate and ovary. Cyclophosphamide is an example of a commonly used alkylating agent. Nitrosoureas act similarly to akylating agents and also inhibit changes necessary for DNA repair. These agents cross the blood-brain barrier and are therefore used to treat brain tumors, lymphomas, multiple myeloma, and malignant melanoma. Carmustine and lomustine are the major drugs in this category. Antimetabolites are drugs that block cell growth by interfering with certain activities, usually DNA synthesis. Once ingested into the cell they halt normal development and reproduction. All drugs in this category affect the cell during the "S" phase of the cell cycle. Antimetabolites may be used in the treatment of acute and chronic leukemias, choriocarcinoma, and some tumors of the gastrointestinal tract, breast and ovary. Nonlimiting examples of commonly used antimetabolites are 6-mercaptopurine and 5-fluorouracil (5FU). Antitumor antibiotics are a diverse group of compounds. In general, they act by binding with DNA and preventing RNA synthesis. These agents are widely used in the treatment of a variety of cancers. The most commonly used drugs in this group are doxorubicin (Adriamycin), mitomycin-C, and bleomycin. Plant (vinca) alkaloids are anti-tumor agents derived from plants. These drugs act specifically by blocking cell division during mitosis. They are commonly used in the treatment of acute lymphoblastic leukemia, Hodgkin's and non-Hodgkin's lymphomas, neuroblastomas, Wilms' tumor, and cancers of the lung, breast and testes. Vincristine and vinblastine are commonly used agents in this group. Steroid hormones are useful in treating some types of tumors. This class includes adrenocorticosteroids, estrogens, antiestrogens, progesterones, and androgens. Although their specific mechanism of action is not clear, steroid hormones modify the growth of certain hormone-dependent cancers. Tamoxifen is an example, which is used for estrogen dependent breast cancer. All of the above-mentioned tumor species may be treated by the inventive BAM-conjugate molecules comprising as the BAM any of the above antitumor agents.

One group of cytotoxic or anti-tumor drugs, which may be used as the BAM component of the "BAM-conjugate" of the invention is selected from alkylating drugs, antimetabolica, cytostatics or drugs related to hormone treatment. In this context, it is preferred to select as cytotoxic or anti-tumor drugs compounds of metal, in particular platin (derivative) and taxol classes. In particular, the drug moiety is selected from the group of drugs consisting of, for example, cisplatin, transplatin, satraplatin, oxaliplatin, carboplatin, nedaplatin, chlorambucil, cyclophosphamide, mephalan, azath ioprin, fluorouracil, (6)-mercaptopurine, methrexate, nandrolone, aminogluthemide, medroxyprogesteron, megestrolacetate, procarbazin, docetaxel, paclitaxel, irinotecan, epipodophyllotoxin, podophyllotoxin, vincristine, vinblastine, docetaxel, daunomycin, daunorubicin, doxorubicin, mitoxantrone, topotecan, bleomycin, gemcitabine, fludarabine, navelbine and 5-FUDR. Particularly preferred is the class of metal-containing anticancer drugs, *e.g.,* the class of platinum compounds.

Further cytotoxic or anti-tumor drugs, which may be used as the BAM component of the "BAM-conjugate" of the invention are Alitretinoin, Altretamine, Azathioprine, Bicalutamide, Busulfan, Bortezomib, Capecitabine, Carfilzomib, Cyclophosphamide, Exemestane, Letrozole, Finasteride, Fostamatinib, Gefitinib, Imatinib, Lenalidomide, Marizomib, Megestrol Acetate, Nilotinib, Triptorelin, Temozolomide, Mifepristone, Tretinoin, Tamoxifen, Teniposide, Peplomycin sulfate or the class of camptothecins.

Further cytotoxic or anti-tumor drugs that may be used as the BAM component of the "BAM-conjugate" of the invention are radionuclides (*e.g.,* alpha particle emitting radionucleids) such as those commonly used in target cancer

therapies well known in the art. Non-limiting examples of such radionucleids that may be of use according to the invention include Molybdenum-99, Technetium-99m, Bismuth-213, Chromium-51, Cobalt-60, Copper-64, Dysprosium-165, Erbium-169, Holmium-166, Iodine-125, Iodine-131, Iridium-192, Iron-59, Lutetium-177, Palladium-103, Phosphorus-32, Potassium-42, Rhenium-186, Rhenium-188, Samarium-153, Selenium-75, Sodium-24, Strontium-89, Xenon-133, Ytterbium-169, Ytterbium-177, Yttrium-90, Radioisotopes of caesium, gold and ruthenium. Also included are Cyclotron Radioisotopes such as Carbon-11, Nitrogen-13, Oxygen-15, Fluorine-18, Cobalt-57, Gallium-67, Indium-111, Iodine-123, Krypton-81 m, Rubidium-82, Strontium-92, Thallium-201. Alternatively or additionally, any of the above non-limiting examples may also be implemented for diagnostic or imaging uses.

Another group of cytotoxic or anti-tumor drugs, which may be used as the BAM component of the "BAM-conjugate" of the invention are indolocarbazole compounds, *e.g,* staurosporin (and its analogues) and rebeccamycin. It is to be mentioned that compounds belonging to the class of anilinoquinazolines (*e.g.,* gefitinib) are also particularly preferred as the BAM component of the BAM-conjugates of the invention.

A further group of cytotoxic or anti-tumor drugs, which may be used as the BAM component of the "BAM-conjugate" of the invention may be selected from inhibitors of topoisomerases, such as irinotecan, or mitotic kinesins or DHFR.

Additionally, cytotoxic or anti-tumor drugs, which may be used as the BAM component of the "BAM-conjugate" of the invention can be selected from factors inhibiting or stimulating cell proliferation (PDGF), intracellular pathways, *e.g.,* the RAS/RAF signaling pathway, such as a member of the RAF/MEK/ERK signaling pathway (*e.g.,* RAF-1) or mitogen-activated protein kinase pathway, CMGC kinase family (containing CDK (cyclin dependent-kinases), MAPK, GSK3, CLK), Ser/Thr kinases that belong to the AGC kinase family containing PKA, PKG, PKC kinase families, receptor tyrosine kinases involved, e.g., in neovascularization and tumor progression, including vascular endothelial growth factor receptor (VEGFR)-2, VEGFR-3, platelet-derived growth factor receptor $\beta$, Flt-3, the endothelin (ET) system, that includes ET-1, ET-2, ET-3, and the $ET_A$ receptor ($ET_{AR}$) and $ET_{BR}$, and c-KIT, which are targeted by, e.g., inhibiting their function, and members of the IGF-family, such as IGF-1, IGF-2, IGF-1 R, IGF2R, etc.

Another group of cytotoxic or anti-tumor drugs, which may be used as the BAM component of the "BAM-transporter conjugate" of the invention may be selected from inhibitors that target tumor cell proliferation and tumor angiogenesis. Particularly preferred in this context are small molecule antitumor kinase inhibitors directed toward targets on malignant cells and/or vascular cells have antiangiogenic activity. Kinase inhibitors such as those directed toward EGFR, Her2/neu, BCR-ABL, c-KIT, PKC, Raf and PI3, are antiangiogenic by virtue of blocking secretion of angiogenic factors by affected malignant cells. Kinase inhibitors such as those directed toward VEGFR2, VEGFR1, PDGFR, PKC, Raf and PI3, are antiangiogenic by effects on vascular cells. Examples of synthetic inhibitors of cyclin dependent kinases (CDKIs) are, *e.g.,* olomoucine, flavopiridol, butyrolactone and their derivatives and thus constrain tumor cell proliferation. On the other hand, antitumor compounds suitable as the BAM component of the inventive peptide-conjugate/BAM-conjugate molecule may be selected from activators of apoptosis programs in cancer cells (*e.g.,* staurosporine) or by down-regulating antiapoptotic proteins, *e.g.,* Bcl-2.

Non-limiting examples of antineoplastic agents that may be used as BAMs according to the methods of the invention include, but are not limited to, aldesleukin, alemtuzumab, alitretinoin, allopurinol, altretamine, amifostine, anastrozole, abiraterone, arsenic, axitinib, azacitidine, bendamustine, bexarotene, bleomycin, bortezomib, busulfan, cabazitaxel, calusterone, capecitabine, carboplatin, carfilzomib, carmustine, carmustine, celecoxib, chlorambucil, cisplatin, cladribine, clofarabine, crizotinib, cyclophosphamide, cytarabine, dacarbazine, dactinomycin, actinomycin D, dasatinib, daunorubicin, decitabine, dexrazoxane, docetaxel, doxorubicin, entinostat, epirubicin, eribulin, erlotinib, estramustine, etoposide, everolimus, exemestane, fostamatinib, floxuridine, fludarabine, fluorouracil, 5-FU, fulvestrant, gefitinib, gemcitabine, hydroxyurea, idarubicin, lenalidomide, ifosfamide, imatinib, iomustine, irinotecan, isotretinoin, ixabepilone, lapatinib, lenalidomide, letrozole, leucovorin, levamisole, lomustine, CCNU, marizomib, meclorethamine, nitrogen mustard, melphalan, L-PAM, mercaptopurine, 6-MP, mertansine, mesna, methotrexate, methoxsalen, mitomycin, mitotane, mitoxantrone, nandrolone, nelarabine, nilotinib, oxaliplatin, paclitaxel, pamidronate, pazopanib, pegademase, pemetrexed, pentostatin, pipobroman, plerixafor, plicamycin, mithramycin, porfimer, pralatrexate, procarbazine, quinacrine, rapamycin, romidepsin, ruxolitinib, sorafenib, streptozocin, sunitinib, tamoxifen, temozolomide, temsirolimus, teniposide, VM-26, testolactone, thalidomide, thioguanine, 6-TG, thiotepa, topotecan, toremifene, tretinoin, ATRA, uracil mustard, valrubicin, vandetanib, vemurafenib, verteporfin, vinblastine, vincristine, vinorelbine, vismodegib, vorinostat, zoledronate, nucleoside analogues AZT, b-D-arabinofuranose, vidarabine, 2-chlorodeoxyadenosine, intercalating drugs, kinase inhibitors, cofarabine, laromustine, clophosphamide, asparaginase, dexamethasone, prednisone and lestaurtinib. The above-listed antineoplastic agents may be used in accordance with the methods disclosed herein not only in connection with neoplastic diseases, but also in the treatment, prevention and/or amelioration of other diseases, or symptoms thereof, as is known in the art, *e.g.,* in connection with the treatment, prevention and/or amelioration of anti-inflamatory or autoimmune diseases, and/or symptoms thereof.

In a particular embodiment, the invention relates to the compound according to the invention, wherein the cytotoxic or antineoplastic agent is an azonafide.

In certain embodiments, the antineoplastic agent may be any member of the azonafide family. Azonafide (2-[2'-(dimethylamino)ethyl]-1,2-dihydro-3H-dibenz[de, h]isoquinoline-1,3-dione) is the parent of a series of anthracene-containing antitumor agents. The azonafides are a series of anthracene-based DNA intercalators which inhibit tumor cell growth *in vitro* at low nanomolar concentrations and are not affected by the multidrug resistance phenomenon (MDR). The compound according to the invention may be any azonafide known in the art. The skilled person is aware of methods to conjugate azonafides to the reactive moiety Z of the compound of the invention or to a linker molecule. It is to be understood that chemical modification of the azonafide, the linker and/or the reactive moiety Z may be required to conjugate the azonafide to the compound of the invention.

In a particular embodiment, the azonafide may be any one of the compounds disclosed in Sami et al., J. Med. Chem. 1996, 39(8), p.1609-1618; Sami et al., J. Med. Chem. 1996, 39(25), p.4978-4987; or Mayr et al., Anti-Cancer Drugs 1999, 10(2), p.163-170. More particularly, the azonafide may be any one of the azonafide shown in Fig.15. That is, in one embodiment, the azanofide may be any one of compounds 35, 38a, 38b or 46 as shown in Fig.15. In a particular embodiment, the azonafide may be compound 38a (herein also referred to as zonafide 38a) as shown in Fig.15.

In certain embodiments, the azonafide may be conjugated to the compound of the invention via a linker. In certain embodiments, azonafide may be conjugated to the linker via a free -OH group. In certain embodiments, said free OH-group may be conjugated to the linker via an ester bond. In certain embodiments, the azonafide comprising a free -OH group may be conjugated to a linker comprising an NHS ester. In certain embodiments, the linker may comprise the motif $N_3$-$PEG_4$. In certain embodiments, the linker may be $N_3$-$PEG_4$-NHS.

However, it has to be noted that the skilled person is aware of numerous other methods to conjugate an azonafide to the compound of the invention, either directly to the reactive moiety Z or indirectly via a linker.

In a particular embodiment, the invention relates to the compound according to the invention, wherein the cytotoxic or antineoplastic agent is "p53 Upregulated Modulator of Apoptosis" (PUMA).

In certain embodiments, the antineoplastic agent may be a polypeptide. In particular, the polypeptide may be a protein, e.g. the protein "p53 Upregulated Modulator of Apoptosis" (PUMA). PUMA is a pro-apoptotic protein, member of the Bcl-2 protein family. High activity of PUMA is known to be toxic to cancer cells and various drugs are currently in development that aim at inducing PUMA activity in cancer cells. With the compound of the invention, it may be possible to deliver PUMA specifically to cancer cells and induce apoptosis in these cells. The skilled person is aware of methods to produce PUMA and to conjugate it to the compound of the invention either directly or via a linker. The sequences of the known isoforms of human PUMA are set forth in SEQ ID NO:88 to 91). In certain embodiments, the antineoplastic agent is human PUMA as set forth in SEQ ID NO:88, 89, 90 or 91. Preferably, the antineoplastic agent is human PUMA as set forth in SEQ ID NO: 89 or 91.

In a particular embodiment, the invention relates to the compound according to the invention, wherein the cytotoxic or antineoplastic agent is SN-38.

In certain embodiments, the antineoplastic agent may be SN-38. SN-38 is the active metabolite of irinotecan (an analog of camptothecin - a topoisomerase I inhibitor) but has 1000 times more activity than irinotecan itself. *In vitro* cytotoxicity assays show that the potency of SN-38 relative to irinotecan varies from 2- to 2000-fold. The skilled person is aware of methods to conjugate SN-38 to the reactive moiety Z of the compound of the invention or to a linker molecule. It is to be understood that chemical modification of SN-38, the linker and/or the reactive moiety Z may be required to conjugate SN-38 to the compound of the invention.

In a particular embodiment, the invention relates to the compound according to the invention, wherein the cytotoxic or antineoplastic agent is 6-thioguanine.

In certain embodiments, the antineoplastic agent may be 6-thioguanine. 6-thioguanine is in the antimetabolite family of medications. It is a purine analogue of guanine and works by disrupting DNA and RNA. The skilled person is aware of methods to conjugate 6-thioguanine to the reactive moiety Z of the compound of the invention or to a linker molecule. It is to be understood that chemical modification of 6-thioguanine, the linker and/or the reactive moiety Z may be required to conjugate 6-thioguanine to the compound of the invention.

In certain embodiments, 6-thioguanine may be coupled directly to the reactive moiety Z, in particular via a disulfide bond. In certain embodiments, the reactive moiety Z is L-cysteine.

In a particular embodiment, the invention relates to the compound according to the invention, wherein the cytotoxic or antineoplastic agent is a pro-apoptotic peptide.

In certain embodiments, the antineoplastic agent may be a pro-apoptotic peptide. Pro-apoptotic peptides are peptides that induce intrinsic apoptosis pathways in cells, preferably in cancer cells. Various pro-apoptotic peptides are known in the art and are described, for example, in WO 2010/112471, WO 2003/083441 and WO 2015/001045. Further pro-apoptotic peptides that may be conjugated to the compound of the invention have been disclosed by Min et al.; Archives of Pharmacal Research, 2018, 41, p.594-616. The skilled person is aware of methods to conjugate pro-apoptotic peptides to the reactive moiety Z of the compound of the invention or to a linker molecule. It is to be understood that chemical modification of the pro-apoptotic peptide, the linker and/or the reactive moiety Z may be required to conjugate a pro-apoptotic peptide to the compound of the invention.

In a particular embodiment, the invention relates to the compound according to the invention, wherein the payload is an imaging agent.

In certain embodiments, the payload may be an imaging agent. The imaging agent may be any imaging agent known in the art. The compound of the invention, when conjugated to an imaging agent, may be used in the visualization of cells and/or tissues *in vitro* and/or *in vivo.* When a compound is used for the visualization of a target cell or tissue *in vitro,* care has to be taken an imaging agent is selected that is functional under the imaging conditions. Similar criteria apply for imaging agents that are used for the visualization of target cells and/or tissues *in vivo.* However, for *in vivo* applications, additional care has to be taken that the imaging agent is biocompatible, e.g. has no toxic or otherwise detrimental effects on the subject that compound of the invention comprising the imaging agent is administered to.

The term "imaging agent" as used herein refers to any element, molecule, functional group, compound, fragments thereof or moiety that facilitates detection of an agent (e.g., the compound of the invention) to which it is conjugated. Examples of imaging agents include, but are not limited to: various ligands, radionuclides, fluorescent dyes (for specific exemplary fluorescent dyes, see below), chemiluminescent agents (such as, for example, acridinum esters, stabilized dioxetanes, and the like), bioluminescent agents, spectrally resolvable inorganic fluorescent semiconductor nanocrystals (i.e., quantum dots), metal nanoparticles (e.g., gold, silver, copper, platinum, etc.) nanoclusters, paramagnetic metal ions, enzymes (for specific examples of enzymes, see below), colorimetric labels (such as, for example, dyes, colloidal gold, and the like), biotin, dioxigenin, haptens, and proteins for which antisera or monoclonal antibodies are available. It is to be understood that the imaging agent may be conjugated to the compound of the invention directly or indirectly via a linker, or that the imaging agent may be comprised in a molecule that is conjugated to the compound of the invention directly or indirectly via a linker. The skilled person would understand which imaging agents may be conjugated directly to the compound of the invention or a linker and which imaging agents need to be embedded in a molecule that can be conjugated to the compound of the invention or a linker. For example, if the imaging agent is a radionuclide, the radionuclide is preferably embedded in a molecule that can be conjugated to the reactive moiety Z of the compound of the invention or to the linker.

The term "image", as used herein, is understood to mean a visual display or any data representation that may be interpreted for visual display. For example, a three-dimensional image may include a dataset of values of a given quantity that varies in three spatial dimensions. A three-dimensional image (e.g., a three-dimensional data representation) may be displayed in two-dimensions (e.g., on a two-dimensional screen, or on a two-dimensional printout). In certain embodiments, the term "image" may refer to, for example, to a multidimensional image (e.g., a multi-dimensional (e.g., four dimensional) data representation) that is displayed in two-dimensions (e.g., on a two-dimensional screen, or on a two-dimensional printout). The term "image" may refer, for example, to an optical image, an x-ray image, an image generated by: positron emission tomography (PET), magnetic resonance, (MR) single photon emission computed tomography (SPECT), and/or ultrasound, and any combination of these.

In certain embodiments, the compound of the invention may be conjugated to one or more contrast/imaging agents (e.g., fluorescent dyes, (chelated) radioinuclides (SPECT, PET), MR-active agents, CT-agents).

In certain embodiments, a contrast/imaging agent may be conjugated to the compound of the invention for medical or biological imaging. The imaging techniques encompassed In certain embodiments may include positron emission tomography (PET), single photon emission computed tomography (SPECT), computerized tomography (CT), magnetic resonance imaging (MRI), optical bioluminescence imaging, optical fluorescence imaging, and combinations thereof. In certain embodiments, the contrast/imaging agent may be any molecule, substance or compound known in the art for PET, SPECT, CT, MRI, and optical imaging. The contrast agent may be radionuclides, radiometals, positron emitters, beta emitters, gamma emitters, alpha emitters, paramagnetic metal ions, and supraparamagnetic metal ions. The contrast agents include, but are not limited to, iodine, fluorine, Cu, Zr, Lu, At, Yt, Ga, In, Tc, Gd, Dy, Fe, Mn, Ba and $BaSO_4$.

It is to be understood that certain imaging, in particular radionuclides, are not bound directly to the compound of the invention or the linker but are comprised in a complexing or chelator which can be conjugated to the reactive moiety Z of the compound of the invention or the linker. Chelators that may be conjugated to the compound of the invention include, but are not limited to, 1,4,7,10-tetraazacyclododecane-1,4,7,10-tetraacetic acid (DOTA), diethylenetriaminepentaacetic (DTPA), desferrioxamine (DFO) and triethylenetetramine (TETA), 1,4,8,11-tetraazabicyclo[6.6.2]hexadecane-4,11-diacetic acid (CB-TE2A); ethylenediaminetetraacetic acid (EDTA); ethylene glycolbis(2-aminoethyl)-N,N,N',N'-tetraacetic acid (EGTA); 1,4,8,11-tetraazacyclotetradecane-1,4,8,11-tetraacetic acid (TETA); ethylenebis-(2-4 hydroxy-phenylglycine) (EHPG); 5-Cl-EHPG; 5BrEHPG; 5-Me-EHPG; 5t-Bu-EHPG; 5-sec-Bu-EHPG; benzodiethylenetriamine pentaacetic acid (benzo-DTPA); dibenzo-DTPA; phenyl-DTPA, diphenyl-DTPA; benzyl-DTPA; dibenzyl-DTPA; bis-2(hydroxybenzyl)-ethylene-diaminediacetic acid (HBED) and derivatives thereof; Ac-DOTA; benzo-DOTA; dibenzo-DOTA; 1,4,7-triazacyclononane N,N',N"-triacetic acid(NOTA); benzo-NOTA; benzo-TETA, benzo-DOTMA, where DOTMA is 1,4,7,10-tetraazacyclotetradecane-1,4,7,10-tetra(methyl tetraacetic acid), benzo-TETMA, where TETMA is 1,4,8, 11-tetraazacyclotetradecane-1,4,811-(methyl tetraacetic acid); derivatives of 1,3-propylenediaminetetraacetic acid (PDTA); triethylenetetraaminehexaacetic acid (TTHA); derivatives of 1,5,10-N,N',N"-tris(2,3-dihydroxybenzoyl)-tricatecholate (LICAM); and 1,3,5-N,N',N"-tris(2,3-dihydroxybenzoyl)aminomethylbenzene (MECAM), or other metal chelators. In certain embodiments, the payload may comprise more than one chelator.

In certain embodiments, the imaging agent may be a fluorescent reporter. In certain embodiments, the fluorescent reporter may be a near infrared or far red dye. In certain embodiments, the fluorescent reporter may be selected from the group consisting of a fluorophore, fluorochrome, dye, pigment, fluorescent transition metal, and fluorescent protein. In certain embodiments, the fluorescent reporter is selected from the group consisting of Cy5, Cy5.5, Cy2, FITC, TRITC, Cy7, FAM, Cy3, Cy3.5, Texas Red, ROX, HEX, JA133, AlexaFluor 488, AlexaFluor 546, AlexaFluor 633, AlexaFluor 555, AlexaFluor 647, DAPI, TMR, R6G, GFP, enhanced GFP, CFP, ECFP, YFP, Citrine, Venus, YPet, CyPet, AMCA, Spectrum Green, Spectrum Orange, Spectrum Aqua, Lissamine and Europium.

In certain embodiments, imaging is performed in normal lighting settings. In certain embodiments, imaging is performed with some to zero levels of ambient lighting settings.

The imaging methods herein may be used with a number of different fluorescent probe species (or, as in embodiments using a tandem bioluminescent reporter/fluorescent probe, the fluorescent species thereof), for example, (1) probes that become activated after target contact (e.g., binding or interaction) (Weissleder et al., Nature Biotech., 17:375-378, 1999; Bremer et al., Nature Med., 7:743-748, 2001; Campo et al., Photochem. Photobiol. 83:958-965, 2007); (2) wavelength shifting beacons (Tyagi et al., Nat. Biotechnol, 18:1191-1196, 2000); (3) multicolor (e.g., fluorescent) probes (Tyagi et al., Nat. Biotechnol., 16:49-53, 1998); (4) probes that have high binding affinity to targets, e.g., that remain within a target region while non specific probes are cleared from the body (Achilefu et al., Invest. Radiol, 35:479-485, 2000; Becker et al., Nature Biotech. 19:327-331, 2001; Bujai et al., J. Biomed. Opt. 6:122-133, 2001; Ballou et al. Biotechnol. Prog. 13:649-658, 1997; and Neri et al., Nature Biotech 15:1271-1275, 1997); (5) quantum dot or nanoparticle-based imaging probes, including multivalent imaging probes, and fluorescent quantum dots such as amine T2 MP EviTags ® (Evident Technologies) or Qdot® Nanocrystals (Invitrogen™); (6) non-specific imaging probes e.g., indocyanine green, AngioSense® (VisEn Medical); (7) labeled cells (e.g., such as cells labeled using exogenous fluorophores such as VivoTag™ 680, nanoparticles, or quantum dots, or by geneticallymanipulating cells to express fluorescent or luminescent proteins such as green or red fluorescent protein; and/or (8) X-ray, MR, ultrasound, PET or SPECT contrast agents such as gadolinium, metal oxide nanoparticles, X-ray contrast agents including iodine based imaging agents, or radioisotopic form of metals such as copper, gallium, indium, technetium, yttrium, and lutetium including, without limitation, $^{99m}Tc$, $^{111}In$, $^{64}Cu$, $^{67}Ga$, $^{186}Re$, $^{188}Re$, $^{153}Sm$, $^{177}Lu$, and $^{67}Cu$. The relevant text of the above-referenced documents are incorporated by reference herein.

Another group of suitable imaging agents are lanthanide metal-ligand probes. Fluorescent lanthanide metals include europium and terbium. Fluorescence properties of lanthanides are described in Lackowicz, 1999, Principles of Fluorescence Spectroscopy, 2nd Ed., Kluwar Academic, New York, the relevant text incorporated by reference herein.

Furthermore, imaging agents used in the embodiment of this invention can be conjugated to molecules capable of eliciting photodynamic therapy. These include, but are not limited to, Photofrin, Lutrin, Antrin, aminolevulinic acid, hypericin, benzoporphyrin derivative, and select porphyrins. In certain embodiments, two or more probe species are graphically distinguished, e.g., are displayed with different colors (e.g., green and red, e.g., green and blue), to separately represent the two lymphatic drainage pathways and/or nodes.

In certain embodiments, the representations of two or more probe species are superimposed on a graphical display, or the overlapping portion is represented with a different (e.g., a third) color (e.g., yellow). For example, for a lymphatic drainage pathway that both drains the extremity and leads to the tumor site, the pathway may contain both first and second probe species (corresponding, respectively, to a first and second color on the display), and the region of overlap on the display is assigned a new color different from the first and second color. The color may indicate that the associated node should not be removed, to avoid lymphedema.

In general, fluorescent quantum dots used in the practice of the elements of this invention are nanocrystals containing several atoms of a semiconductor material (including but not limited to those containing cadmium and selenium, sulfide, or tellurium; zinc sulfide, indium-antimony, lead selenide, gallium arsenide, and silica or ormosil), which have been coated with zinc sulfide to improve the properties of the fluorescent agents.

In certain embodiments, imaging agents have excitation and emission wavelengths in the red and near infrared spectrum, e.g., in the range 550-1300 or 400-1300 nm or from about 440 to about 1100 nm, from about 550 to about 800 nm, or from about 600 to about 900 nm.

Use of this portion of the electromagnetic spectrum maximizes tissue penetration and minimizes absorption by physiologically abundant absorbers such as hemoglobin (<650 nm) and water (>1200 nm). Imaging agents with excitation and emission wavelengths in other spectrums, such as the visible and ultraviolet light spectrum, can also be employed in the methods of the embodiments of the present invention. In particular, fluorophores such as certain carbocyanine or polymethine fluorescent fluorochromes or dyes can be used to construct optical imaging agents, e.g. U.S. Pat. No. 6,747,159 to Caputo et al. (2004); U.S. Pat. No. 6,448,008 to Caputo et al. (2002); U.S. Pat. No. 6,136,612 to Delia Ciana et al. (2000); U.S. Pat. No. 4,981,977 to Southwick, et al. (1991); 5,268,486 to Waggoner et al. (1993); U.S. Pat. No. 5,569,587 to Waggoner (1996); 5,569,766 to Waggoner et al. (1996); U.S. Pat. No. 5,486,616 to Waggoner et al.

(1996); U.S. Pat. No. 5,627,027 to Waggoner (1997); U.S. Pat. No. 5,808,044 to Brush, et al. (1998); U.S. Pat. No. 5,877,310 to Reddington, et al. (1999); U.S. Pat. No. 6,002,003 to Shen, et al. (1999); U.S. Pat. No. 6,004,536 to Leung et al. (1999); U.S. Pat. No. 6,008,373 to Waggoner, et al. (1999); U.S. Pat No. 6,043,025 to Minden, et al. (2000); U.S. Pat. No. 6,127,134 to Minden, et al. (2000); U.S. Pat. No. 6,130,094 to Waggoner, et al. (2000); U.S. Pat. No. 6,133,445 to Waggoner, et al. (2000); U.S. Pat. No. 7,445,767 to Licha, et al. (2008); U.S. Pat. No. 6,534,041 to Licha et al. (2003); U.S. Pat. No. 7,547,721 to Miwa et al. (2009); U.S. Pat. No. 7,488,468 to Miwa et al. (2009); U.S. Pat. No. 7,473,415 to Kawakami et al. (2003); also WO 96/17628, EP 0 796 I B I, EP 1 181 940 B1, EP 0 988 060 B1, WO 98/47538, WO 00/16810, EP 1 113 822 B1, WO 01/43781, EP 1 237 583 A1, WO 03/074091, EP 1 480 683 B1, WO 06/072580, EP 1 833 513 A1, EP 1 679 082 A1, WO 97/40104, WO 99/51702, WO 01/21624, and EP 1 065 250 A1; and Tetrahedron Letters 41, 9185-88 (2000).

Exemplary fluorochromes for imaging agents include, for example, the following: Cy5.5, Cy5, Cy7.5 and Cy7 (GE® Healthcare); AlexaFluor660, AlexaFluor680, AlexaFluor790, and AlexaFluor750 (Invitrogen); VivoTag™680, VivoTag™-S680, VivoTag ™-S750 (VISEN Medical); Dy677, Dy682, Dy752 and Dy780 (Dyomics ®); DyLight ® 547, and/or DyLight ® 647 (Pierce); HiLyte Fluor™ 647, HiLyte Fluor™ 680, and HiLyte Fluor™ 750 (AnaSpec ®); IRDye ® 800CW, IRDye ® 800RS, and IRDye ® 700DX (Li-Cor ®); ADS780WS, ADS830WS, and ADS832WS (American Dye Source); XenoLight CF™ 680, XenoLight CF™ 750, XenoLight CF™ 770, and XenoLight DiR (Caliper ® Life Sciences); and Kodak ® X-SIGHT ® 650, Kodak ® X-SIGHT 691, Kodak ® X-SIGHT 751 (Carestream ® Health).

Suitable means for imaging, detecting, recording or measuring the compound of the invention comprising an imaging agent may also include, for example, a flow cytometer, a laser scanning cytometer, a fluorescence micro-plate reader, a fluorescence microscope, a confocal microscope, a brightfield microscope, a high content scanning system, and like devices. More than one imaging techniques may be used at the same time or consecutively to detect the compound of the invention comprising an imaging agent. In one embodiment, optical imaging is used as a sensitive, high-throughput screening tool to acquire multiple time points in the same subject, permitting semi-quantitative evaluations of tumor marker levels. This offsets the relatively decreased temporal resolution obtained with PET, although PET is needed to achieve adequate depth penetration for acquiring volumetric data, and to detect, quantitate, and monitor changes in receptor and/or other cellular marker levels as a means of assessing disease progression or improvement, as well as stratifying patients to suitable treatment protocols.

The systems and methods described herein can be used with other imaging approaches such as the use of devices including but not limited to various scopes (microscopes, endoscopes), catheters and optical imaging equipment, for example computer based hardware for tomographic presentations.

In certain embodiments, the systems and methods can be used in the detection, characterization and/or determination of the localization of a disease, especially early disease, the severity of a disease or a disease-associated condition, the staging of a disease, and monitoring and guiding various therapeutic interventions, such as surgical procedures, and monitoring and/or development of drug therapy and delivery, including cell based therapies. In certain embodiments, the methods can also be used in prognosis of a disease or disease condition.

In a particular embodiment, the invention relates to a pharmaceutical composition comprising the payload conjugate according to the invention and a pharmaceutically acceptable carrier or excipient.

As detailed herein, the compounds of the invention comprise or consist of a payload-conjugate (*i.e.*, a BAM or an imaging agent conjugated to the compound of the invention either directly or indirectly via a linker) that may effect targeting of the payload to the a target cell or tissue. In certain embodiments, the payload-conjugate may also effect intracellular transport of the payload into a target cell. Accordingly, in preferred embodiments, the invention encompasses a pharmaceutical composition comprising a compound of the invention, *e.g.,* a payload-conjugate, and a pharmaceutically acceptable carrier or excipient.

Such a carrier or excipient includes, but is not limited to, saline, buffered saline, dextrose, water, glycerol, ethanol, and/or combinations thereof. The pharmaceutical compositions also may include additional therapeutic agents for the treatment of the given disease being treated. The formulation is made to suit the mode of administration. In general, methods of administering polypeptides are well known in the art and can be applied to administration of the conjugates of the invention.

Administration is by any of the routes normally used for introducing a compound into ultimate contact with blood. Suitable methods of administering such conjugates in the context of the present invention to a patient are available including oral and parenteral routes. Although more than one route can be used to administer a particular composition, a particular route can often provide a more immediate and more effective action or reaction than another route.

Preferably the payload-conjugates of the invention are administered by parenteral modes of administration, in particular by intravenous, intraperitoneal, intramuscular, intradermal, subcutaneous intrathecal, intraocular, retrobulbar, intrapulmonary or intraarticular means. Such administration routes and appropriate formulations are generally known to those of skill in the art. Formulations suitable for parenteral administration include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include

suspending agents, solbilizers, thickening agents, stabilizers, and preservatives. Carrier polypeptide-target polypeptide conjugates can also be administered via liposomes.

The molecules and compounds of the invention, alone or in combination with other suitable components, can also be made into aerosol formulations (*i.e.*, they can be "nebulized") to be administered via inhalation. Aerosol formulations can be placed into pressurized acceptable propellants, such as dichlorodifluoromethane, propane, nitrogen, and the like.

In a preferred embodiment, the pharmaceutical compositions of the invention are provided in lyophilized form to be reconstituted prior to administration. Buffers and solutions for the reconstitution of the pharmaceutical compositions may be provided along with the pharmaceutical formulation to produce aqueous compositions of the present invention for administration.

Pharmaceutically acceptable carriers are determined in part by the particular composition being administered, as well as by the particular method used to administer the composition. Accordingly, there are a wide variety of suitable formulations of pharmaceutical compositions of the present invention. Pharmaceutically acceptable carriers and excipients are well known in the art, and one or more conjugates of the invention can be formulated into pharmaceutical compositions by well-known methods (see, *e.g.,* Remington: The Science and Practice of Pharmacy, 21st edition, A. R. Gennaro, Ed., Mack Publishing Company (2005); Pharmaceutical Formulation Development of Peptides and Proteins, S. Frokiaer and L. Hovgaard, Eds., Taylor & Francis (2000); and Handbook of Pharmaceutical Excipients, 3rd edition, A. Kibbe, Ed., Pharmaceutical Press (2000)).

Pharmaceutical compositions comprising one or more payload-conjugates of the invention are optionally tested in one or more appropriate *in vitro* and/or *in vivo* animal models of disease, to confirm efficacy, tissue metabolism, and to estimate dosages, according to methods well known in the art. Thus, it is understood that the suitable dose of a composition according to the present invention will depend upon the age, health and weight of the recipient, kind of concurrent treatment, if any, frequency of treatment, and the nature of the effect desired. However, the dosage is tailored to the individual subject, as is determinable by one of skill in the art, without undue experimentation. The total dose of therapeutic agent may be administered in multiple doses or in a single dose. In certain embodiments, the compositions are administered alone, in other embodiments the compositions are administered in conjunction with other therapeutics directed to the disease or directed to other symptoms thereof.

The dose administered to a patient, in the context of the present invention, is sufficient to effect a beneficial therapeutic response in the patient over time, or, *e.g.,* to inhibit infection by a pathogen, to reduce or prevent the symptoms of a disease state, or other appropriate activity, depending on the application. The dose is determined by the efficacy of a particular composition/formulation, and the activity, stability or serum half-life of the BAM polypeptide conjugate employed and the condition of the patient, as well as the body weight or surface area of the patient to be treated. The size of the dose is also determined by the existence, nature, and extent of any adverse side-effects that accompany the administration of a particular composition/formulation, or the like in a particular patient.

In a particular embodiment, the invention relates to the payload conjugate according to the invention or the pharmaceutical composition according to the invention for use as a medicament.

That is, the payload-conjugate of the invention or a pharmaceutical composition comprising the payload conjugate of the invention may be used in the detection and/or treatment of medical conditions in a subject.

The compounds of the invention interact specifically with proteoglycans, in particular, chondroitin-4-sulfate (C4S), chondroitin-6-sulfate (C4S) and/or keratan sulfate (KS). Thus, it is to be understood that some compounds of the invention may be more suitable for the detection and/or treatment of a specific medical conditions than others. For example, in certain embodiments, compounds that specifically bind to C4S are preferably used in the detection and/or treatment of medical conditions that involve cells synthesizing detectable and/or elevated amounts of C4S. In other embodiments, compounds that specifically bind to C6S are preferably used in the detection and/or treatment of medical conditions that involve cells synthesizing detectable and/or elevated amounts of C6S. In further embodiments, compounds that specifically bind to KS are preferably used in the detection and/or treatment of medical conditions that involve cells synthesizing detectable and/or elevated amounts of KS.

In a particular embodiment, the invention relates to the payload conjugate according to the invention or the pharmaceutical composition according to the invention for use in the treatment of cancer.

The compounds of the invention are envisioned as transporter moieties that effect transport across the cell membrane and into the cytoplasm and/or nucleus of a cell. It is common to all of the therapeutic compounds (*i.e.*, BAMs) disclosed herein that they have to cross the cell membrane in order to act as anti-cancer drugs. By coupling compounds belonging to these classes (compounds directly damaging the DNA in the nucleus of the cell, effecting the synthesis or breakdown of the mitotic spindles or stopping the synthesis of pre-DNA molecule building blocks) to the compound of the invention, the entry of the anti-cancer compounds into the cell is enhanced and/or their solubility is enhanced, thereby increasing the efficacy of these therapeutic compounds. In turn, increased cell take-up and, preferably, better solubility of these compounds in the aqueous environment (*e.g.*, the cytosol) allows to lower the dosage of the therapeutic anti-cancer compound may be achieved.

It is to be understood that use of the compound of the invention in the treatment of cancer is only contemplated for cancers that are characterized by malignant cells comprising detectable and/or elevated amounts of proteoglycans, in particular C4S, C6S and/or KS, on their cell surface. Examples of cancers that are characterized by malignant cells comprising detectable and/or elevated amounts of proteoglycans are disclosed herein. Further, the skilled person is enabled by the disclosure of this invention to identify a compound according to the invention that can specifically bind to a cell comprising a specific proteoglycan.

In particular, it has been shown by the inventors that the compound of the invention can bind to colorectal cancer cell lines (Examples 4 and 5; Figures 6 and 7). Thus, in certain embodiments, the compound of the invention may be used in the treatment of colorectal cancer. In certain embodiments, the compound used in the treatment of colorectal cancer comprises one, two, three or four peptide moieties binding specifically to C4S, in particular, any of the peptide moieties comprising an amino acid sequence set forth in SEQ ID NOs:37-79. In certain embodiments, the compound used in the treatment of colorectal cancer comprises one, two, three or four peptide moieties comprising the amino acid sequence set forth in SEQ ID NO:37.

The term "colorectal cancer" as used herein includes a broadly accepted medical definition that defines colorectal cancer as the intestine downstream of the small intestine (ie, the large intestine (colon), including the cecum, ascending colon, transverse colon, descending colon, sigmoid colon). And the medical condition characterized by cell cancer of the rectum. Further, as used herein, the term "colorectal cancer" includes medical conditions characterized by cellular cancers of the duodenum and the small intestine (jejunum and ileum).

Further, it has been shown by the inventors that the compound of the invention can bind to lymphoma cell lines (Examples 4; Figure 5). Thus, in certain embodiments, the compound of the invention may be used in the treatment of lymphoma. In certain embodiments, the compound used in the treatment of lymphoma comprises one, two, three or four peptide moieties binding specifically to C4S, in particular any of the peptide moieties comprising an amino acid sequence set forth in SEQ ID NOs:37-79. In certain embodiments, the compound used in the treatment of lymphoma comprises one, two, three or four peptide moieties comprising the amino acid sequence set forth in SEQ ID NO:37.

The term "lymphoma" as used herein refers to a malignant growth of B or T cells in the lymphatic system. "Lymphoma" includes numerous types of malignant growths, including Hodgkin's Lymphoma and non-Hodgkin's lymphoma.

Further, it has been shown by the inventors that the compound of the invention can bind to leukemia cell lines (Examples 4; Figure 5). Thus, in certain embodiments, the compound of the invention may be used in the treatment of leukemia. In certain embodiments, the compound used in the treatment of leukemia comprises one, two, three or four peptide moieties binding specifically to C4S or C6S, in particular any of the peptide moieties comprising an amino acid sequence set forth in SEQ ID NOs:37-79. In certain embodiments, the compound used in the treatment of leukemia comprises one, two, three or four peptide moieties comprising the amino acid sequence set forth in SEQ ID NO:37.

The term "leukemia" as used herein means any disease involving the progressive proliferation of abnormal leukocytes found in hemopoietic tissues, other organs and usually in the blood in increased numbers.

When using the payload conjugates according to the invention for targeting malignant or cancerous cells, the compound of the invention may be conjugated to any cytotoxic or antineoplastic agent disclosed herein. However, it is preferred that the cytotoxic or antineoplastic agent, when using the payload conjugates of the invention for the treatment of cancer, is an azonafide, PUMA, SN-38, 6-thioguanine or a pro-apoptotic peptide.

In a particular embodiment, the invention relates to the payload conjugate according to the invention or the pharmaceutical composition according to the invention for use in the treatment of disorders involving leukocytes, wherein the payload conjugate specifically binds to chondroitin-4-sulfate.

In a particular embodiment, the invention relates to the payload conjugate or the pharmaceutical composition for use according to the invention, wherein the disorder involving leukocytes is selected from a neoplastic disease, an inflammatory disease, an autoimmune disease, an immunodeficiency disease, a viral infection, a bacterial infection, a protozoan infection or a parasitic infection.

In certain embodiments, the compounds of the invention target specifically C4S proteoglycan. These proteoglycans are preferentially expressed in conjunction with the surface proteins, *e.g.,* CD68, of leukocytes. Indeed, it is likely that leukocytes express no other form of proteoglycan. Accordingly, the compounds of the invention that specifically bind to C4S allow the specific targeting of leukocytes. Therefore, the compounds and methods of the invention facilitate the efficient delivery of cargo moieties, *e.g.,* BAMs, to leukocytes and provides a general means of selectively delivering a substance of interest into a leukocyte. Thus, it is preferred that the BAM to be conjugated to the compound of the invention be any substance the person skilled in the art knows or expects to exert an effect on leukocyte activity, including therapeutic effects (such as treating, preventing, attenuating or ameliorating a disease), or for the purpose of labeling leukocytes such as for diagnostic purposes or for purposes of scientific research. Of particular interest is the targeting of myeloid cells.

In a particular embodiment, the invention relates to the payload conjugate according to the invention or the pharmaceutical composition according to the invention for use in the treatment of a disease or condition in which leukocytes have a pathophysiological involvement: neutrophilia, neutropenia, leukopenia, basopenia, basophilia, eosinopenia, eosinophilia, idiopathic hypereosinophilic syndrome, lymphocyctic leukocytosis, lymphocytosis, lymphocytopenia, monocytosis, monocytopenia, May Hegglin Anomaly, Pelger-Huet Anomaly, Alder-Reilly Anomaly, Chedial-Higashi syndrome, Job's syndrome (hyper-IgE), lazy leukocyte syndrome, congenital C3 deficiency, chronic granulomatrous disease, leukocyte, glucose-6-phosphate dehydrogenase deficiency, myeloperoxidase deficiency-benign, severe combined immunodeficiency disease, DiGeorge's syndrome, Nezelof's syndrome, infantile sex-linked agammaglobulinemia, common variable hypogammaglobulinemia, mucopolysaccharidosis, lipodoses, Gaucher disease, Niemann-pick disease, Fabry disease, Farber's disease, gangliosidoses; Tay Sachs, Sandhoff disease, Krabbe disease, metachromatic, leukodystrophy, Wolman's disease, leukemia, acute lymphatic leukemia (L1, L2, L3), chronic lymphatic leukemia, acute myelogenous leukemia (AML), undifferentiated AML (MO), myeloblastic leukemia (M1), myeloblastic leukemia (M2), promyelocytic leukemia (M3), myelomonocytic leukemia (M4), monocytic leukemia (M5), erytholeukemia (M6), megakaryoblastic leukemia (M7), chronic myelogenous leukemia.

The BAM-conjugates of the invention may be used for the treatment, amelioration, prophylaxis, or diagnosis of a wide range of diseases or conditions in which leukocytes have a pathophysiological involvement. Non-limiting exemplary conditions involving primary or secondary leukocyte dysfunction include: neutrophilia, neutropenia, leukopenia, basopenia, basophilia, eosinopenia, eosinophilia, idiopathic hypereosinophilic syndrome, lymphocytic leukocytosis, lymphocytosis, lymphocytopenia, monocytosis, monocytopenia, May Hegglin Anomaly, Pelger-Huet Anomaly, Alder-Reilly Anomaly, Chedial-Higashi syndrome, Job's syndrome (hyper-IgE), lazy leukocyte syndrome, congenital C3 deficiency, chronic granulomatous disease, leukocyte, glucose-6-phosphate dehydrogenase deficiency, myeloperoxidase deficiency-benign, severe combined immunodeficiency disease, DiGeorge's syndrome, Nezelof's syndrome, infantile sex-linked agammaglobulinemia, common variable hypogammaglobulinemia, mucopolysaccharidosis, lipodoses, Gaucher disease, Niemann-pick disease, Fabry disease, Farber's disease, gangliosidoses; Tay Sachs, Sandhoff disease, Krabbe disease, metachromatic leukodystrophy, Wolman's disease, leukemia, acute lymphocytic leukemia (L1, L2, L3), chronic lymphocytic leukemia, all forms of acute myelogenous leukemia (AML), including undifferentiated AML (MO), myeloblastic leukemia (M1), myeloblastic leukemia, (M2), promyelocytic leukemia, (M3), myelomonocytic leukemia (M4), monocytic leukemia (M5), erytholeukemia (M6), megakaryoblastic leukemia, (M7), chronic myelogenous leukemia as well as undifferentiated or biphenotypic acute leukemias (leukemias that have both lymphocytic and myeloid features), and all forms of lymphomas including Hodgkin lymphoma, T-cell lymphoma, B-cell lymphoma, lymphoplasmacytic lymphoma, Waldenstrom macroglobulinemia, multiple myeloma, and follicular lymphoma.

In a particular embodiment, the invention relates to the payload conjugate according to the invention or the pharmaceutical composition according to the invention for use according to the invention, wherein the disorder involving leukocytes is a neoplastic disease, in particular wherein the neoplastic disease is leukemia.

The payload-conjugates of the invention are of use, either additionally or alternatively, in the diagnosis and/or treatment of diseases wherein the eitiology is primarily dysfunction of myeloid or lymphoid cells. Non-limiting examples of such diseases include the various forms of acute myelogenous leukemia (AML), in particular, Acute Lymphoblastic Leukemia, Acute Myeloblastic Leukemia, Acute Myelogenous Leukemia, Acute Myeloid Leukemia, Acute Promyelocytic Leukemia, Acute Undifferentiated Leukemia, B-cell Chronic Lymphocytic Leukemia, B-cell (acute) Leukemia, Chronic Lymphocytic Leukemia, Chronic Myelocytic Leukemia, Myeloid Leukemia, pre B-cell Leukemia, T-cell Leukemia, T-cell Acute Lymphoblastic Leukemia, T-cell Leukemia (in lung carcinoma), Cutaneous T-cell Leukemia, Human Monocytic Leukemia, Mast cell Leukemia, Mixed Linkage Leukemia, Hairy cell leukemia, T-cell prolymphocytic leukemia, Large granular lymphocytic leukemia, Adult T-cell leukemia, and all forms of lymphomas including Hodgkin lymphoma, T-cell lymphoma, B-cell lymphoma, lymphoplasmacytic lymphoma, Waldenstrom macroglobulinemia, multiple myeloma, and follicular lymphoma.

In a preferred embodiment, the "payload- conjugate" of the invention may be used for the diagnosis and/or treatment or amelioration of various haematological malignancies, such as acute lymphoblastic leukemia (ALL), chronic lymphocytic leukemia, chronic myeloid (or myelogenous) leukemia (CML), all forms of lymphoma and myeloma, and, in particular, all forms of acute myeloid (or myelogenous) leukemia (AML).

AML is a heterogeneous group of diseases, characterized by uncontrolled proliferation of clonal neoplastic precursor cells, and impaired production of normal haematopoiesis, leading to neutropenia, anaemia and thrombocytopenia. For prognostic and therapeutic purposes the World Health Organization (WHO) has provided the following broad classification for AML:

AML with certain genetic abnormalities, including

- AML with a translocation between chromosomes 8 and 21
- AML with a translocation or inversion in chromosome 16
- AML with changes in chromosome 11
- APL (M3), which usually has translocation between chromosomes 15 and 17.

AML with multilineage dysplasia (more than one abnormal myeloid cell type is involved).

AML related to previous chemotherapy or radiation.

AML not otherwise specified, including

- Undifferentiated AML (MO)
- AML with minimal maturation (M1)
- AML with maturation (M2)
- Promyelocytic leukemia, (M3),
- Acute myelomonocytic leukemia (M4)
- Acute monoblastic leukemia (M5a) acute monocytic leukemia (M5b)
- Acute erythroid leukemia (M6)
- Acute megakaryoblastic leukemia (M7)
- Acute basophilic leukemia
- Acute panmyelosis with fibrosis
- Myeloid sarcoma (also known as granulocytic sarcoma or chloroma).

Undifferentiated or biphenotypic acute leukemias (leukemias that have both lymphocytic and myeloid features). Sometimes called ALL with myeloid markers, AML with lymphoid markers, or mixed lineage leukemias.
Any of the conventional chemotherapeutic agents as specified above may be used as the biologically active moiety conjugated to the transporter peptides of the invention for the treatment of AML.
Preferably, for the treatment of AML, the BAM is selected from one of the following chemotherapeutic agents:

- anti-metabolites such as cytosine arabinosides including cytarabine, purine analogues such as thioguanine, anti-folates including trimethopin and pemetred, or pyrimidine analogues including gemcitabine and flouroacil;
- topisomerase inhibitors, such as the anthracyclines, including daunorubicin, adriamycin (doxorubicin) epirubicin, idarubicin, anamycin, and MEN 10755; the quinoline alkaloids, including camptothecin, SN-38, DX-8951f, topotecan, 9-aminocamptothecin, BN 80915, irinotecan, DB 67, BNP 1350, exatecan, lurtotecan, ST 1481 , and CKD 602; the podophyllotoxin analogues etoposide and teniposide, and the anthracenediones, mitoxantrone and amsacrine or other natural products include azathioprine; brequinar; phenoxizone biscyclopeptides, such as dactinomycin; basic glycopeptides, e.g., bleomycin; anthraquinone glycosides, such as plicamycin (mithrmycin); anthracenediones, e.g., mitoxantrone; azirinopyrrolo indolediones, e.g., mitomycin; macrocyclic immunosuppressants, e.g., cyclosporine, FK-506 (tacrolimus, prograf), rapamycin, etc.; and the like;
- agents that interfere with microtubule assembly, such as the family of vinca alkaloids including vinca alkaloids include vinblastine, vincristine; vinorelbine, vindesine; vindolineand vincamine; the taxanes and diterpenes, including paclitaxel and docetaxel,
- metal complexes, e.g., cisplatin (cis-DDP), carboplatin, etc.; ureas, e.g., hydroxyurea; and hydrazines, *e.g.,* N-methylhydrazine, arsenic trioxide;
- retinoids, e.g., vitamin A, 13-cis-retinoic acid, trans-retinoic acid, isotretinoin, etc.; carotenoids, e.g., beta-carotene, vitamin D, etc
- azonafides
- pro-apoptotic peptides, PUMA.

A growing body of evidence documents that the myeloid malignancies, including the various forms of AML are caused by genetic mutations that constitutively activate enzymes involved in signal transduction. Accordingly, in certain embodiments, the BAM-conjugates of the invention may comprise a BAM moiety designed to target these enzymes, downstream mediators of these enzymes or one or more sequelae of their constitutive activity. Non-limiting examples of such enzymes and/or downstream effectors include farnesyl transferase; vascular endothelial growth factor and other angiogenic proteins; enzymes that contribute to the enhanced proliferative and survival capacity of leukemia blasts, such as *FLT3* or RAS; enzymes that contribute to impaired hematopoietic differentiation, such as HDAC inhibitors, the anti-apoptotic gene *BCL-2* , inhibitors of M-CSF, af10, alox 12, arhgef12, arnt, axl, bax, bad, bcl, bcl3, bcl6, btg1, cav1, cbfb, cdc23, cdh17, cdx2, cebpa, clc, cr1, crebbp, dek, dleu1, dleu2, egfr, ets1, evi2a, evi2b, foxo3a, fus, gli2, gmps, hox11, hoxa9, irf1, kit, laf4 , lcp1, ldb1, lmo1, lmo2, lyl1, madh5, mll3, mllt2, mllt3, mov10l1, mtcp1, myc, nfkb2, notch1, notch3, npm1, nup214 , nup98, p53, pbx1, pbx2, pbx3, pbxp1, pitx2, pml, rab7, rgs2, runx1, set, sp140, tal1, tal2, tcl1b, tcl6, thra, tra,and znfn1a1.
In line with this, more recently, genotype specific drug targets for the treatment of the various forms of AML have been identified. Such targeted therapies include in particular protein-kinase inhibitors, including inhibitors of tyrosine

kinases (Flt3, c-kit, BCR-ABL), Aurora kinases and other components of intracellular signaling pathways (e.g., Ras).

In a preferred embodiment, the biologically active moiety conjugated to the transporter moiety of the invention may be an agent which interferes with such aberrant signal transduction, such as farnesyl transferase inhibitors, histone deacetylase and proteosome inhibitors, antiangiogenesis agents, FLT3 inhibitors, apoptosis inhibitors or inhibitors of M-CSF.

These agents may selectively inhibit a specific component of the signaling pathway or may interact with various components, such as the multi-kinase inhibitor sorafenib.

Protein kinase inhibitors which may be used in accordance with the present invention include crizotinib, gefitinib, imatinib, erlotinib, pazopanib, sunitinib, sorafenib, dasatenib, lapatinib, lestaurtinib, nilotinib, ruxolitinib, tandutinib, vandetanib, cediranib, seliciclib, midostaurin, L-21649, ABT-869, dovitinib and PKC412, tipifarnib, AFG206, AFG210, and AHL196, AUZ454 and ATH686,SU5416 and SU6668.

Non-limiting examples of BAMs that may be used in the treatment haematological malignancies other than acute myeloid leukemia according to the methods disclosed herein include protease inhibitors (*e.g.*, bortezomib, carfilzomib, marizomib), mTOR inhibitors (*e.g.*, rapamycin), inhibitors of protein kinases primarily expressed in leukocytes (*e.g.*, fostamatinib) and epigenetic modifying drugs (compounds acting on the writing and/or erasing or the epigenetic code, *e.g.,* vorinostat).

In a particular embodiment, the invention relates to the payload conjugate according to the invention or the pharmaceutical composition according to the invention for use according to the invention, wherein the disorder involving leukocytes is an inflammatory disease, an autoimmune disease and/or immunodeficiency disease.

Exemplary conditions and diseases which may be treated, prevented, attenuated or ameliorated by modulating leukocyte activity include acute or chronic inflammatory conditions, including chronic obstructive pulmonary disease (COPD). rheumatoid arthritis (RA), psoriatic arthritis, inflammatory bowel disease, psoriasis, lupus, asthma, allergic reactions, and autoimmune diseases, including but not limited to Acute disseminated encephalomyelitis (ADEM), Addison's disease, Agammaglobulinemia, Alopecia areata, Amyotrophic Lateral Sclerosis, Ankylosing Spondylitis, Antiphospholipid syndrome, Antisynthetase syndrome, Atopic allergy, Atopic dermatitis, Autoimmune aplastic anemia, Autoimmune cardiomyopathy, Autoimmune enteropathy, Autoimmune hemolytic anemia, Autoimmune hepatitis, Autoimmune inner ear disease, Autoimmune lymphoproliferative syndrome, Autoimmune peripheral neuropathy, Autoimmune pancreatitis, Autoimmune polyendocrine syndrome, Autoimmune progesterone dermatitis, Autoimmune thrombocytopenic purpura, Autoimmune urticaria, Autoimmune uveitis, Balo disease/Balo concentric sclerosis, Behçet's disease, Berger's disease, Bickerstaff's encephalitis, Blau syndrome, Bullous pemphigoid, Cancer, Castleman's disease, Celiac disease, Chagas disease, Chronic inflammatory demyelinating polyneuropathy, Chronic recurrent multifocal osteomyelitis, Chronic obstructive pulmonary disease, Churg-Strauss syndrome, Cicatricial pemphigoid, Cogan syndrome, Cold agglutinin disease, Complement component 2 deficiency, Contact dermatitis, Cranial arteritis, CREST syndrome, Crohn's disease (including either of the two types of idiopathic inflammatory bowel disease "IBD"), Cushing's Syndrome, Cutaneous leukocytoclastic angiitis, Dego's disease, Dercum's disease, Dermatitis herpetiformis, Dermatomyositis, Diabetes mellitus type 1, Diffuse cutaneous systemic sclerosis, Dressler's syndrome, Drug-induced lupus, Discoid lupus erythematosus, Eczema, Endometriosis, Enthesitis-related arthritis, Eosinophilic fasciitis, Eosinophilic gastroenteritis, Epidermolysis bullosa acquisita, Erythema nodosum, Erythroblastosis fetalis, Essential mixed cryoglobulinemia, Evan's syndrome, Fibrodysplasia ossificans progressiva, Fibrosing alveolitis (or Idiopathic pulmonary fibrosis), Gastritis, Gastrointestinal pemphigoid, Giant cell arteritis, Glomerulonephritis, Goodpasture" syndrome, Graves' disease, Guillain-Barré syndrome (GBS), Hashimoto's encephalopathy, Hashimoto's thyroiditis, Henoch-Schonlein purpura, Herpes gestationis aka Gestational Pemphigoid, Hidradenitis suppurativa, Hughes-Stovin syndrome, Hypogammaglobulinemia, Idiopathic inflammatory demyelinating diseases, Idiopathic pulmonary fibrosis, Idiopathic thrombocytopenic purpura (See Autoimmune thrombocytopenic purpura), IgA nephropathy, Inclusion body myositis, Chronic inflammatory demyelinating polyneuropathy, Interstitial cystitis, Juvenile idiopathic arthritis aka Juvenile rheumatoid arthritis, Kawasaki's disease, Lambert-Eaton myasthenic syndrome, Leukocytoclastic vasculitis, Lichen planus, Lichen sclerosus, Linear IgA disease (LAD), Lou Gehrig's disease (Also Amyotrophic lateral sclerosis), Lupoid hepatitis aka Autoimmune hepatitis, Lupus erythematosus, Majeed syndrome, Menière's disease, Microscopic polyangiitis, Miller-Fisher syndrome see Guillain-Barre Syndrome, Mixed connective tissue disease, Morphea, Mucha-Habermann disease aka Pityriasis lichenoides et varioliformis acuta, Multiple sclerosis, Myasthenia gravis, Myositis, Narcolepsy, Neuromyelitis optica (also Devic's disease), Neuromyotonia, Occular cicatricial pemphigoid, Opsoclonus myoclonus syndrome, Ord's thyroiditis, Palindromic rheumatism, PANDAS (pediatric autoimmune neuropsychiatric disorders associated with streptococcus), Paraneoplastic cerebellar degeneration, Paroxysmal nocturnal hemoglobinuria (PNH), Parry Romberg syndrome, Parsonage-Turner syndrome, Pars planitis, Pemphigus vulgaris, Pernicious anaemia, Perivenous encephalomyelitis, POEMS syndrome, Polyarteritis nodosa, Polymyalgia rheumatica, Polymyositis, Primary biliary cirrhosis, Primary sclerosing cholangitis, Progressive inflammatory neuropathy, Psoriasis, Psoriatic arthritis, Pyoderma gangrenosum, Pure red cell aplasia, Rasmussen's encephalitis, Raynaud phenomenon, Relapsing polychondritis, Reiter's syndrome, Restless leg syndrome, Retroperitoneal fibrosis, Rheumatoid arthritis, Rheumatic fever, Sarcoidosis, Schizophrenia, Schmidt syndrome another form of

APS, Schnitzler syndrome, Scleritis, Scleroderma, Serum Sickness, Sjögren's syndrome, Spondyloarthropathy, Still's disease see Juvenile Rheumatoid Arthritis, Stiff person syndrome, Subacute bacterial endocarditis (SBE), Susac's syndrome, Sweet's syndrome, Sydenham chorea see PANDAS, Sympathetic ophthalmia, Systemic lupus erythematosis (e.g. Lupus erythematosus), Takayasu's arteritis, Temporal arteritis (also known as "giant cell arteritis"), Thrombocytopenia, Tolosa-Hunt syndrome, Transverse myelitis, Ulcerative colitis (one of two types of idiopathic inflammatory bowel disease "IBD"), Undifferentiated connective tissue disease different from Mixed connective tissue disease, Undifferentiated spondyloarthropathy, Urticarial vasculitis, Vasculitis, Vitiligo, and Wegener's granulomatosis.

The compounds of the invention may be used in the treatment or prevention of inflammatory conditions, diseases or conditions associated with autoimmune disease and disorders; and/or or amelioration of the symptoms of such inflammatory conditions and/or autoimmune disease. Non-limiting examples of conditions that may be treated with the compounds and methods of the invention include Acute disseminated encephalomyelitis (ADEM), Addison's disease, Agammaglobulinemia, Alopecia areata, Amyotrophic Lateral Sclerosis, Ankylosing Spondylitis, Antiphospholipid syndrome, Antisynthetase syndrome, Atopic allergy, Atopic dermatitis, Autoimmune aplastic anemia, Autoimmune cardiomyopathy, Autoimmune enteropathy, Autoimmune hemolytic anemia, Autoimmune hepatitis, Autoimmune inner ear disease, Autoimmune lymphoproliferative syndrome, Autoimmune peripheral neuropathy, Autoimmune pancreatitis, Autoimmune polyendocrine syndrome, Autoimmune progesterone dermatitis, Autoimmune thrombocytopenic purpura, Autoimmune urticaria, Autoimmune uveitis, Balo disease/Balo concentric sclerosis, Behçet's disease, Berger's disease, Bickerstaff's encephalitis, Blau syndrome, Bullous pemphigoid, Cancer, Castleman's disease, Celiac disease, Chagas disease, Chronic inflammatory demyelinating polyneuropathy, Chronic recurrent multifocal osteomyelitis, Chronic obstructive pulmonary disease, Churg-Strauss syndrome, Cicatricial pemphigoid, Cogan syndrome, Cold agglutinin disease, Complement component 2 deficiency, Contact dermatitis, Cranial arteritis, CREST syndrome, Crohn's disease (one of two types of idiopathic inflammatory bowel disease "IBD"), Cushing's Syndrome, Cutaneous leukocytoclastic angiitis, Dego's disease, Dercum's disease, Dermatitis herpetiformis, Dermatomyositis, Diabetes mellitus type 1, Diffuse cutaneous systemic sclerosis, Dressler's syndrome, Drug-induced lupus, Discoid lupus erythematosus, Eczema, Endometriosis, Enthesitis-related arthritis, Eosinophilic fasciitis, Eosinophilic gastroenteritis, Epidermolysis bullosa acquisita, Erythema nodosum, Erythroblastosis fetalis, Essential mixed cryoglobulinemia, Evan's syndrome, Fibrodysplasia ossificans progressiva, Fibrosing alveolitis (or Idiopathic pulmonary fibrosis), Gastritis, Gastrointestinal pemphigoid, Giant cell arteritis, Glomerulonephritis, Goodpasture's syndrome, Graves' disease, Guillain-Barré syndrome (GBS), Hashimoto's encephalopathy, Hashimoto's thyroiditis, Henoch-Schonlein purpura, Herpes gestationis aka Gestational Pemphigoid, Hidradenitis suppurativa, Hughes-Stovin syndrome, Hypogammaglobulinemia, Idiopathic inflammatory demyelinating diseases, Idiopathic pulmonary fibrosis, Idiopathic thrombocytopenic purpura (See Autoimmune thrombocytopenic purpura), IgA nephropathy, Inclusion body myositis, Chronic inflammatory demyelinating polyneuropathy, Interstitial cystitis, Juvenile idiopathic arthritis aka Juvenile rheumatoid arthritis, Kawasaki's disease, Lambert-Eaton myasthenic syndrome, Leukocytoclastic vasculitis, Lichen planus, Lichen sclerosus, Linear IgA disease (LAD), Lou Gehrig's disease (Also Amyotrophic lateral sclerosis), Lupoid hepatitis aka Autoimmune hepatitis, Lupus erythematosus, Majeed syndrome, Ménière's disease, Microscopic polyangiitis, Miller-Fisher syndrome see Guillain-Barre Syndrome, Mixed connective tissue disease, Morphea, Mucha-Habermann disease aka Pityriasis lichenoides et varioliformis acuta, Multiple sclerosis, Myasthenia gravis, Myositis, Narcolepsy, Neuromyelitis optica (also Devic's disease), Neuromyotonia, Occular cicatricial pemphigoid, Opsoclonus myoclonus syndrome, Ord's thyroiditis, Palindromic rheumatism, PANDAS (pediatric autoimmune neuropsychiatric disorders associated with streptococcus), Paraneoplastic cerebellar degeneration, Paroxysmal nocturnal hemoglobinuria (PNH), Parry Romberg syndrome, Parsonage-Turner syndrome, Pars planitis, Pemphigus vulgaris, Pernicious anaemia, Perivenous encephalomyelitis, POEMS syndrome, Polyarteritis nodosa, Polymyalgia rheumatica, Polymyositis, Primary biliary cirrhosis, Primary sclerosing cholangitis, Progressive inflammatory neuropathy, Psoriasis, Psoriatic arthritis, Pyoderma gangrenosum, Pure red cell aplasia, Rasmussen's encephalitis, Raynaud phenomenon, Relapsing polychondritis, Reiter's syndrome, Restless leg syndrome, Retroperitoneal fibrosis, Rheumatoid arthritis, Rheumatic fever, Sarcoidosis, Schizophrenia, Schmidt syndrome another form of APS, Schnitzler syndrome, Scleritis, Scleroderma, Serum Sickness, Sjögren's syndrome, Spondyloarthropathy, Still's disease see Juvenile Rheumatoid Arthritis, Stiff person syndrome, Subacute bacterial endocarditis (SBE), Susac's syndrome, Sweet's syndrome, Sydenham chorea see PANDAS, Sympathetic ophthalmia, Systemic lupus erythematosis see Lupus erythematosis, Takayasu's arteritis, Temporal arteritis (also known as "giant cell arteritis"), Thrombocytopenia, Tolosa-Hunt syndrome, Transverse myelitis, Ulcerative colitis (one of two types of idiopathic inflammatory bowel disease "IBD"), Undifferentiated connective tissue disease different from Mixed connective tissue disease, Undifferentiated spondyloarthropathy, Urticarial vasculitis, Vasculitis, Vitiligo, and Wegener's granulomatosis.

Non-limiting Examples of BAMs that may used in the treatment of such conditions include COX-2 inhibitors, prednisone, pazopanib, famotidine, dalfampridine, pegloticase, esomeprazole, aspirin, celecoxib, diclofenac, valdecoxib, rofecoxib, diflunisal, etodolac, fenoprofen, flurbiprofen, ibuprofen, indomethacin, ketoprofen, ketorolac, mefenamic acid, meloxicam, nabumetone, naproxen, oxaprozin, piroxicam, sulindac, tolmetin, lansoprazole, meclofenamate, triamcinolone, methylprednisolone, betamethasone, budesonide, prednisolone, hydrocortisone, dexamethasone and cortisone.

In certain embodiments, the payload conjugate of the invention may be used in the treatment of infection. Exemplary conditions involving secondary leukocyte dysfunction include infections by any one of the following pathogens: HIV, Epstein Barr Virus, Morbillivirus (measles), Paramyxovirus, Rubivirus, Herpes Virus Type 6, Herpes Virus, Dengue Virus, Herpes Simplex Virus 1, Herpes Simplex Virus 2, Parvovirus, Respiratory Syncytial Virus, Variola Virus, Varicella, Flavivirus, Human T-lymphotropic virus Type 1, Human T-lymphotropic virus Type 2, Human T-lymphotropic virus Type 3, Human T-lymphotropic virus Type 4, Hepatitis A Virus, Hepatitis B Virus, Hepatitis C Virus, Hepatitis D Virus, Hepatitis E Virus, Lassa Virus, Influenza A, Subtypes H1N1 and H3N2 of Influenza A, Influenza B, or Influenza C Virus or Trypanosomatid protozoa, including *Leishmania.* Most preferably, the disease involving secondary leukocyte dysfunction is HIV infection and AIDS or leishmaniasis.

In a particular embodiment, the invention relates to the payload conjugate according to the invention or the pharmaceutical composition according to the invention for use according to the invention, wherein the disorder involving leukocytes is a viral infection, in particular wherein the viral infection is caused by HIV, Epstein Barr Virus, Morbillivirus, Paramyxovirus, Rubivirus, Herpes Virus, Dengue Virus, Herpes Simplex virus, Parvovirus, Respiratory Syncytial Virus, Variola Virus, Varicella, Flavivirus, Human T-lymphotropic Virus, Hepatitis A, B, C, D, or E Virus, Lassa Virus, or Influenza Virus, in particular wherein in the viral infection is caused by HIV.

It is well established that leukocytes, in particular macrophages play a crucial role in HIV-1 infection. They are among the first cells infected by HIV-1, and have been proposed to form a reservoir of HIV-1 in infected persons. They also serve as a means of spreading virus to other tissues such as the brain. Moreover, the virus may be stored here while remaining hidden from potentially neutralizing antibodies or other forms of immune response.

In light of the above, the compounds of the invention, which selectively target macrophages and facilitate intracellular transport are ideally suited as carrier moieties of intracellular HIV agents.

Thus, the BAM which may be conjugated to the transport moiety of the invention may in particular be

- an NRTI (nucleoside or nucleotide reverse transcriptase inhibitor) including zidovudine, didanosine, stavudine, lamivudine, emtricitabine, abacavir and Tenofovir, apricitabine elvucitabine, OBP-601, amdoxovir, epivir, retrovir, *stavudine, zalcitabine, AZT;,* stavudine, 4-Ed4T, Amdoxovir, Dexelvucitabine, DOT (dioxolane thymidine), Fosalvudine, GS-9131, GS-9148, IDX12899, IDX12989, Quinolones, Racivir, Thiovir, TSAO-T derivatives;
- an NNRTI (non-nucleoside reverse transcriptase inhibitor) including nevirapine, delaviradme, efavirenz, etravinne and rilpivi[pi]ne, etravirine, HBY097, IDX899, V026048, RDEA806, RDEA427, RDEA640, UK-435061; or
- a protease inhibitor including ritonavir, tipranavir, saquinavir, nelfinavir, indinavir, amprenavir, fosamprenavir, atazanavir, lopinavir, darunavir (TMC-1 14), lasinavir and brecanavir (VX-385), GRL-02031, SPI-256.

In certain embodiments, the compound of the invention may also be used in the treatment or prevention of diseases and conditions associated with protozoal infections or other infectious conditions, and/or or amelioration of the symptoms associated therewith. Non-limiting examples of such diseases and conditions that may be treated with the compounds and methods of the invention include Acinetobacter infections, Actinomycosis, African sleeping sickness (African trypanosomiasis), AIDS (Acquired immunodeficiency syndrome), Amebiasis, Anaplasmosis, Anthrax, Arcanobacterium haemolyticum infection, Argentine hemorrhagic fever, Ascariasis, Aspergillosis, Astrovirus infection, Babesiosis, Bacillus cereus infection, Bacterial pneumonia, Bacterial vaginosis (BV), Bacteroides infection, Balantidiasis, Baylisascaris infection, BK virus infection, Black piedra, Blastocystis hominis infection, Blastomycosis, Bolivian hemorrhagic fever, Borrelia infection, Botulism (and Infant botulism), Brazilian hemorrhagic fever, Brucellosis, Burkholderia infection, Buruli ulcer, Calicivirus infection (Norovirus and Sapovirus), Campylobacteriosis, Candidiasis (Moniliasis; Thrush), Cat-scratch disease, Cellulitis, Chagas Disease (American trypanosomiasis), Chancroid, Chickenpox, Chlamydia, Chlamydophila pneumoniae infection, Cholera, Chromoblastomycosis, Clonorchiasis, Clostridium difficile infection, Coccidioidomycosis, Colorado tick fever (CTF), Common cold (Acute viral rhinopharyngitis; Acute coryza), Creutzfeldt-Jakob disease (CJD), Crimean-Congo hemorrhagic fever (CCHF), Cryptococcosis, Cryptosporidiosis, Cutaneous larva migrans (CLM), Cyclosporiasis, Cysticercosis, Cytomegalovirus infection, Dengue fever, Dientamoebiasis, Diphtheria, Diphyllobothriasis, Dracunculiasis, Ebola hemorrhagic fever, Echinococcosis, Ehrlichiosis, Enterobiasis (Pinworm infection), Enterococcus infection, Enterovirus infection, Epidemic typhus, Erythema infectiosum (Fifth disease), Exanthem subitum (Sixth disease), Fasciolopsiasis, Fasciolosis, Fatal familial insomnia (FFI), Filariasis, Food poisoning by Clostridium perfringens, Free-living amebic infection, Fusobacterium infection, Gas gangrene (Clostridial myonecrosis), Geotrichosis, Gerstmann-Straussler-Scheinker syndrome (GSS), Giardiasis, Glanders, Gnathostomiasis, Gonorrhea, Granuloma inguinale (Donovanosis), Group A streptococcal infection, Group B streptococcal infection, Haemophilus influenzae infection, Hand, foot and mouth disease (HFMD), Hantavirus Pulmonary Syndrome (HPS), Helicobacter pylori infection, Hemolytic-uremic syndrome (HUS), Hemorrhagic fever with renal syndrome (HFRS), Hepatitis A, Hepatitis B, Hepatitis C, Hepatitis D, Hepatitis E, Herpes simplex, Histoplasmosis, Hookworm infection, Human bocavirus infection, Human ewingii ehrlichiosis, Human granulocytic anaplasmosis (HGA), Human metapneumovirus infection, Human monocytic ehrlichiosis, Human papillomavirus (HPV) infection, Human parainfluenza virus infection, Hymenolepiasis, Epstein-Barr

Virus Infectious Mononucleosis (Mono), Influenza (flu), Isosporiasis, Kawasaki disease, Keratitis, Kingella kingae infection, Kuru, Lassa fever, Legionellosis (Legionnaires' disease), Legionellosis (Pontiac fever), Leishmaniasis, Leprosy, Leptospirosis, Listeriosis, Lyme disease (Lyme borreliosis), Lymphatic filariasis (Elephantiasis), Lymphocytic choriomeningitis, Malaria, Marburg hemorrhagic fever (MHF), Measles, Melioidosis (Whitmore's disease), Meningitis, Meningococcal disease, Metagonimiasis, Microsporidiosis, Molluscum contagiosum (MC), Mumps, Murine typhus (Endemic typhus), Mycoplasma pneumonia, Mycetoma , Myiasis, Neonatal conjunctivitis (Ophthalmia neonatorum), (New) Variant Creutzfeldt-Jakob disease (vCJD, nvCJD), Nocardiosis, Onchocerciasis (River blindness), Paracoccidioidomycosis (South American blastomycosis), Paragonimiasis, Pasteurellosis, Pelvic inflammatory disease (PID), Pertussis (Whooping cough), Plague, Pneumococcal infection, Pneumocystis pneumonia (PCP), Pneumonia, Poliomyelitis, Prevotella infection, Primary amoebic meningoencephalitis (PAM), Progressive multifocal leukoencephalopathy, Psittacosis, Q fever, Rabies, Rat-bite fever, Respiratory syncytial virus infection, Rhinosporidiosis, Rhinovirus infection, Rickettsial infection, Rickettsialpox, Rift Valley fever (RVF), Rocky mountain spotted fever (RMSF), Rotavirus infection, Rubella, Salmonellosis, SARS (Severe Acute Respiratory Syndrome), Scabies, Schistosomiasis, Sepsis, Shigellosis (Bacillary dysentery), Shingles (Herpes zoster), Smallpox (Variola), Sporotrichosis, Staphylococcal food poisoning, Staphylococcal infection, Strongyloidiasis, Syphilis, Taeniasis, Tetanus (Lockjaw), Tinea barbae (Barber's itch), Tinea capitis (Ringworm of the Scalp), Tinea corporis (Ringworm of the Body), Tinea cruris (Jock itch), Tinea manuum (Ringworm of the Hand), Tinea nigra, Tinea pedis (Athlete's foot), Tinea unguium (Onychomycosis), Tinea versicolor (Pityriasis versicolor), Toxocariasis (Ocular Larva Migrans (OLM)), Toxocariasis (Visceral Larva Migrans (VLM)), Toxoplasmosis, Trichinellosis, Trichomoniasis, Trichuriasis (Whipworm infection), Tuberculosis, Tularemia, Ureaplasma urealyticum infection, Venezuelan equine encephalitis, Venezuelan hemorrhagic fever, Viral pneumonia, West Nile Fever, White piedra (Tinea blanca), Yersinia pseudotuberculosis infection, Yersiniosis, Yellow fever and Zygomycosis.

Non-limiting Examples of BAMs that may used in the treatment of such conditions include chloroquine, mefloquine, primaquine, proguanil hydrochloride, proguanil hydrochloride with atovaquone, pyrimethamine, sulfadoxine, quinine, quinoline, doxycycline, clindamycin, artesunate, diloxanide, metronidazole, tinidazole, mepacrine hydrochloride, amphotericin, pentamidine, pyrimethamine, sulfadiazine, azithromycin, atovaquone, trimethoprim-sulphamethoxazole, trimethoprim, dapsone, atovaquone, pentamidine isetionate, amodiaquine, chloroguanide, eflornithine, hydroxychloroquine, iodoquinol, meglumine antimonate, melarsoprol, nifurtimox, paromomycin, sodium stibogluconate, suramin, tryparsamide.

In a particular embodiment, the invention relates to the payload conjugate according to the invention or the pharmaceutical composition according to the invention for use according to the invention, wherein the disorder involving leukocytes is a protozoan infection, in particular wherein the protozoan infection is Leishmaniasis.

*Leishmania* are protozoa belonging to the order Kinetoplastida and the family Trypanosomatidae. Leishmaniasis is a vector-borne disease that is transmitted by sandflies. Human infection is caused by about 30 different species that infect mammals. These include the *L. donovani* complex with 3 species (*L. donovani, L. infantum,* and *L. chagasi);* the *L. mexicana* complex with 3 main species (*L. mexicana, L. amazonensis,* and *L. venezuelensis); L. tropica; L. major, L. aethiopica;* and the subgenus *Viannia* with 4 main species (*L. (V.) braziliensis, L. (V.) guyanensis, L. (V.) panamensis,* and *L. (V.) peruviana).* The different species are morphologically indistinguishable, but they can be differentiated by isoenzyme analysis, molecular methods, or monoclonal antibodies.

*Leishmaniasis* is responsible for various types of diseases, including visceral leishmaniasis, cutaneous leishmaniasis (of localized or diffuse type) and mucocutaneous leishmaniasis. This variability of the clinical features results from both the diversity of the *Leishmania* species and the immune response of the hosts.

In humans as well as in other mammals, the parasite is inoculated through the skin as a flagellated, extracellular promastigote by its arthropod vector. In its mammalian host, *Leishmania* promastigotes must first evade complement-mediated lysis until they are engulfed by a macrophage. The *Leishmania* promastigote then differentiates into the more resistant amastigotes stage and remains an obligate intracellular pathogen infecting the hematopoietic cells of the monocyte/macrophage lineage. Sequestering itself inside the cells of the host allows *Leishmania* to escape many of the immune responses that would otherwise be directed against it. However, in order to survive the antimicrobial activities of its host cell and to prevent activation of an effective immune response, the various *Leishmania* species have evolved complex mechanisms to subvert normal macrophage function, particularly those involved in immune surveillance and macrophage activation, at either the protein or gene expression level.

In addition to repressing the microbicidal activities of the host macrophage, *Leishmania* inhibits the ability of the host cell to display parasite antigens to other components of the immune system. Thus, *Leishmania* prevents the activation of an effective immune response by inhibiting production of a number of cytokines, particularly those involved in the inflammatory response (IL-1 and tumor necrosis factor alpha [TNF-$\alpha$]) or in T-lymphocyte activation (IL-12)). The powerful ability of *Leishmania* to shut down the macrophage antimicrobial machinery allows the parasite to avoid the initial innate immune response and create a safe environment for proliferation. Moreover, the hidden location within the host macrophage is largely responsible for the lack of effective chemotherapy as it impairs the accession of therapeutic drugs.

In order to enhance the antileishmanial activity and to reduce toxicity of the medication, liposomal drug delivery systems have been developed so as to target the mononuclear phagocyte system. Thus, for example, liposomal amphotericin B has been used to treat visceral leishmaniasis.

Nevertheless, to date, pentavalent antimony (Sb), which has been developed more than 50 years ago, remains the first-line treatment for the treatment of Leishmaniasis, despite of its poor efficacy and side effects. There is thus a substantial need for an effective antileishmanial agent, which selectively targets the macrophage host cells so as to increase antileishmanial activity and to reduce toxicity. By virtue of their ability to specifically target myeloid cells, the compounds of the invention may be used to transport and deliver agents with anti-leishmanial activity to the infected macrophage host cells.

Thus, in accordance with one preferred embodiment of the invention, the BAM which may be conjugated to form the BAM-conjugate to the invention may be an agent with anti-protozoal activity, including but not limited to chloroquine, mefloquine, primaquine, proguanil hydrochloride, proguanil hydrochloride with atovaquone, pyrimethamine, sulfadoxine, quinine, quinoline, doxycycline, clindamycin, artesunate, diloxanide, metronidazole, tinidazole, mepacrine hydrochloride, amphotericin, pentamidine, pyrimethamine, sulfadiazine, azithromycin, atovaquone, trimethoprim-sulphamethoxazole, trimethoprim, dapsone, atovaquone, pentamidine isetionate, amodiaquine, chloroguanide, eflornithine, hydroxychloroquine, iodoquinol, meglumine antimonate, melarsoprol, nifurtimox, paromomycin, sodium stibogluconate, suramin and tryparsamide.

In a particular embodiment, the invention relates to the payload conjugate according to the invention or the pharmaceutical composition according to the invention for use in the treatment of a heart disease or condition, a disease or condition of the ovary or testis, a disease or condition of the central nervous system, or a C6S accumulation disease, in particular wherein the payload conjugate specifically binds to chondroitin-6-sulfate.

C6S is preferentially expressed relative to other proteoglycans, by certain tissues, for example, tissues of the central nervous system, heart, ovary and testis. Accordingly, the invention encompasses the use of the compounds of the invention, e.g., BAM-conjugates, to specifically target cells expressing C6S. The invention also encompasses the use of the compounds of the invention, e.g., BAM-conjugates, to facilitate transport of the BAM moiety into cells expressing C6S.

That is, in certain embodiments, the compounds of the invention binding specifically to C6S may find particular use in the diagnosis and/or treatment of C6S and KS accumulation diseases such as Morquio syndrome. BAMs that may be conjugated to the compound of the invention for the treatment of Morquio syndrome include, without limitation, elosulfase alfa (Vimizim). Alternatively, the compound of the invention binding specifically to C6S may be conjugated to an enzyme that hydrolyzes C6S to reduce the levels of accumulated C6S in the subject’s body.

Thus, in a particular embodiment, the invention relates to the payload conjugate according to the invention or the pharmaceutical composition according to the invention for use in the treatment of a C6S accumulation disease, wherein said disease is Morquio syndrome.

Morquio syndrome, also known as Mucopolysaccharidosis Type IV (MPS IV), is a rare metabolic disorder in which the body cannot process certain types of sugar molecules called glycosaminoglycans (AKA GAGs, or mucopolysaccharides). In Morquio syndrome, the specific GAG which builds up in the body is keratan sulfate and/or chondroitin-6-sulfate. This birth defect, which is autosomal recessive, is a type of lysosomal storage disorder. The buildup of GAGs in different parts of the body causes symptoms in many different organ systems.

The compounds of the invention binding specifically to C6S may also find particular use in the targeting of cells of the central nervous system, heart, ovary and testis. Thus, the compounds of the invention may find particular use in the diagnosis, treatment, prevention or amelioration of symptoms associated with diseases or conditions in which these cells are pathophysiologically involved or have pathophysiological involvement. For example, nonlimiting examples of diseases or conditions affecting heart tissue that may be treated using the compound of the invention include angina, arrhythmia, atrial fibrillation, dilated cardiomyopathy, hypertrhophic cardiomiopahthy, congestive heart failure, endocarditis, heart rhythm disorders, myocarditis, pericarditis, premature ventricular contractions, and Wolff-Parkinson-White syndrome. It is to be understood that the compounds of the invention are conjugated to a BAM that is suitable for the treatment of the respective medical condition. That is, any BAM that is known in the art to be suitable for the treatment of a specific medical condition may be conjugated to the compounds of the invention.

Accordingly, in a particular embodiment, the invention relates to the payload conjugate according to the invention or the pharmaceutical composition according to the invention for use in the treatment of a heart disease or condition, in particular wherein said disease or condition is angina, arrhythmia, atrial fibrillation, dilated cardiomyopathy, hypertrhophic cardiomiopahthy, congestive heart failure, endocarditis, heart rhythm disorders, myocarditis, pericarditis, premature ventricular contractions, or Wolff-Parkinson-White syndrome.

The compounds of the invention binding specifically to C6S may find particular use in the treatment of diseases and conditions involving tissues of the ovary or testis. Non-limiting examples of such diseases and conditions affecting the ovary or testis that may be treated using the molecules and compounds of the invention include epididymitis, orchitis, spermatocele, varicocele, male hypogonadism, testicular cancer, infertility, ovarian cyst, polycystic ovary syndrome,

premature ovarian failure, and ovarian cancer. It is to be understood that the compounds of the invention are conjugated to a BAM that is suitable for the treatment of the respective medical condition. That is, any BAM that is known in the art to be suitable for the treatment of a specific medical condition may be conjugated to the compounds of the invention.

Accordingly, in a particular embodiment, the invention relates to the payload conjugate according to the invention or the pharmaceutical composition according to the invention for use in the treatment of a disease or condition of the ovary or testis, in particular wherein said disease or condition is epididymitis, orchitis, spermatocele, varicocele, male hypogonadism, testicular cancer, infertility, ovarian cyst, polycystic ovary syndrome, premature ovarian failure, or ovarian cancer.

The compounds of the invention binding specifically to C6S may find particular use in the targeting of cells of the central nervous system. Thus, the compounds of the invention may find particular use in the diagnosis, treatment, prevention or amelioration of symptoms associated with diseases or conditions affecting such cells, *e.g.,* glial cells. Nonlimiting examples of such diseases or conditions affecting the central nervous system that may be treated using the molecules and compound of the invention include glial-related conditions of the central nervous system, anxiety disorders, depression, epilepsy, spinal muscular atrophy, amyotrophic lateral sclerosis, Alzheimer's disease, autism, Ataxia Telangiectasia, Niemann Pick disease Type C, cerebellar degeneration, Machado-Joseph Disease, olivopontocerebella atrophy, spinocerebella ataxias, sporadic ataxia, multiple system Atrophyatrophy, Parkinson's disease, Parkison's syndrome, gait ataxia, herpes zoster, viral encephalitis, Japanese encephalitis, bacterial encephalitis, toxoplasmosis, malaria, amoebic meningoencephalitis, Cryptococcus neoformans encephalitis, Lyme disease, streptococci meningoencephalitis, staphylococci meningoencephalitis, astrocytoma, glioblastoma, oligodendroglioma, ependymoma, medulloblastoma and meningioma. It is to be understood that the compounds of the invention are conjugated to a BAM that is suitable for the treatment of the respective medical condition. That is, any BAM that is known in the art to be suitable for the treatment of a specific medical condition may be conjugated to the compounds of the invention.

Accordingly, in a particular embodiment, the invention relates to the payload conjugate according to the invention or the pharmaceutical composition according to the invention for use in the treatment of a disease or condition of the central nervous system, in particular wherein said disease or condition is a glial-related disease of the central or peripheral nervous system, an anxiety disorder, depression, epilepsy, spinal muscular atrophy, amyotrophic lateral sclerosis, Alzheimer's disease, autism, Ataxia Telangiectasia, Niemann Pick disease Type C, cerebellar degeneration, Machado-Joseph Disease, olivopontocerebella atrophy, spinocerebella ataxias, sporadic ataxia, multiple system Atrophyatrophy, Parkinson's disease, Parkison's syndrome, gait ataxia, herpes zoster, viral encephalitis, Japanese encephalitis, bacterial encephalitis, toxoplasmosis, malaria, amoebic meningoencephalitis, Cryptococcus neoformans encephalitis, Lyme disease, streptococci meningoencephalitis, staphylococci meningoencephalitis, astrocytoma, glioblastoma, oligodendroglioma, ependymoma, medulloblastoma or meningioma.

In a particular embodiment, the invention relates to the payload conjugate according to the invention or the pharmaceutical composition according to the invention for use in the treatment of a corneal disease or condition, a retina disease or condition, a disease or condition of the central nervous system, or a keratan sulfate accumulation disease, in particular wherein the payload conjugate specifically binds to keratan sulfate.

KS is preferentially expressed relative to other proteoglycans, by certain tissues, for example, tissues of the eye (*e.g.*, cornea), the central nervous system, and the endometrial lining of the uterus. Accordingly, the invention encompasses the use of the compounds of the invention, *e.g.*, BAM-conjugates, to specifically target cells expressing KS and/or tissues comprising such cells. The invention also encompasses the use of the compounds of the invention, *e.g.*, BAM-conjugates, to facilitate transport of the BAM moiety into cells expressing KS, *e.g.*, corneal cells.

That is, in certain embodiments, the compounds of the invention may find particular use in the diagnosis, treatment of KS accumulation diseases such as Morquio syndrome. BAMs that may be conjugated to the compound of the invention for the treatment of Morquio syndrome include, without limitation, elosulfase alfa (Vimizim). Alternatively, the compound of the invention binding specifically to KS may be conjugated to an enzyme that hydrolyzes KS to reduce the levels of accumulated KS in the subject's body.

Thus, in a particular embodiment, the invention relates to the payload conjugate according to the invention or the pharmaceutical composition according to the invention for use in the treatment of a keratan sulfate accumulation disease, wherein said disease is Morquio syndrome.

The compounds of the invention binding specifically to KS may also find particular use in the targeting of tissues or cells of the eye, *e.g.*, corneal cells. Thus, the compounds of the invention may find particular use in the treatment, prevention or amelioration of symptoms associated with diseases or conditions in which these cells are pathophysiologically involved or have pathophysiological involvement. Nonlimiting examples of diseases or conditions affecting corneal cells that may be treated using the molecules and compound of the invention include corneal ulcer, corneal flash burn, corneal abrasion, corneal neovascularization, keratitis, ocular herpes, pterygium, trachomacorneal abrasion, corneal dystrophy, Fuch's dystrophy, iridocorneal endothelial syndrome, lattice dystrophy, map-dot-fingerprint dystrophy, corneal infection, ocular herpes, and dry eye. It is to be understood that the compounds of the invention are conjugated to a BAM that is suitable for the treatment of the respective medical condition. That is, any BAM that is known in the art

to be suitable for the treatment of a specific medical condition may be conjugated to the compounds of the invention.

Accordingly, in a particular embodiment, the invention relates to the payload conjugate according to the invention or the pharmaceutical composition according to the invention for use in the treatment of a corneal disease or condition, in particular wherein said disease or condition is corneal ulcer, corneal flash burn, corneal abrasion, corneal neovascularization, keratitis, ocular herpes, pterygium, trachoma, corneal dystrophy, Fuch's dystrophy, iridocorneal endothelial syndrome, lattice dystrophy, map-dot-fingerprint dystrophy, corneal infection, or dry eye.

The compounds of the invention binding specifically to KS may find particular use in the targeting of cells and tissues of the retina. Thus, the compounds of the invention may find particular use in the diagnosis, treatment, prevention or amelioration of symptoms associated with diseases or conditions affecting such cells. Non-limiting examples of such diseases and conditions affecting the retina that may be treated using the molecules and compounds of the invention include age-related macular degeneration, diabetic retinopathy, retinitis pigmentosa, retinal degeneration, macular hole, retinoblastoma, glaucoma and onchocerciasis. It is to be understood that the compounds of the invention are conjugated to a BAM that is suitable for the treatment of the respective medical condition. That is, any BAM that is known in the art to be suitable for the treatment of a specific medical condition may be conjugated to the compounds of the invention.

Accordingly, in a particular embodiment, the invention relates to the payload conjugate according to the invention or the pharmaceutical composition according to the invention for use in the treatment of a retina disease or condition, in particular wherein said disease or condition is age-related macular degeneration, diabetic retinopathy, retinitis pigmentosa, retinal degeneration, macular hole, retinoblastoma, glaucoma or onchocerciasis.

The compounds of the invention binding specifically to KS may find particular use in the targeting of cells of the central nervous system. Thus, the compounds of the invention may find particular use in the diagnosis, treatment, prevention or amelioration of symptoms associated with diseases or conditions affecting such cells. Nonlimiting examples of such diseases or conditions that may be treated using the molecules and compound of the invention include anxiety disorders, depression, epilepsy, spinal muscular atrophy, amyotrophic lateral sclerosis, Alzheimer's disease, autism, Ataxia Telangiectasia, Niemann Pick disease Type C, cerebellar degeneration, Machado-Joseph disease, olivopontocerebella atrophy, spinocerebella ataxias, sporadic ataxia, multiple system atrophy, Parkinson's disease, Parkison's syndrome, gait ataxia, herpes zoster, viral encephalitis, Japanese encephalitis, bacterial encephalitis, toxoplasmosis, malaria, amoebic meningoencephalitis, Cryptococcus neoformans encephalitis, Lyme disease, streptococci meningoencephalitis, staphylococci meningoencephalitis, astrocytoma, glioblastoma, oligodendroglioma, ependymoma, medulloblastoma and meningioma. It is to be understood that the compounds of the invention are conjugated to a BAM that is suitable for the treatment of the respective medical condition. That is, any BAM that is known in the art to be suitable for the treatment of a specific medical condition may be conjugated to the compounds of the invention.

Accordingly, in a particular embodiment, the invention relates to the payload conjugate according to the invention or the pharmaceutical composition according to the invention for use in the treatment of a disease or condition of the central nervous system, in particular wherein said disease or condition is an anxiety disorder, depression, epilepsy, spinal muscular atrophy, amyotrophic lateral sclerosis, Alzheimer's disease, autism, Ataxia Telangiectasia, Niemann Pick disease Type C, cerebellar degeneration, Machado-Joseph disease, olivopontocerebella atrophy, spinocerebella ataxias, sporadic ataxia, multiple system atrophy, Parkinson's disease, Parkison's syndrome, gait ataxia, herpes zoster, viral encephalitis, Japanese encephalitis, bacterial encephalitis, toxoplasmosis, malaria, amoebic meningoencephalitis, Cryptococcus neoformans encephalitis, Lyme disease, streptococci meningoencephalitis, staphylococci meningoencephalitis, astrocytoma, glioblastoma, oligodendroglioma, ependymoma, medulloblastoma or meningioma.

The compounds of the invention binding specifically to KS may find particular use in the targeting of cells of the endometrial lining of the uterus. Thus, the compounds of the invention may find particular use in the diagnosis, treatment, prevention, regulation of physiological function or amelioration of symptoms associated with diseases, conditions or physiological functions related to such cells and tissues. Nonlimiting examples of such diseases, conditions or physiological functions that may be treated using the molecules and compounds of the invention include endometrial cancer, endometrial hyperplasia, endometrial polyp, uterine bleeding, endometriosis, adenomyosis, cervicitis, infertility, contraception, and regulation of the menstrual cycle. It is to be understood that the compounds of the invention are conjugated to a BAM that is suitable for the treatment of the respective medical condition. That is, any BAM that is known in the art to be suitable for the treatment of a specific medical condition may be conjugated to the compounds of the invention.

Accordingly, in a particular embodiment, the invention relates to the payload conjugate according to the invention or the pharmaceutical composition according to the invention for use in the treatment of a disease or condition of the endometrium, in particular wherein said disease or condition is endometrial cancer, endometrial hyperplasia, endometrial polyp, uterine bleeding, endometriosis, adenomyosis, cervicitis, infertility, contraception or regulation of the menstrual cycle.

In a particular embodiment, the invention relates to the payload conjugate according to the invention or the pharmaceutical composition according to the invention for use in diagnostics.

That is, in certain embodiments, the payload conjugate according to the invention may comprise an imaging

agent and may be used in diagnostic methods, in particular in imaging or visualization methods. The payload conjugate according to the invention may comprise any imaging that can be conjugated to the compound of the invention and is suitable for *in vitro* or *in vivo* visualization of cells. In particular the payload conjugate according to the invention may comprise any of the imaging agents disclosed herein.

The compounds of the invention interact specifically with proteoglycans, in particular, chondroitin-4-sulfate (C4S), chondroitin-6-sulfate (C4S) and/or keratan sulfate (KS). Thus, it is to be understood that some compounds of the invention may be more suitable for the detection and/or diagnosis of a specific medical conditions than others. For example, in certain embodiments, payload conjugates that specifically bind to C4S are preferably used in the diagnosis of cells that synthesize detectable and/or elevated amounts of C4S. In other embodiments, payload conjugates that specifically bind to C6S are preferably used in the diagnosis of cells that synthesize detectable and/or elevated amounts of C6S. In further embodiments, payload conjugates that specifically bind to KS are preferably used in the diagnosis of cells that synthesize detectable and/or elevated amounts of KS.

In a particular embodiment, the invention relates to the payload conjugate according to the invention or the pharmaceutical composition according to the invention for use in the visualization of cells comprising chondroitin-4-sulfate, chondroitin-6-sulfate and/or keratan sulfate on their cell surface and/or tissues comprising cells comprising chondroitin-4-sulfate, chondroitin-6-sulfate and/or keratan sulfate on their cell surface.

It has been shown herein that the compounds of the invention bind specifically to proteoglycans on the surface of cells, in particular on the surface of mammalian cells. Thus, the compounds of the invention may be used, without limitation, in immunohistochemistry assays to visualize cells and/or tissues comprising a proteoglycan that can be specifically bound by a payload conjugate comprising an imaging agent. It is to be understood that the payload conjugate may be used for the visualization of cells *in vitro, in vivo* or *in situ.* Visualization may be performed by any suitable method known in the art, preferably one of the methods disclosed herein. The skilled person is aware of imaging agents that are suitable for a particular visualization method.

In certain embodiments, the payload conjugate of the invention may comprise a compound that specifically binds to C4S and an imaging agent. Such payload conjugates may be used for the visualization of cells and/or tissues comprising C4S on their surface. In particular, a payload conjugate that binds specifically to C4S and comprises an imaging agent may be used for the visualization of leukocytes.

Thus, in a particular embodiment, the invention relates to the payload conjugate or the pharmaceutical composition for use according to the invention, wherein the cells comprising chondroitin-4-sulfate are leukocytes.

In certain embodiments, the payload conjugate of the invention may comprise a compound that specifically binds to C6S and an imaging agent. Such payload conjugates may be used for the visualization of cells and/or tissues comprising C6S on their surface. In particular, a payload conjugate that binds specifically to C6S and comprises an imaging agent may be used for the visualization of tissues of the central nervous system, heart, ovary and testis.

Thus, in a particular embodiment, the invention relates to the payload conjugate or the pharmaceutical composition for use according to the invention, wherein the cells and/or tissues comprising chondroitin-6-sulfate are cells and/or tissues of the central nervous system, heart, ovary or testis.

In certain embodiments, the payload conjugate of the invention may comprise a compound that specifically binds to KS and an imaging agent. Such payload conjugates may be used for the visualization of cells and/or tissues comprising KS on their surface. In particular, a payload conjugate that binds specifically to KS and comprises an imaging agent may be used for the visualization of cells and/or tissues of the eye (*e.g.*, cornea), the central nervous system, and the endometrial lining of the uterus.

Thus, in a particular embodiment, the invention relates to the payload conjugate or the pharmaceutical composition for use according to the invention, wherein the cells and/or tissues comprising keratan sulfate are cells and/or tissues of the eye (*e.g.*, cornea), the central nervous system, or the endometrial lining of the uterus.

The payload conjugate of the invention comprising an imaging agent may further be used in the diagnosis of diseases or medical conditions. It is to be understood that diagnostic methods comprising the payload conjugate of the invention may be indicative of a certain disease or medical condition, but may require additional diagnostic tests to diagnose a subject with a certain disease or medical condition with sufficient certainty. Thus, the payload conjugate or pharmaceutical composition of the invention may be used in combination with other diagnostic tests when diagnosing a disease or medical condition in a subject.

Accordingly, in a particular embodiment, the invention relates to the payload conjugate according to the invention or the pharmaceutical composition according to the invention for use in the diagnosis of diseases involving cells and/or tissues comprising proteoglycans on the surface.

In certain embodiments, a payload conjugate specifically binding to C4S and comprising an imaging agent may be used in the diagnosis of disorders involving leukocytes, in particular any of the disorders disclosed herein. That is, payload conjugates comprising an imaging agent and binding specifically to C4S may be used in the diagnosis of neutrophilia, neutropenia, leukopenia, basopenia, basophilia, eosinopenia, eosinophilia, idiopathic hypereosinophilic syndrome, lymphocytic leukocytosis, lymphocytosis, lymphocytopenia, monocytosis, monocytopenia, May Hegglin

Anomaly, Pelger-Huet Anomaly, Alder-Reilly Anomaly, Chedial-Higashi syndrome, Job's syndrome (hyper-IgE), lazy leukocyte syndrome, congenital C3 deficiency, chronic granulomatous disease, leukocyte, glucose-6-phosphate dehydrogenase deficiency, myeloperoxidase deficiency-benign, severe combined immunodeficiency disease, DiGeorge's syndrome, Nezelof's syndrome, infantile sex-linked agammaglobulinemia, common variable hypogammaglobulinemia, mucopolysaccharidosis, lipodoses, Gaucher disease, Niemann-pick disease, Fabry disease, Farber's disease, gangliosidoses; Tay Sachs, Sandhoff disease, Krabbe disease, metachromatic leukodystrophy, Wolman's disease, leukemia, acute lymphocytic leukemia (L1, L2, L3), chronic lymphocytic leukemia, all forms of acute myelogenous leukemia (AML), including undifferentiated AML (M0), myeloblastic leukemia (M1), myeloblastic leukemia, (M2), promyelocytic leukemia, (M3), myelomonocytic leukemia (M4), monocytic leukemia (M5), erytholeukemia (M6), megakaryoblastic leukemia, (M7), chronic myelogenous leukemia as well as undifferentiated or biphenotypic acute leukemias (leukemias that have both lymphocytic and myeloid features), and all forms of lymphomas including Hodgkin lymphoma, T-cell lymphoma, B-cell lymphoma, lymphoplasmacytic lymphoma, Waldenstrom macroglobulinemia, multiple myeloma, and/or follicular lymphoma.

Accordingly, in a particular embodiment, the invention relates to the payload conjugate or pharmaceutical composition for use according to the invention, wherein the proteoglycan on the surface of the cells and/or tissues is chondroitin-4-sulfate and/or wherein the cells are leukocytes.

In certain embodiments, a payload conjugate specifically binding to C6S and comprising an imaging agent may be used in the diagnosis of disorders involving tissues of the central nervous system, heart, ovary or testis, in particular any of the disorders disclosed herein. That is, payload conjugates comprising an imaging agent and binding specifically to C6S may be used in the diagnosis of angina, arrhythmia, atrial fibrillation, dilated cardiomyopathy, hypertrhophic cardiomiopahthy, congestive heart failure, endocarditis, heart rhythm disorders, myocarditis, pericarditis, premature ventricular contractions, or Wolff-Parkinson-White syndrome, epididymitis, orchitis, spermatocele, varicocele, male hypogonadism, testicular cancer, infertility, ovarian cyst, polycystic ovary syndrome, premature ovarian failure, ovarian cancer, a glial-related disease of the central or peripheral nervous system, an anxiety disorder, depression, epilepsy, spinal muscular atrophy, amyotrophic lateral sclerosis, Alzheimer's disease, autism, Ataxia Telangiectasia, Niemann Pick disease Type C, cerebellar degeneration, Machado-Joseph Disease, olivopontocerebella atrophy, spinocerebella ataxias, sporadic ataxia, multiple system Atrophyatrophy, Parkinson's disease, Parkison's syndrome, gait ataxia, herpes zoster, viral encephalitis, Japanese encephalitis, bacterial encephalitis, toxoplasmosis, malaria, amoebic meningoencephalitis, Cryptococcus neoformans encephalitis, Lyme disease, streptococci meningoencephalitis, staphylococci meningoencephalitis, astrocytoma, glioblastoma, oligodendroglioma, ependymoma, medulloblastoma and/or meningioma.

Accordingly, in a particular embodiment, the invention relates to the payload conjugate or pharmaceutical composition for use according to the invention, wherein the proteoglycan on the surface of the cells and/or tissues is chondroitin-6-sulfate and/or wherein the cells and/or tissues are cells and/or tissues of the central nervous system, heart, ovary or testis.

In certain embodiments, a payload conjugate specifically binding to KS and comprising an imaging agent may be used in the diagnosis of disorders involving tissues of the eye (*e.g.*, cornea), the central nervous system, or the endometrial lining of the uterus, in particular any of the disorders disclosed herein. That is, payload conjugates comprising an imaging agent and binding specifically to C6S may be used in the diagnosis of corneal ulcer, corneal flash burn, corneal abrasion, corneal neovascularization, keratitis, ocular herpes, pterygium, trachoma, corneal dystrophy, Fuch's dystrophy, iridocorneal endothelial syndrome, lattice dystrophy, map-dot-fingerprint dystrophy, corneal infection, dry eye, age-related macular degeneration, diabetic retinopathy, retinitis pigmentosa, retinal degeneration, macular hole, retinoblastoma, glaucoma, onchocerciasis, an anxiety disorder, depression, epilepsy, spinal muscular atrophy, amyotrophic lateral sclerosis, Alzheimer's disease, autism, Ataxia Telangiectasia, Niemann Pick disease Type C, cerebellar degeneration, Machado-Joseph disease, olivopontocerebella atrophy, spinocerebella ataxias, sporadic ataxia, multiple system atrophy, Parkinson's disease, Parkison's syndrome, gait ataxia, herpes zoster, viral encephalitis, Japanese encephalitis, bacterial encephalitis, toxoplasmosis, malaria, amoebic meningoencephalitis, Cryptococcus neoformans encephalitis, Lyme disease, streptococci meningoencephalitis, staphylococci meningoencephalitis, astrocytoma, glioblastoma, oligodendroglioma, ependymoma, medulloblastoma, meningioma, endometrial cancer, endometrial hyperplasia, endometrial polyp, uterine bleeding, endometriosis, adenomyosis, cervicitis, infertility, contraception and/or regulation of the menstrual cycle.

Accordingly, in a particular embodiment, the invention relates to the payload conjugate or pharmaceutical composition for use according to the invention, wherein the proteoglycan on the surface of the cells and/or tissues is keratan sulfate and/or wherein the cells and/or tissues are cells and/or tissues of the eye, in particular the cornea, the central nervous system, or the endometrial lining of the uterus.

In a particular embodiment, the invention relates to the payload conjugate according to the invention or the pharmaceutical composition according to the invention for use in the diagnosis of diseases associated with elevated levels of proteoglycans on malignant cells, in particular wherein the proteoglycans are chondroitin-4-sulfate, chondroitin-6-sulfate and/or keratan sulfate.

That is, the compounds of the invention, in particular when conjugated to an imaging agent, may be used in the diagnosis of medical conditions associated with elevated levels of proteoglycans on malignant cells. Disclosed herein are compounds that bind specifically to chondroitin-4-sulfate, chondroitin-6-sulfate and/or keratan sulfate. Thus, in certain embodiments, the invention relates to payload conjugates that specifically bind to C4S and that can be used in the diagnosis of diseases associated with elevated levels of C4S on malignant cells. In other embodiments, the invention relates to payload conjugates that specifically bind to C6S and that can be used in the diagnosis of diseases associated with elevated levels of C6S on malignant cells. In further embodiments, the invention relates to payload conjugates that specifically bind to KS and that can be used in the diagnosis of diseases associated with elevated levels of KS on malignant cells.

It is known in the art that Morquio syndrome is characterized by the accumulation of glycosaminoglycans, in particular C6S and KS, in the subject's body. Thus, the payload conjugates of the invention may be used in the diagnosis of Morquio syndrome, in particular wherein the payload conjugates specifically to C4S and/or C6S.

Accordingly, in a particular embodiment, the invention relates to the payload conjugate according to the invention or pharmaceutical composition according to the invention for use in the diagnosis of Morquio syndrome.

## Synthesis of the compounds of the present invention

Compounds of the present invention were synthesized using Automated Peptide Synthesizers, either Activo-P11 (Activotec) or Microwave Liberty Blue (CEM). The synthesis was performed by standard Fmoc solid-phase chemistry on Rink Amide resin (capacity: 0.18 - 0.22 mmol/g, scale: 0.05 - 0.2 mmol) using DMF (N,N-dimethylformamide) as solvent.

For compounds according to scheme (I) with two P peptide moieties, the first steps of coupling were performed according to scheme (IIIa):

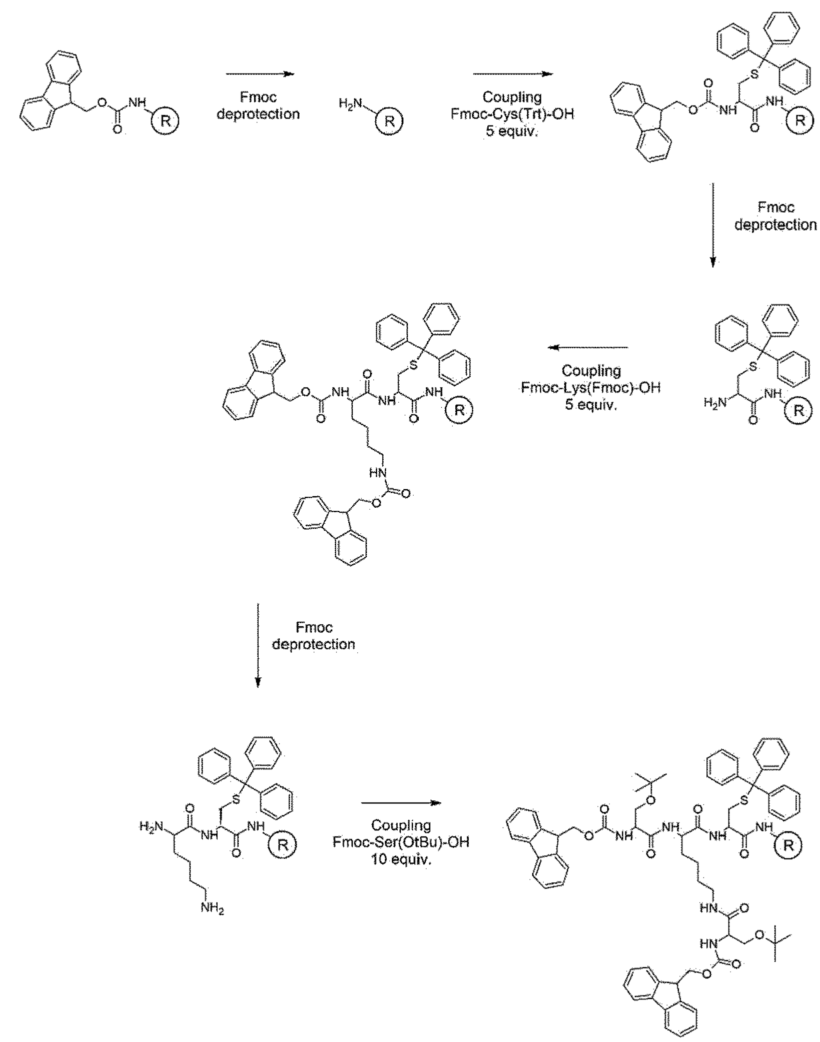


(IIIa)

wherein R in a circle represents solid support resin.

Couplings with Activo-P11 were carried out once for each amino acid (single coupling, 1-hour, room temperature) by using hexafluorophosphate benzotriazole tetramethyl uronium (HBTU) and N,N-diisopropylethylamine (DIPEA) in the conditions as follows: 5-10 equiv. of 0.5 M amino acid (typically, 5 equivalents in the case of Cys or Lys building blocks, 10 equivalents in the case of Ser building block, as well as all further couplings), 4.8-9.6 equiv. respectively of 0.48 M HBTU, 15-30 equiv. respectively of 1 M DIPEA. Couplings with Microwave Liberty Blue are carried out differently for each amino acid (single or double couplings, 4 or 10 minutes, 50° C or 90° C) by using diisopropylcarbodiimide (DIC), a carbodiimide-type coupling reagent, and coupling additive ethyl cyano(hydroxyimino)acetate, known under commercial name of Oxyma Pure, as follows: 5-10 equiv. of 0.2 M amino acid (typically, 5 equivalents in the case of first Cys coupling or second Lys coupling, 10 equivalents in the case of a third coupling of Ser building block, as well as all further couplings), 5-10 equiv. respectively of 0.25 M DIC, 5-10 equiv. respectively of 0.5 M Oxyma Pure.

Fluorenylmethyloxycarbonyl (Fmoc) protecting groups were removed using either 20% v/v piperidine in DMF or 10% w/v piperazine in N-methyl-2-pyrrolidone (NMP).

For compounds according to scheme (I) with three P peptide moieties, the first steps of coupling were performed according to scheme (IIIb):

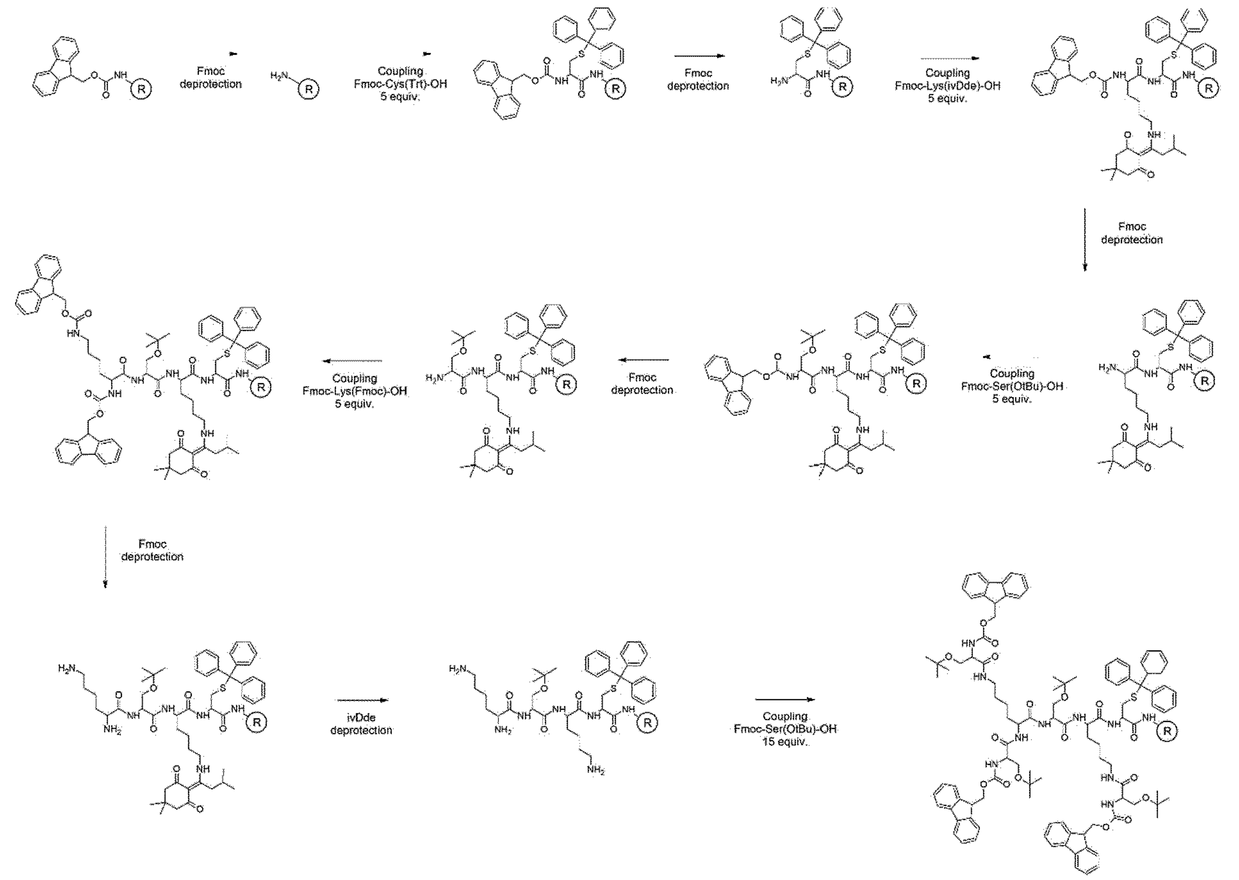


(IIIb)

wherein R represents solid support resin.

Couplings with Activo-P11 were carried out once for each amino acid (single coupling, 1-hour, room temperature in the conditions as follows: 5-15 equiv. of 0.5 M amino acid (typically, 5 equivalents except for the fifth coupling of Ser building block, as well as all further couplings, whereby 15 equivalents are used), 4.8-14.4 equiv. respectively of 0.48 M HBTU, 15-45 equiv. respectively of 1 M DIPEA. Couplings with Microwave Liberty Blue were carried out differently for each amino acid (single or double couplings, 4 or 10 minutes, 50° C or 90° C) as follows: 5-15 equiv. of 0.2 M amino acid (typically, 5 equivalents except for the fifth coupling of Ser building block, as well as all further couplings, whereby 15 equivalents are used), 5-15 equiv. respectively of 0.25 M DIC, 5-15 equiv. respectively of 0.5 M Oxyma Pure.

Fmoc protecting groups were removed using either 20% v/v piperidine in DMF or 10% w/v piperazine in NMP. Deprotection of side chain of Fmoc-L-Lys(ivDde) (also referred to as N-$\alpha$-Fmoc-N-$\varepsilon$-1-(4,4-dimethyl-2,6-dioxocyclohex-1-ylidene)-3-methylbutyl-L-lysine) was performed after the first 4 couplings using 2% v/v hydrazine in DMF. In certain embodiments of the present invention, Fmoc-L-Lys(Mtt), also referred to as N-$\alpha$-Fmoc-N-$\varepsilon$-4-methyltrityl-L-lysine, can be used instead of Fmoc-L-Lys(ivDde). In such case, Mtt is removed after the first 4 couplings using 1% v/v trifluoroacetic acid (TFA) in dichloromethane (DCM).

For compounds according to scheme (I) with four P peptide moieties, the first steps of coupling were performed according to scheme (IIIc):

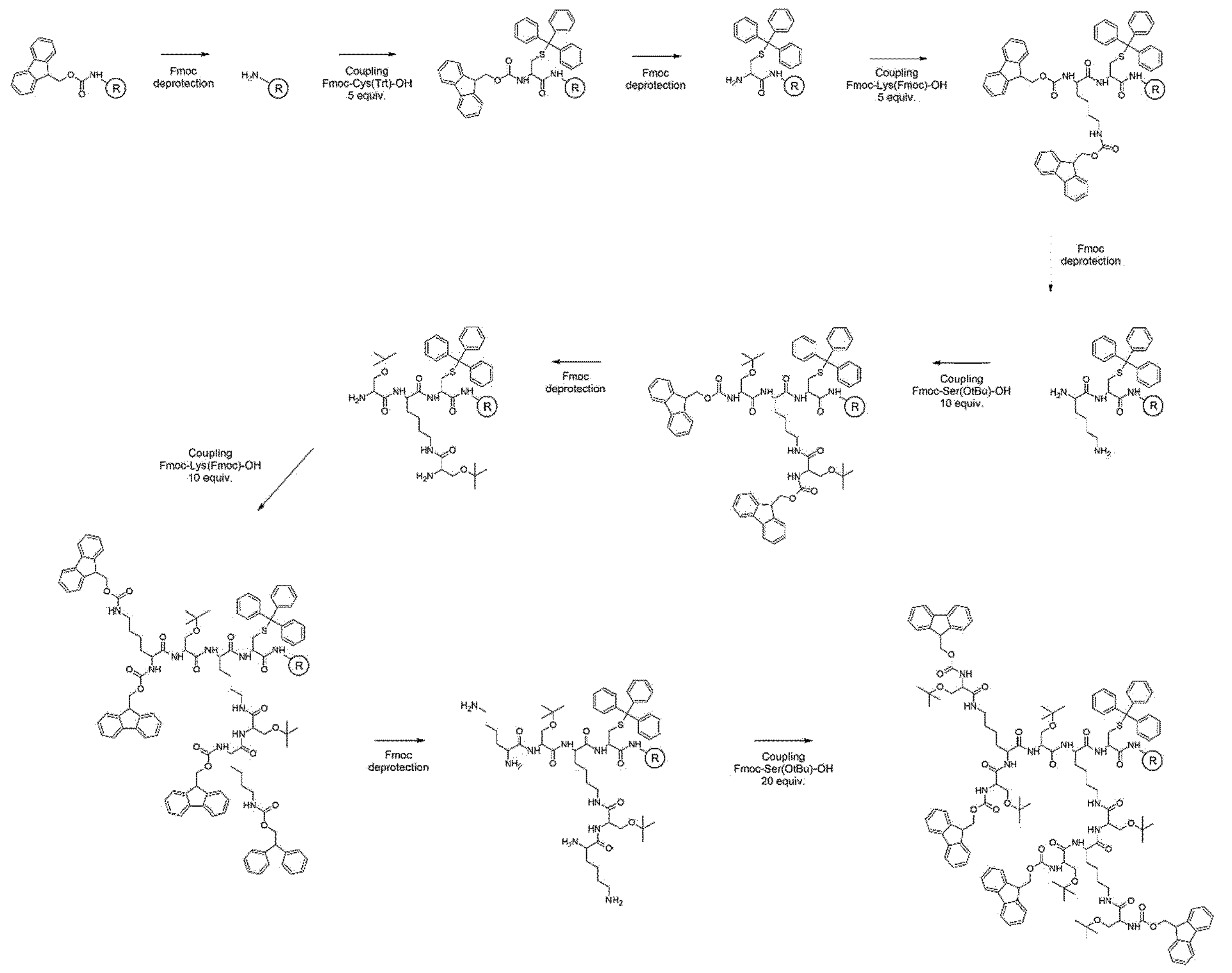


(IIIc)

wherein R represents solid support resin.

Couplings with Activo-P11 were carried out once for each amino acid (single coupling, 1-hour, room temperature) in the conditions as follows: 5-10-20 equiv. of 0.5 M amino acid (typically, first and second coupling of Cys and Lys building blocks, respectively, were carried out using 5 equivalents, third and fourth couplings of Ser and Lys building blocks, respectively, were carried out using 10 equivalents, and fifth coupling of Ser building block, as well as all further couplings, was carried out using 20 equivalents), 4.8-9.6-19.2 equiv. respectively of 0.48 M HBTU, 15-30-60 equiv. respectively of 1 M DIPEA. Couplings with Microwave Liberty Blue were carried out differently for each amino acid (single or double couplings, 4 or 10 minutes, 50° C or 90° C) as follows: 5-10-20 equiv. of 0.2 M amino acid (typically, first and second coupling of Cys and Lys building blocks, respectively, were carried out using 5 equivalents, third and fourth couplings of Ser and Lys building blocks, respectively, were carried out using 10 equivalents, and fifth coupling of Ser building block, as well as all further couplings, was carried out using 20 equivalents), 5-10-20 equiv. respectively of 0.25 M DIC, 5-10-20 equiv. respectively of 0.5 M Oxyma Pure.

Fmoc groups were removed using either 20% v/v piperidine in DMF or 10% w/v piperazine in NMP.

In the exemplary compounds of the present invention, D-histidine residue is typically attached to the branched peptides obtained according to reaction schemes (IIIa-c). In further synthetic steps, peptide chains are further built up by attachment onto said D-histidine residues by the means of solid state synthesis, as discussed herein. In the exemplary embodiments of the present invention, the following peptide sequences are used:

51: RYFvRIKYR
21: RRFvYIYRR
28: RYFvRIYRR

Peptides were acetylated with 10% v/v $Ac_2O$/ 10% v/v DIPEA / 40% v/v DMF/ 40% v/v DCM.

The so obtained peptide compounds were cleaved from the resin under shaking in reducing conditions (90% v/v TFA, 2.5% v/v water, 2.5% v/v Triisopropyl silane, 5% w/v Phenol) for 2.5 hrs at room temperature. The resin was removed by filtration, peptides were precipitated with cold diethyl ether and incubation at -20° C, and pelleted by centrifugation. These steps were repeated twice, peptides were dried at open air and stored at -20° C until purification, or purified immediately.

Peptides were dissolved in 0.5-1 ml solvent B (99.9% v/v ACN (acetonitrile) and 0.1% v/v TFA) and 3.5-4 ml solvent A (99.9% v/v H2O and 0.1% v/v TFA) and treated as follows: sonication, centrifugation, filtration of supernatant (0.45 $\mu$m filter) and injection of 4 ml. Crude peptides were purified using a RP-HPLC preparative system: flow of 35 ml-min$^{-1}$ and linear gradient, which varies from product to product, of solvent B in 25 min (Table 3). Fractions were checked for presence of the desired product using a RP-UHPLC-MS system: flow of 0.620 ml-min$^{-1}$ and linear gradient of 5-95% v/v solvent B in 4 min (Table 2), and fractions with the desired product were pooled together and lyophilized. Purity was checked using a RP-UHPLC-MS system: flow of 0.620 ml·min$^{-1}$ and linear gradient of 5-95% v/v solvent B in either 4 min or 10 min (Table 2 and Table 4).

As described in further Examples, the so-obtained peptides may be further derivatized using the reactivity of cysteine moiety. In certain Examples discussed herein, the C-terminal cysteine residue was replaced by propargylglycine residue, so that the reactivity of the triple bond comprised therein may be used for further derivatization by using the click chemistry protocol.

**Analysis of peptides, reactions and HPLC fractions**

Crude peptides, reaction mixtures and fractions from HPLC purification were analyzed using a reversed-phase UHPLC column placed in an Agilent LC-MS system: flow rate of 0.620 ml-min$^{-1}$ and linear gradient of 5-95% v/v solvent B in 4 min (Table 2).

***Table 2. Gradient used for the LCMS analysis of peptides, reactions and HPLC fractions.*** *Solvents: A, 99.97% v/v $H_2O$ and 0.03% v/v TFA; B, 99.97% v/v MeCN and 0.03% v/v TFA.*

| Time (min) | Flow (ml/min) | Solvent A (%) | Solvent B (%) |
|---|---|---|---|
| 0.00 | 0.620 | 95 | 5 |
| 0.10 | 0.620 | 95 | 5 |
| 4.10 | 0.620 | 5 | 95 |
| 6.90 | 0.620 | 5 | 95 |
| 7.00 | 0.620 | 95 | 5 |

Crude peptides and reaction mixtures were purified using a reversed-phase HPLC column placed in a Waters preparative system: flow rate of 35 ml-min$^{-1}$ and linear gradient, which varies from product to product, of solvent B in 25 min (Table 3).

***Table 3. Gradient used for the HPLC purification of peptides and reactions.*** *Solvents: A, 99.9% v/v $H_2O$ and 0.1% v/v TFA; B, 99.9% v/v MeCN and 0.1% v/v TFA.*

| Time (min) | Flow (ml/min) | Solvent A (%) | Solvent B (%) |
|---|---|---|---|
| 0 | 35 | XX | XX |
| 25 | 35 | XX | XX |
| 30 | 35 | 2 | 98 |
| 31 | 35 | 2 | 98 |
| 36 | 0 | 90 | 10 |

Purity of lyophilized purified compounds was assessed using a reversed-phase UHPLC column part of an LC-MS system: flow rate of 0.620 ml-min$^{-1}$ and linear gradient of 5-95% v/v solvent B in 4 min (Table 2) or 10 min (Table 4)

***Table 4. Gradient used for the LC-MS purity analysis of lyophilized final products.*** *Solvents: A, 99.97% v/v H2O and 0.03% v/v TFA; B, 99.97% v/v MeCN and 0.03% v/v TFA.*

| Time (min) | Flow (ml/min) | Solvent A (%) | Solvent B (%) |
|---|---|---|---|
| 0.00 | 0.620 | 95 | 5 |
| 0.10 | 0.620 | 95 | 5 |
| 10.10 | 0.620 | 5 | 95 |
| 12.90 | 0.620 | 5 | 95 |
| 13.00 | 0.620 | 95 | 5 |

**Preparative examples**

**Preparative example 1**

The following compounds were prepared according to the general method described above:

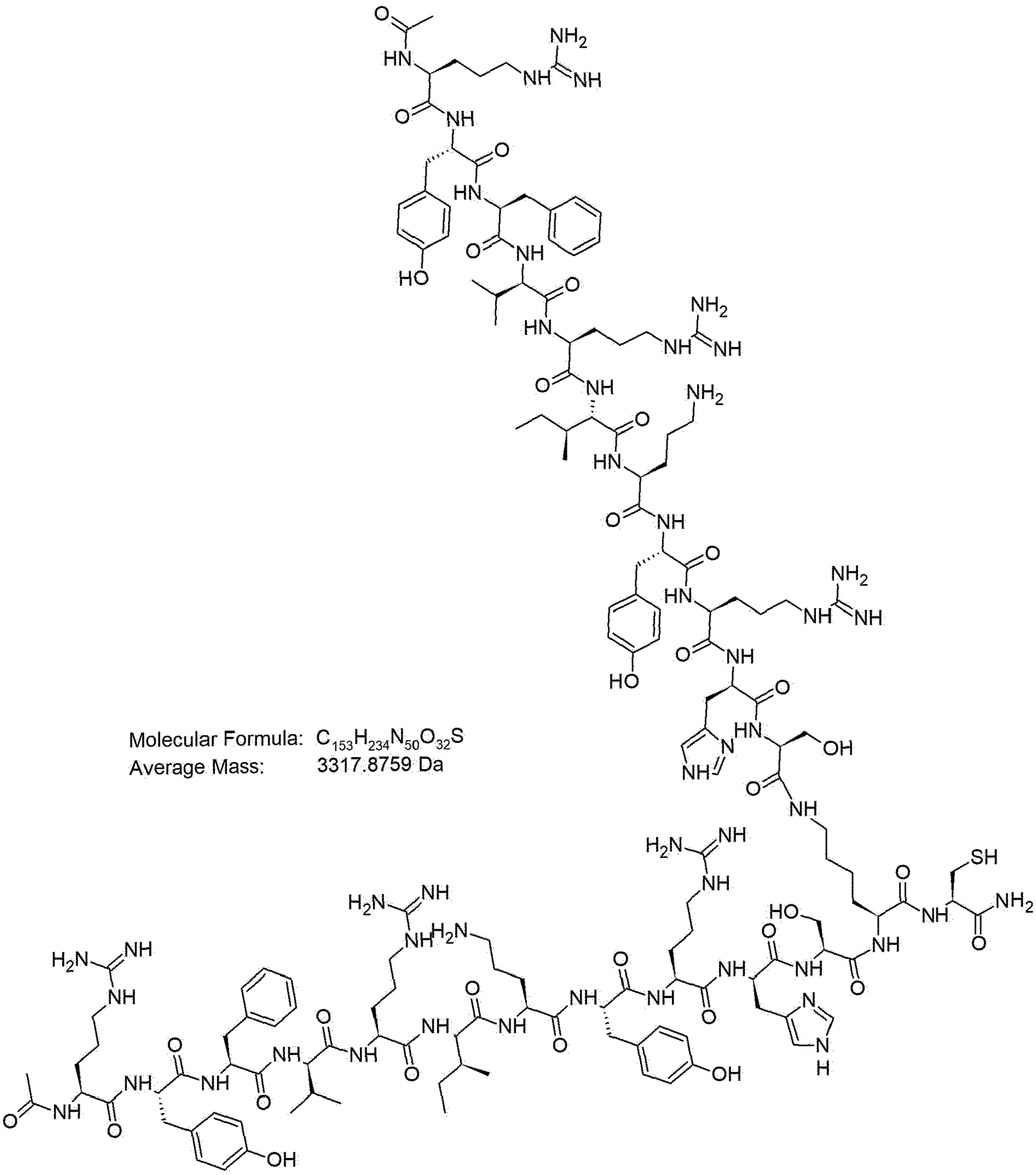

Molecular Formula: $C_{153}H_{234}N_{50}O_{32}S$
Average Mass: 3317.8759 Da

**(51-hS)$_2$KC**

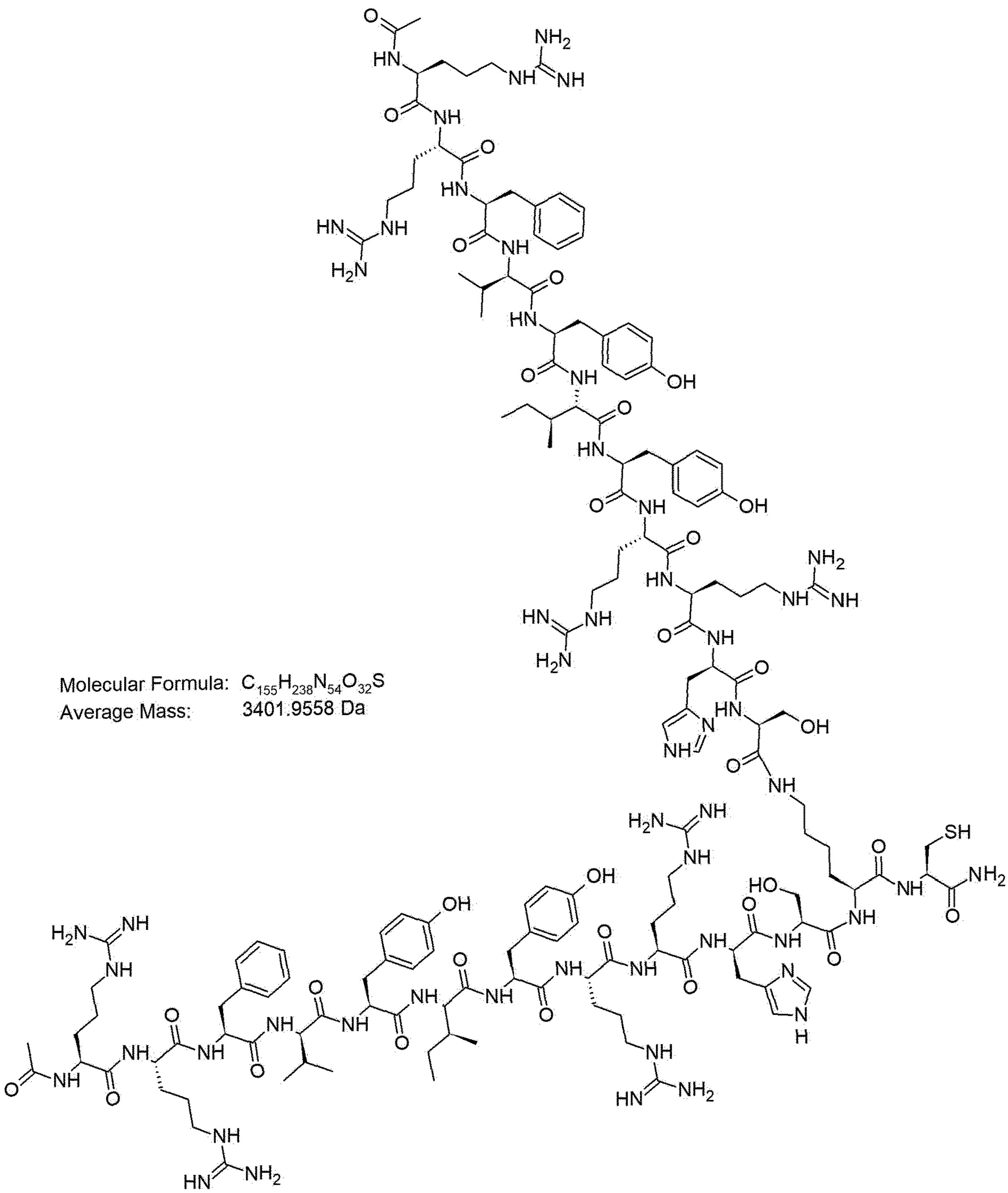

Molecular Formula: $C_{155}H_{238}N_{54}O_{32}S$
Average Mass: 3401.9558 Da

**$(21\text{-hS})_2$KC**

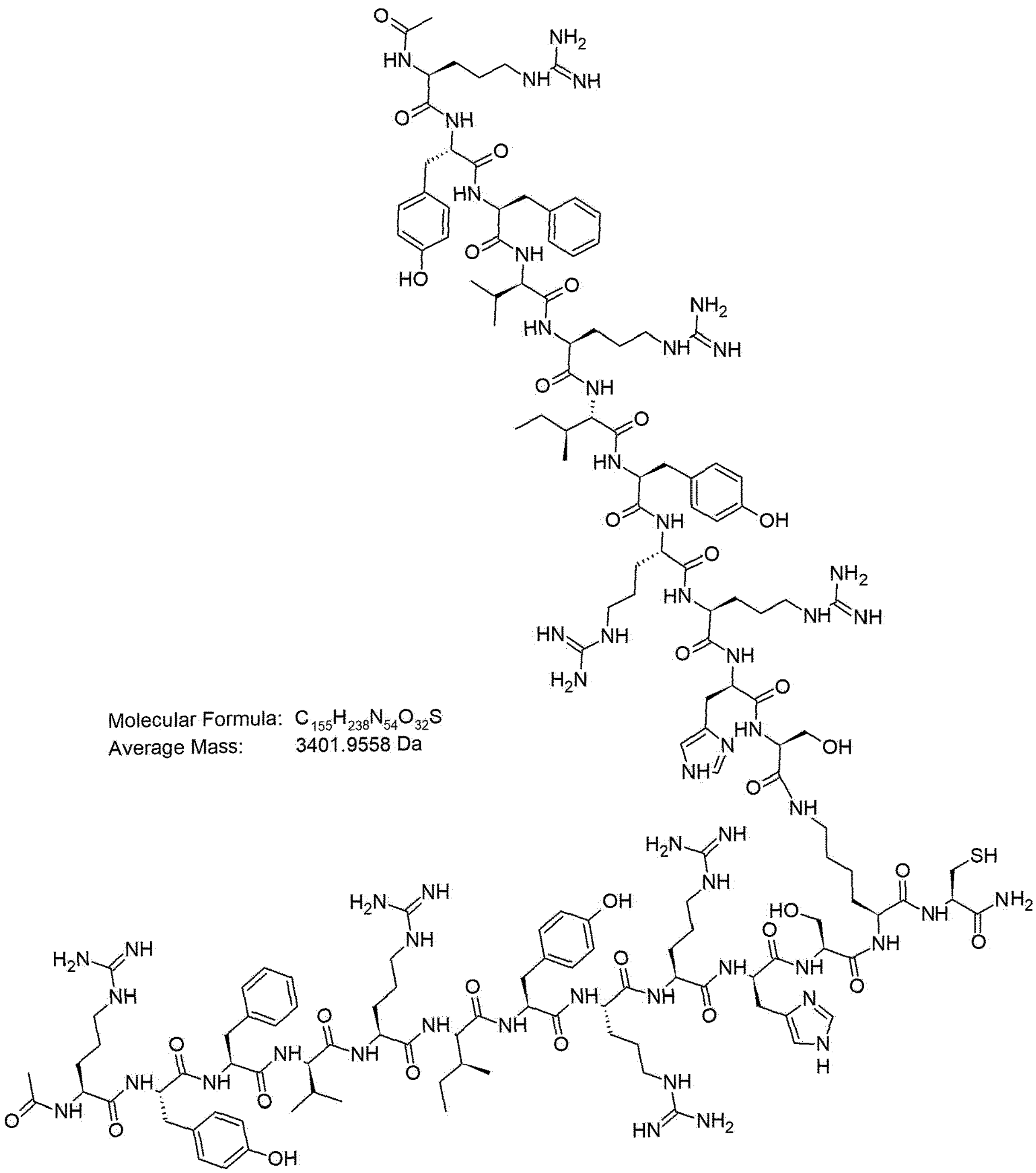

Molecular Formula: $C_{155}H_{238}N_{54}O_{32}S$
Average Mass: 3401.9558 Da

**(28-hS)$_2$KC**

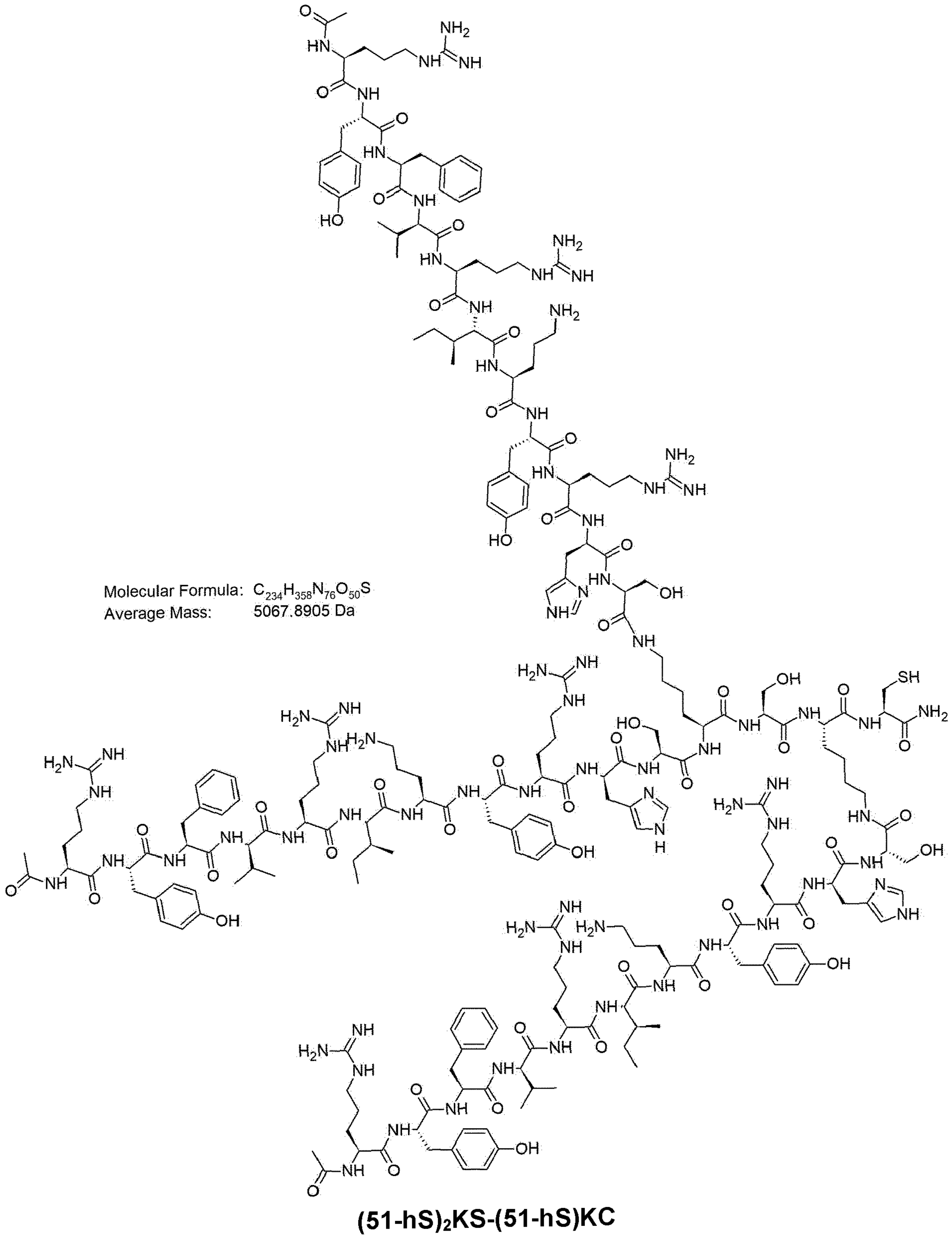

Molecular Formula: $C_{234}H_{358}N_{76}O_{50}S$
Average Mass: 5067.8905 Da

**(51-hS)$_2$KS-(51-hS)KC**

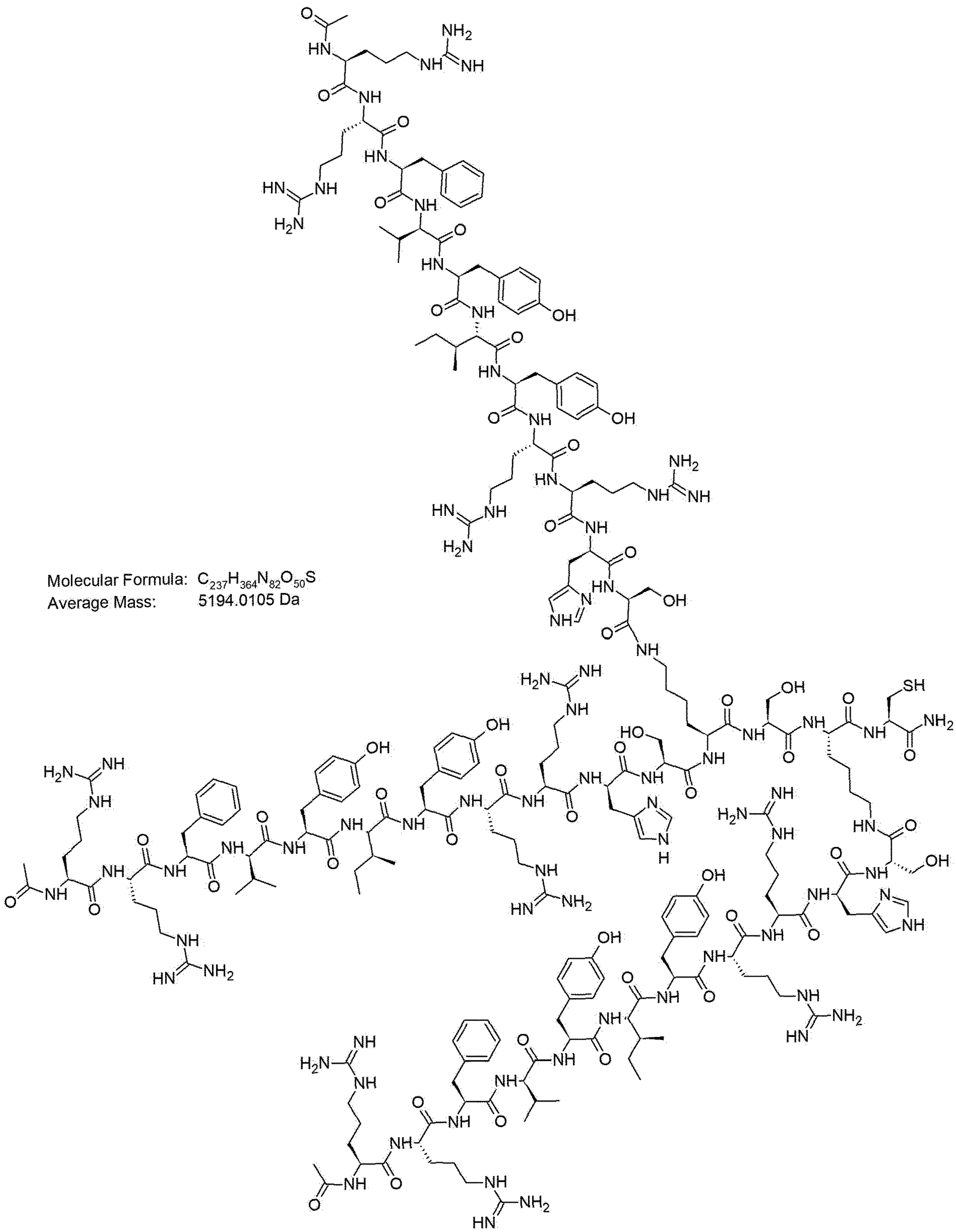

Molecular Formula: $C_{237}H_{364}N_{82}O_{50}S$
Average Mass: 5194.0105 Da

**(21-hS)$_2$KS-(21-hS)KC**

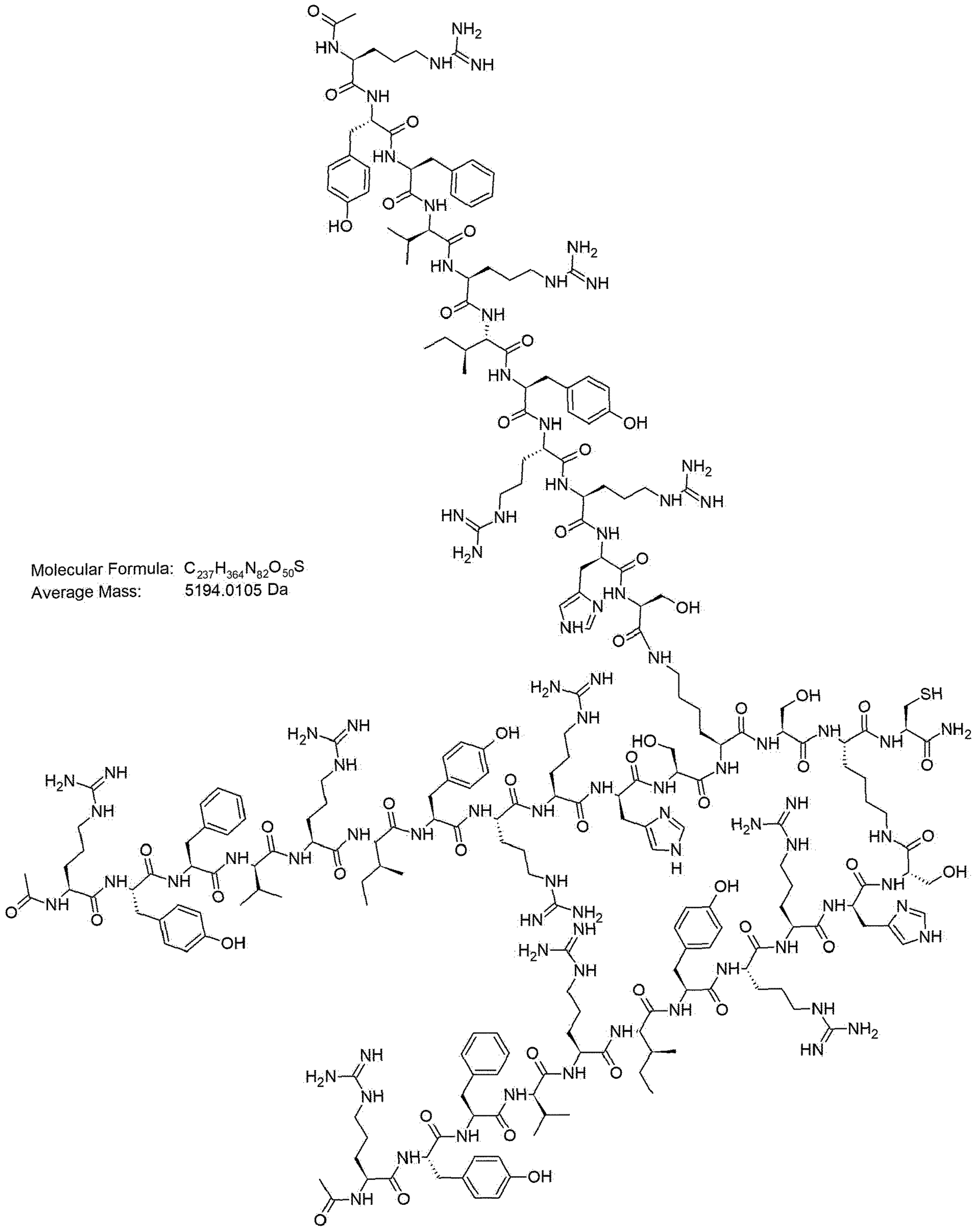

Molecular Formula: $C_{237}H_{364}N_{82}O_{50}S$
Average Mass: 5194.0105 Da

**(28-hS)$_2$KS-(28-hS)KC**

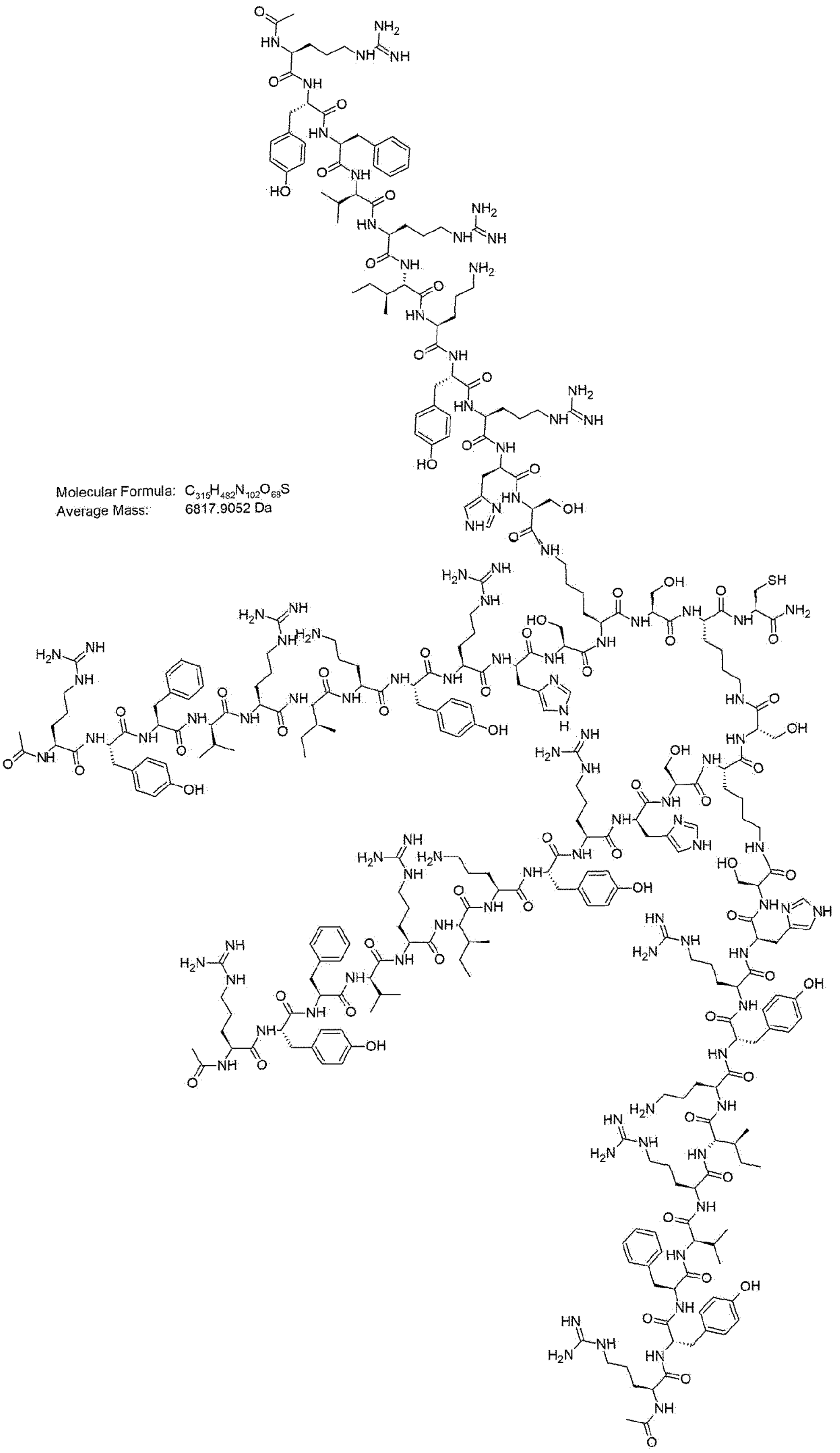

Molecular Formula: $C_{315}H_{482}N_{102}O_{68}S$
Average Mass: 6817.9052 Da

**[(51-hS)$_2$KS]$_2$KC**

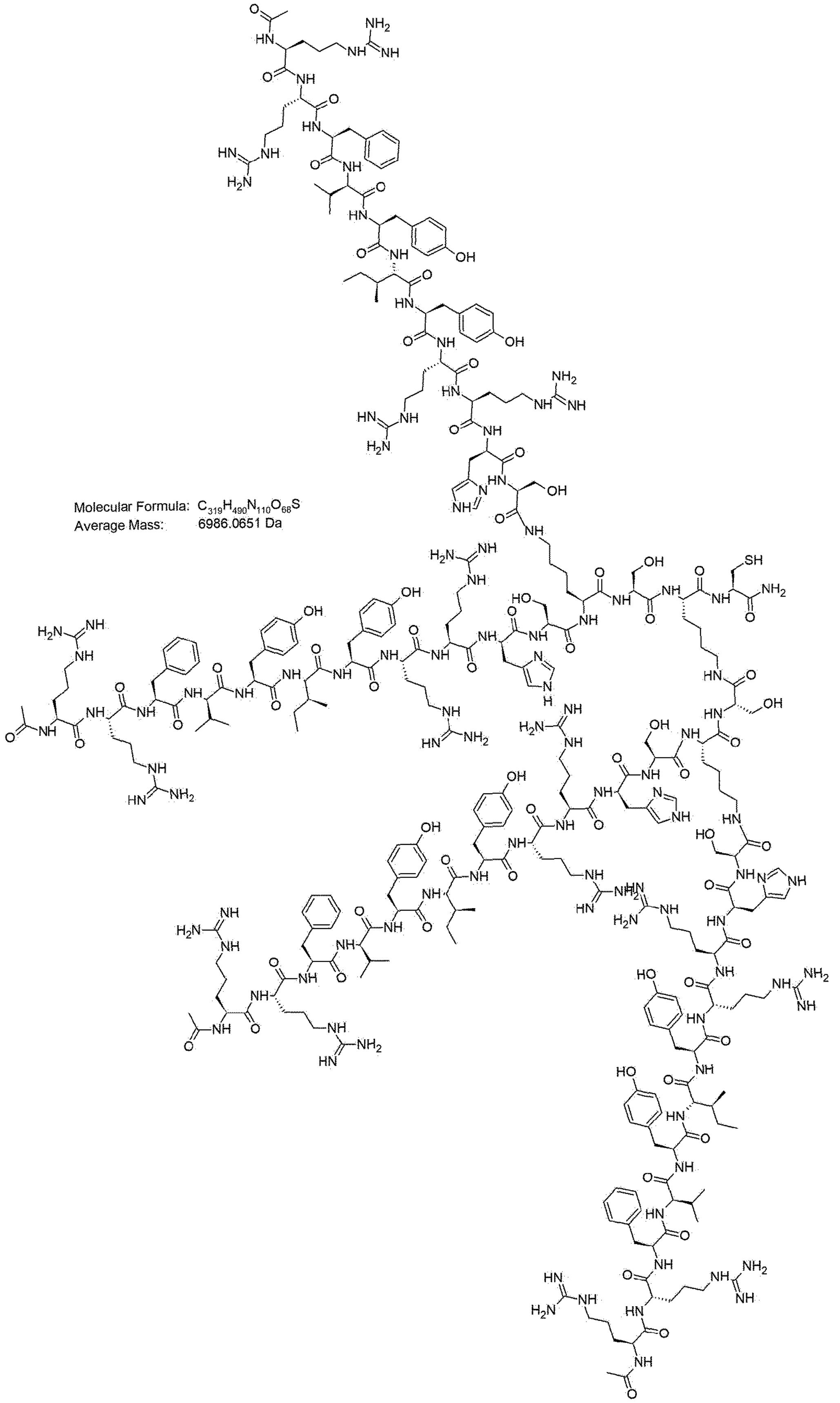

Molecular Formula: $C_{319}H_{490}N_{110}O_{68}S$
Average Mass: 6986.0651 Da

**[(21-hS)$_2$KS]$_2$KC**

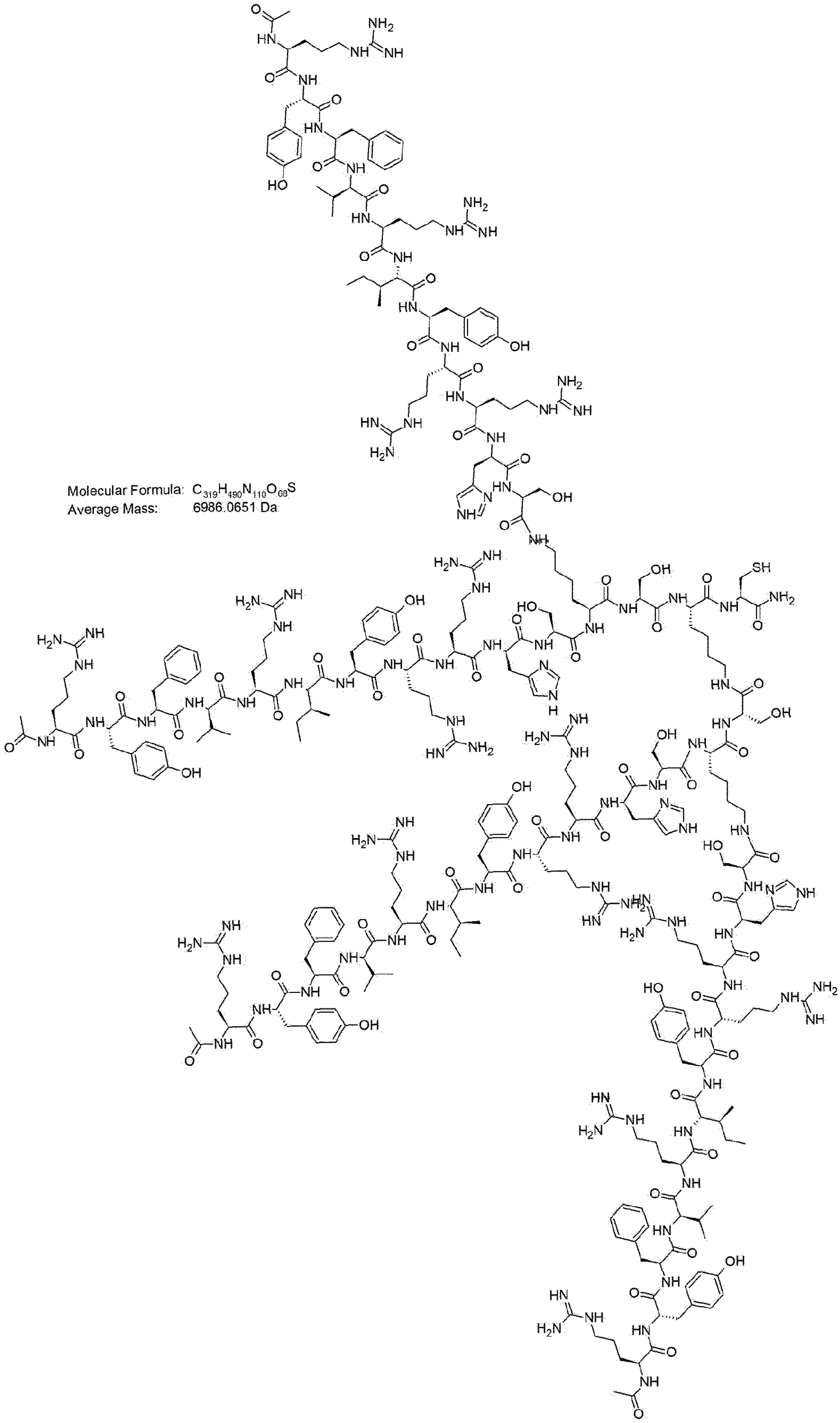

Molecular Formula: $C_{319}H_{490}N_{110}O_{68}S$
Average Mass: 6986.0651 Da

**[(28-hS)$_2$KS]$_2$KC**

The peptide compounds were analyzed by LC-MS according to the general methods described herein. The LC-Ms analysis of instant peptides is summarized in Table 5. Briefly, batch powders (if available) have been dissolved with solvent A. Alternatively, stock solutions (if available) have been diluted with solvent A. Purity assessed using a reversed-phase UHPLC column in an LC-MS system: flow rate of 0.620 ml-min$^{-1}$ and linear gradient of 5-95% v/v solvent B in 10 min (Table 4).

Table 4.1. Analysis of prepared peptides

| Compound name | Batch no. | Retention time (min) | Purity (%) | Average mass (Da) | Observed m/z | Calculated m/z |
|---|---|---|---|---|---|---|
| **(51-hS)$_2$KC** | D-043 | 2.954 | 98.166 | 3317.9 | 664.6 - 830.4 - 1106.8 | 664.6 (z=5) - 830.5 (z=4) - 1107.0 (z=3) |
| **(21-hS)$_2$KC** (A) | C-005 (18S003) | 3.188 | 97.749 | 3402.0 | 681.0 - 851.3 -1135.6 | 681.4 - 851.5 - 1135.0 |
| **(28-hS)$_2$KC** (B) | B-165 | 3.025 | 69.624 | 3402.0 | 681.4 - 851.4 -1134.8 | 681.4 - 851.5 - 1135.0 |
| **(51-hS)$_2$KS-(51-hS)KC** (C) | D-055 (19S102) | 3.322 | 91.259 | 5067.9 | 845-4 - 1014.3 1267.6 | 845.7 (z=-) - 1014.6 (z=-) - 1268.0 (z=4) |
| **(21-hS)$_2$KS-(21-hS)KC** | C-073 | 3.175 | 93.760 | 5194.0 | 743-0 - 866-7 - 1039-8 - 1299.4 | 743-0 - 866-7 - 1039-8-1299.5 |
| **(28-hS)$_2$KS-(28-hS)KC** | C-095 | 3.228 | 97.066 | 5194.0 | 743-0 - 866-6 - 1039-8 - 1299.4 | 743-0 - 866-7 - 1039-8-1299.5 |
| **[(51-hS)$_2$KS]$_2$KC** | D-141 | 3.272 | 92.331 | 6817.9 | 758-6 - 853-2 - 975.0 - 1137.2 -1364.6 | 758.6 (z=-) - 853.2 (z=-) - 975.0 (z=-) - 1137.3 (z=6) - 1364.6 (z=5) |
| **[(21-hS)$_2$KS]$_2$KC** | D-059 | 3.252 | 92.430 | 6986.1 | 777-3 - 874-2 - 999-0 - 1165.3 -1398.2 | 777-2 - 874-3 - 999-0 - 1165.4 -1398.2 |
| **[(28-hS)$_2$KS]$_2$KC** | D-060 | 3.329 | 95.157 | 6986.1 | 777-2 - 874-2 | 777-2 - 874-3 |
| | | | | | - 999-0 - 1165.4 -1398.2 | - 999-0 - 1165.4 -1398.2 |

**Notes:**
(A) Sample prepared from stock solution 18S003 (10% DMSO in PBS pH 7.4) originating from batch C-005 (powder not available). Detected masses correspond to disulfide bond formation between sulfhydryl side chains of C-terminal cysteine residues of two **(21-hS)$_2$KC** peptides (dimer of a dimer).
(B) Detected masses in the remaining part of the peak (approx. 30% of the total area) correspond to sulfonated side reactions (Δmass of +80 Da) occurring during peptide cleavage from the resin. Sulfonyl side chain protecting groups of arginine residues can release sulfonyl cations that might trigger undesired modifications on Trp, Ser, Thr, Tyr or Arg. Scavengers could reduce sulfonation side reactions and phenol has been added as quenching agent.
(C)Sample prepared from stock solution 19S102 (100% DMSO) originating from batch D-055 (powder not available). Detected masses correspond to the disulfide bond formation between sulfhydryl side chains of C-terminal cysteine residues of two **(51-hS)$_2$KS-(51-hS)KC** peptides (dimer of a trimer).

**Preparative example 2 - preparation of compounds with alternative branching parts**

Possible variants have been explored in either one of the two formats and can be therefore considered common options, even if not all combinations have been effectively prepared. The linkers as discussed below comprise the structures as defined with respective SEQ IDs.

- Linker 0: reference formats

  - SEQ ID NO 1: h4-S3-K2-C1
  - SEQ ID NO 2: h6-S5-K4-S3-K2-C1

The exemplary linkers that comprise the structures according to these SEQ IDs are depicted below. It is understood herein that the linkers are connected to other parts of the molecule, herein depicted independently as -R.

SEQ ID NO:1

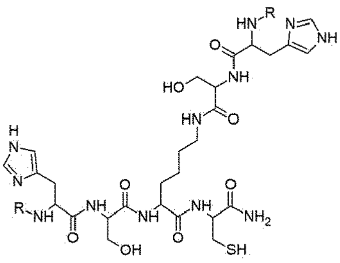

SEQ ID NO:2

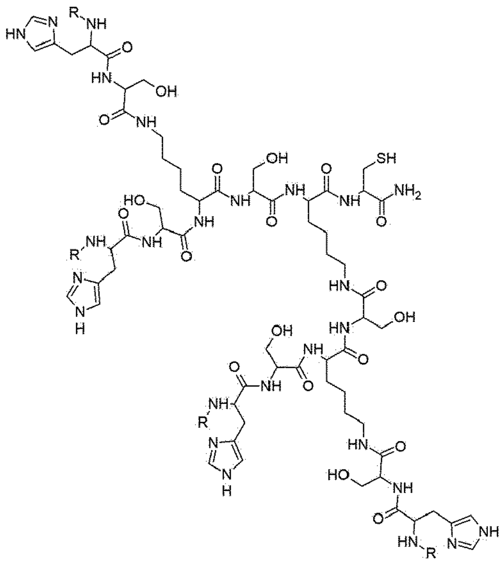

- Linker 1: additional (β)Alanine residue

  - SEQ ID NO 3: h6-S5-K4-(β)Ala-S3-K2-C1 between S3 and K4
  - SEQ ID N O 4: h6-S5-K4-S3-(β)Ala -K2-C1 between K2 and S3 The exemplary linkers that comprise the structures according to these SEQ IDs are depicted below. It is understood herein that the linkers are connected to other parts of the molecule, herein depicted independently as -R

SEQ ID NO:3

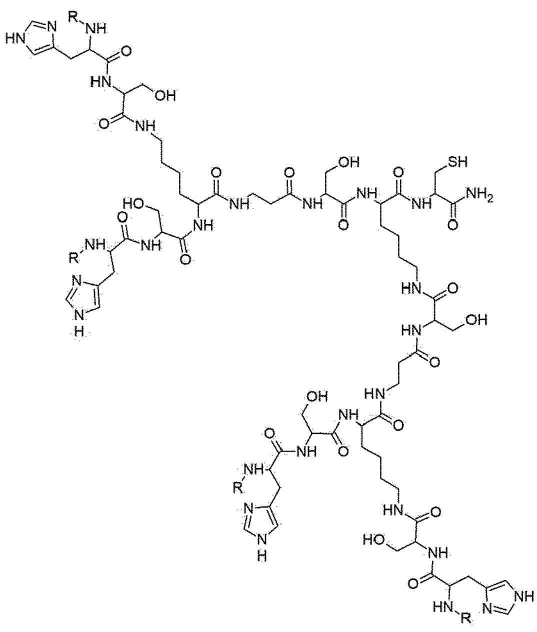

SEQ ID NO:4

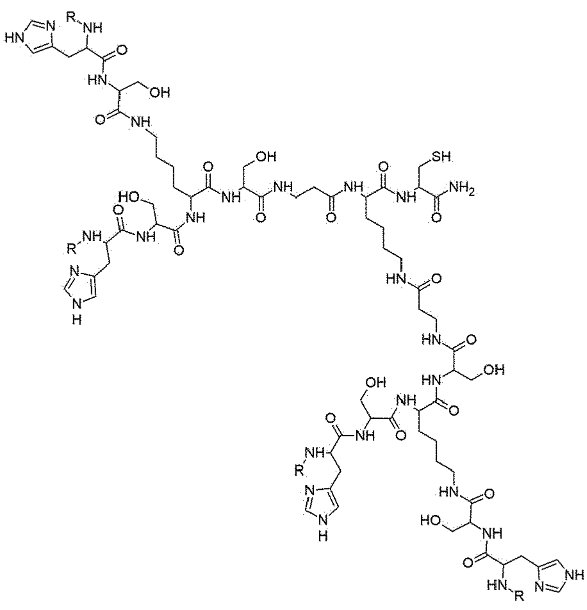

- Linker 2: additional PEG units between C1 and K2

  - SEQ ID NO 5: h6-S5-K4-S3-K2-PEG(1/5/16/36)-C1
  - SEQ ID NO 6: h4-S3-K2-PEG(1/5/16/36)-C1

The exemplary linkers that comprise the structures according to these SEQ IDs are depicted below. It is understood herein that the linkers are connected to other parts of the molecule, herein depicted independently as -R.

SEQ ID NO:5

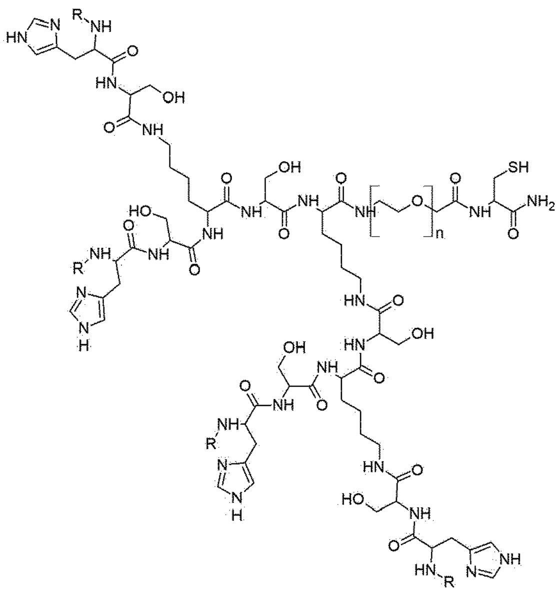

SEQ ID NO:6

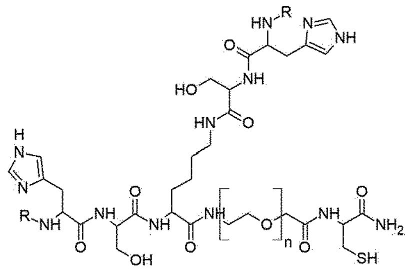

- Linker 3: PEG units replacing S3

  - SEQ ID NO 7: h6-S5-K4-PEG(1/3/5/12)-K2-C1
  - SEQ ID NO 8: h4-PEG (1/3/5/12)-K2-C1

The exemplary linkers that comprise the structures according to these SEQ IDs are depicted below. It is understood herein that the linkers are connected to other parts of the molecule, herein depicted independently as -R.

SEQ ID NO:7

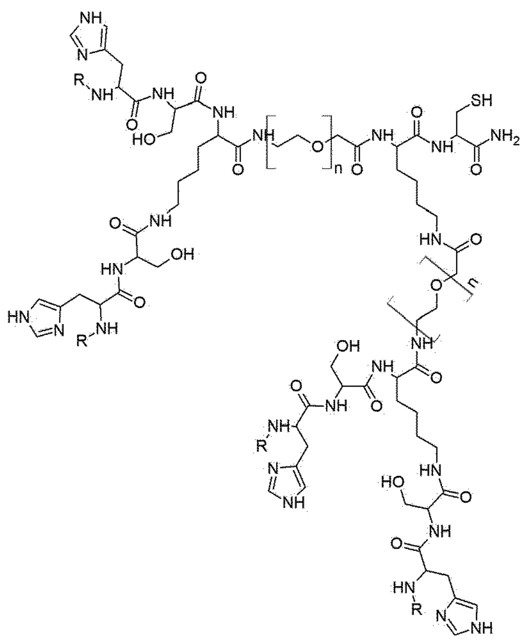

SEQ ID NO:8

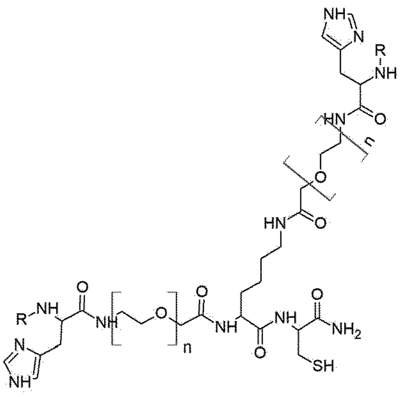

- Linker 4: symmetric divalent amino acid replacing K2 and/or K4

  - SEQ ID NO 9: h6-S5-diAA-S3-diAA-C1
  - SEQ ID NO 10: h4-S3-diAA-C1

The exemplary linkers that comprise the structures according to these SEQ IDs are depicted below. It is understood herein that the linkers are connected to other parts of the molecule, herein depicted independently as -R.

SEQ ID NO:9

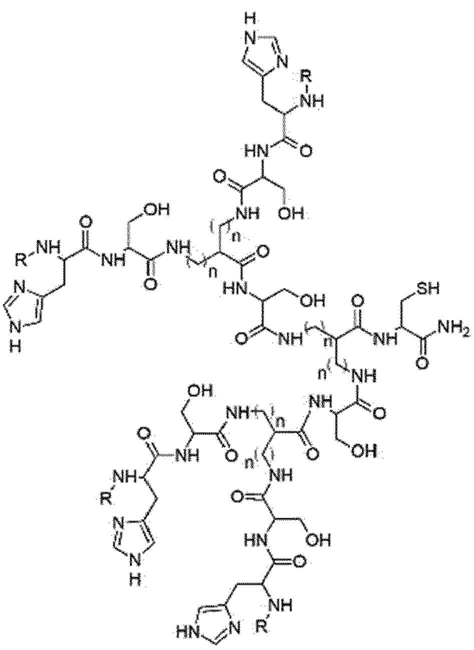

SEQ ID NO:10

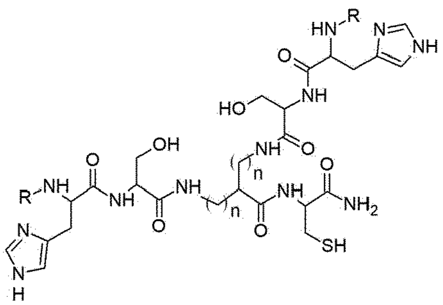

- Linker 5: Gly replacing S3 and/or S5
    - SEQ ID NO 11: h6-S5-K4-G-K2-C1
    - SEQ ID NO 12: h6-G-K4-S3-K2-C1
    - SEQ ID NO 13: h6-G-K4-G-K2-C1
    - SEQ ID NO 14: h4-G-K2-C1

The exemplary linkers that comprise the structures according to these SEQ IDs are depicted below. It is understood herein that the linkers are connected to other parts of the molecule, herein depicted independently as -R.

SEQ ID NO:11

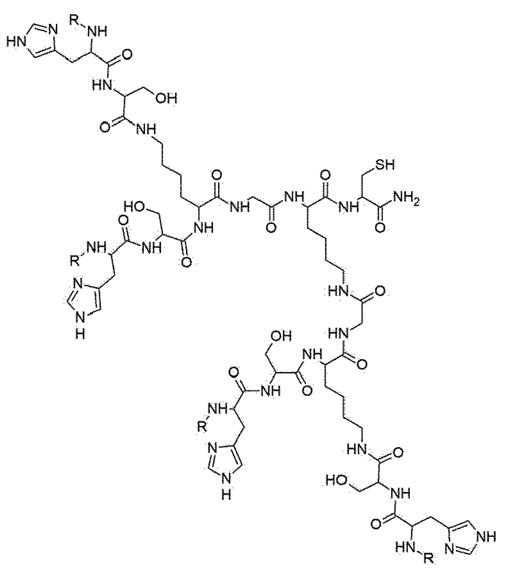

SEQ ID NO:12

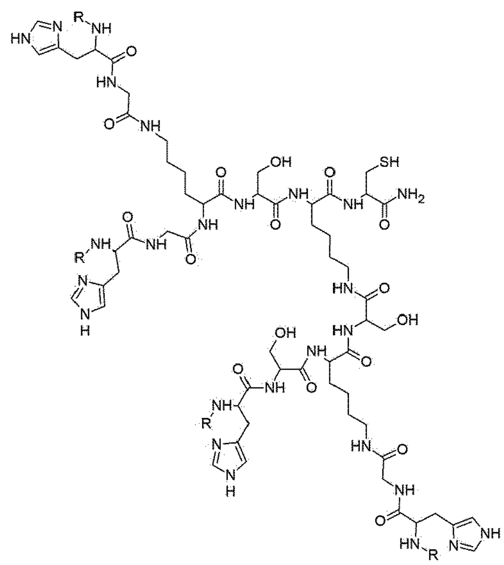

SEQ ID NO:13

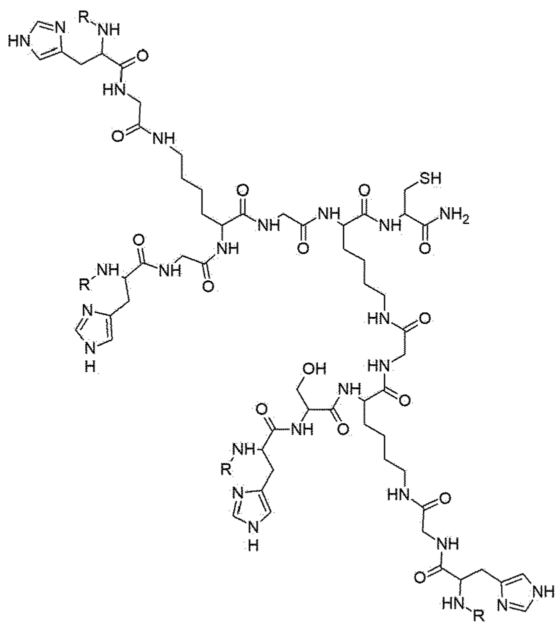

SEQ ID NO:14

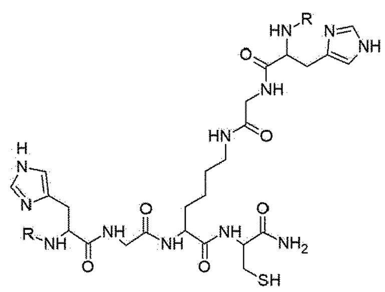

- Linker 6: symmetric divalent amino acid K2 and/or K4 + Gly replacing S3 and/or S5

- SEQ ID NO 15: h6-S5-diAA-G-diAA-C1
- SEQ ID NO 16: h6-G-diAA-S3-diAA-C1
- SEQ ID NO 17: h6-G-diAA-G-diAA-C1
- SEQ ID NO 18: h4-G-diAA-C1

The exemplary linkers that comprise the structures according to these SEQ IDs are depicted below. It is understood herein that the linkers are connected to other parts of the molecule, herein depicted independently as -R.

SEQ ID NO:15

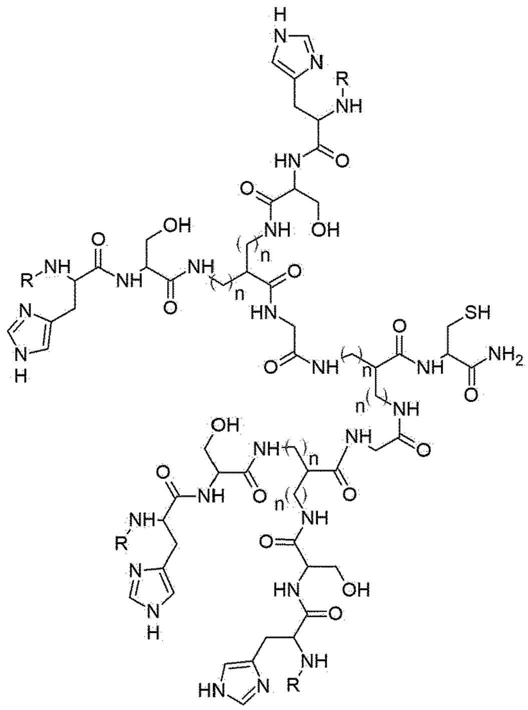

SEQ ID NO:16

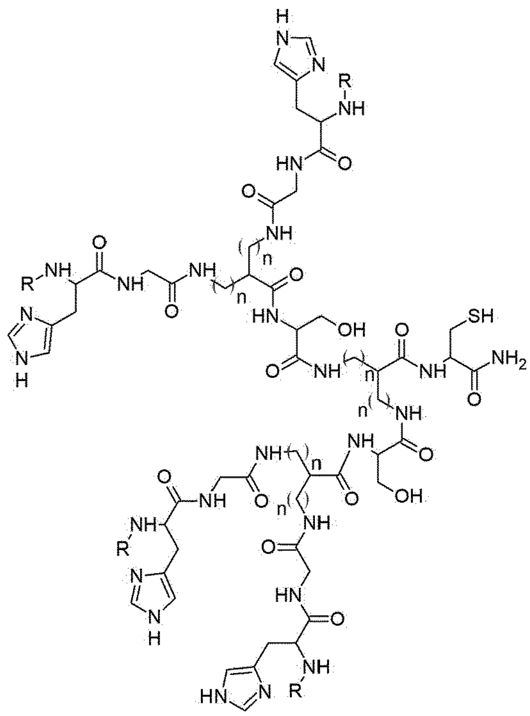

SEQ ID NO:17

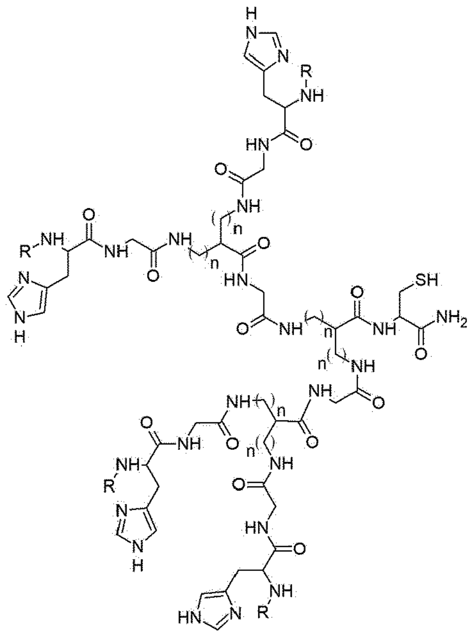

SEQ ID NO:18

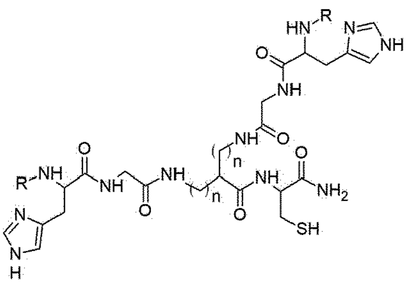

- Linker 7: Propargylglycine residue replacing C1

  - SEQ ID NO 19: h4-S3-K2-X
  - SEQ ID NO 20: h6-S5-K4-S3-K2-X

The exemplary linkers that comprise the structures according to these SEQ IDs are depicted below. It is understood herein that the linkers are connected to other parts of the molecule, herein depicted independently as -R.

SEQ ID NO:19

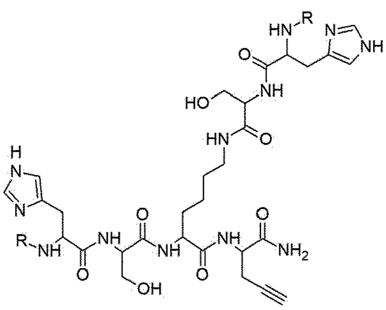

SEQ ID NO:20

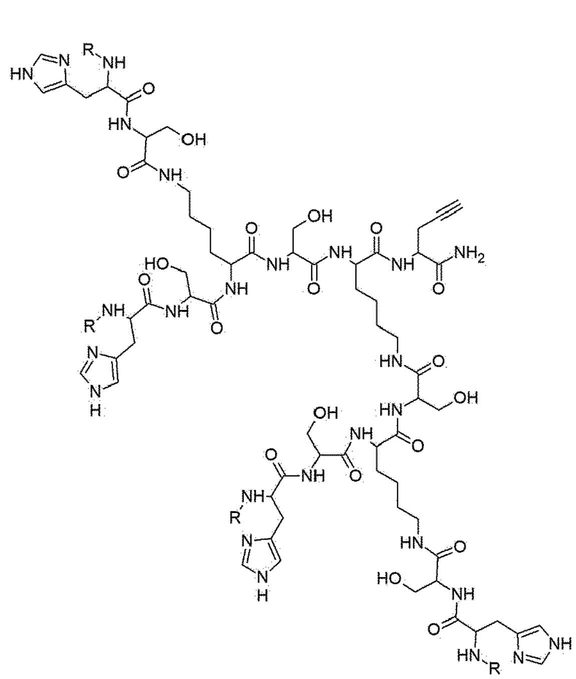

- Linker 8: Lysine biotinylated residue replacing C1

    - SEQ ID NO 21: h4-S3-K2-K(Biotin)
    - SEQ ID NO 22: h6-S5-K4-S3-K2-K(Biotin)

The exemplary linkers that comprise the structures according to these SEQ IDs are depicted below. It is understood herein that the linkers are connected to other parts of the molecule, herein depicted independently as -R.

SEQ ID NO:21

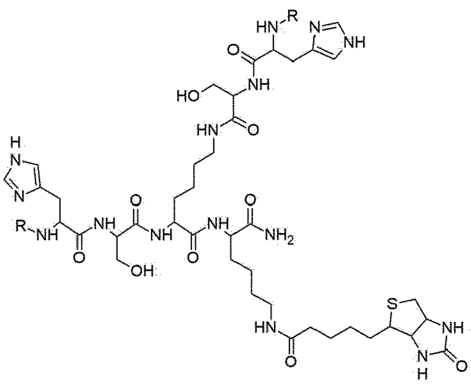

SEQ ID NO:22

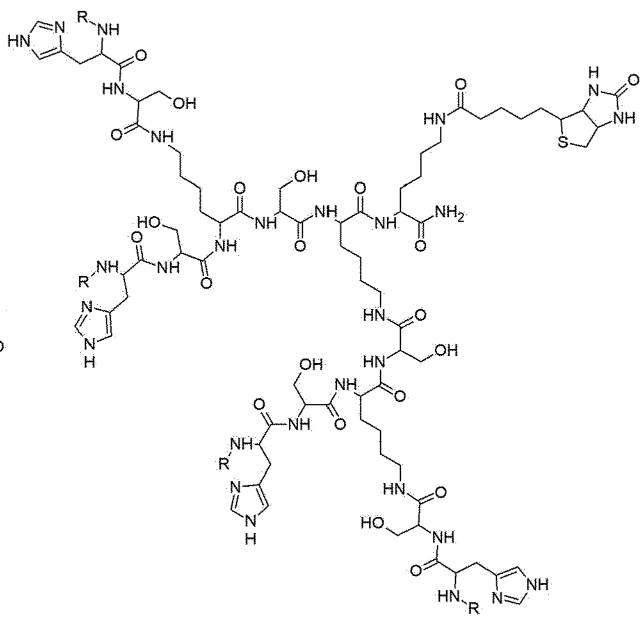

- Linker 9: h4-K2-C1 without S3 (or S5)

    - SEQ ID NO 23: h4-K2-C1
    - SEQ ID NO 24: h6-K4-S3-K2-C1

The exemplary linkers that comprise the structures according to these SEQ IDs are depicted below. It is understood herein that the linkers are connected to other parts of the molecule, herein depicted independently as -R.

SEQ ID NO:23

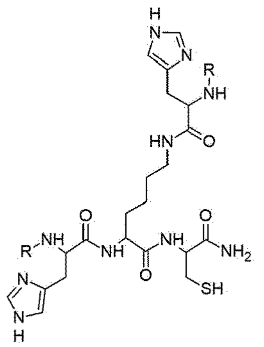

SEQ ID NO:24

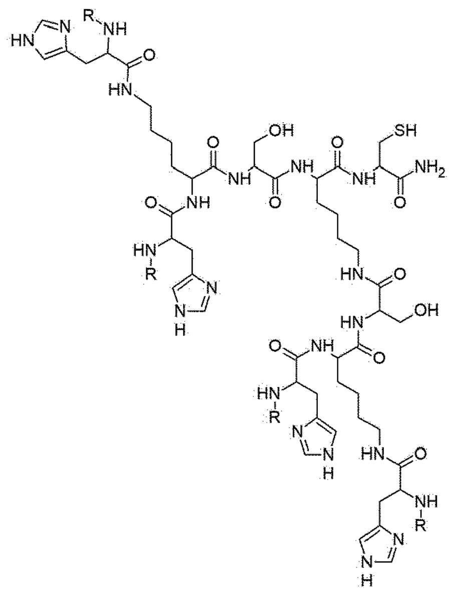

- Linker 10: without S3 (or S5) + PEG between C1 and K2

  - SEQ ID NO 25: h4-K2-PEG(1)-C1
  - SEQ ID NO 26: h6-K4-S3-K2-PEG(1)-C1

The exemplary linkers that comprise the structures according to these SEQ IDs are depicted below. It is understood herein that the linkers are connected to other parts of the molecule, herein depicted independently as -R.

SEQ ID NO:25

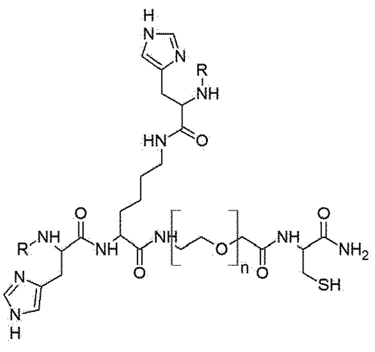

SEQ ID NO:26

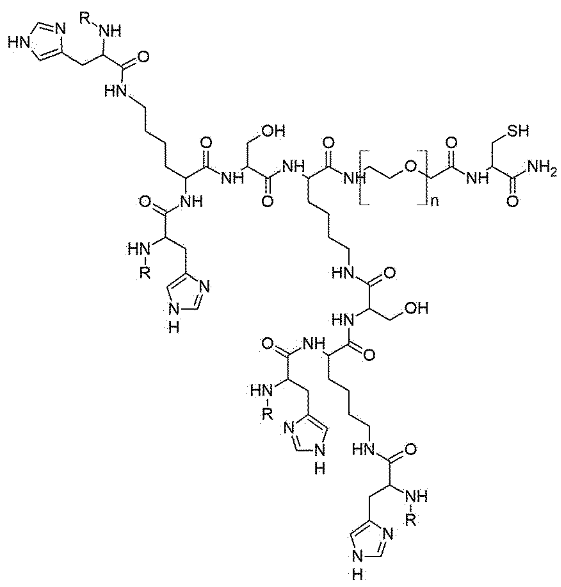

- Linker 11: additional Ser after S3 (or S5)

  - SEQ ID NO 27: h4-S-S3-K2-C1

- SEQ ID NO 28: h6-S-S5-K4-S3-K2-C1

The exemplary linkers that comprise the structures according to these SEQ IDs are depicted below. It is understood herein that the linkers are connected to other parts of the molecule, herein depicted independently as -R.

SEQ ID NO:27

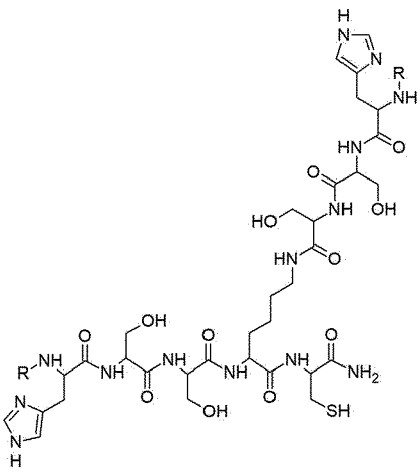

SEQ ID NO:28

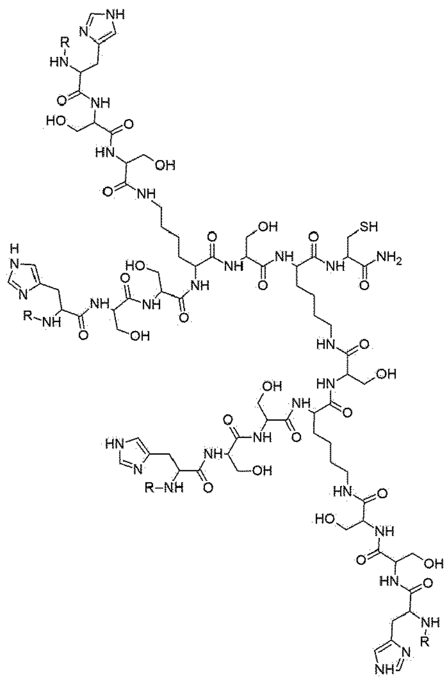

- Linker 12: double additional Ser between C1 and K2

  - SEQ ID NO 29: h4-S3-K2-S-S-C1
  - SEQ ID NO 30: h6-S5-K4-S3-K2-S-S-C1

The exemplary linkers that comprise the structures according to these SEQ IDs are depicted below. It is understood herein that the linkers are connected to other parts of the molecule, herein depicted independently as -R.

SEQ ID NO:29

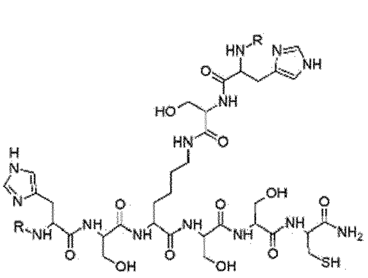

SEQ ID NO:30

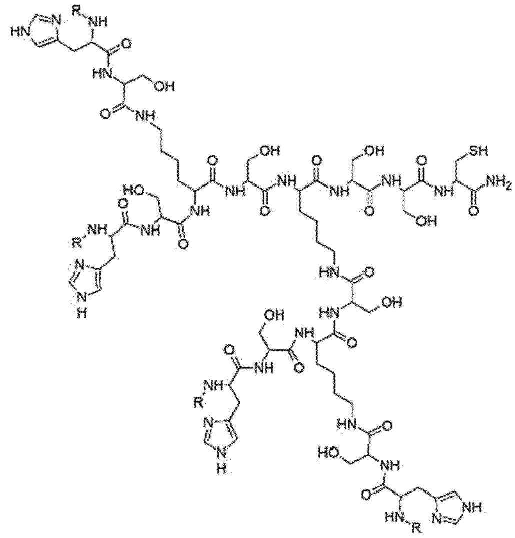

Batch powders (if available) have been dissolved with solvent A. Alternatively, stock solutions (if available) have been diluted with solvent A. Purity assessed using a reversed-phase UHPLC column in an LC-MS system: flow rate of 0.620 ml-min$^{-1}$ and linear gradient of 5-95% v/v solvent B in 10 min (Table 4).

Table 4.2. Analysis of prepared peptides

| Compound name | Batch no. | Retention time (min) | Purity (%) | Average mass (Da) | Observed m/z | Calculated m/z |
|---|---|---|---|---|---|---|
| **[(51-hS)$_2$KS]$_2$K-PEG1-C** | C-148 | 3.235 | 79.692 | 6919.02 | 769.7 - 866.0 - 989.4 - 1154.1 - 1384.3 | 769.8 (Z=9) - 865.9 (Z=8)- 989.4 (Z=7) - 1154.2 (Z=6) - 1384.8 (Z=5) |
| **[(51-hS)$_2$KS]$_2$K-PEG5-C** | C-153 | 3.224 | 80.262 | 7109.36 | 790.7 - 889.5 - 1016.5 - 1185.8 - 1422.7 | 790.9 (Z=9) -889.7 (Z=8) - 1016.6 (Z=7) - 1185.9 (Z=6) - 1422.9 (Z=5) |
| **[(51-hS)$_2$KS-PEG1]$_2$K C** | C-152 | 3.211 | 100.000 | 6846.33 | 978.7 - 1141.6 - 1370.2 | 979.0 (Z=7) - 1142.1 (Z=6) - 1370.3 (Z=5) |
| **[(51-hS)$_2$KS-PEG5]$_2$K C** | C-181 | 3.239 | 100.000 | 7226.44 | 904.1 - 1033.2 - 1205.3 - 1446.2 | 904.3 (Z=8) - 1033.3 (Z=7) - 1205.4 (Z=6) - 1446.3 (Z=5) |
| **[(51-hS)$_2$KS]$_2$KK (Biotin)** | A-082 | 3.207 | 100.000 | 7069.4 | 884.5 - 1010.7 - 1179.3 - 1414.7 | 884.7 (Z=8) - 1010.9 (Z=7) - 1179.2 (Z=6) - 1414.9 (Z=5) |
| **[(51-hS)$_2$ (diAA)S]$_2$ (diAA)C** | C-188 | 3.225 | 100.000 | 6733.7 | 842.5 - 962.5 - 1123.3 - 1347.6 | 842.7 (Z=8) -963.0 (Z=7) - 1123.3 (Z=6) - 1347.7 (Z=5) |
| **[(51-hG)$_2$KG]$_2$KC** | D-029 | 3.214 | 98.700 | 6637.6 | 738.6 - 830.8 - 949.0 - 1107.2 - 1328.4 | 738.5 (Z=9) -830.7 (Z=8) - 949.2 (Z=7) - 1107.3 (Z=6) - 1328.5 (Z=5) |
| **[(51-hG)$_2$ (diAA)G]$_2$ (diAA)C** | D-028 | 3.207 | 100.000 | 6553.4 | 820.0 - 937.0 - 1093.1 - 1311.6 | 820.2 (Z=8) - 937.2 (Z=7)- 1093.2 (Z=6) - 1311. 7 (Z=5) |
| **(51-h)$_2$KC** | C-046 | 2.982 | 100.000 | 3143.8 | 1048.7 | 1048.9 (Z=3) |

(continued)

| Compound name | Batch no. | Retention time (min) | Purity (%) | Average mass (Da) | Observed m/z | Calculated m/z |
|---|---|---|---|---|---|---|
| **$(51\text{-hSS})_2$KC** | C-056 | 2.897 | 73.259 | 3492.0 | 873.9 - 1164.7 | 874.0 (Z=4) - 1165.0 (Z=3) |
| Unless otherwise stated, diAA refers in Table 4.2 to a compound or moiety as defined as diAA herein, wherein n is 1. | | | | | | |

**Preparative Example 3 - Preparation of biotinylated derivatives of $(51\text{-hS})_2$KC, $(51\text{-hS})_2$KS-(51-hS)KC and $[(51\text{-hS})_2\text{KS}]_2$KC**

5 mg of each peptide (1 molar equivalent) $(51\text{-hS})_2$KC, $(51\text{-hS})_2$KS-(51-hS)KC and $[(51\text{-hS})_2\text{KS}]_2$KC were weighted, and dissolved in 3 ml of 1x 10 mM PBS pH 7.4. Corresponding 2 molar equivalents of Mal-Biotin, that is 1.36 mg, 0.89 mg and 0.66 mg, respectively, according to the formula:

Peptide (g) / Peptide (MW) = Peptide (moles), corresponding to 1 molar equivalent

Peptide (moles) x 2 molar equivalents = Mal-Biotin (moles)

Mal-Biotin (moles) x Mal-Biotin (MW) = Mal-Biotin (g)

were dissolved in 300 μL of glacial acetic acid and mixed with solution of each peptide in PBS. The reaction mixture was kept shaking at room temperature protected from light. The progress of the reaction was monitored by using RP-UHPLC-MS system: flow of 0.620 $ml\text{-}min^{-1}$ and linear gradient of 5-95% v/v solvent B in 4 min, and typically the reaction was completed within 24 hours. The product of each reaction was purified using a RP-HPLC preparative system: flow of 35 $ml\text{-}min^{-1}$ and linear gradient of 10-40% v/v solvent B in 25 min.

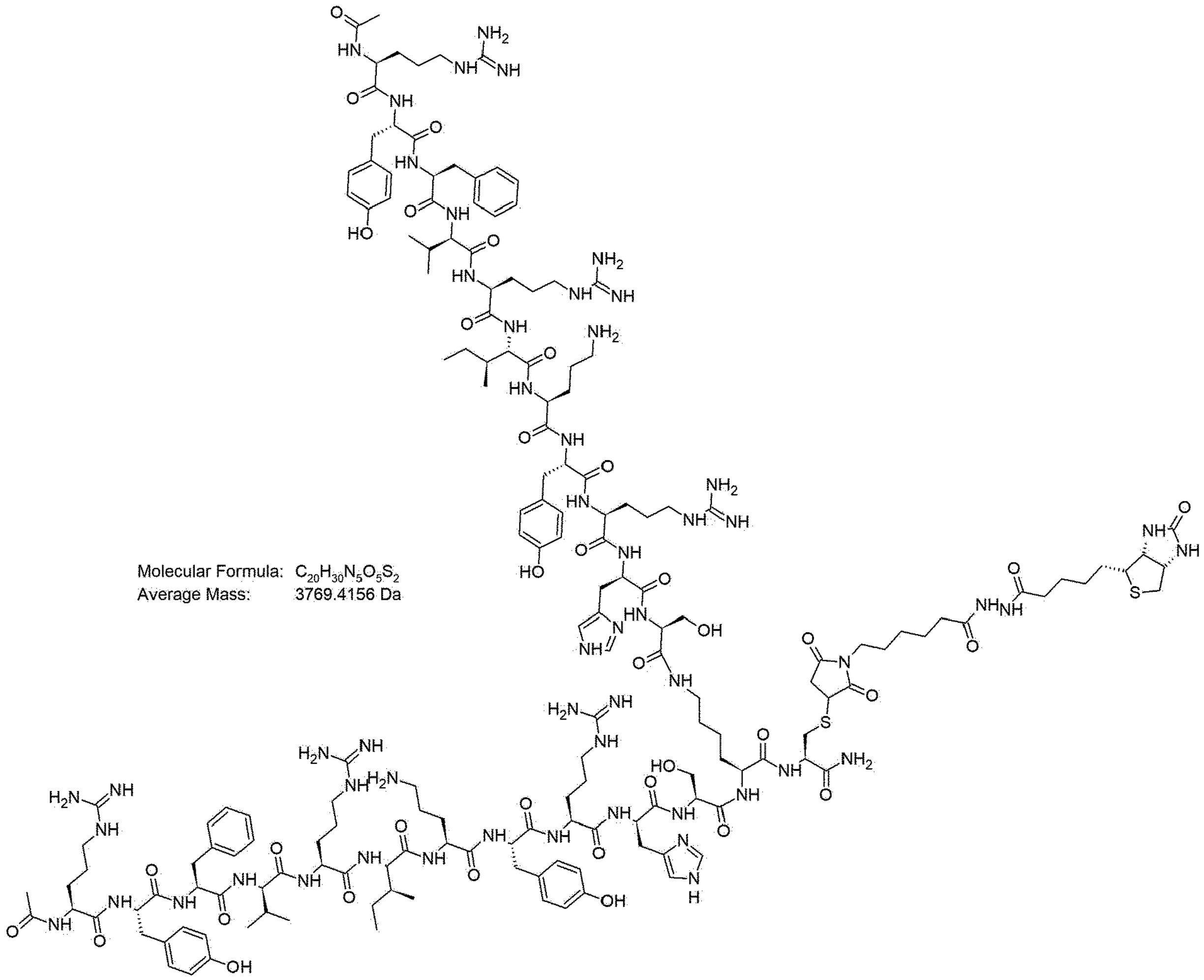

Molecular Formula: $C_{20}H_{30}N_5O_5S_2$
Average Mass: 3769.4156 Da

**(51-hS)$_2$KC-Mal-Biotin**

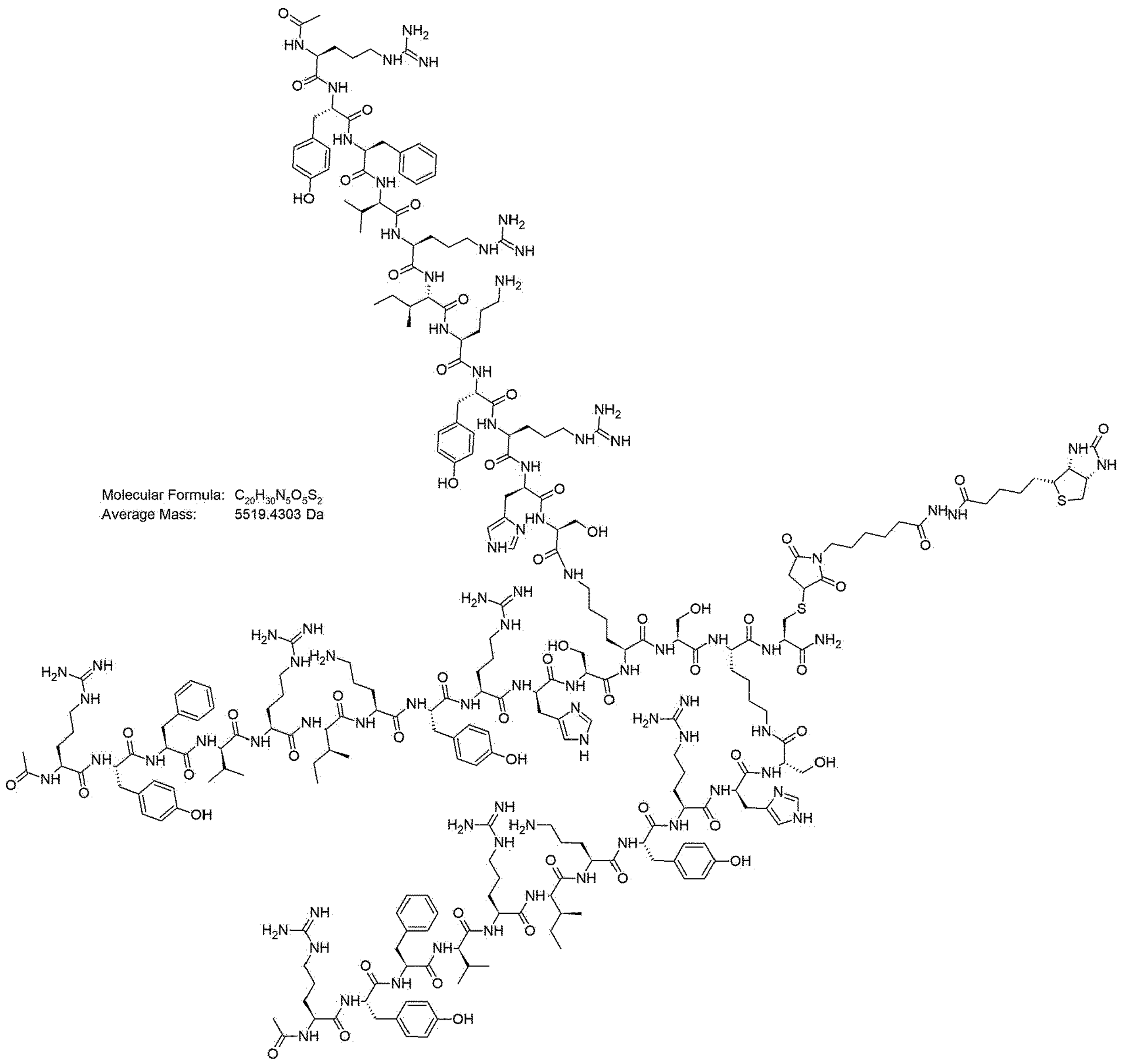

Molecular Formula: $C_{20}H_{30}N_5O_5S_2$
Average Mass: 5519.4303 Da

**(51-hS)$_2$KS-(51-hS)KC-Mal-Biotin**

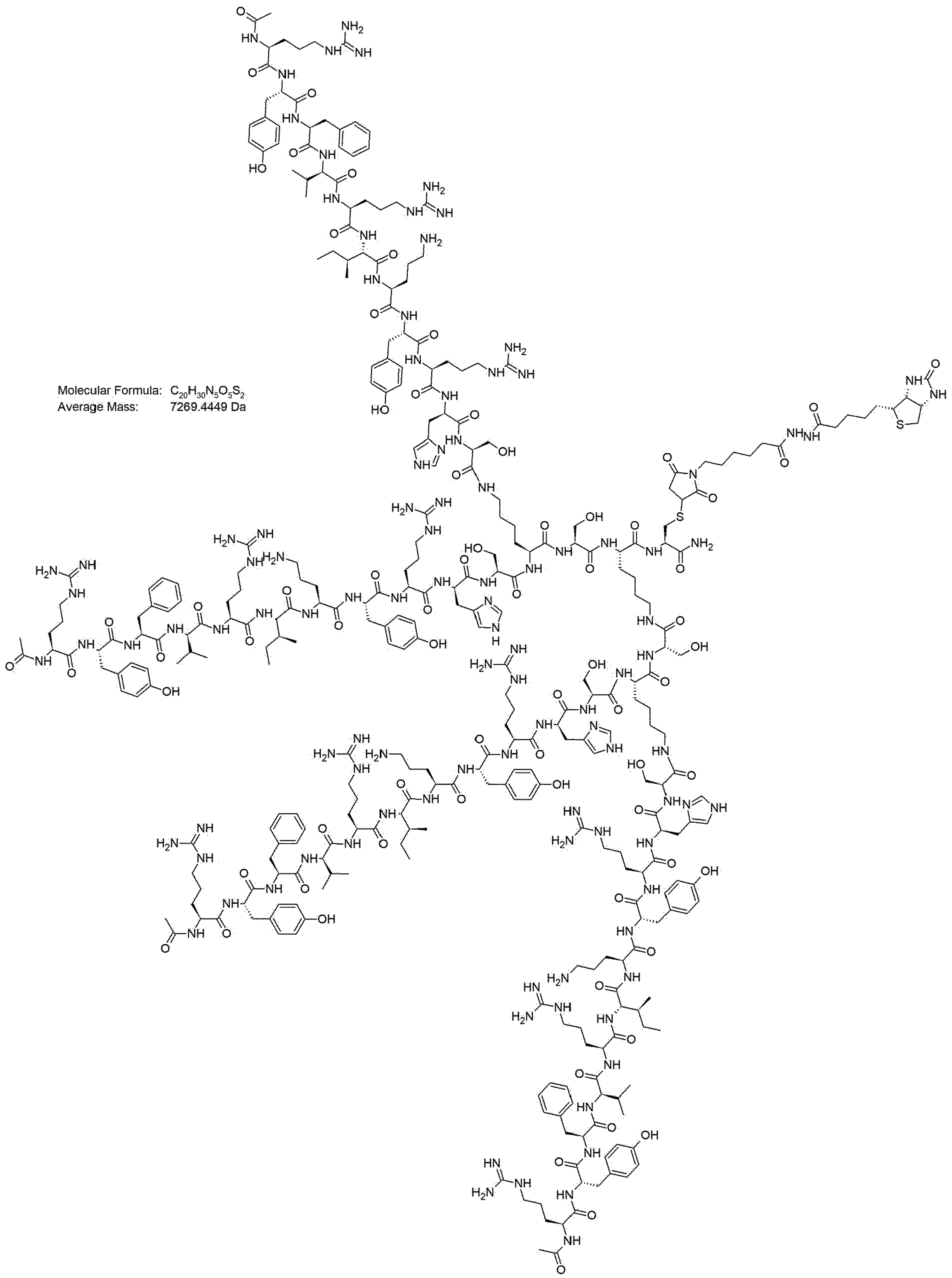

Molecular Formula: $C_{20}H_{30}N_5O_5S_2$
Average Mass: 7269.4449 Da

**[(51-hS)$_2$KS]$_2$KC-Mal-Biotin**

Batch powders (if available) have been dissolved with solvent A. In alternative, stock solutions (if available) have been diluted with solvent A. Purity assessed using a reversed-phase UHPLC column in an LC-MS system: flow rate of 0.620 ml-min$^{-1}$ and linear gradient of 5-95% v/v solvent B in 10 min (Table 4).

C-Mal-Biotin derivatives of $(21\text{-hS})_2$KC, $(21\text{-hS})_2$KS-(21-hS)KC, $[(21\text{-hS})_2\text{KS}]_2$KC, $(28\text{-hS})_2$KC, $(28\text{-hS})_2$KS-(28-hS)KC, and $[(28\text{-hS})_2\text{KS}]_2$KC were prepared according to the same protocol as C-Mal-Biotin derivatives of $(51\text{-hS})_2$KC, $(51\text{-hS})_2$KS-(51-hS)KC, and $[(51\text{-hS})_2\text{KS}]_2$KC.

Table 4.3. Analysis of prepared peptides

| Compound name | Batch no. | Retention time (min) | Purity (%) | Average mass (Da) | Observed m/z | Calculated m/z |
|---|---|---|---|---|---|---|
| $(51\text{-hS})_2$K C-Mal-Biotin | B-067 | 1.494 | 91.318 | 3769.4 | 539.4 - 629.0 - 754.7 - 943.2 | 539.5 (Z=7) - 629.2 (Z=6) - 754.9 (Z=5) - 943.4 (Z=4) |
| $(51\text{-hS})_2$KS-(51-hS)K C-Mal-Biotin (A) | B-164 (175154) | 1.811 | 55.798 | 5519.4 | 552.8 - 614.0 - 690.8 - 789.4 - 920.8 | 553.0 (Z=10) - 614.3 (Z=9) - 690.9 (Z=8) - 789.5 (Z=7) - 920.9 (Z=6) |
| $[(51\text{-hS})_2\text{KS}]_2$K C-Mal-Biotin | C-161 | 1.684 | 89.360 | 7269.4 | 606.6 - 661.8 - 727.8 - 808.6 - 909.6 - 1039.3 | 606.8 (Z=12) - 661.9 (Z=11) - 728.0 (Z=10) - 808.7 (Z=9) - 909.7 (Z=8) - 1039.5 (Z=7) |
| **Notes:** (A) Sample prepared from stock solution 17S154 (15% DMSO in PBS pH 7.4) originating from batch powder B-164 (powder not available). | | | | | | |

**Preparative example 4 - preparation of Mal-680RD derivatives of $(51\text{-hS})_2$KC, $(51\text{-hS})_2$KS-(51-hS)KC and $[(51\text{-hS})_2\text{KS}]_2$KC**

5 mg of each $(51\text{-hS})_2$KC, $(51\text{-hS})_2$KS-(51-hS)KC and $[(51\text{-hS})_2\text{KS}]_2$KC, corresponding to 1 molar equivalent, were weighed out and dissolved in 800 μL of 1x 10 mM PBS pH 7.4. Corresponding 1.2 molar equivalents of Mal-680RD (disodium salt), that is 1.86 mg, 1.22 mg and 0.91 mg, respectively, according to the formula: Peptide (g) / Peptide (MW) = Peptide (moles), corresponding to 1 molar equivalent Peptide (moles) x 2 molar equivalents = Mal-680RD (disodium salt) (moles) Mal-680RD (disodium salt) (moles) x Mal-680RD (disodium salt) (MW) = Mal-680RD (disodium salt) (g)

were dissolved in 200 μL of DMSO, and mixed with solution of the peptide. The reaction mixture was kept shaking at room temperature protected from light. The progress of the reaction was monitored by using RP-UHPLC-MS system: flow of 0.620 $ml\text{-}min^{-1}$ and linear gradient of 5-95% v/v solvent B in 4 min, and typically the reaction was complete within 1.5 hours. Products of the reactions were purified using a RP-HPLC preparative system: flow of 35 $ml\text{-}min^{-1}$ and linear gradient of 10-40% v/v solvent B in 25 min for $(51\text{-hS})_2$KC-Mal-680RD and $(51\text{-hS})_2$KS-(51-hS)KC-Mal-680RD, and 15-45% v/v solvent B in 25 min for $[(51\text{-hS})_2\text{KS}]_2$KC-Mal-680RD. Fractions were checked for presence of the desired product using a RP-UHPLC-MS system: flow of 0.620 $ml\text{-}min^{-1}$ and linear gradient of 5-95% v/v solvent B in 4 min, and fractions with the desired product were pooled together and lyophilized. Purity was checked using a RP-UHPLC-MS system: flow of 0.620 $ml\text{-}min^{-1}$ and linear gradient of 5-95% v/v solvent B in 10 min.

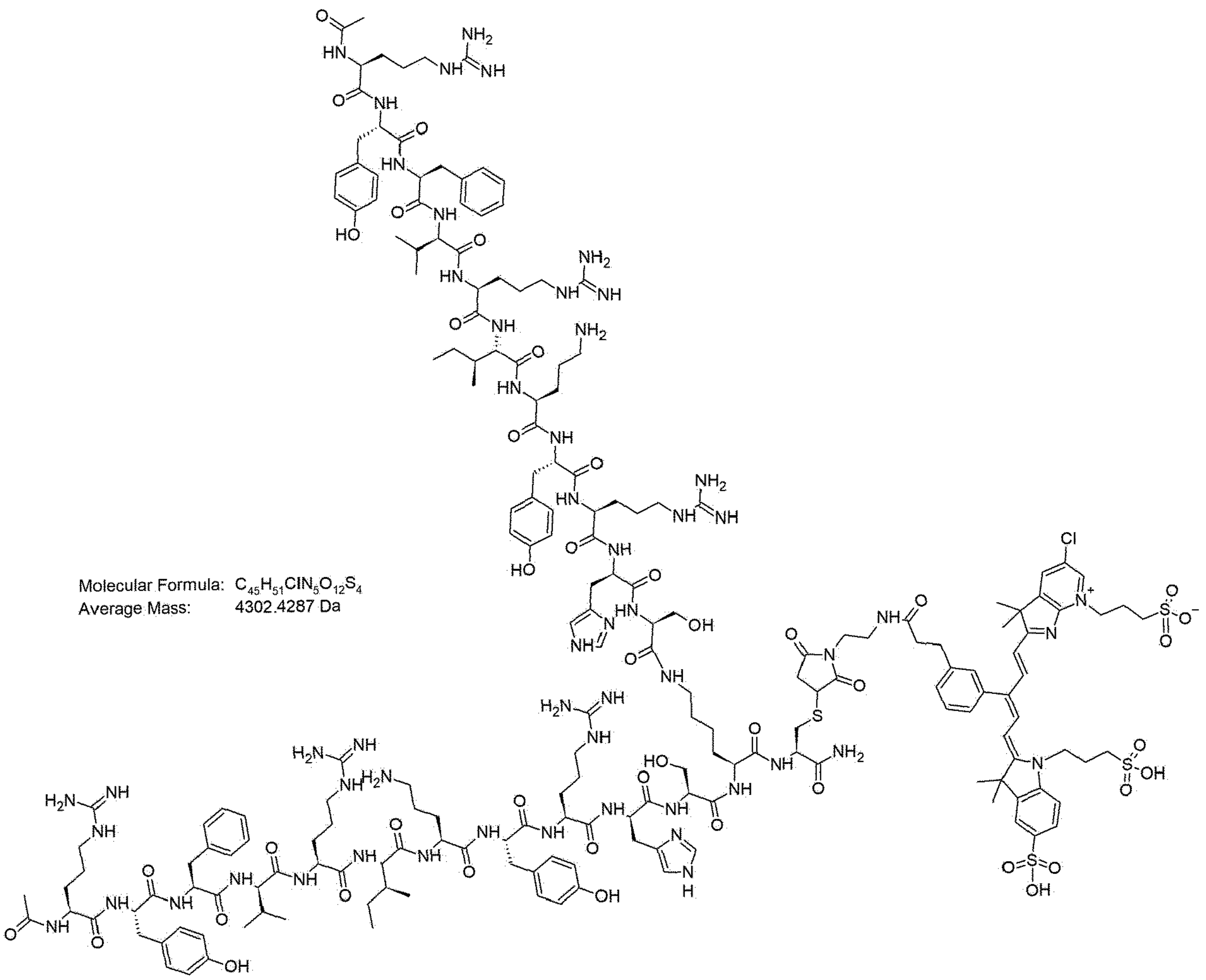

Molecular Formula: $C_{45}H_{51}ClN_5O_{12}S_4$
Average Mass: 4302.4287 Da

**(51-hS)$_2$KC-Mal-680RD**

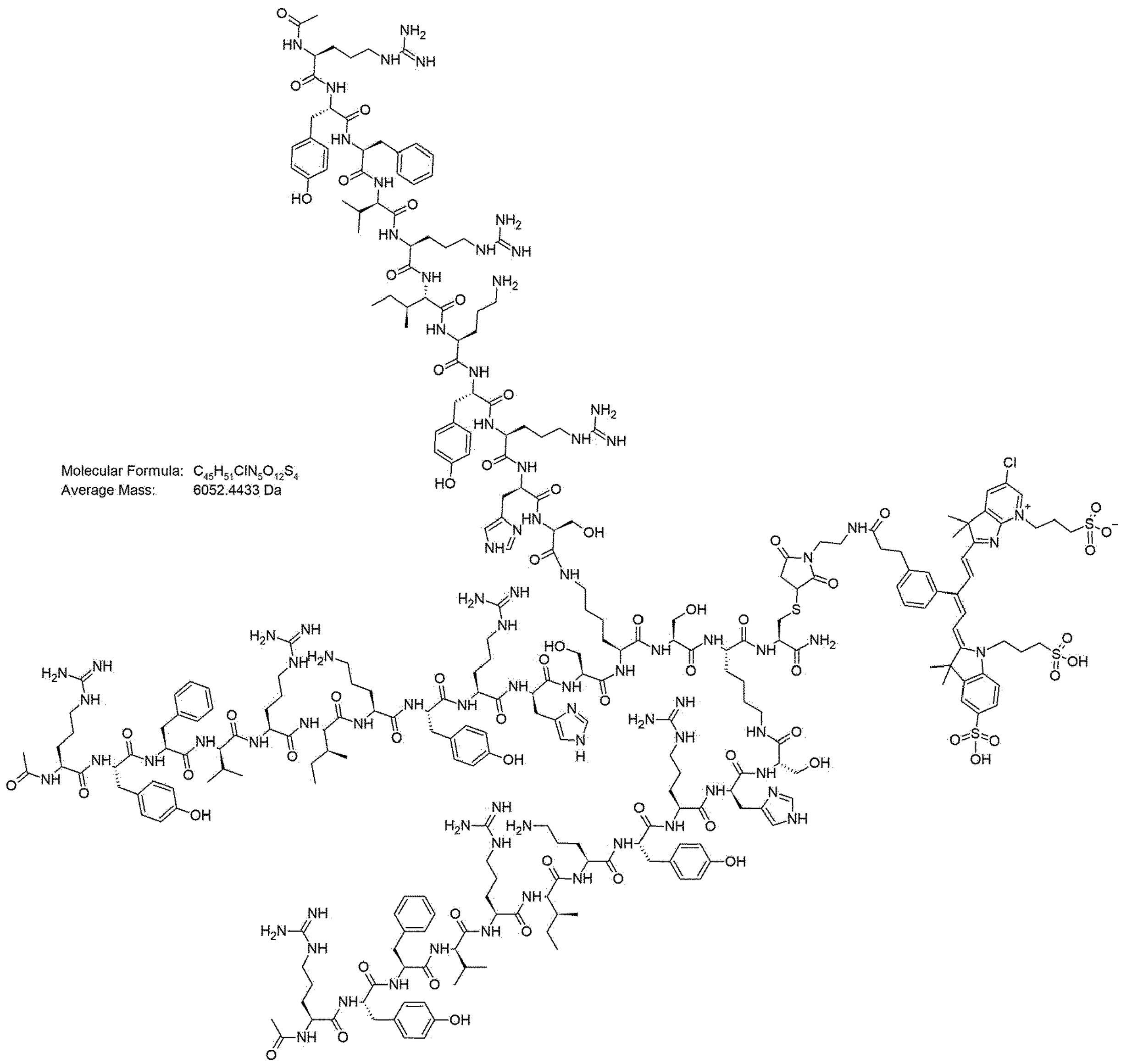

Molecular Formula: $C_{45}H_{51}ClN_5O_{12}S_4$
Average Mass: 6052.4433 Da

**(51-hS)$_2$KS-(51-hS)KC-Mal-680RD**

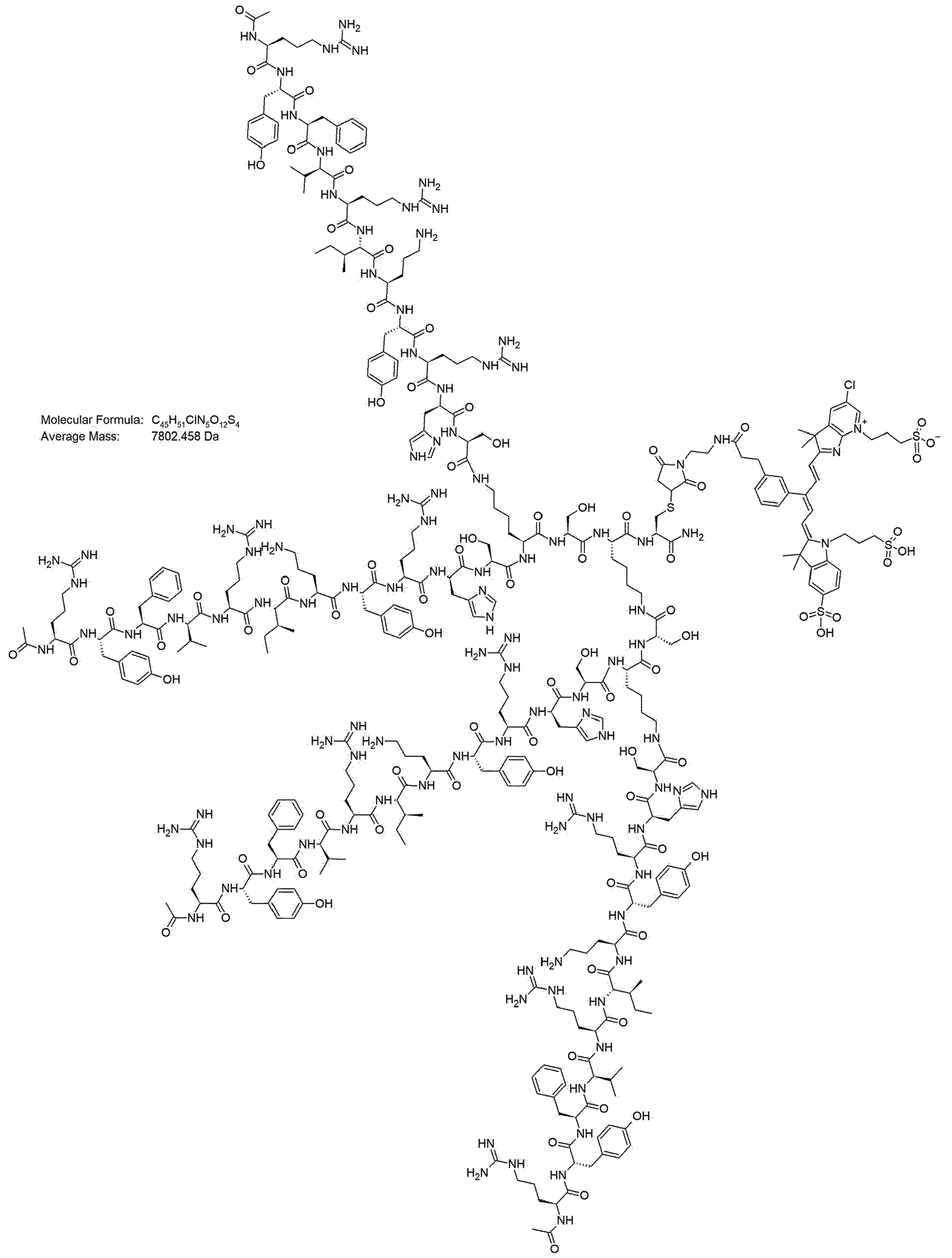

Molecular Formula: $C_{45}H_{51}ClN_5O_{12}S_4$
Average Mass: 7802.458 Da

**[(51-hS)₂KS]₂KC-Mal-680RD**

Batch powders (if available) have been dissolved with solvent A. In alternative, stock solutions (if available) have been diluted with solvent A. Purity assessed using a reversed-phase UHPLC column in an LC-MS system: flow rate of 0.620 ml-min$^{-1}$ and linear gradient of 5-95% v/v solvent B in 10 min (Table 4).

C-Mal-680RD derivatives of $(21\text{-hS})_2$KC, $(21\text{-hS})_2$KS-(21-hS)KC, $[(21\text{-hS})_2\text{KS}]_2$KC, $(28\text{-hS})_2$KC, $(28\text{-hS})_2$KS-(28-hS)KC, and $[(28\text{-hS})_2\text{KS}]_2$KC were prepared according to the same protocol as C-Mal-680RD derivatives of $(51\text{-hS})_2$KC, $(51\text{-hS})_2$KS-(51-hS)KC, and $[(51\text{-hS})_2\text{KS}]_2$KC.

Table 4.4. Analysis of prepared peptides

| Compound name | Batch no. | Retention time (min) | Purity (%) | Average mass (Da) | Observed m/z | Calculated m/z |
|---|---|---|---|---|---|---|
| $(51\text{-hS})_2$KC -Mal-680RD | D-044 | 3.490 | 98.902 | 4302.4 | 718.0 - 861.4 - 1076.6 - 1435.0 | 718.1 (Z=6) - 861.5 (Z=5) - 1076.6 (Z=4) - 1435.2 (Z=3) |
| $(21\text{-hS})_2$KC -Mal-680RD (A) | B-183 | 3.519 | 90.686 | 4386.5 | 732.0 - 878.3 - 1097.4 - 1462.9 | 732.1 (Z=6) - 878.3 (Z=5) - 1097.6 (Z=4) - 1463.2 (Z=3) |
| $(28\text{-hS})_2$KC -Mal-680RD (A) | C-036 | 3.567 | 67.415 | 4386.5 | 732.0 - 878.2 - 1097.6 - 1463.3 | 732.1 (Z=6) - 878.3 (Z=5) - 1097.6 (Z=4) - 1463.2 (Z=3) |
| $(51\text{-hS})_2$KS-(51-hS)KC - Mal-680RD | D-057 | 3.482 | 96.165 | 6052.4 | 865.6 - 1009.6 - 1211.4 | 865.6 (Z=7)-1009.7 (Z=6) - 1211.5 (Z=5) |
| $(21\text{-hS})_2$KS-(21-hS)KC - Mal-680RD (A)(B) | C-150 (185112) | 3.465 | 98.475 | 6178.6 | 773.3 - 883.6 - 1030.7 - 1236.6 | 773.3 (Z=8) - 883.7 (Z=7) - 1030.8 (Z=6) - 1236.7 (Z=5) |
| $(28\text{-hS})_2$KS-(28-hS)KC - Mal-680RD (A) | C-164 | 3.558 | 82.808 | 6178.6 | 773.3 - 883.6 - 1030.8 - 1236.6 | 773.3 (Z=8) - 883.7 (Z=7) - 1030.8 (Z=6) - 1236.7 (Z=5) |
| $[(51\text{-hS})_2\text{KS}]_2$KC -Mal-680RD | D-145 | 3.459 | 98.721 | 7802.4 | 781.3 - 867.8-976.2 - 1115.6 - 1301.4 | 781.3 (Z=10) - 867.9 (Z=9) - 976.3 (Z=8) - 1115.6 (Z=7)-1301.4 (Z=6) |
| $[(21\text{-hS})_2\text{KS}]_2$KC -Mal-680RD (A) | D-004 | 3.470 | 98.850 | 7970.6 | 725.8 - 798.0 - 886.6 - 997.2 - 1139.6 - 1329.4 | 725.6 (Z=11) - 798.1 (Z=10) - 886.6 (Z=9) - 997.3 (Z=8) - 1139.7 (Z=7)-1329.4 (Z=6) |
| $[(28\text{-hS})_2\text{KS}]_2$KC -Mal-680RD (A) | D-005 | 3.572 | 98.546 | 7970.6 | 798.0 - 886.5 - 997.3 - 1139.6 - 1329.4 | 798.1 (Z=10) - 886.6 (Z=9) - 997.3 (Z=8) - 1139.7 (Z=7)-1329.4 (Z=6) |

**Notes:**
(A) Sequence variants of 51, namely 21 and 28, synthetized in the three formats and modified as well with Mal-680RD by applying the same protocol
(B) Sample prepared from stock solution 18S112 (15% DMSO in PBS pH 7.4) originating from batch C-150 (powder not available).

**Preparative example 5 - preparation of Mal-Alexa488 derivatives of $(51\text{-hS})_2$KC, $(51\text{-hS})_2$KS-(51-hS)KC and $[(51\text{-hS})_2\text{KS}]_2$KC**

5 mg of each $(51\text{-hS})_2$KC, $(51\text{-hS})_2$KS-(51-hS)KC and $[(51\text{-hS})_2\text{KS}]_2$KC, corresponding to 1 molar equivalent, were weighed out and dissolved in 850 $\mu$L of 1x 10 mM PBS pH 7.4. Corresponding 1.5 molar equivalents of Mal-Alexa488 (monosodium salt), that is 1.63 mg, 1.07 mg and 0.79 mg, respectively, according to the formula:

Peptide (g) / [Peptide (MW) = Peptide (moles), corresponding to 1 molar equivalent

Peptide (moles) x 1.5 molar equivalents = Mal-Alexa488 (monosodium salt) (moles)

Mal-Alexa488 (monosodium salt) (moles) x Mal-Alexa488 (monosodium salt) (MW) = Mal-Alexa488 (monosodium salt) (g)

were dissolved in 150 μL of DMSO, and mixed with solution of the peptide. The reaction mixture was kept shaking at room temperature protected from light. The progress of the reaction was monitored by using RP-UHPLC-MS system: flow of 0.620 ml-min$^{-1}$ and linear gradient of 5-95% v/v solvent B in 4 min, and typically the reaction was complete within 1 to 4 hours. Products of the reactions were purified using a RP-HPLC preparative system: flow of 35 ml-min$^{-1}$ and linear gradient of 10-40% v/v solvent B in 25 min. Fractions were checked for presence of the desired product using a RP-UHPLC-MS system: flow of 0.620 ml-min$^{-1}$ and linear gradient of 5-95% v/v solvent B in 4 min, and fractions with the desired product were pooled together and lyophilized. Purity was checked using a RP-UHPLC-MS system: flow of 0.620 ml-min$^{-1}$ and linear gradient of 5-95% v/v solvent B in 10 min.

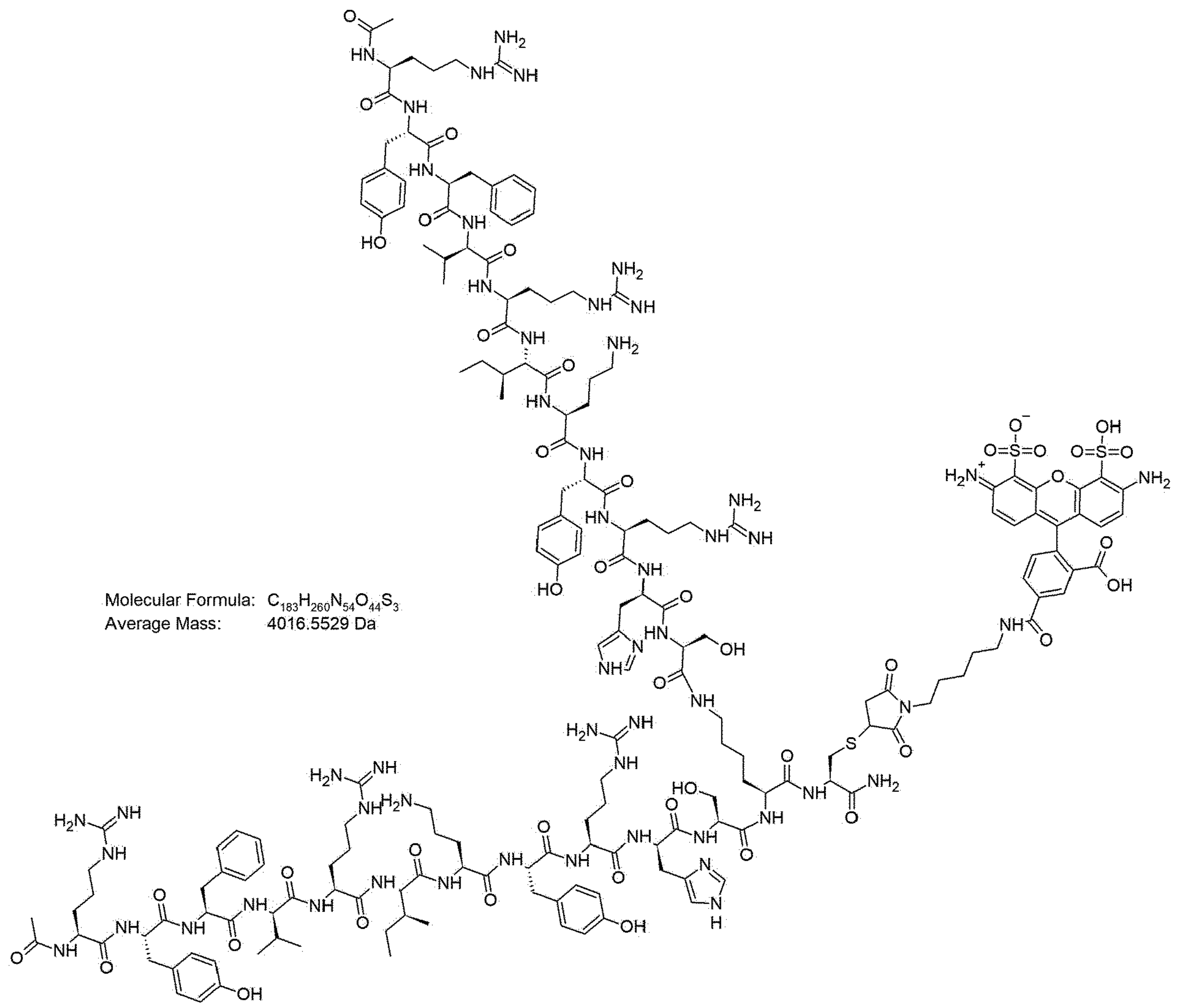

Molecular Formula: $C_{183}H_{260}N_{54}O_{44}S_3$
Average Mass: 4016.5529 Da

**(51-hS)$_2$KC-Mal-Alexa488**

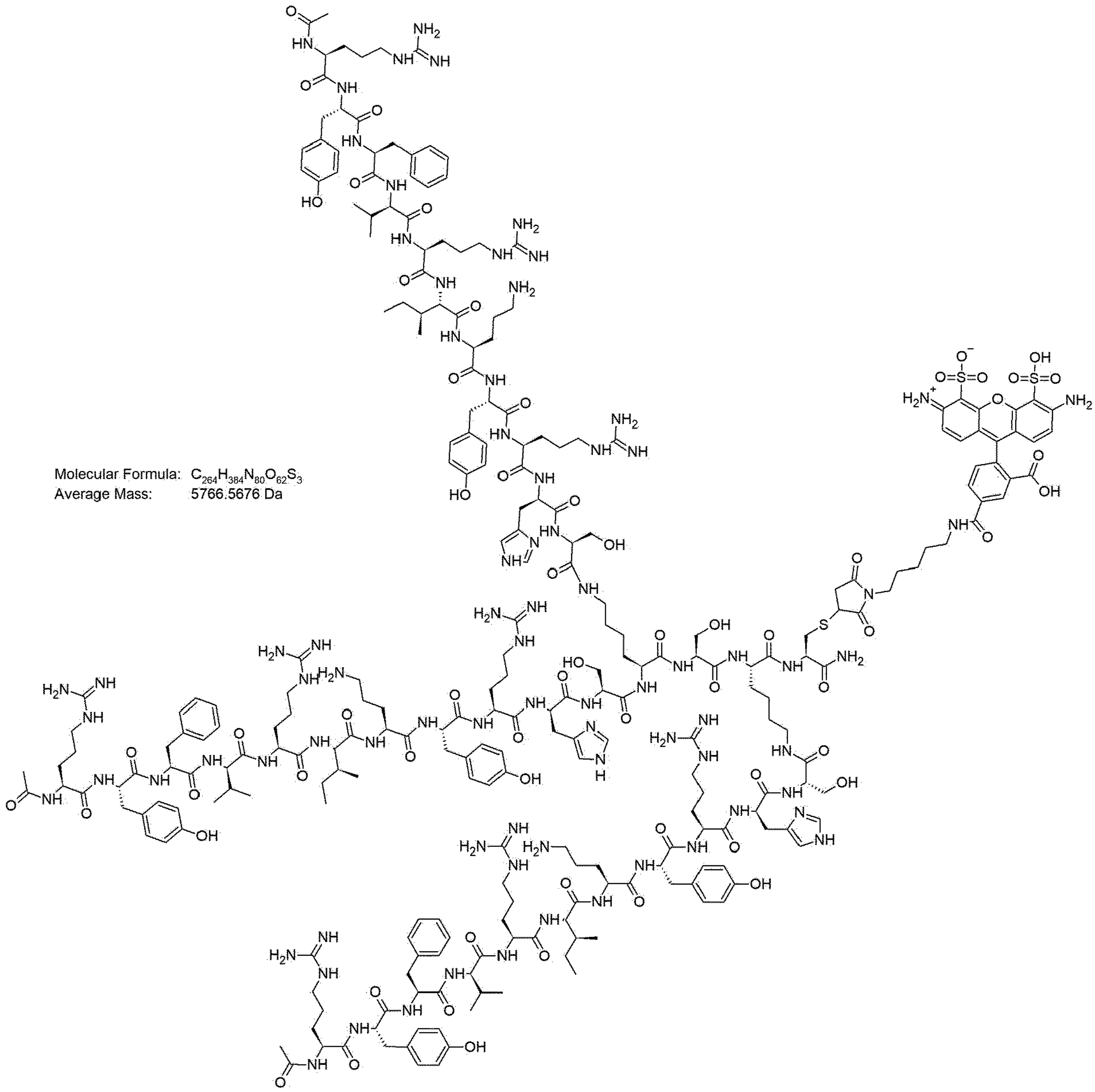

Molecular Formula: $C_{264}H_{384}N_{80}O_{62}S_3$
Average Mass: 5766.5676 Da

**(51-hS)$_2$KS-(51-hS)KC-Mal-Alexa488**

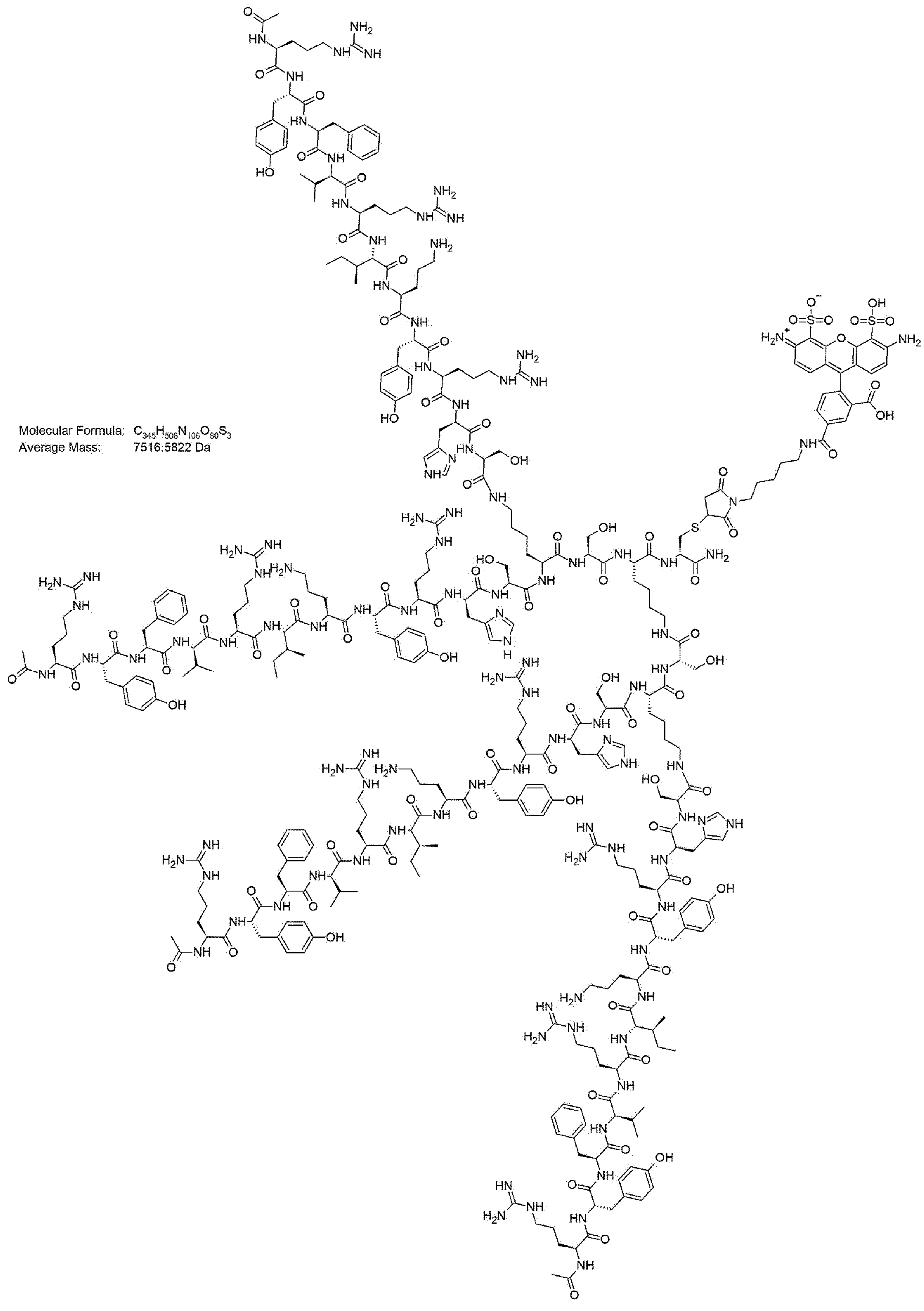

Molecular Formula: $C_{345}H_{508}N_{106}O_{80}S_3$
Average Mass: 7516.5822 Da

**[(51-hS)$_2$KS]$_2$KC-Mal-Alexa488**

Batch powders (if available) have been dissolved with solvent A. In alternative, stock solutions (if available) have been diluted with solvent A. Purity assessed using a reversed-phase UHPLC column in an LC-MS system: flow rate of 0.620 $ml\cdot min^{-1}$ and linear gradient of 5-95% v/v solvent B in 10 min (Table 4).

Table 4.5 Analysis of prepared peptides

| Compound name | Batch no. | Retention time (min) | Purity (%) | Average mass (Da) | Observed m/z | Calculated m/z |
|---|---|---|---|---|---|---|
| $(51\text{-hS})_2$KC -Mal-Alexa488 | D-054 | 1.699 | 96.165 | 4016.5 | 502.9 - 574.7 - 670.2 - 804.2 - 1005.0 - 1339.6 | 503.1 (Z=8) - 574.8 (Z=7) - 670.4 (Z=6) - 804.3 (Z=5) - 1005.1 (Z=4) - 1339.9 (Z=3) |
| $(51\text{-hS})_2$KS-(51-hS)KC - Mal-Alexa488 | D-164 | 1.652 | 99.707 | 5766.5 | 525.2 - 577.5 - 641.6 - 721.6 - 824.6 - 962.0 - 1154.2 | 525.2 (Z=11) - 577.7 (Z=10) - 641.7 (Z=9) - 721.8 (Z=8) - 824.8 (Z=7) - 962.1 (Z=6) - 1154.3 (Z=5) |
| $[(51\text{-hS})_2\text{KS}]_2$KC -Mal-Alexa488 | D-153 | 1.717 | 99.647 | 7516.5 | 579.2 - 627.2 - 684.2 - 752.6 - 836.0 - 940.5 - 1074.7 - 1253.5 | 579.2 (Z=13) - 627.4 (Z=12) - 684.3 (Z=11) - 752.7 (Z=10) - 836.2 (Z=9) - 940.6 (Z=8) - 1074.8 (Z=7) - 1253.8 (Z=6) |

**Preparative example 6 - preparation of Mal-Val-Cit-Azonafide derivatives of $(51\text{-hS})_2$KC, $(51\text{-hS})_2$KS-(51-hS)KC and $[(51\text{-hS})_2\text{KS}]_2$KC**

Mal-Val-Cit-Azonafide was prepared according to the following synthetic scheme:

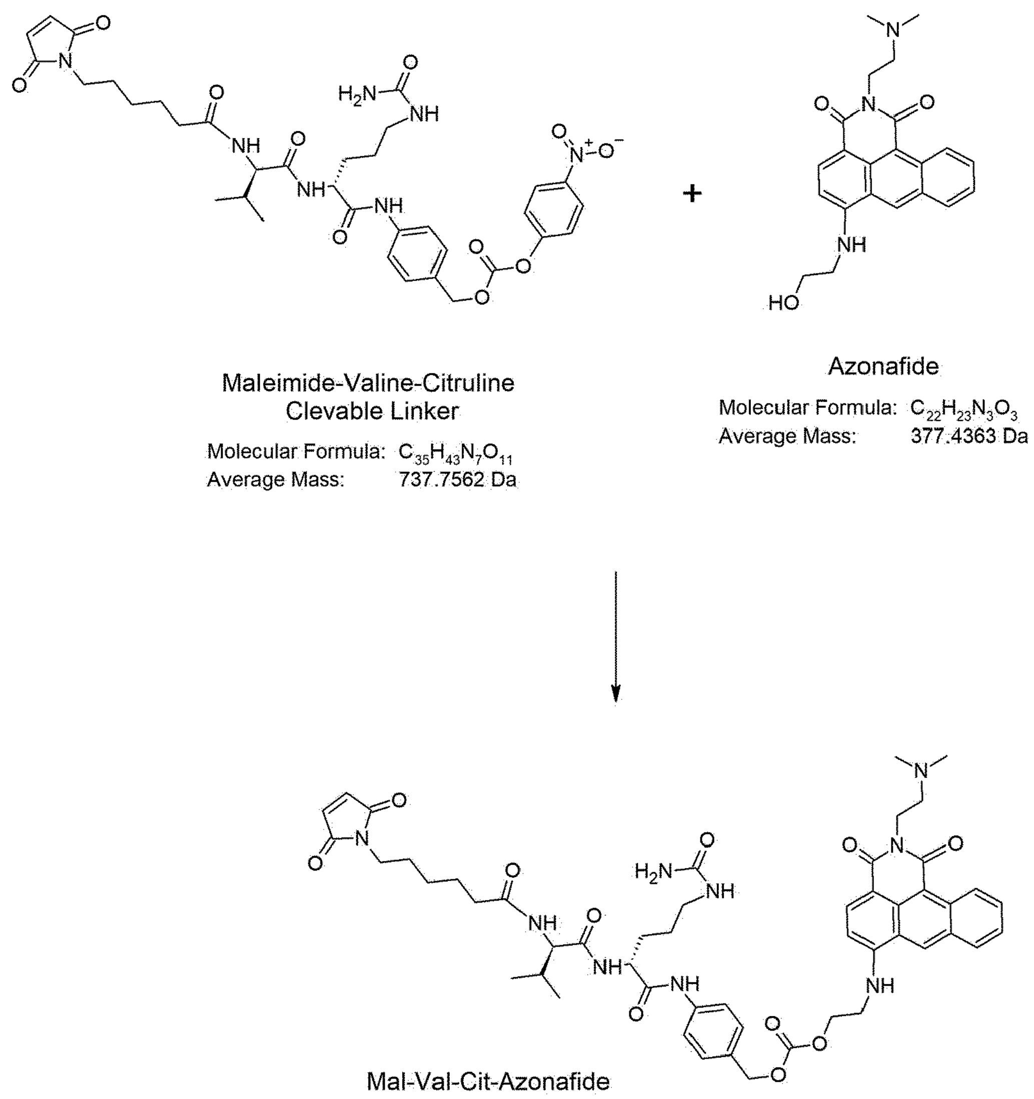

Maleimide-Valine-Citruline Clevable Linker

Molecular Formula: $C_{35}H_{43}N_7O_{11}$

Average Mass: 737.7562 Da

Azonafide

Molecular Formula: $C_{22}H_{23}N_3O_3$

Average Mass: 377.4363 Da

Mal-Val-Cit-Azonafide

Molecular Formula: $C_{51}H_{61}N_9O_{11}$

Average Mass: 976.0837 Da

20 mg of Azonafide were dissolved in 500 μL of DMSO. 80 mg (2 equivalents) of Maleimide-valine-citruline (Mal-Val-Cit-PAP-PNP) were dissolved in 500 μL of DMSO. Both solutions were mixed together, and 40 μL DIPEA (4 equivalents) were added to the resulting solution. The reaction mixture was kept shaking at room temperature protected from light. The progress of the reaction was monitored by using RP-UHPLC-MS system: flow of 0.620 ml-min$^{-1}$ and linear gradient of 5-95% v/v solvent B in 4 min, and typically the reaction was complete within 3 to 5 days. Products of the reactions were purified using a RP-HPLC preparative system: flow of 35 ml-min$^{-1}$ and linear gradient of 10-40% v/v solvent B in 25 min. Fractions were checked for presence of the desired product using a RP-UHPLC-MS system: flow of 0.620 ml-min$^{-1}$ and linear gradient of 5-95% v/v solvent B in 4 min, and fractions with the desired product were pooled together and lyophilized. Purity was checked using a RP-UHPLC-MS system: flow of 0.620 ml-min$^{-1}$ and linear gradient of 5-95% v/v solvent B in 4 min.

5 mg of each $(51\text{-hS})_2$KC, $(51\text{-hS})_2$KS-(51-hS)KC and $[(51\text{-hS})_2\text{KS}]_2$KC, corresponding to 1 molar equivalent, were weighed out and dissolved in 500 μL of 1x 10 mM PBS pH 7.4. Corresponding 1.2 molar equivalents of Mal-Val-Cit-Azonafide obtained as described herein, that is 1.77 mg, 1.16 mg and 0.86 mg, respectively, according to the formula:

Peptide (g) / [Peptide (MW) = Peptide (moles), corresponding to 1 molar equivalent

Peptide (moles) x 1.2 molar equivalents = Mal-Val-Cit-Azonafide (moles)

Mal-Val-Cit-Azonafide (moles) x Mal-Val-Cit-Azonafide (MW) = Mal-Val-Cit-Azonafide (g)

were dissolved in 500 μL of DMSO, and mixed with the solution of the peptide. The reaction mixture was kept shaking at room temperature protected from light. The progress of the reaction was monitored by using RP-UHPLC-MS system: flow of 0.620 ml-min$^{-1}$ and linear gradient of 5-95% v/v solvent B in 4 min, and typically the reaction was complete within 2 to 6 hours. Products of the reactions were purified using a RP-HPLC preparative system: flow of 35 ml-min$^{-1}$ and linear gradient of 10-40% v/v solvent B in 25 min. Fractions were checked for presence of the desired product using a RP-UHPLC-MS system: flow of 0.620 ml-min$^{-1}$ and linear gradient of 5-95% v/v solvent B in 4 min, and fractions with the desired product were pooled together and lyophilized. Purity was checked using a RP-UHPLC-MS system: flow of 0.620 ml-min$^{-1}$ and linear gradient of 5-95% v/v solvent B in 4 min.

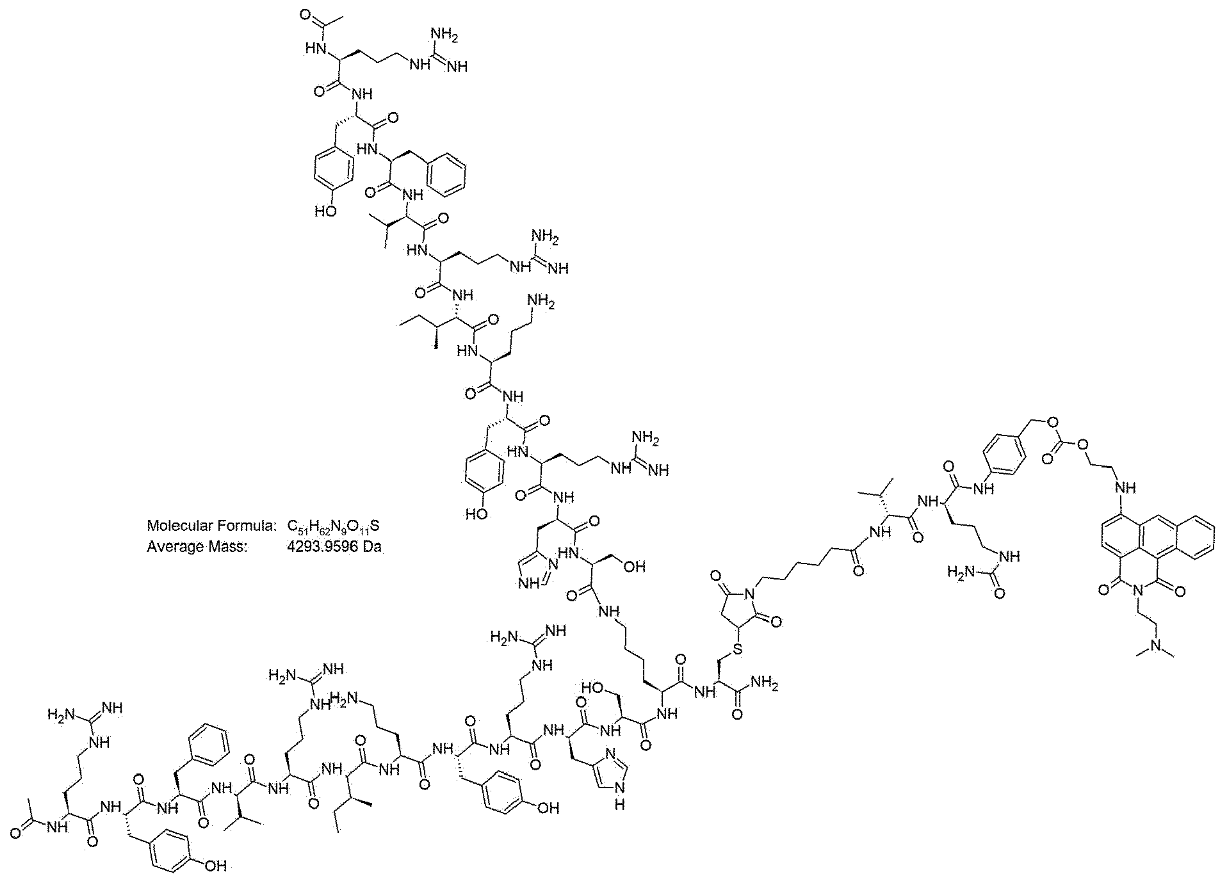

Molecular Formula: $C_{51}H_{62}N_9O_{11}S$
Average Mass: 4293.9596 Da

**(51-hS)$_2$KC-Mal-Val-Cit-Azonafide**

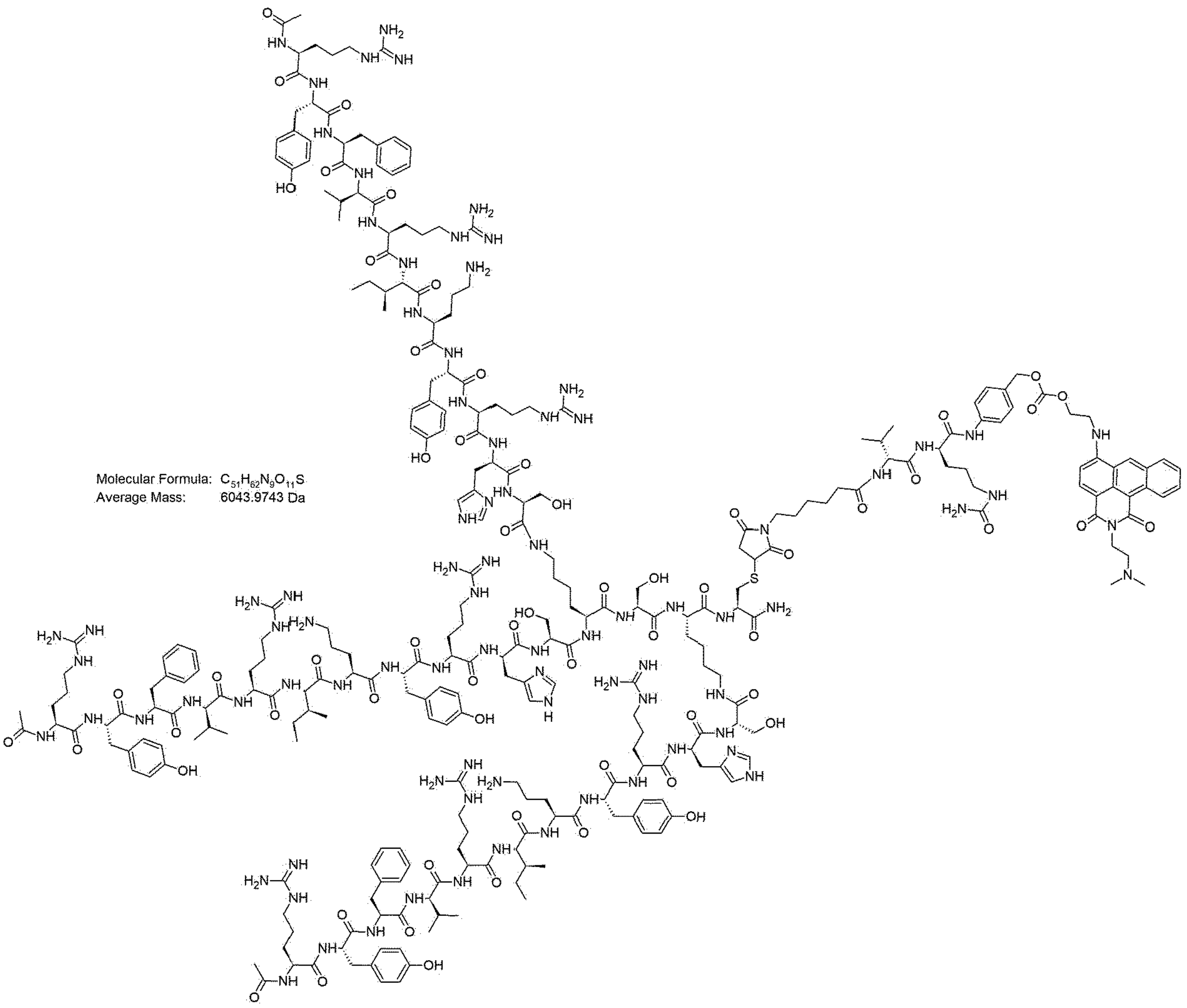

Molecular Formula: $C_{51}H_{62}N_9O_{11}S$
Average Mass: 6043.9743 Da

**(51-hS)$_2$KS-(51-hS)KC-Mal-Val-Cit-Azonafide**

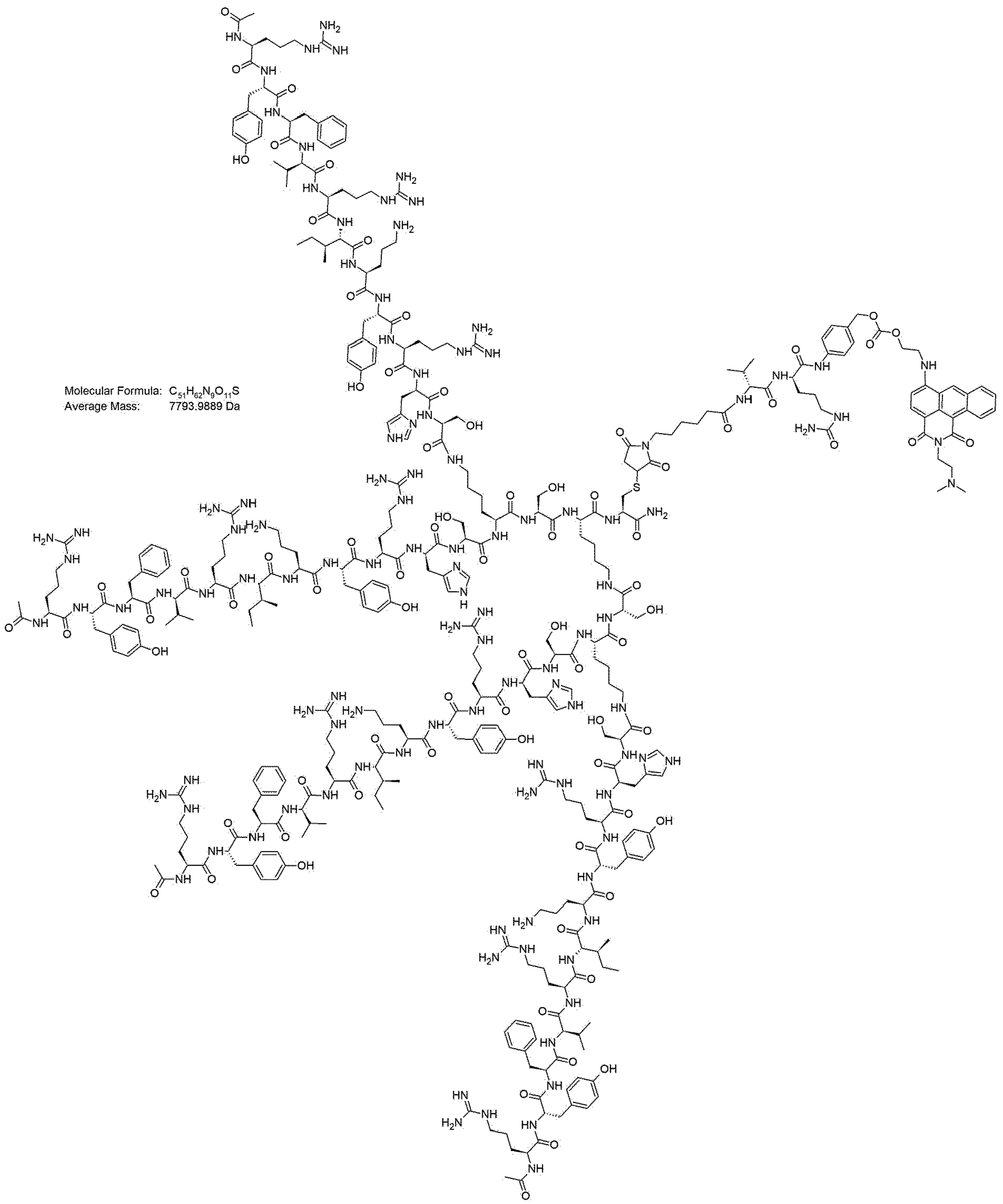

Molecular Formula: $C_{51}H_{62}N_{9}O_{11}S$
Average Mass: 7793.9889 Da

## [(51-hS)$_2$KS]$_2$KC-Mal-Val-Cit-Azonafide

Batch powders (if available) have been dissolved with solvent A. Alternatively, stock solutions (if available) have been diluted with solvent A. Purity assessed using a reversed-phase UHPLC column in an LC-MS system: flow rate of 0.620 ml-min$^{-1}$ and linear gradient of 5-95% v/v solvent B in 4 min (Table 2).

Table 4.6. Analysis of prepared peptides

| Compound name | Batch no. | Retention time (min) | Purity (%) | Average mass (Da) | Observed m/z | Calculated m/z |
|---|---|---|---|---|---|---|
| Mal-Val-Cit-Azonafide | E-020 | 2.673 | 96.676 | 975.5 | 976.4 | 976.5 (Z=1) |
| $(51\text{-hS})_2$KC -Mal-Val-Cit-Azonafide | E-021 | 2.252 | 98.462 | 4294.0 | 716.6 - 859.7 - 1074.4 - 1432.3 | 716.7 (Z=6) - 859.8 (Z=5) - 1074.5 (Z=4) - 1432.3 (Z=3) |
| $(51\text{-hS})_2$KS-(51-hS)KC - Mal-Val-Cit-Azonafide | D-152 | 2.290 | 95.575 | 6044.0 | 864.3 - 1008.2 - 1209.7 | 864.4 (Z=7) - 1008.3 (Z=6) - 1209.8 (Z=5) |
| $[(51\text{-hS})_2\text{KS}]_2$KC -Mal-Val-Cit-Azonafide | D-135 | 2.135 | 94.157 | 7794.0 | 867.2 - 975.3 - 1114.2 - 1299.8 - 1559.6 - 1949.2 | 867.0 (Z=9) - 975.3 (Z=8) - 1114.4 (Z=7)-1300.0 (Z=6) - 1559.8 (Z=5) - 1949.5 (Z=4) - |

**Preparative example 7 - Preparation of 6-thioguanine (6TG) derivatives of $(51\text{-hS})_2$KC, $(51\text{-hS})_2$KS-(51-hS)KC and $[(51\text{-hS})_2\text{KS}]_2$KC**

30 mg of each $(51\text{-hS})_2$KC, $(51\text{-hS})_2$KS-(51-hS)KC and $[(51\text{-hS})_2\text{KS}]_2$KC, corresponding to 1 molar equivalent, were weighed out and dissolved in 500 μL of 1x 10 mM PBS pH 7.4. Corresponding 1.5 molar equivalents of 6-thioguanine according to the formula:

Peptide (g) / [Peptide (MW) = Peptide (moles), corresponding to 1 molar equivalent

Peptide (moles) x 1.5 molar equivalents = 6-thioguanine (moles)

6-thioguanine (moles) x 6-thioguanine (MW) = 6-thioguanine (g)

that is 0.38 mg, 0.25 mg and 0.18 mg, respectively, were dissolved in 500 μL of DMSO, and mixed with solution of the peptide. 1.5 molar equivalents of 2,3-dichloro-5,6-cyano-1,4-benzoquinone, that is 0.51 mg, 0.34 mg and 0.25 mg, respectively, were dissolved in 100 μL of DMSO, and added dropwise to the reaction mixture. The reaction mixture was kept shaking at room temperature protected from light. The progress of the reaction was monitored by using RP-UHPLC-MS system: flow of 0.620 ml-min$^{-1}$ and linear gradient of 5-95% v/v solvent B in 4 min, and typically the reaction was complete within 2 hours. Products of the reactions were purified using a RP-HPLC preparative system: flow of 35 ml-min$^{-1}$ and linear gradient of 10-40% v/v solvent B in 25 min. Fractions were checked for presence of the desired product using a RP-UHPLC-MS system: flow of 0.620 ml-min$^{-1}$ and linear gradient of 5-95% v/v solvent B in 4 min, and fractions with the desired product were pooled together and lyophilized. Purity was checked using a RP-UHPLC-MS system: flow of 0.620 ml-min$^{-1}$ and linear gradient of 5-95% v/v solvent B in 10 min.

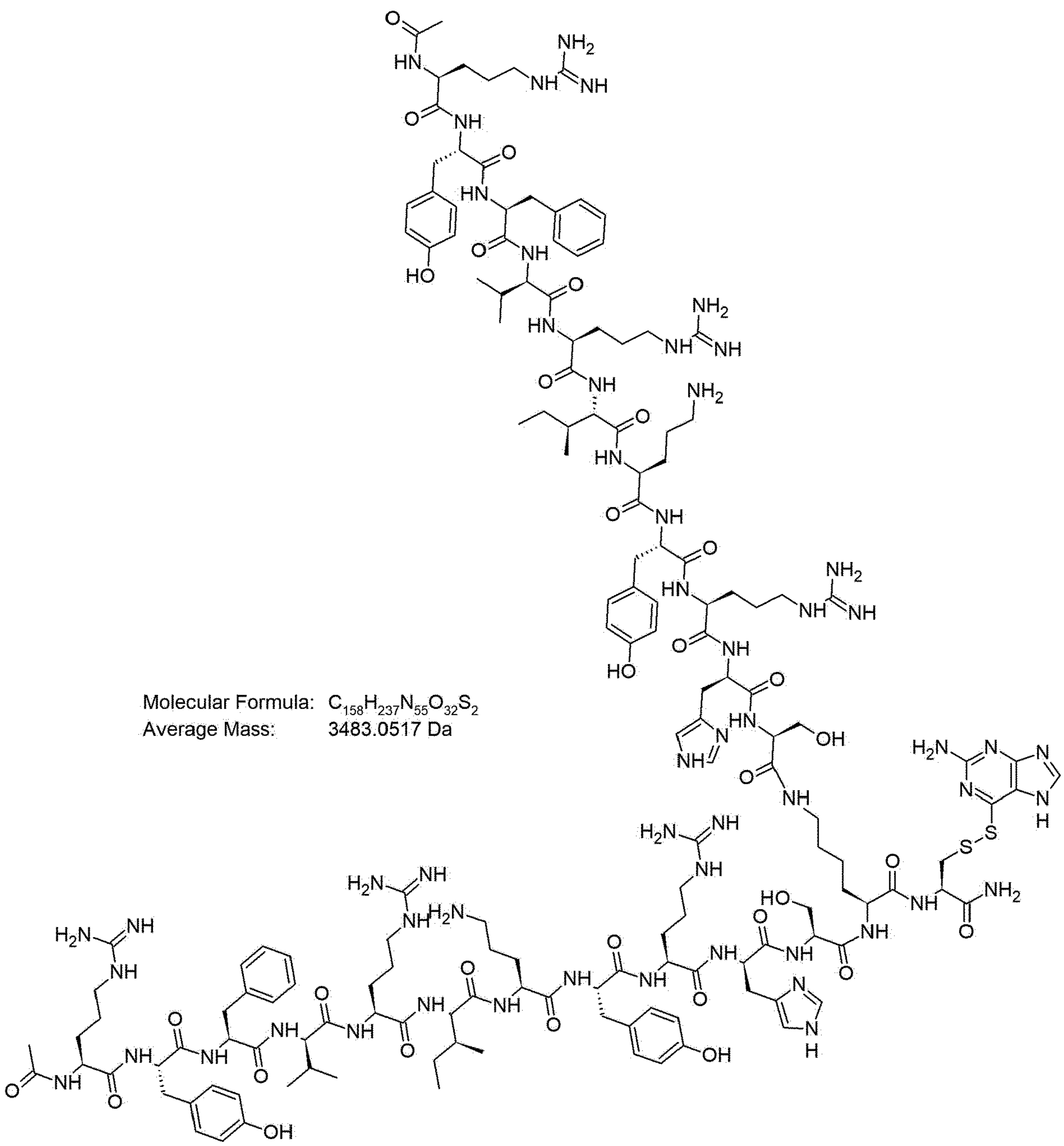

Molecular Formula: $C_{158}H_{237}N_{55}O_{32}S_2$
Average Mass: 3483.0517 Da

**(51-hS)$_2$KC-6-thioguanine**

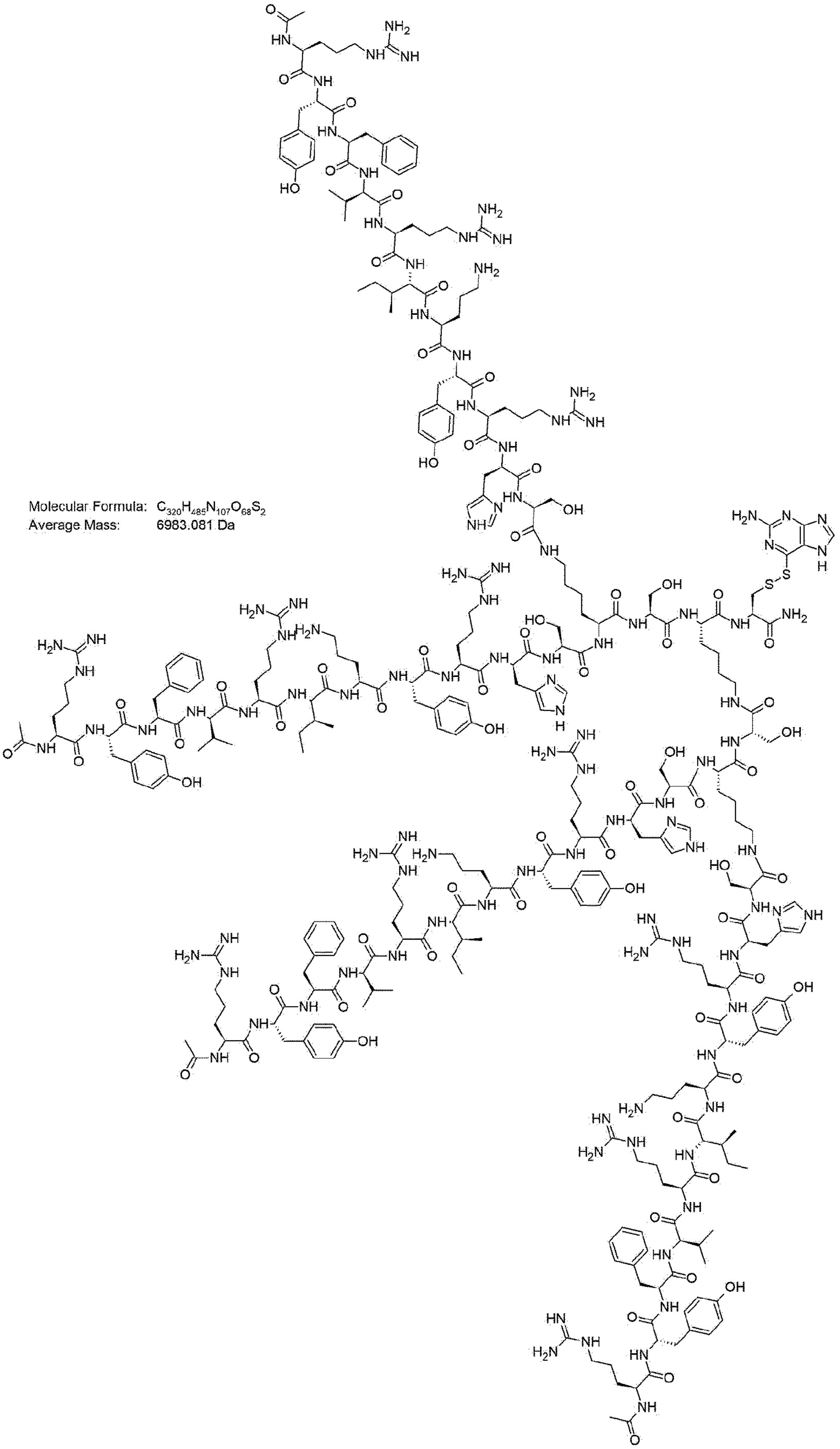

Molecular Formula: $C_{320}H_{485}N_{107}O_{68}S_2$
Average Mass: 6983.081 Da

**[(51-hS)$_2$KS]$_2$KC-6-thioguanine**

Batch powders (if available) have been dissolved with solvent A. In alternative, stock solutions (if available) have been diluted with solvent A. Purity assessed using a reversed-phase UHPLC column in an LC-MS system: flow rate of 0.620 ml-min$^{-1}$ and linear gradient of 5-95% v/v solvent B in 10 min (Table 4).

Table 4.7. Analysis of prepared peptides .

| Compound name | Batch no. | Retention time (min) | Purity (%) | Average mass (Da) | Observed m/z | Calculated m/z |
|---|---|---|---|---|---|---|
| $(51\text{-hS})_2$KC-6TG | E-018 | 2.934 | 80.610 | 3483.1 | 581.5 - 697.6-871.6 - 1161.9 | 581.5 (Z=6) - 697.6 (Z=5) - 871.8 (Z=4) - 1162.0 (Z=3) |
| $[(51\text{-hS})_2\text{KS}]_2$KC-6TG | E-033 | 3.189 | 32.574 | 6983.1 | 699.4 - 776.8 - 873.8 - 998.6 - 1164.8 - 1397.6 | 699.3 (Z=10) - 776.9 (Z=9) - 873.9 (Z=8) - 998.6 (Z=7)- 1164.9 (Z=6) - 1397.6 (Z=5) |

Purity assessed using a reversed-phase UHPLC column in an LC-MS system: flow rate of 0.620 ml-min$^{-1}$ and linear gradient of 5-50% v/v solvent B in 4 min (not shown).

Table 4.8. Analysis of prepared peptides (different batche from above).

| Compound name | Batch no. | Retention time (min) | Purity (%) | Average mass (Da) | Observed m/z | Calculated m/z |
|---|---|---|---|---|---|---|
| $(51\text{-hS})_2$KC -6TG | E-028 | 2.577 | 98.337 | 3483.1 | 581.5 - 697.5-871.6 - 1161.8 | 581.5 (Z=6) - 697.6 (Z=5) - 871.8 (Z=4) - 1162.0 (Z=3) |
| $[(51\text{-hS})_2\text{KS}]_2$KC -6TG | E-031 | 2.766 | 31.833 | 6983.1 | 776.9 - 874.0 - 998.5 - 1164.8 - 1397.5 | 776.9 (Z=9) - 873.9 (Z=8) - 998.6 (Z=7)-1164.9 (Z=6) - 1397.6 (Z=5) |

**Preparative example 8 - preparation of $[(51\text{-hS})_2\text{KS}]_2$K-propargylglycine**

The peptide compound comprising four copies of the peptide sequence 51 according to the following formula:

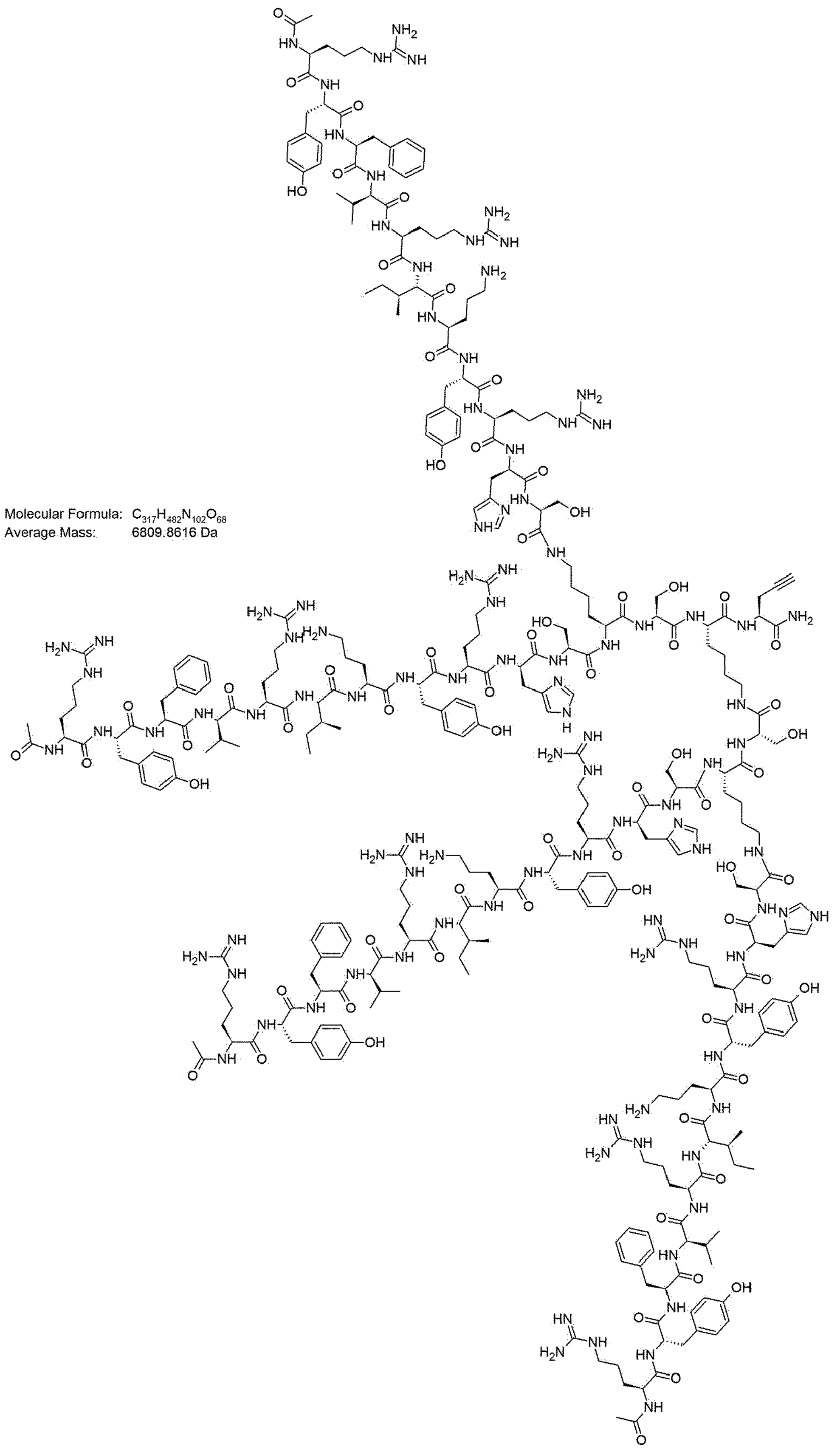

Molecular Formula: $C_{317}H_{482}N_{102}O_{68}$
Average Mass: 6809.8616 Da

**[(51-hS)$_2$KS]$_2$K-propargylglycine**

was prepared according to the general protocol described herein and as in Scheme IIIc, with the exception that in the

first step an Fmoc-protected propargylglycine was attached to the solid support resin.

Purification

Peptide was cleaved as previously described (see section: Synthesis of the compounds of the present invention), dissolved in 0.5 ml solvent B (99.9% v/v IACN and 0.1% v/v TFA) and 3.5 ml solvent A (99.9% v/v $H_2O$ and 0.1% v/v TFA) and treated as follows: sonication, centrifugation, filtration of supernatant (0.45 μm filter) and injection of 4 ml. Crude peptide was purified using a RP-HPLC preparative system: flow of 35 ml-min$^{-1}$ and linear gradient of 12-45% v/v solvent B in 25 min. Fractions were checked for presence of the desired product using a RP-UHPLC-MS system: flow of 0.620 ml-min$^{-1}$ and linear gradient of 5-95% v/v solvent B in 4 min, and fractions with the desired product were pooled together and lyophilized. Purity was checked using a RP-UHPLC-MS system: flow of 0.620 ml-min$^{-1}$ and linear gradient of 5-95% v/v solvent B in 4 min.

**Preparative example 9 - preparation of [(51-hS)$_2$KS]$_2$KX-triazole linker-PEG4-Azonafide**

N3-PEG4-Azonafide was prepared according to the following scheme:

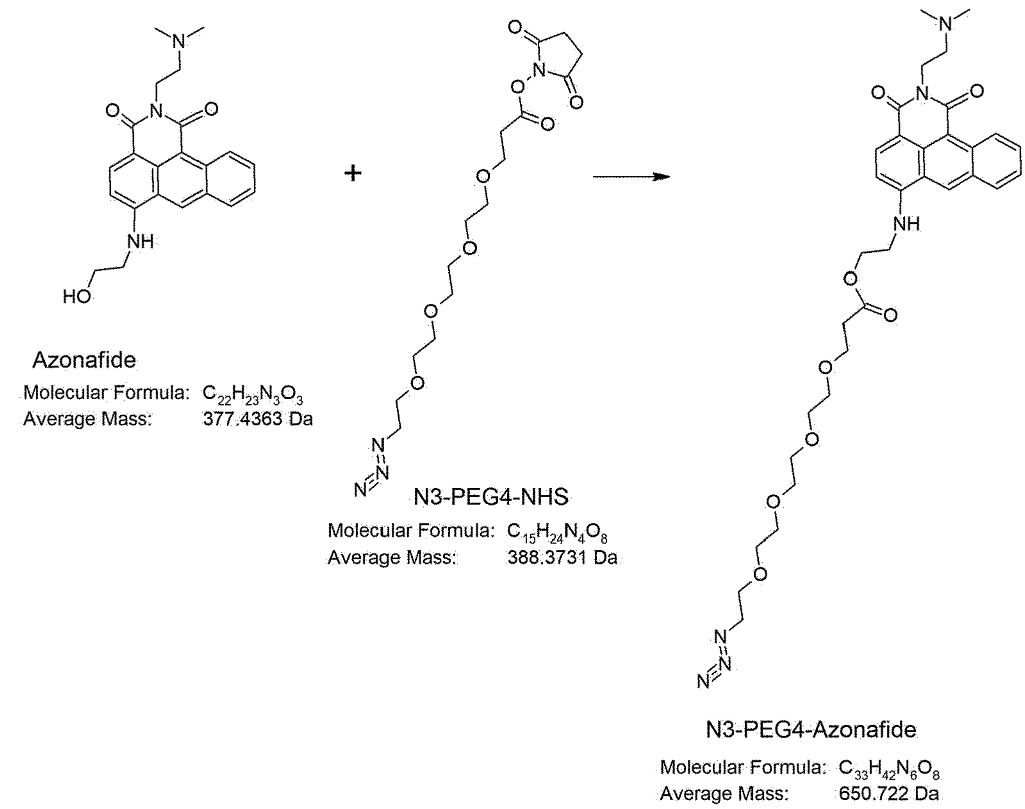

Azonafide
Molecular Formula: $C_{22}H_{23}N_3O_3$
Average Mass: 377.4363 Da

N3-PEG4-NHS
Molecular Formula: $C_{15}H_{24}N_4O_8$
Average Mass: 388.3731 Da

N3-PEG4-Azonafide
Molecular Formula: $C_{33}H_{42}N_6O_8$
Average Mass: 650.722 Da

30 mg of Azonafide were dissolved in 500 μL DMSO and 100 mg of N3-PEG4-NHS were dissolved in 200 μL DMSO, and both solutions were mixed. 70 μL of DIPEA were added to the solution, and the reaction mixture was shaken at room temperature protected from light. The progress of the reaction was monitored by using RP-UHPLC-MS system: flow of 0.620 ml-min$^{-1}$ and linear gradient of 5-95% v/v solvent B in 4 min, and the reaction was complete after 3 days. Products of the reactions were purified using a RP-HPLC preparative system: flow of 35 ml-min$^{-1}$ and linear gradient of 10-40% v/v solvent B in 25 min. Fractions were checked for presence of the desired product using a RP-UHPLC-MS system: flow of 0.620 ml-min$^{-1}$ and linear gradient of 5-95% v/v solvent B in 4 min, and fractions with the desired product were pooled together and lyophilized. Purity was checked using a RP-UHPLC-MS system: flow of 0.620 ml-min$^{-1}$ and linear gradient of 5-95% v/v solvent B in 4 min.

5 mg of [(51-hS)$_2$KS]$_2$K-propargylglycine (1 molar equivalent) was weighted out and dissolved in DMSO at a final concentration of 10 mM. 0.72 mg of N3-PEG4-Azonafide (1.5 molar equivalents) obtained as described above were weighed out according to the formula:

$$\text{Peptide (g) / [Peptide (MW) = Peptide (moles), corresponding to 1 molar equivalent}$$

$$\text{Peptide (moles) x 1.5 molar equivalents = N3-PEG4-Azonafide (moles)}$$

$$\text{N3-PEG4-Azonafide (moles) x N3-PEG4-Azonafide (MW) = N3-PEG4-Azonafide (g)}$$

and dissolved in DMSO at a final concentration of 20 mM. Peptide compound solution was diluted to 2 mM, and N3-PEG4-Azonafide was dilued to 3 mM. 80 mM aqueous stock solution copper sulphate was obtained by dissolving $CuSO_4$ x 5 $H_2O$ in water. 100 mM sodium ascorbate aqueous solution was prepared. By mixing of both peptide compound and N3-PEG4-Azonafide solutions together with addition of 80 mM copper sulfate aqueous solution and 100 mM sodium ascorbate aqueous solution, with addition of water and/or DMSO as required, a solution with final concentration of peptide compound of 1mM, N3-PEG4-Azonafide of 1.5 mM, copper sulfate of 2 mM and sodium ascorbate of 10 mM, with final amount of DMSO of 50% v/v, was obtained. The reaction mixture was shaken at room temperature protected from light. The progress of the reaction was monitored by using RP-UHPLC-MS system: flow of 0.620 ml-min$^{-1}$ and linear gradient of 5-95% v/v solvent B in 4 min, and the reaction was complete after 2 hours. Products of the reactions were purified using a RP-HPLC preparative system: flow of 35 ml-min$^{-1}$ and linear gradient of 10-40% v/v solvent B in 25 min. Fractions were checked for presence of the desired product using a RP-UHPLC-MS system: flow of 0.620 ml-min$^{-1}$ and linear gradient of 5-95% v/v solvent B in 4 min, and fractions with the desired product were pooled together and lyophilized. Purity was checked using a RP-UHPLC-MS system: flow of 0.620 ml-min$^{-1}$ and linear gradient of 5-95% v/v solvent B in 4 min.

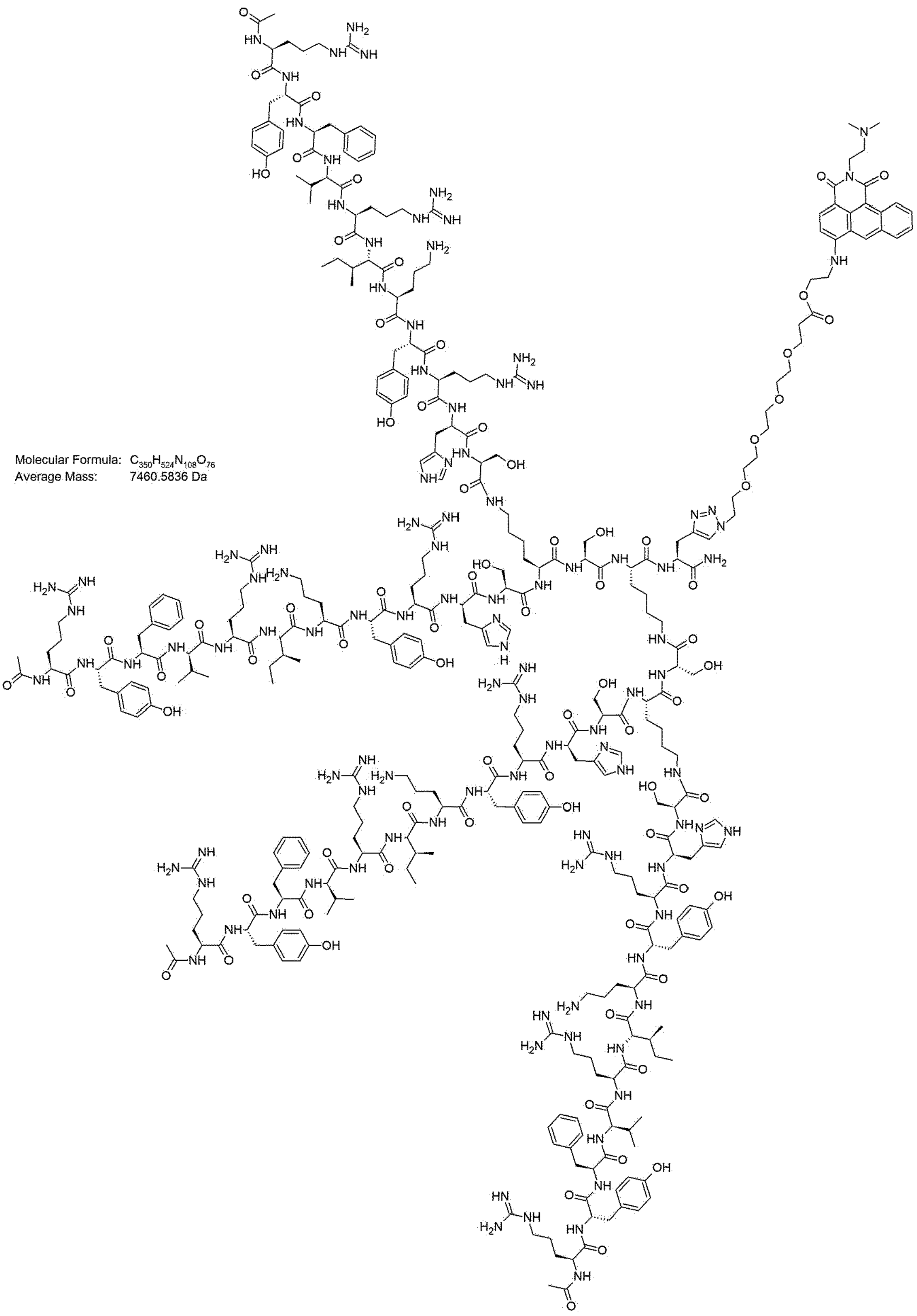

Molecular Formula: $C_{350}H_{524}N_{108}O_{76}$
Average Mass: 7460.5836 Da

**[(51-hS)$_2$KS]$_2$K-triazole linker-PEG4-Azonafide**

**Preparative example 10 - preparation of [(51-hS)$_2$KS]$_2$K-triazole linker-680RD**

5 mg of [(51-hS)$_2$KS]$_2$K-propargylglycine (1 molar equivalent) were dissolved in DMSO to a concentration of 10 mM. 1.06 mg of N3-680RD (disodium salt) (1.3 molar equivalents) were weighed out according to formula:

Peptide (g) / [Peptide (MW) = Peptide (moles), corresponding to 1 molar equivalent

Peptide (moles) x 1.3 molar equivalents = N3-680RD (disodium salt) (moles)

N3-680RD (disodium salt) (moles) x N3-680RD (disodium salt) (MW) = N3-680RD (disodium salt) (g)

and dissolved in DMSO to a concentration of 20 mM. By mixing of both solutions together with addition of 100 mM copper sulfate aqueous solution and 500 mM sodium ascorbate aqueous solution, with addition of water and/or DMSO as required, a solution with final concentration of peptide compound of 1mM, N3-680RD (disodium salt) of 1.3 mM, copper sulfate of 2 mM and sodium ascorbate of 10 mM, with final amount of DMSO of 75% v/v, was obtained. The reaction mixture was shaken at room temperature protected from light. The progress of the reaction was monitored by using RP-UHPLC-MS system: flow of 0.620 ml-min$^{-1}$ and linear gradient of 5-95% v/v solvent B in 4 min, and the reaction was complete after 2 hours. Products of the reactions were purified using a RP-HPLC preparative system: flow of 35 ml-min$^{-1}$ and linear gradient of 10-40% v/v solvent B in 25 min. Fractions were checked for presence of the desired product using a RP-UHPLC-MS system: flow of 0.620 ml-min$^{-1}$ and linear gradient of 5-95% v/v solvent B in 4 min, and fractions with the desired product were pooled together and lyophilized. Purity was checked using a RP-UHPLC-MS system: flow of 0.620 ml-min$^{-1}$ and linear gradient of 5-95% v/v solvent B in 4 min.

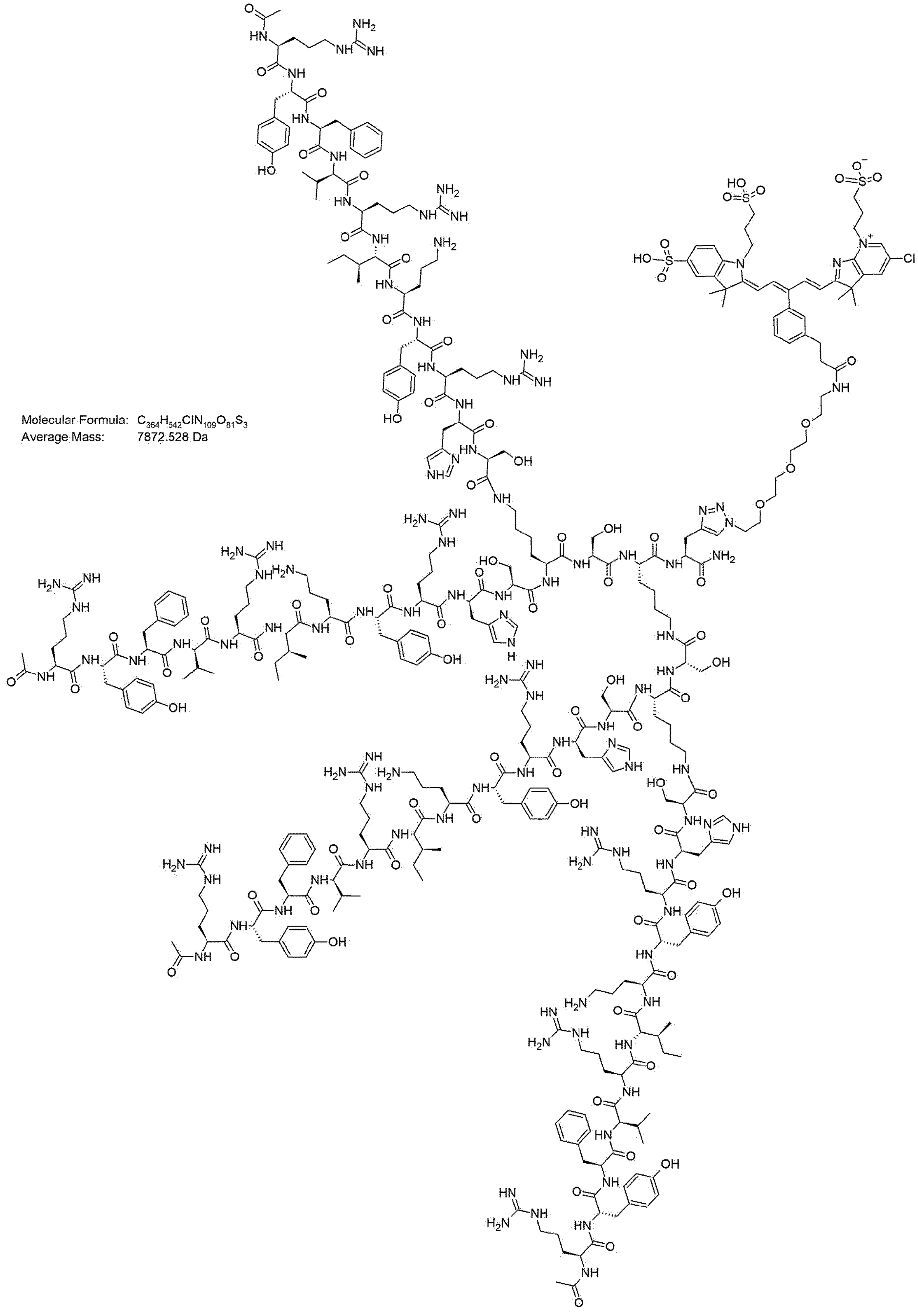

Molecular Formula: $C_{364}H_{542}ClN_{109}O_{81}S_3$
Average Mass: 7872.528 Da

**[(51-hS)$_2$KS]$_2$K-triazole linker-680RD**

**Preparative example 11 - preparation of [(51-hS)$_2$KS]$_2$K-triazole linker-Alexa488**

5 mg of [(51-hS)$_2$KS]$_2$K-propargylglycine (1 molar equivalent) were dissolved in DMSO to a concentration of 10 mM. 0.76 mg of N3-Alexa488(di-triethylammonium salt) (1.2 molar equivalents) were weighed out according to formula:

Peptide (g) / [Peptide (MW) = Peptide (moles), corresponding to 1 molar equivalent

Peptide (moles) x 1.2 molar equivalents = N3-Alexa488(di-triethylammonium salt) (moles)

N3-Alexa488(di-triethylammonium salt) (moles) x N3-Alexa488(di-triethylammonium salt) (MW) = N3-Alexa488(di-triethylammonium salt) (g)

and dissolved in DMSO to a concentration of 20 mM. By mixing of both solutions together with addition of 80 mM copper sulfate aqueous solution , 100 mM sodium ascorbate aqueous solution, 20 mM tris(3-hydroxypropyltriazolylmethyl)amine (THTPA) aqueous solution, with addition of water and/or DMSO as required, a solution with final concentration of peptide compound of 1 mM, N3-Alexa488(di-triethylammonium salt) of 1.2 mM, copper sulfate of 4 mM and sodium ascorbate of 20 mM, and THTPA of 10 mM, with final amount of DMSO of 60% v/v, was obtained. The reaction mixture was shaken at room temperature protected from light. The progress of the reaction was monitored by using RP-UHPLC-MS system: flow of 0.620 ml-min$^{-1}$ and linear gradient of 5-95% v/v solvent B in 4 min, and the reaction was complete after 48 hours. Products of the reactions were purified using a RP-HPLC preparative system: flow of 35 ml-min$^{-1}$ and linear gradient of 10-40% v/v solvent B in 25 min. Fractions were checked for presence of the desired product using a RP-UHPLC-MS system: flow of 0.620 ml-min$^{-1}$ and linear gradient of 5-95% v/v solvent B in 4 min, and fractions with the desired product were pooled together and lyophilized. Purity was checked using a RP-UHPLC-MS system: flow of 0.620 ml-min$^{-1}$ and linear gradient of 5-95% v/v solvent B in 4 min.

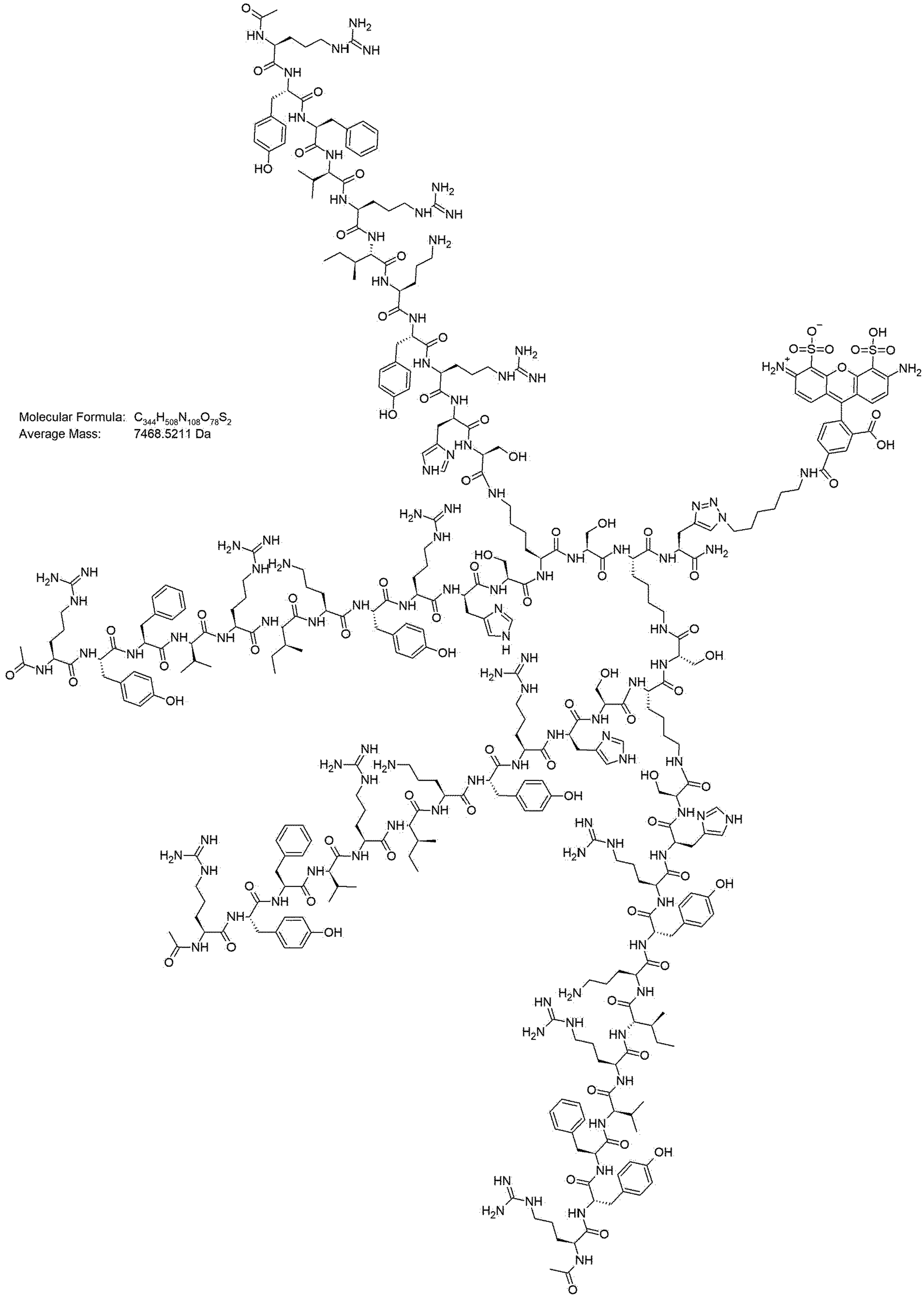

Molecular Formula: $C_{344}H_{508}N_{108}O_{78}S_2$
Average Mass: 7468.5211 Da

**[(51-hS)$_2$KS]$_2$K-triazole linker-Alexa488**

Batch powders (if available) have been dissolved with solvent A. Alternatively, stock solutions (if available) have been diluted with solvent A. Purity assessed using a reversed-phase UHPLC column in an LC-MS system: flow rate of 0.620 ml-min$^{-1}$ and linear gradient of 5-95% v/v solvent B in 4 min (Table 2).

Table 5 Analysis of prepared peptides.

| Compound name | Batch no. | Retention time (min) | Purity (%) | Average mass (Da) | Observed m/z | Calculated m/z |
|---|---|---|---|---|---|---|
| $[(51\text{-hS})_2KS]_2K$ - propargylglycine | D-012 | 1.858 | 94.700 | 6809.9 | 682.0 - 757.8 - 973.8 - 1135.8 - 1362.8 | 682.0 (Z=10) - 757.7 (Z=9) - 973.8 (Z=7) - 1136.0 (Z=6) - 1363.0 (Z=5) |
| N3-PEG4-Azonafide | D-015 | 2.476 | 100.000 | 650.7 | 651.3 | 651.7 (Z=1) |
| $[(51\text{-hS})_2KS]_2K$ - triazole linker-PEG4-Azonafide | D-033 | 1.870 | 100.000 | 7460.6 | 622.6 - 747.0 - 933.4 - 1066.5 -1244.2 - 1492.9 | 622.7 (Z=12) - 747.1 (Z=10) - 933.6 (Z=8) - 1066.8 (Z=7)-1244.4 (Z=6) - 1493.1 (Z=5) |
| $[(51\text{-hS})_2KS]_2K$ - triazole linker-680RD | D-030 | 2.105 | 100.000 | 7872.5 | 985.0 - 1125.7 -1313.2 - 1575.4 | 985.1 (Z=8) - 1125.7 (Z=7) - 1313.1 (Z=6)-1575.5 (Z=5) |
| $[(51\text{-hS})_2KS]_2K$ - triazole linker-Alexa488 | D-049 | 2.036 | 96.135 | 7468.5 | 746.0 - 1245.6 - 1494.4 | 747.9 (Z=10) - 1245.8 (Z=6) - 1494.7 (Z=5) |

**Experimental Examples**

**Example 1 - Binding of compound 51-hSC-Mal-Biotin to different proteoglycans**

The aim of this study was to determine the binding affinity of a single peptide moiety to different proteoglycans. To this end, an ELISA procedure was employed.

The compound tested in this Example is:

51-hSC-Mal-Biotin: A single copy of peptide with SEQ ID NO:37 conjugated to a biotin moiety.

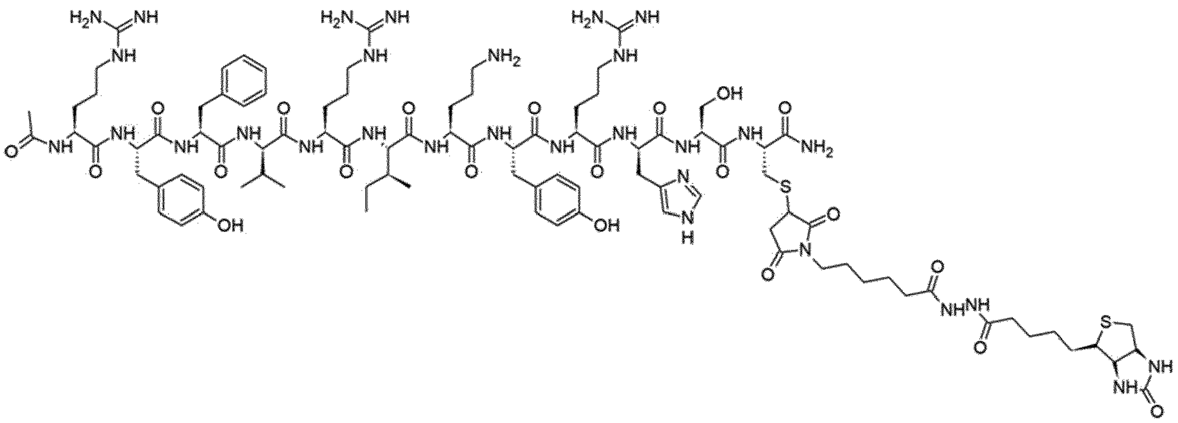

ELISA plates were coated with various purified GAGs (Sigma, AMBIO) in PBS pH 7.4 for 2 hours at 37°C. Plates were blocked overnight at 4°C with 200 μl of PBS pH 7.4, Tween 0.05% + BSA 3% + PEG400 2% , then washed twice with fresh buffer TSCM pH 6.4 ((Tris 20 mM, NaCl 150 mM, BSA 1%, Tween 0.05%, 2 mM MgCl2, 2mM CaCl2). Binding of the different peptides at serial dilution was conducted in TSCMP (TSCM + 2% (w/v) PEG 600) pH 6.4 for 1 hour at 37°C (100ul per well). Plates were washed three times with fresh TSCM pH 6.4 (200ul per well) and SAV-HRP was added at a dilution of 1/8000 (100ul per well) for 20min at room temperature. Following 3 washes with PBS pH 7.4,

3,3',5,5'-tetramethylbenzidine (TMB) substrate was added for 10min (100ul per well), protected from light. The reaction was stopped by addition of 100μl of 1M $H_2SO_4$ and the absorbance at 450nm was measured.

The GAGs tested in this example were: C4Str: chondroitin-4-sulfate(tracheal); C4Sns: chondroitin-4-sulfate (nasal); CSPG: chondroitin sulfate proteoglycan; C6S: chondroitin-6-sulfate; HA: hyaluronic acid; HEP: heparin; HS: heparan sulfate; DS: dermatan sulfate; PBS: PBS negative control.

The results shown in Figure 1 suggest that the 51-hSC-Mal-Biotin peptide binds with the highest affinity to C4S and C6S and moderate affinity for heparan sulfate (HS).

### Example 2 - Binding of compounds $(51\text{-hS})_2$KC-Mal-Biotin, $(51\text{-hS})_2$KS-(51-hS)KC-Mal-Biotin and $[(51\text{-hS})_2\text{KS}]_2$KK(Biotin) to purified C4S

The aim of this study was to determine the binding affinity of compounds according to the invention comprising a biotin moiety and either 2, 3 or 4 peptide moieties to the purified C4S target in comparison to the modified control protein VAR2-Biotin. To this end, an ELISA procedure was employed.

The compounds tested in this Example are:

$(51\text{-hS})_2$KC-Mal-Biotin:
Two copies of SEQ ID NO:37 linked with the structure of SEQ ID NO:31 and further comprising the reactive moiety L-cys conjugated to biotin-maleimide (N-Biotinoyl-N'-(6-maleimidohexanoyl)hydrazide) (see preparative Example 3).

$(51\text{-hS})_2$KS-(51-hS)KC-Mal-Biotin:
Three copies of SEQ ID NO:37 linked with the structure of SEQ ID NO:33 and further comprising the reactive moiety L-cys conjugated to biotin-maleimide (N-Biotinoyl-N'-(6-maleimidohexanoyl)hydrazide) (see preparative Example 3).

$[(51\text{-hS})_2\text{KS}]_2$KK(Biotin):
Four copies of SEQ ID NO:37 linked with a structure comprising SEQ ID NO:22.

ELISA plates were coated with purified C4S (CS-A obtained from nasal bovine, Sigma) in PBS pH 7.4 for 2 hours at 37°C. Plates were blocked overnight at 4°C with 200 μl of PBS pH 7.4, Tween 0.05% + BSA 3% + PEG400 2% , then washed twice with fresh TSCM buffer pH 6.4 ((Tris 20 mM, NaCl 150 mM, BSA 1%, Tween 0.05%, 2 mM $MgCl_2$, 2mM $CaCl_2$). Binding of the different peptides at serial dilution was conducted in TSCMP (TSCM + 2% (w/v) PEG 600) pH 6.4 for 1 hour at 37°C (100μl per well). Plates were washed three times with fresh TSCM pH 6.4 (200μl per well) and SAV-HRP was added at a dilution of 1/8000 (100μl per well) for 20min at room temperature. Following 3 washes with PBS pH 7.4, 3,3' ,5,5' -tetramethylbenzidine (TMB) substrate was added for 10min (100μl per well), protected from light. The reaction was stopped by addition of 100μl of 1M $H_2SO_4$ and the absorbance at 450nm was measured.

The results are shown in Figure 2. Figure 2 shows that the binding affinity of the tested compounds increase with the degree of peptide oligomerization. In particular, compounds comprising three or four copies of the peptide bind to C4S with higher affinity than compounds comprising only two copies of the peptide.

### Example 3 - Binding of $(51\text{-hS})_2$KC-Mal-Biotin, $(51\text{-hS})_2$KS-(51-hS)KC-Mal-Biotin and $[(51\text{-hS})_2\text{KS}]_2$KK(Biotin) to CHO cells

The aim of this experiment was to the determine binding affinity of $(51\text{-hS})_2$KC-Mal-Biotin, $(51\text{-hS})_2$KS-(51-hS)KC-Mal-Biotin and $[(51\text{-hS})_2\text{KS}]_2$KK(Biotin) (see Example 2 for explanation) for CHO-K1 - wild type cells with GAG present. As a control, the binding affinity of the same compounds was determined for the GAG-deficient mutant cell line CHO-pgsA-745.

Cell lines in exponential growth phase were cultured in complete culture medium with 10% FBS and grown in 75$cm^2$ cell culture flasks. On the day of the experiment, cells were harvested and transferred in V-bottom culture plates, washed with PBS 1X supplemented with BSA 3% and then incubated with $(51\text{-hS})_2$KC-Mal-Biotin, $(51\text{-hS})_2$KS-(51-hS)KC-Mal-Biotin, $[(51\text{-hS})_2\text{KS}]_2$KK(Biotin) and VAR2-Biotin for 30min at 4°C in PBS 1X supplemented with BSA 3% (and eventually PEG600 2%) at various dilutions (serial dilutions).

After the incubation, cells were washed in PBS 1X supplemented with BSA 3%. For visualization, cells were incubated for 15min at 4°C with streptavidin coupled to 680RD fluorochrome. After the incubation, cells were washed in PBS 1X supplemented with 3% BSA and then incubated for at least 15min at 4°C with 4',6-diamidino-2-phenylindole (DAPI) (Thermo Fisher Scientific) in order to exclude dead cells. Samples were directly applied to a flow cytometer. Data generated on the flow cytometer was analyzed using Flowjo™ 10.6.0. Labeled peptide used in this assay are shown in Table 6.

Table 6

| Peptide/Compound | Concentration (nM) | Medium | Timepoint (min) and Temperature (°C) |
|---|---|---|---|
| **[(51-hS)$_2$KS]$_2$KK(Biotin)** | 1000 | Medium + BSA 3% + PEG600 2% | 30min 4°C |
| | 200 | | |
| **(51-hS)$_2$KS-(51-hS)KC-Mal-Biotin** | 40 | | |
| | 8 | | |
| **VAR2-Biotin** | 1.6 | | |

Results presented in Figure 3 show that binding on CHO-K1 cells indicates a clear hierarchy between the three tested compounds. Indeed, [(51-hS)$_2$KS]$_2$KK(Biotin) shows highest binding affinity for CHO-K1 cells. [(51-hS)$_2$KS]$_2$KK(Biotin) binds 2.8x better than (51-hS)$_2$KS-(51-hS)KC-Mal-Biotin which binds itself 17.9x better than (51-hS)$_2$KC-Mal-Biotin (Figure 3A). Moreover, binding of compounds on CHO-pgsA-745, which are defective in GAG expression, reveals that all peptides are selective for GAGs since fluorescence of (51-hS)$_2$KC-Mal-Biotin, (51-hS)$_2$KS-(51-hS)KC-Mal-Biotin, and [(51-hS)$_2$KS]$_2$KK(Biotin) is reduced 4.6-fold, 209.9-fold and 5.7-fold, respectively, as compared with fluorescence on CHO-K1 cells (Figure 3B).

**Example 4 - Impact on binding of branching modifications of [(51-hS)$_2$KS]$_2$KC targeting peptides on CHO cells**

The aim of this study was to test whether the introduction of PEG1 or PEG5 in the structure of [(51-hS)$_2$KS]$_2$KC-Mal-680RD was impacting the binding and specificity towards GAGs on CHO-K1 (WT) and CHO-pgsA-745 (GAG-) cells.

The compounds tested in this Example are:

[(51-hS)$_2$KS]$_2$KC-Mal-680RD:
Four copies of SEQ ID NO:37 linked with the structure of SEQ ID NO:35 and further comprising the reactive moiety L-Cys conjugated to the dye IRDye 680RD Maleimide (see Preparative Example 4. Compounds containing (PEG1) or (PEG5) building blocks were prepared according to analogous protocol.

[(51-hS)$_2$KS]$_2$K(PEG1)C-Mal-680RD:
Four copies of SEQ ID NO:37 linked with a structure comprising SEQ ID NO:5, wherein n is 1, and further comprising the reactive moiety L-Cys conjugated to the dye IRDye 680RD Maleimide.

[(51-hS)$_2$K(PEG1)]$_2$KC-Mal-680RD:
Four copies of SEQ ID NO:37 linked with a structure comprising SEQ ID NO:7, wherein n is 1, and further comprising the reactive moiety L-Cys conjugated to the dye IRDye 680RD Maleimide.

[(51-hS)$_2$K(PEG5)]$_2$KC-Mal-680RD:
Four copies of SEQ ID NO:37 linked with a structure comprising SEQ ID NO:7, wherein n is 5, and further comprising the reactive moiety L-Cys conjugated to the dye IRDye 680RD Maleimide.

Tumor cell lines in exponential growth phase were cultured in complete culture medium with 10% FBS and grown in 75cm$^2$ cell culture flasks. On the day of the experiment, cells were harvested and transferred in V-bottom culture plates, washed with PBS 1X supplemented with BSA 3% and then incubated with compounds of the present invention coupled with 680RD fluorochrome for 30min at 4°C in PBS 1X supplemented with BSA 3% and PEG600 2% at various dilutions (serial dilutions).

After the incubation, cells were washed in PBS 1X supplemented with BSA 3%.

After the incubation, cells were washed in PBS 1X supplemented with 3% BSA and then incubated for at least 15min at 4°C with DAPI (Thermo Fisher Scientific) in order to exclude dead cells and samples are directly acquired on a flow cytometer. Data generated on the flow cytometer was analyzed using Flowjo™ 10.6.0.

The reagents used in the experiment are presented in Tables 7 and 8

Table 7 Peptides with Peg1 design

| Peptide/Compound | Concentration (nM) | Medium | Timepoint (min) and Temperature (°C) |
|---|---|---|---|
| $[(51\text{-hS})_2KS]_2KC$-Mal-680RD | 1000 | Medium + BSA 3% + PEG600 2% | 30min 4°C |
| | 200 | | |
| $[(51\text{-hS})_2KS]_2K$(PEG1)C-Mal-680RD | 40 | | |
| | 8 | | |
| $[(51\text{-hS})_2K(\text{PEG1})]_2KC$-Mal-680RD | 1.6 | | |

Table 8 Peptides with Peg5 design

| Peptide/Compound | Concentration (nM) | Medium | Timepoint (min) and Temperature (°C) |
|---|---|---|---|
| $[(51\text{-hS})_2KS]_2KC$-Mal-680RD | 1000 | Medium + BSA 3% + PEG600 2% | 30min 4°C |
| | 500 | | |
| | 250 | | |
| $[(51\text{-hS})_2K(\text{PEG5})]_2KC$-Mal-680RD | 125 | | |
| | 63 | | |
| | 31 | | |
| | 16 | | |

These experiments clearly indicate that the introduction of a PEG1 or PEG5 at defined positions has no negative impact on binding and selectivity of $[(51\text{-hS})_2KS]_2KC$-Mal-680RD on CHO-K1 and CHO-pgsA-745 cells (Figure 4A and 4B). Instead it leads to an increased binding as observed with $[(51\text{-hS})_2K(\text{PEG1})]_2KC$-Mal-680RD and $[(51\text{-hS})_2K(\text{PEG5})]_2KC$-Mal-680RD, as shown in Figures 4 and 5. This shows that both the solubility and the binding of instant compounds could potentially be improved by the addition of these PEG molecules.

**Example 5 - Binding of compounds of the invention to tumor cell lines**

The aim of this study was to determine the affinity of the peptide compounds of the present invention to tumor cells, in particular to HCT-116 (colorectal), SKOV-3 (ovarian), lymphoma (U937) and leukemia (HL-60) cell lines to characterize the binding affinity of Phi targeting peptide towards tumoral cells.

In this Example, the same compounds and controls were used as in Example 2.

Tumor cell lines in exponential growth phase were cultured in complete culture medium with 10% FBS and grown in 75cm$^2$ cell culture flasks. On the day of the experiment, cells were harvested and transferred in V-bottom culture plates, washed with PBS 1X supplemented with BSA 3% and then incubated with the compounds for 30min at 4°C in PBS 1X supplemented with BSA 3% and PEG600 2% at various dilutions (serial dilutions).

After the incubation, cells were washed in PBS 1X supplemented with BSA 3%. For biotinylated compounds, cells are then incubated for 15min at 4°C with streptavidin coupled to 680RD fluorochrome.

After the incubation, cells were washed in PBS 1X supplemented with 3% BSA and then incubated for at least 15min at 4°C with DAPI (Thermo Fisher Scientific) in order to exclude dead cells and samples are directly applied to a flow cytometer. Data generated using the flow cytometer was analyzed using Flowjo™ 10.6.0 Fluorescence geometric mean (via the use of streptavidin-680RD) of samples was standardized by subtracting the fluorescence geometric mean of the negative control (no compound) and the addition of 1. Experimental design is outlined in Table 9.

Table 9

| Peptide/Compound | Concentration (nM) | Medium | Timepoint (min) and Temperature (°C) |
|---|---|---|---|
| **[(51-hS)$_2$KS]$_2$KK(Biotin)** | 1000/5000 | Medium + BSA 3% + PEG600 2% | 30min 4°C |
| | 200/1581 | | |
| **(51-hS)$_2$KS-(51-hS)KC-Mal-Biotin** | 40/500 | | |
| **(51-hS)$_2$KC-Mal-Biotin** | 8/158 | | |
| **VAR2-Biotin** | 1.6/50 | | |

Binding in PBS 1X supplemented with BSA 3% on HL-60 and U937 cells indicates a clear hierarchy between the four tested compounds. Indeed, VAR2-Biotin is the best binder on both cell lines. VAR2-Biotin binds 5 to 5.5x better than [(51-hS)$_2$KS]$_2$KK(Biotin) which binds itself between 4x (HL-60 cells) and 3x (U937 cells) better than (51-hS)$_2$KS-(51-hS)KC-Mal-Biotin. (51-hS)$_2$KC-Mal-Biotin binding is reduced by at least 10x as compared with (51-hS)$_2$KS-(51-hS)KC-Mal-Biotin (Fig. 6) Further, in the analogous experiment wherein (21-hS)$_2$KC-Mal-Biotin, (51-hS)2KS-(51-hS)KC-Mal-Biotin, and [(51-hS)$_2$KS]$_2$KK(Biotin) were used, the oligomerization increased binding affinity of the peptides to colorectal cell line, HCT116, as depicted in Fig. 7, was demonstrated.

**Example 6 - Internalization of [(51-hS)$_2$KS]$_2$KC-Mal-Alexa488 in adherent cells**

The aim of this study was the assessment of internalization capacity of [(51-hS)2KS]2KC-Mal-Alexa488 and comparison with internalization capacity of VAR2-Alexa488 control in adherent tumor cells, colon carcinoma HCT116.

The compounds tested in this Example are:

[(51-hS)$_2$KS]$_2$KC-Mal-Alexa488:
Four copies of SEQ ID NO:37 linked with the structure of SEQ ID NO:35 and further comprising the reactive moiety L-Cys conjugated to the dye Alexa Fluor 488 $C_5$ maleimide (see preparative Example 5).

Alexa488-COOH

VAR2-Alexa488

Adherent cell lines in exponential growth phase were cultured in complete culture medium with 10% FBS and grown in flat-bottom culture plates. On the day of the experiment, cells were washed with culture medium supplemented with 3% BSA (Sigma-Aldrich) and then incubated with peptides coupled with A488 fluorochrome for various timepoints (from 5 to 160min) at a single concentration or at various dilutions (serial dilutions) at a determined timepoint at 37°C in the corresponding culture medium supplemented with 3% BSA. After the incubation, cells were washed in PBS 1X and detached using trypsin. Following trypsinization, cells were washed in PBS 1X supplemented with 3% BSA and then incubated for at least 15min at 4°C with DAPI (Thermo Fisher Scientific) in order to exclude dead cells. Right before acquisition on a flow cytometer, samples were divided in two. The first series of samples was acquired directly on a flow cytometer whereas the second series was supplemented with Trypan Blue in order to observe the internal fluorescence only. The percentage of internalization was further determined.

Fluorescence geometric mean of samples is standardised by subtracting fluorescence geometric mean of negative control (no compound) and the addition of 1. Experimental design is summarized in Table 10.

**Table 10.**

| Peptide/Compound | Concentration (nM) | Medium | Timepoint (min) |
|---|---|---|---|
| $[(51\text{-hS})_2KS]_2$KC-Mal-Alexa488 | 200 | Medium + BSA 3% | 160 |
| | | | 80 |
| Carboxylic acid-Alexa488 | | | 40 |
| | | | 20 |
| VAR2-Alexa488 | | | 10 |
| | | | 5 |
| | | | 0 |

$[(51\text{-hS})_2KS]_2$KC-Mal-Alexa488 is internalised to a higher extent than VAR2-Alexa488 in HCT-116 cells, 85% vs. 75% respectively and faster: 70% of $[(51\text{-hS})_2KS]_2$KC-Mal-Alexa488 is internalised after 40min vs. 120min for VAR2-Alexa488) - see Figure 8.

**Example 7 - Internalization of peptides in suspension cells**

The aim of this study was to determine internalisation capacity of $[(51\text{-hS})_2KS]_2$KC-A488 over time in L-363, H929 and RPMI-8226 cells as compared with VAR2-Alexa488 and carboxylic acid-Alexa488 controls. The same compounds were used as in Example 6.

Suspension cell lines in exponential growth phase were cultured in complete culture medium with 10% FBS and grown in 75cm$^2$ cell culture flasks (supplemented with 10% FBS, Sodium Pyruvate 1mM and β-mercaptoethanol 0.05mM for H-929 cells). On the day of the experiment, cells were transferred in V-bottom culture plates, washed with culture medium supplemented with 3% BSA (Sigma-Aldrich) and then incubated with compounds for different times (from 5 to 160min - Table 11) at a single concentration or at different concentrations (serial dilutions) at a determined timepoint at 37°C in the corresponding culture medium supplemented with 3% BSA. After the incubation, cells were washed in PBS 1X supplemented with 3% BSA and then incubated for at least 15min at 4°C with DAPI (Thermo Fisher Scientific) in order to exclude dead cells.

Right before application onto a flow cytometer, samples were split in two. The first series of samples was applied directly onto a flow cytometer whereas the second series was supplemented with Trypan Blue in order to observe the internal fluorescence only. Based on this effect, the percentage of internalization was determined.

Fluorescence geometric mean of samples is standardised by subtracting fluorescence geometric mean of negative control (no compound) and the addition of 1.

**Table 11**

| Peptide/Compound | Concentration (nM) | Medium | Timepoint (min) |
|---|---|---|---|
| $[(51\text{-hS})_2KS]_2$KC-Mal-Alexa488 | 200 | Medium + BSA 3% | 160 |
| | | | 80 |
| Carboxylic acid-Alexa488 | | | 40 |
| | | | 20 |
| VAR2-Alexa488 | | | 10 |
| | | | 5 |
| | | | 0 |

Contrary to adherent cells (Example 7), the capacity of cells growing in suspension to internalize the compounds of the present invention depends on the cell type. Leukemia cells such as HL-60 or K562 internalize compounds well (70-90% of internalization reached after 40 to 80 min - Figure 9), multiple myeloma cells, such as L363 and H929 internalize it less and at a lower rate (40-65% after 160min - Figure 10).

**Example 8 - Internalization of peptides is GAG dependent**

The aim of this experiment was to determine the internalization of the $[(51\text{-hS})_2\text{KS}]_2$KC-Mal-Alexa488 to the wild-type CHO-K1 cells, which express GAG and the mutant cell line CHO-pgsA-745, which lacks GAG expression.

The compounds tested in this Example are:

$[(51\text{-hS})_2\text{KS}]_2$KC-Mal-Alexa488:

Four copies of SEQ ID NO:37 linked with the structure of SEQ ID NO:35 and further comprising the reactive moiety L-Cys conjugated to the dye Alexa Fluor 488 $C_5$ maleimide (see preparative Example 5).

Adherent cell lines in exponential growth phase were cultured in complete culture medium with 10% FBS and grown in flat-bottom culture plates. On the day of the experiment, cells were washed with culture medium supplemented with 3% BSA (Sigma-Aldrich) and then incubated with compounds coupled with A488 fluorochrome for different times (from 5 to 160min) at a single concentration or at various dilutions (serial dilutions) at a determined timepoint at 37°C in the corresponding culture medium supplemented with 3% BSA.

After the incubation, cells were washed in PBS 1X and detached using trypsin. Following trypsinization, cells were washed in PBS 1X supplemented with 3% BSA and then incubated for at least 15min at 4°C with DAPI (Thermo Fisher Scientific) in order to exclude dead cells. Right before application onto a flow cytometer, samples were split in two. The first series of samples was acquired directly on a flow cytometer whereas the second series was supplemented with Trypan Blue to quantify only the internal fluorescence. Using this effect, the percentage of internalization was determined.

As presented in Figure 11, internalization of the peptide is efficient only in the CHO-K1 cell line, reaching maximum internalization after 70 - 80 min, whereas no internalization is observed in the mutant cells. Therefore it is concluded that internalization of the fluorescently labelled peptide is GAG dependent.

**Example 9** - ***In vivo*** **biodistribution of $[(51\text{-hS})_2\text{KS}]_2$KC-Mal-680RD in an HCT116 xenografted mice model**

The aim of the study was to evaluate the *in vivo* biodistribution of a fluorescent-labelled compound, namely $[(51\text{-hS})_2\text{KS}]_2$KC-Mal-680RD, in an HCT 116 tumor xenografted mouse model, using Optical Imaging.

The compounds tested in this Example are:

$[(51\text{-hS})_2\text{KS}]_2$KC-Mal-680RD:
Four copies of SEQ ID NO:37 linked with the structure of SEQ ID NO:35 and further comprising the reactive moiety L-Cys conjugated to the dye IRDye 680RD Maleimide (see preparative Example 4).

$[(51\text{-hS})_2\text{KS}]_2$KC:
Four copies of SEQ ID NO:37 linked with the structure of SEQ ID NO:35 and further comprising the unconjugated reactive moiety L-Cys.

For this purpose, HCT 116 cells were subcutaneously inoculated into the shoulder of athymic nude mice, and then the fluorescently-labelled compound was administered intravenously.

Optical Imaging was used to evaluate fluorescent signals in mice 0.5, 1, 3, 8, 24, and 72 hours after the administration of $[(51\text{-hS})_2\text{KS}]_2$KC-Mal-680RD at 3 different concentrations. Flow cytometry was also used to evaluate the tumor target expression in xenografted mice.

HCT-116 cells were grown in McCoy's 5a medium supplemented with 10% foetal bovine serum, 100 IU/mL penicillin and 100 μg/mL streptomycin. The trypan-blue exclusion test was used to assess cell viability before cells inoculation. An aliquot (10 μL) of cell suspension was mixed with an equal amount of 0.4% trypan blue solution in PBS. 10 μL of the final solution was introduced in a counting chamber (hemocytometer). Viable cells appear round and bright, while damaged cells appear deep blue because of the incorporation of the trypan dye. Cell viability was expressed as % of viable cells over total cells). Cell cultures with viability of less than 70% were discarded.

HCT 116 cells were collected and washed two times with PBS. One million of HCT 116 cells were suspended in 1:1 Matrigel™: McCoy's 5a (total volume 0.15 mL) and then injected subcutaneously into the shoulder of each female mouse of groups A, B, C.

Animals were randomly assigned to the following experimental groups, Table 12.

**Table 12.** animal treatments outlines; *this group received 4 nmoles of fluorescent labeled $[(51\text{-hS})_2KS]_2KC$-Mal-680RD and 16nmoles of unlabeled $[(51\text{-hS})_2KS]_2KC$.

| Group | No. of animals | Animal ID | Test Item | Dose fluorochrome (nmol/mouse) | Conc. [uM] | Injected volume [μL] | Admin. route | Analysis time (h) |
|---|---|---|---|---|---|---|---|---|
| A | 3F | 1-2-3 | $[(51\text{-hS})_2KS]_2KC$ -Mal-680RD | 4 | 40 | 100 | i.v. | Imaging: 0.5, 1,3, 8, 24, 72 |
| B | 3F | 4-5-6 | $[(51\text{-hS})_2KS]_2KC$ -Mal-680RD | 20 | 200 | 100 | i.v. | Imaging: 0.5, 1, 3, 8, 24, 72 |
| C | 3F | 7-8-9 | $[(51\text{-hS})_2KS]_2KC$ -Mal-680RD + $[(51\text{-hS})_2KS]_2KC$ | 4* | 40 + 160 | 100 | i.v. | Imaging: 0.5, 1, 3, 8, 24, 72 |

All animals were dosed once according to Table 12 when the tumors reached approximately 500-600 $mm^3$. The solutions were injected intravenously via the tail vein at the administration volume of 100 μL. After each injection the tail vein was flushed with 100μL of sterile saline solution. All animals were individually inspected before the treatment and 0.5, 1, 3, 8, 24, and 72 hours after treatment.

Clinical signs of the animals, such as posture, movement, breath, external body appearance, and any other relevant clinical sign were recorded. Mice of groups A, B, and C were anesthetized under 3-4 % Sevoflurane gas anaesthesia (O2 95.0 %) and imaged 0.5, 1, 3, 8, 24, and 72 hours post treatments. Mice were irradiated with filtered light of wavelength 675 nm and an image of emission light intensity was collected at 720 nm wavelength. Field of View, fstop, Binning and Exposition Time were determined during experimental setting. Regions of interest (ROIs) were selected by an operator over the acquired supine, dorsal, and side views. The signal intensity within the ROIs was determined automatically by the software. At the end of the experiment the mice were sacrificed and tumors were excised to measure ex vivo the fluorescent signal intensity.

The *in vivo* biodistribution of $[(51\text{-hS})_2KS]_2KC$-Mal-680RD in group A, B, and C showed the presence of a strong signal, expressed as Average Radiant Efficiency, in the abdominal region, mainly liver and intestine, suggesting their involvement in the elimination pathways of the compound independently of the administered dose or the presence of blocking compound ($[(51\text{-hS})_2KS]_2KC$).

The *in vivo* biodistribution of $[(51\text{-hS})_2KS]_2KC$-Mal-680RD also showed the presence of a signal, expressed as Average Radiant Efficiency, in the tumor of all groups, mainly in groups A and B. The higher tumor signal in group B is thus dependent on the administered dose and on the presence of blocking compound ($[(51\text{-hS})_2KS]_2KC$). In group A and B and at all imaging times, the tumor to muscle ratio demonstrated a higher signal in tumor, almost two- fold, respective to muscles (Figures 12 and 13).

The *ex vivo* ROI measurements in tumors of all groups showed, as expected, an higher fluorescence signal intensity of group B compared to either group A and C (Figure 14).

Further embodiments of the invention are disclosed in the following numbered items.

1. A compound according to formula (I):

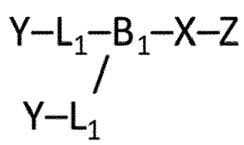

(I)

and all pharmaceutically acceptable salts, polymorphs, and cocrystals thereof, wherein

(A) Z represents a reactive moiety, in particular wherein the reactive moiety is suitable for conjugating a payload to the compound;

(B) X is absent or represents a chemical spacer comprising a single amino acid residue, a dipeptide and/or a moiety comprising 1 to 36 ethylene glycol repeats according to the formula $-NH(CH_2CH_2O)_{1-36}CH_2CO-$;

(C) $B_1$ represents a branching moiety, wherein the branching moiety is derived from an amino acid residue comprising two amino groups and a carboxyl group , wherein $B_1$ is coupled to X through its carboxyl group, and to each $L_1$ through its amino groups;

(D) each $L_1$ independently represents:

(D.i) a single amino acid residue or a dipeptide, preferably wherein the single amino acid residue is L-serine or L-glycine or wherein the dipeptide consists of or comprises L-serine and/or L-glycine;

(D.ii) a moiety comprising 1 to 36 ethylene glycol repeats according to the formula $-NH(CH_2CH_2O)_{1-36}CH_2CO-$; or

(D.iii) is absent;

and

(E) each Y independently represents $P-L_3-$ or a moiety according to formula (II)

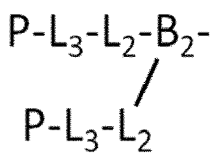

(II)

wherein

(E.i) $B_2$ represents a branching moiety wherein the branching moiety is derived from an amino acid residue comprising two amino groups and a carboxyl group, wherein $B_2$ is coupled to $L_1$ through its carboxyl group, and to each $L_2$ through its amino groups;

(E.ii) each $L_2$ independently represents:

(E.ii.a) a single amino acid residue or a dipeptide, preferably wherein the single amino acid residue is L-serine or L-glycine or wherein the dipeptide consists of or comprises L-serine and/or L-glycine, or
(E.ii.b) is absent;

(E.iii) $L_3$ represents a single amino acid residue, in particular a single D-amino acid residue, in particular a D-histidine residue, coupled to $L_2$ through its carboxylic group and coupled to P through its amino-group, and

(E.iv) P represents a peptide moiety, wherein each P independently represents a polypeptide comprising the amino acid sequence $(AA_1)-(AA_2)-(AA_3)-(AA_4)-(AA_5)-(AA_6)-(AA_7)-(AA_8)-(AA_9)$,

(E.iv.a) wherein $(AA_1)$ represents a single amino acid residue with a positively charged amino acid side chain, or wherein $(AA_1)$ is absent;

(E.iv.b) wherein $(AA_5)$ and $(AA_7)$ each represent a single amino acid residue comprising a positively charged amino acid side chain, a hydrophobic amino acid side chain or an aromatic amino acid side chain;

(E.iv.c) wherein $(AA_2)$, $(AA_3)$, $(AA_4)$ and $(AA_6)$ each represent a single amino acid residue;

(E.iv.d) wherein $(AA_8)$ represents a single amino acid residue, or wherein $(AA_8)$ is absent;

(E.iv.e) wherein ($AA_9$) represents a single amino acid residue comprising a positively charged amino acid side chain, a hydrophobic amino acid side chain or an aromatic amino acid side chain, or wherein ($AA_9$) is absent; and

(E.iv.f) wherein ($AA_8$) and ($AA_9$) are either both present or both absent, wherein when ($AA_8$) and ($AA_9$) are both absent, ($AA_1$), ($AA_3$), ($AA_5$) and ($AA_7$) represent a single amino acid residue comprising a positively charged amino acid side chain;

(E.v) wherein P comprises at least 3 amino acid residues comprising a positively charged amino acid side chain; and

(E.vi) wherein each P is coupled to an α-amino group of an $L_3$ residue through the α-carboxyl group of residue ($AA_7$) and/or ($AA_9$).

2. The compound according to item 1, wherein when Y represents

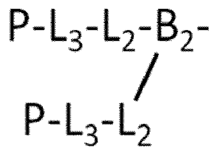

(II)

wherein $L_1$ is not absent.

3. The compound according to item 1 or 2, wherein the branching moiety $B_1$ and/or $B_2$ is derived from L-lysine; L-ornithine, α-aminoglycine, α,γ-diaminopropionic acid, α,γ-diaminobutyric acid, and amino acid according to formula

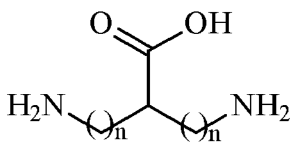

wherein n is selected from 1, 2, 3, and 4.

4. The compound according to item 3, wherein the branching moiety $B_1$ and/or $B_2$ is derived from L-lysine or L-ornithine; wherein when $L_1$ and/or $L_2$ are coupled to an amino group comprised in the amino acid side chain of $B_1$ and/or $B_2$, respectively, $L_1$ and/or $L_2$ are not absent.

5. The compound according to any one of items 1 to 4, wherein the reactive moiety Z is an amino acid residue, in particular wherein the amino acid is selected from a group consisting of: L-cysteine, L-C-propargylglycine, L-biocytin, and $NH_2CH_2CONHSO_2(CH_2)_3COOH$, or wherein the reactive moiety Z is L-cysteamine or 4-aminobutanethiol.

6. The compound according to any one of items 1 to 5, wherein the single amino acid comprised in the chemical spacer X is L-serine, or, wherein the dipeptide comprised in the chemical spacer X comprises or consists of L-serine.

7. The compound according to any one of items 1 to 6, wherein the compound comprises the structure:

(D-His)-(L-Ser)-(L-Lys)-(L-Cys) (SEQ ID NO:1);
(D-His)-(L-Ser)-(L-Lys)-(L-Ser)-(L-Lys)-(L-Cys) (SEQ ID NO:2);
(D-His)-(L-Ser)-(L-Lys)-(βAla)-(L-Ser)-(L-Lys)-(L-Cys) (SEQ ID NO:3);
(D-His)-(L-Ser)-(L-Lys)-(L-Ser)-(βAla)-(L-Lys)-(L-Cys) (SEQ ID NO:4);
(D-His)-(L-Ser)-(L-Lys)-(L-Ser)-(L-Lys)-(PEG1)-(L-Cys) (SEQ ID NO:5);
(D-His)-(L-Ser)-(L-Lys)-(PEG1/5/16/36)-(L-Cys) (SEQ ID NO:6);
(D-His)-(L-Ser)-(L-Lys)-(PEG1/3/5)-(L-Lys)-(L-Cys) (SEQ ID NO:7);
(D-His)-(PEG2/5)n-(L-Lys)-(L-Cys) (SEQ ID NO:8);

(D-His)-(L-Ser)-(diAA)-(L-Ser)-(diAA)-(L-Cys) (SEQ ID NO:9);
(D-His)-(L-Ser)-(diAA)-(L-Cys) (SEQ ID NO:10);
(D-His)-(L-Ser)-(L-Lys)-(Gly)-(L-Lys)-(L-Cys) (SEQ ID NO:11);
(D-His)-(Gly)-(L-Lys)-(L-Ser)-(L-Lys)-(L-Cys) (SEQ ID NO:12);
(D-His)-(Gly)-(L-Lys)-(Gly)-(L-Lys)-(L-Cys) (SEQ ID NO:13);
(D-His)-(Gly)-(L-Lys)-(L-Cys) (SEQ ID NO:14);
(D-His)-(L-Ser)-(diAA)-(Gly)-(diAA)-(L-Cys) (SEQ ID NO:15);
(D-His)-(Gly)-(diAA)-(L-Ser)-(diAA)-(L-Cys) (SEQ ID NO:16);
(D-His)-(Gly)-(diAA)-(Gly)-(diAA)-(L-Cys) (SEQ ID NO:17);
(D-His)-(Gly)-(diAA)-(L-Cys) (SEQ ID NO:18);
(D-His)-(L-Ser)-(L-Lys)-(L-PAG) (SEQ ID NO:19);
(D-His)-(L-Ser)-(L-Lys)-(L-Ser)-(L-Lys)-(L-PAG) (SEQ ID NO:20);
(D-His)-(L-Ser)-(L-Lys)-(L-Bct) (SEQ ID NO:21);
(D-His)-(L-Ser)-(L-Lys)-(L-Ser)-(L-Lys)-(L-Bct) (SEQ ID NO:22);
(D-His)-(L-Lys)-(L-Cys) (SEQ ID NO:23);
(D-His)-(L-Lys)-(L-Ser)-(L-Lys)-(L-Cys) (SEQ ID NO:24);
(D-His)-(L-Lys)-(PEG1)-(L-Cys), wherein n is 1 (SEQ ID NO:25);
(D-His)-(L-Lys)-(L-Ser)-(L-Lys)-(PEG1)-(L-Cys), (SEQ ID NO:26);
(D-His)-(L-Ser)-(L-Ser)-(L-Lys)-(L-Cys) (SEQ ID NO:27);
(D-His)-(L-Ser)-(L-Ser)-(L-Lys)-(L-Ser)-(L-Lys)-(L-Cys) (SEQ ID NO:28);
(D-His)-(L-Ser)-(L-Lys)-(L-Ser)-(L-Ser)-(L-Cys) (SEQ ID NO:29); or
(D-His)-(L-Ser)-(L-Lys)-(L-Ser)-(L-Lys)-(L-Ser)-(L-Ser)-(L-Cys) (SEQ ID NO:30), wherein (PEG1/5/16/36). (PEG1/3/5), (PEG2/5) and (PEG1) represent a moiety according to the formula $-NH(CH_2CH_2O)_nCH_2CO-$, wherein n is: 1, 5, 16 or 36 for (PEG1/5/16/36); 1, 3 or 5 for (PEG1/3/5); 2 or 5 for (PEG2/5); 1 for (PEG1),

and wherein diAA represents an amino acid according to formula

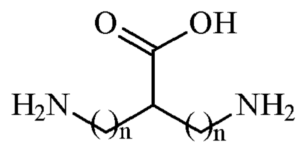

wherein n is selected from 1, 2, 3, and 4.

8. The compound according to any one of items 1 to 7, wherein the compound comprises the structure:
(D-His)-(L-Ser)-(L-Lys[ε-N-(L-Ser)-(D-His)])-(L-Cys) (SEQ ID NO:31);

(D-His)-(L-Ser)-(L-Lys[ε-N-(L-Ser)-(L-Lys[ε-N-(L-Ser)-(D-His)]-(L-Ser)-(D-His)])-(L-Cys) (SEQ ID NO:32);

(D-His)-(L-Ser)-(L-Lys[ε-N-(L-Ser)-(D-His)])-(L-Ser)-(L-Lys[ε-N-(L-Ser)-(D-His)])-(L-Cys) (SEQ ID NO:33);

(D-His)-(L-Ser)-(L-Lys[ε-N-(L-Ser)-(L-Lys[ε-N-(L-Ser)-(D-His)])-(L-Ser)-(D-His)])-(L-Ser)-(L-Lys[ε-N-(L-Ser)-(D-His)])-(L-Cys) (SEQ ID NO:34);

(D-His)-(L-Ser)-(L-Lys[ε-N-(L-Ser)-(D-His)])-(L-Ser)-(L-Lys[ε-N-(L-Ser)-(L-Lys[ε-N-(L-Ser)-(D-His)])-(L-Ser)-(D-His)])-(L-Cys) (SEQ ID NO:35);

or

(D-His)-(L-Ser)-(L-Lys[ε-N-(L-Ser)-(L-Lys[ε-N-(L-Ser)-(D-His)])-(L-Ser)-(D-His)])-(L-Ser)-(L-Lys[ε-N-(L-Ser)-(L-Lys[ε-N-(L-Ser)-(D-His)])-(L-Ser)-(D-His)])-(L-Cys) (SEQ ID NO:36).

9. The compound according to any one of items 1 to 8, wherein in at least one, at least two, at least three or all peptide moieties P, at least two of the amino acid residues ($AA_1$), ($AA_5$), ($AA_7$) and ($AA_9$) comprise an amino acid residue with a positively charged amino acid side chain.

10. The compound according to any one of items 1 to 9, wherein in at least one, at least two, at least three or all peptide moieties P, amino acid residue ($AA_9$) comprises a positively charged amino acid side chain, in particular wherein ($AA_9$) is selected from a group consisting of: L-arginine, L-lysine and L-2-amino-4-guanidinobutyric acid.

11. The compound according to any one of items 1 to 10, wherein in at least one, at least two, at least three or all peptide moieties P, at least one of the amino acid residues ($AA_2$) and/or ($AA_5$) and at least one of the amino acid residues ($AA_7$) and/or ($AA_8$) comprises a positively charged amino acid side chain.

12. The compound according to any one of items 1 to 11, wherein the amino acid comprising the positively charged amino acid side chain in position ($AA_2$) and/or ($AA_5$) is selected from a group consisting of: L-arginine, D-arginine and L-2-amino-4-guanidinobutyric acid; and/or wherein the amino acid comprising the positively charged amino acid side chain in position ($AA_7$) and/or ($AA_8$) is selected from a group consisting of: L-arginine, L-lysine and L-ornithine.

13. The compound according to any one of items 10 to 12, wherein in at least one, at least two, at least three or all peptide moieties P, between 2 and 5 amino acid residues comprise a hydrophobic and/or aromatic amino acid side chain.

14. The compound according to any one of items 10 to 13, wherein in at least one, at least two, at least three or all peptide moieties P, amino acid residue ($AA_3$) is L-phenylalanine and/or amino acid residue ($AA_6$) is L-isoleucine or D-isoleucine.

15. The compound according to any one of items 10 to 14, wherein at least one, at least two, at least three or all peptide moieties P comprise a sequence independently selected from a group consisting of:

RYFvRIOYR (SEQ ID NO:37);
RRFvYIYRR (SEQ ID NO:38);
RYFvRIYRR (SEQ ID NO:39);
RYFvrIOYR (SEQ ID NO:40);
RYFvRiOYR (SEQ ID NO:41);
RYFvriOYR (SEQ ID NO:42);
RRFvYIYKR (SEQ ID NO:43);
RRFqYIYKR (SEQ ID NO:44);
RYFvRIYKR (SEQ ID NO:45);
RYFvRIYRK (SEQ ID NO:46);
RRFaYIYKR (SEQ ID NO:47);
RRFpYIYKR (SEQ ID N:48);
RYFvRIKYR (SEQ ID NO:49);
RYFqRIKYR (SEQ ID NO:50);
YFvRIOYR (SEQ ID NO:51);
(Agb)YFv(Agb)IKY(Agb) (SEQ ID NO:52);
YFvRIKYR (SEQ ID NO:53);
rYFvRIKYR (SEQ ID NO:54);
RYFvrIKYR (SEQ ID NO:55);
rrFvYIYRR (SEQ ID NO:56);
RSTqRYRVR (SEQ ID NO:57);
RYFvRIKYR (SEQ ID NO:58);

RYFvRIKAR (SEQ ID NO:59);
RAAvRAKYR (SEQ ID NO:60);
RAAvRIKYR (SEQ ID NO:61);
RSTqRYRVR (SEQ ID NO:62);
RSTqRYKVR (SEQ ID NO:63);
RGGgRGKGR (SEQ ID NO:64);
RHHhRHKHR (SEQ ID NO:65);
RWvRVKVR (SEQ ID NO:66);
RLLIRLKLR (SEQ ID NO:67);
RMMmRMKMR (SEQ ID NO:68);
RIIiRIKIR (SEQ ID NO:69); or
RYFVRiKYR (SEQ ID NO:70);

wherein capital letters represent L-amino acids and wherein lower-case letters represent D-amino acids.

16. The compound according to any one of items 1 to 10, wherein at least one, at least two, at least three or all peptide moieties P comprise a positively charged amino acid in position ($AA_1$), ($AA_3$) and ($AA_9$), in particular wherein ($AA_1$) and/or ($AA_9$) are L-arginine and/or wherein ($AA_3$) is L-lysine.

17. The compound according to item 16, wherein at least one, at least two, at least three or all peptide moieties P comprise an amino acid residue comprising a positively charged amino acid side chain in positions ($AA_5$) or ($AA_7$), in particular wherein ($AA_5$) or ($AA_7$) are L-arginine and/or L-lysine.

18. The compound according to item 16 or 17, wherein at least one, at least two, at least three or all peptide moieties P comprise between two and five amino acid residues comprising a hydrophobic and/or aromatic amino acid side chain, in particular wherein the amino acid residues comprising the hydrophobic and/or aromatic side chains are located in positions ($AA_2$), ($AA_4$), ($AA_6$), ($AA_8$) and/or one of positions ($AA_5$) or ($AA_7$).

19. The compound according to any one of items 16 to 18, wherein at least one, at least two, at least three or all peptide moieties P comprise a sequence independently selected from a group consisting of:

RYKvFIKYR (SEQ ID NO:71);
RYKvAIKYR (SEQ ID NO:72);
RYKvRIAYR (SEQ ID NO:73);
RYKvRIFYR (SEQ ID NO:74);
RYKvKFIYR (SEQ ID NO:75);
RYKvFIRYR (SEQ ID NO:76);
RYKvRFIYR (SEQ ID NO:77);
RMKiVMKFR (SEQ ID NO:78); or
RFKfFFKFR (SEQ ID NO:79);

wherein capital letters represent L-amino acids and wherein lower-case letters represent D-amino acids.

20. The compound according to any one of items 1 to 10, wherein at least one, at least two, at least three or all peptide moieties P comprise an amino acid residue comprising a positively charged amino acid side chain in positions ($AA_1$), ($AA_3$), ($AA_5$) and ($AA_7$), in particular wherein ($AA_1$) and/or ($AA_5$) are L-arginine and/or wherein ($AA_3$) and/or ($AA_7$) are L-lysine.

21. The compound according to item 20, wherein at least one, at least two, at least three or all peptide moieties P comprise between two and five amino acid residues comprising a hydrophobic and/or aromatic amino acid side chain, in particular wherein the amino acid residues comprising the hydrophobic and/or aromatic side chains are located in positions ($AA_2$), ($AA_4$) and ($AA_6$), and optionally (AA8) and/or ($AA_9$).

22. The compound according to item 20 or 21, wherein in at least one, at least two, at least three or all peptide moieties P, amino acid residues ($AA_8$) and ($AA_9$) are both absent or are both amino acids comprising a positively charged amino acid side chain, a hydrophobic amino acid side chain and/or an aromatic amino acid side chain.

23. The compound according to any one of items 20 to 22, wherein at least one, at least two, at least three or all

peptide moieties P comprise a sequence independently selected from a group consisting of:

RYKvRIK (SEQ ID NO:80);
RYKvRIKYF (SEQ ID NO:81);
RYKvRIKYA (SEQ ID NO:82);
RYKvRIKHH (SEQ ID NO:83);
RMKiRVK (SEQ ID NO:84);
RMKiRVKFM (SEQ ID NO:85);
RLKIRLKLL (SEQ ID NO:86); or
RYKvRIKYr (SEQ ID NO:87);

wherein capital letters represent L-amino acids and wherein lower-case letters represent D-amino acids.

24. The compound according to any one of items 1 to 23, wherein at least one, at least two, at least three or all peptide moieties P comprise at least one D-amino acid, in particular wherein amino acid residue ($AA_4$) and/or ($AA_6$) is a D-amino acid.

25. The compound according to any one of items 1 to 24, wherein at least one, at least two, at least three or all peptide moieties P comprise not more than one amino acid residue comprising a negatively charged amino acid side chain, in particular wherein none of the amino acid residues in position ($AA_1$) to ($AA_3$) and ($AA_5$) to ($AA_9$) comprise an amino acid residue comprising a negatively charged amino acid side chain.

26. The compound according to any one of items 1 to 25, wherein at least one, at least two, at least three or all peptide moieties P are free of amino acid residues comprising a negatively charged amino acid side chain.

27. The compound according to any one of items 1 to 26, wherein at least one, at least two, at least three or all peptide moieties P comprise not more than one proline residue, in particular wherein none of the amino acid residues in position ($AA_1$) to ($AA_3$) and ($AA_5$) to ($AA_9$) comprise a proline residue.

28. The compound according to any one of items 1 to 27, wherein at least one, at least two, at least three or all peptide moieties P are free of proline residues.

29. The compound according to any one of items 1 to 28, wherein at least two, at least three or all peptide moieties P have identical amino acid sequences.

30. The compound according to any one of items 1 to 29 further conjugated to a payload.

31. The payload conjugate according to item 30, wherein the payload is conjugated to the reactive moiety Z.

32. The payload conjugate according to item 31, wherein the payload is directly conjugated to the reactive moiety Z or wherein the payload is conjugated to the reactive moiety Z via a linker.

33. The payload conjugate according to item 32, wherein the linker comprises a PEG moiety, a moiety derived from cadaverine or an alkyl moiety.

34. The payload conjugate according to any one of items 30 to 33, wherein the payload is a biologically active molecule (BAM) or an imaging agent.

35. The payload conjugate according to item 34, wherein the BAM is a mono- or polysaccharide, a cytotoxic agent, an antineoplastic agent, an anti-inflammatory agent, an anti-viral agent, an anti-bacterial agent or an agent for the treatment of protozoan infections.

36. The payload conjugate according to item 35, wherein the antineoplastic agent is a pro-apoptotic peptide.

37. The payload conjugate according to any one of items 30 to 33, wherein the payload is an imaging agent.

38. The payload conjugate according to item 37, wherein the imaging agent comprises a radionuclide, a fluorescent dye, a chemiluminescent agent, a bioluminescent agent, a spectrally resolvable inorganic fluorescent semiconductor

nanocrystal, a metal nanoparticles, a nanocluster, a paramagnetic metal ion, an enzyme, a colorimetric label, biotin, dioxigenin, a hapten or a protein.

39. A pharmaceutical composition comprising the payload conjugate according to any one of items 30 to 38 and a pharmaceutically acceptable carrier or excipient.

40. The payload conjugate according to any one of items 30 to 38 or the pharmaceutical composition according to item 39 for use as a medicament.

41. The payload conjugate according to any one of items 30 to 38 or the pharmaceutical composition according to item 39 for use in the treatment of cancer.

42. The payload conjugate or the pharmaceutical composition according for use according to item 41, wherein the payload conjugate comprises PUMA, SN-38, 6-thioguanine or a pro-apoptopic peptide.

43. The payload conjugate according to any one of items 30 to 38 or the pharmaceutical composition according to item 39 for use in the treatment of disorders involving leukocytes, wherein the payload conjugate specifically binds to chondroitin-4-sulfate.

44. The payload conjugate or the pharmaceutical composition according for use according to item 43, wherein the disorder involving leukocytes is selected from a neoplastic disease, an inflammatory disease, an autoimmune disease, an immunodeficiency disease, a viral infection, a bacterial infection, a protozoan infection or a parasitic infection.

45. The payload conjugate or the pharmaceutical composition according for use according to item 44, wherein the disorder involving leukocytes is a neoplastic disease, in particular wherein the neoplastic disease is leukemia.

46. The payload conjugate or the pharmaceutical composition according for use according to item 44, wherein the disorder involving leukocytes is a viral infection, in particular wherein the viral infection is caused by HIV.

47. The payload conjugate or the pharmaceutical composition according for use according to item 44, wherein the disorder involving leukocytes is a protozoan infection, in particular wherein the protozoan infection is caused by leishmaniasis.

48. The payload conjugate according to any one of items 30 to 38 or the pharmaceutical composition according to item 39 for use in the treatment of a heart disease or condition, a disease or condition of the ovary or testis, a disease or condition of the central nervous system, or a C6S accumulation disease, wherein the payload conjugate specifically binds to chondroitin-6-sulfate.

49. The payload conjugate according to any one of items 30 to 38 or the pharmaceutical composition according to item 39 for use in the treatment of a corneal disease or condition, a retina disease or condition, a disease or condition of the central nervous system, or a keratan sulfate accumulation disease, wherein the payload conjugate specifically binds to keratan sulfate.

50. The payload conjugate or the pharmaceutical composition according for use according to item 49, wherein the C6S accumulation disease and/or the keratan sulfate accumulation disease is Morquio syndrome.

51. The payload conjugate according to any one of items 30 to 38 or the pharmaceutical composition according to item 39 for use in diagnostics.

52. The payload conjugate according to any one of items 30 to 38 or the pharmaceutical composition according to item 39 for use in the visualization of cells comprising chondroitin-4-sulfate, chondroitin-6-sulfate and/or keratan sulfate on their cell surface and/or tissues comprising cells comprising chondroitin-4-sulfate, chondroitin-6-sulfate and/or keratan sulfate on their cell surface.

53. The payload conjugate or the pharmaceutical composition according for use according to item 52,

(A) wherein the cells comprising chondroitin-4-sulfate are leukocytes;

(B) or wherein the cells and/or tissues comprising chondroitin-6-sulfate are cells and/or tissues of the central nervous system, heart, ovary or testis; or

(C) wherein the cells and/or tissues comprising keratan sulfate are cells and/or tissues of the eye, in particular the cornea, the central nervous system, or the endometrial lining of the uterus.

54. The payload conjugate according to any one of items 30 to 38 or the pharmaceutical composition according to item 39 for use in the diagnosis of diseases involving cells and/or tissues comprising proteoglycans on the surface.

55. The payload conjugate or the pharmaceutical composition according for use according to item 54,

(A) wherein the proteoglycan on the surface of the cells and/or tissues is chondroitin-4-sulfate, and wherein the cells are leukocytes;

(B) wherein the proteoglycan on the surface of the cells and/or tissues is chondroitin-6-sulfate, and wherein the cells and/or tissues are cells and/or tissues of the central nervous system, heart, ovary or testis;

(C) wherein the proteoglycan on the surface of the cells and/or tissues is keratan sulfate, and wherein the cells and/or tissues are cells and/or tissues of the eye, the central nervous system, or the endometrial lining of the uterus.

56. The payload conjugate according to any one of items 30 to 38 or the pharmaceutical composition according to item 39 for use in the diagnosis of diseases associated with elevated levels of proteoglycans on malignant cells, in particular wherein the proteoglycans are chondroitin-4-sulfate, chondroitin-6-sulfate and/or keratan sulfate.

57. The payload conjugate according to any one of items 30 to 38 or the pharmaceutical composition according to item 39 for use in the diagnosis of Morquio syndrome.

## Claims

**1.** A compound according to formula (I):

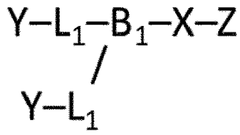

(I)

and all pharmaceutically acceptable salts, polymorphs, and cocrystals thereof, wherein

(A) Z represents a reactive moiety, in particular wherein the reactive moiety is suitable for conjugating a payload to the compound;
(B) X is absent or represents a chemical spacer comprising a single amino acid residue, a dipeptide and/or a moiety comprising 1 to 36 ethylene glycol repeats according to the formula $-NH(CH_2CH_2O)_{1-36}CH_2CO-$;
(C) $B_1$ represents a branching moiety, wherein the branching moiety is derived from an amino acid residue comprising two amino groups and a carboxyl group , wherein $B_1$ is coupled to X through its carboxyl group, and to each $L_1$ through its amino groups;
(D) each $L_1$ independently represents:

(D.i) a single amino acid residue or a dipeptide, preferably wherein the single amino acid residue is L-serine or L-glycine or wherein the dipeptide consists of or comprises L-serine and/or L-glycine;
(D.ii) a moiety comprising 1 to 36 ethylene glycol repeats according to the formula $-NH(CH_2CH_2O)_{1-36}CH_2CO-$; or
(D.iii) is absent;

and
(E) each Y independently represents $P-L_3-$ or a moiety according to formula (II)

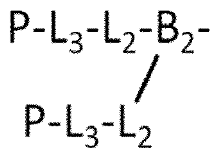

(II)

wherein

(E.i) $B_2$ represents a branching moiety wherein the branching moiety is derived from an amino acid residue comprising two amino groups and a carboxyl group, wherein $B_2$ is coupled to $L_1$ through its carboxyl group, and to each $L_2$ through its amino groups;
(E.ii) each $L_2$ independently represents:

(E.ii.a) a single amino acid residue or a dipeptide, preferably wherein the single amino acid residue is L-serine or L-glycine or wherein the dipeptide consists of or comprises L-serine and/or L-glycine, or
(E.ii.b) is absent;

(E.iii) $L_3$ represents a single amino acid residue, in particular a single D-amino acid residue, in particular a D-histidine residue, coupled to $L_2$ through its carboxylic group and coupled to P through its amino-group, and

(E.iv) P represents a peptide moiety, wherein each P independently represents a polypeptide comprising the amino acid sequence $(AA_1)$-$(AA_2)$-$(AA_3)$-$(AA_4)$-$(AA_5)$-$(AA_6)$-$(AA_7)$-$(AA_8)$-$(AA_9)$,

(E.iv.a) wherein $(AA_1)$ represents a single amino acid residue with a positively charged amino acid side chain, or wherein $(AA_1)$ is absent;
(E.iv.b) wherein $(AA_5)$ and $(AA_7)$ each represent a single amino acid residue comprising a positively charged amino acid side chain, a hydrophobic amino acid side chain or an aromatic amino acid side chain;
(E.iv.c) wherein $(AA_2)$, $(AA_3)$, $(AA_4)$ and $(AA_6)$ each represent a single amino acid residue;
(E.iv.d) wherein $(AA_8)$ represents a single amino acid residue, or wherein $(AA_8)$ is absent;
(E.iv.e) wherein $(AA_9)$ represents a single amino acid residue comprising a positively charged amino acid side chain, a hydrophobic amino acid side chain or an aromatic amino acid side chain, or wherein $(AA_9)$ is absent; and
(E.iv.f) wherein $(AA_8)$ and $(AA_9)$ are either both present or both absent, wherein when $(AA_8)$ and $(AA_9)$ are both absent, $(AA_1)$, $(AA_3)$, $(AA_5)$ and $(AA_7)$ represent a single amino acid residue comprising a positively charged amino acid side chain;

(E.v) wherein P comprises at least 3 amino acid residues comprising a positively charged amino acid side chain; and
(E.vi) wherein each P is coupled to an $\alpha$-amino group of an $L_3$ residue through the $\alpha$-carboxyl group of residue $(AA_7)$ and/or $(AA_9)$.

2. The compound according to claim 1, wherein when Y represents

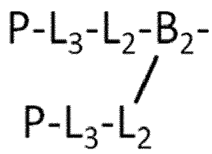

(II)

wherein $L_1$ is not absent, and/or
wherein the branching moiety $B_1$ and/or $B_2$ is derived from L-lysine; L-ornithine, $\alpha$-aminoglycine, $\alpha,\gamma$-diaminopropionic acid, $\alpha,\gamma$-diaminobutyric acid, and amino acid according to formula

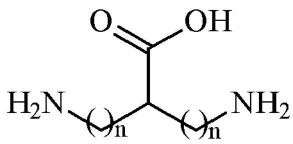

wherein n is selected from 1, 2, 3, and 4.

3. The compound according to claim 2, wherein the branching moiety $B_1$ and/or $B_2$ is derived from L-lysine or L-ornithine; wherein when $L_1$ and/or $L_2$ are coupled to an amino group comprised in the amino acid side chain of $B_1$ and/or $B_2$, respectively, $L_1$ and/or $L_2$ are not absent,
and/or
wherein the reactive moiety Z is an amino acid residue, in particular wherein the amino acid is selected from a group consisting of: L-cysteine, L-C-propargylglycine, L-biocytin, and $NH_2CH_2CONHSO_2(CH_2)_3COOH$, or wherein the reactive moiety Z is L-cysteamine or 4-aminobutanethiol,
and/or
wherein the single amino acid comprised in the chemical spacer X is L-serine, or, wherein the dipeptide comprised in the chemical spacer X comprises or consists of L-serine.

4. The compound according to any one of claims 1 to 3, wherein the compound comprises the structure:

(D-His)-(L-Ser)-(L-Lys)-(L-Cys) (SEQ ID NO:1);

(D-His)-(L-Ser)-(L-Lys)-(L-Ser)-(L-Lys)-(L-Cys) (SEQ ID NO:2);
(D-His)-(L-Ser)-(L-Lys)-(βAla)-(L-Ser)-(L-Lys)-(L-Cys) (SEQ ID NO:3);
(D-His)-(L-Ser)-(L-Lys)-(L-Ser)-(βAla)-(L-Lys)-(L-Cys) (SEQ ID NO:4);
(D-His)-(L-Ser)-(L-Lys)-(L-Ser)-(L-Lys)-(PEG1)-(L-Cys) (SEQ ID NO:5);
(D-His)-(L-Ser)-(L-Lys)-(PEG1/5/16/36)-(L-Cys) (SEQ ID NO:6);
(D-His)-(L-Ser)-(L-Lys)-(PEG1/3/5)-(L-Lys)-(L-Cys) (SEQ ID NO:7);
(D-His)-(PEG2/5)$_n$-(L-Lys)-(L-Cys) (SEQ ID NO:8);
(D-His)-(L-Ser)-(diAA)-(L-Ser)-(diAA)-(L-Cys) (SEQ ID NO:9);
(D-His)-(L-Ser)-(diAA)-(L-Cys) (SEQ ID NO:10);
(D-His)-(L-Ser)-(L-Lys)-(Gly)-(L-Lys)-(L-Cys) (SEQ ID NO:11);
(D-His)-(Gly)-(L-Lys)-(L-Ser)-(L-Lys)-(L-Cys) (SEQ ID NO:12);
(D-His)-(Gly)-(L-Lys)-(Gly)-(L-Lys)-(L-Cys) (SEQ ID NO:13);
(D-His)-(Gly)-(L-Lys)-(L-Cys) (SEQ ID NO:14);
(D-His)-(L-Ser)-(diAA)-(Gly)-(diAA)-(L-Cys) (SEQ ID NO:15);
(D-His)-(Gly)-(diAA)-(L-Ser)-(diAA)-(L-Cys) (SEQ ID NO:16);
(D-His)-(Gly)-(diAA)-(Gly)-(diAA)-(L-Cys) (SEQ ID NO:17);
(D-His)-(Gly)-(diAA)-(L-Cys) (SEQ ID NO:18);
(D-His)-(L-Ser)-(L-Lys)-(L-PAG) (SEQ ID NO:19);
(D-His)-(L-Ser)-(L-Lys)-(L-Ser)-(L-Lys)-(L-PAG) (SEQ ID NO:20);
(D-His)-(L-Ser)-(L-Lys)-(L-Bct) (SEQ ID NO:21);
(D-His)-(L-Ser)-(L-Lys)-(L-Ser)-(L-Lys)-(L-Bct) (SEQ ID NO:22);
(D-His)-(L-Lys)-(L-Cys) (SEQ ID NO:23);
(D-His)-(L-Lys)-(L-Ser)-(L-Lys)-(L-Cys) (SEQ ID NO:24);
(D-His)-(L-Lys)-(PEG1)-(L-Cys), wherein n is 1 (SEQ ID NO:25);
(D-His)-(L-Lys)-(L-Ser)-(L-Lys)-(PEG1)-(L-Cys), (SEQ ID NO:26);
(D-His)-(L-Ser)-(L-Ser)-(L-Lys)-(L-Cys) (SEQ ID NO:27);
(D-His)-(L-Ser)-(L-Ser)-(L-Lys)-(L-Ser)-(L-Lys)-(L-Cys) (SEQ ID NO:28);
(D-His)-(L-Ser)-(L-Lys)-(L-Ser)-(L-Ser)-(L-Cys) (SEQ ID NO:29); or
(D-His)-(L-Ser)-(L-Lys)-(L-Ser)-(L-Lys)-(L-Ser)-(L-Ser)-(L-Cys) (SEQ ID NO:30), wherein (PEG1/5/16/36). (PEG1/3/5), (PEG2/5) and (PEG1) represent a moiety according to the formula $-NH(CH_2CH_2O)_nCH_2CO-$, wherein n is: 1, 5, 16 or 36 for (PEG1/5/16/36); 1, 3 or 5 for (PEG1/3/5); 2 or 5 for (PEG2/5); 1 for (PEG1),

and wherein diAA represents an amino acid according to formula

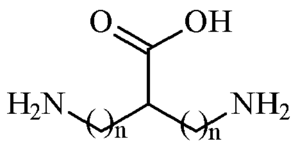

wherein n is selected from 1, 2, 3, and 4,
and/or
wherein the compound comprises the structure:
(D-His)-(L-Ser)-(L-Lys[ε-N-(L-Ser)-(D-His)])-(L-Cys) (SEQ ID NO:31);

(D-His)-(L-Ser)-(L-Lys[ε-N-(L-Ser)-(L-Lys[ε-N-(L-Ser)-(D-His)]-(L-Ser)-(D-His)])-(L-Cys) (SEQ ID NO:32);

(D-His)-(L-Ser)-(L-Lys[ε-N-(L-Ser)-(D-His)])-(L-Ser)-(L-Lys[ε-N-(L-Ser)-(D-His)])-(L-Cys) (SEQ ID NO:33);

(D-His)-(L-Ser)-(L-Lys[ε-N-(L-Ser)-(L-Lys[ε-N-(L-Ser)-(D-His)])-(L-Ser)-(D-His)])-(L-Ser)-(L-Lys[ε-N-(L-Ser)-(D-His)])-(L-Cys) (SEQ ID NO:34);

(D-His)-(L-Ser)-(L-Lys[ε-N-(L-Ser)-(D-His)])-(L-Ser)-(L-Lys[ε-N-(L-Ser)-(L-Lys[ε-N-(L-Ser)-(D-His)])-(L-Ser)-(D-His)])-(L-Cys) (SEQ ID NO:35); or

(D-His)-(L-Ser)-(L-Lys[ε-N-(L-Ser)-(L-Lys[ε-N-(L-Ser)-(D-His)])-(L-Ser)-(D-His)])-(L-Ser)-(L-Lys[ε-N-(L-Ser)-(L-Lys[ε-N-(L-Ser)-(D-His)])-(L-Ser)-(D-His)])-(L-Cys) (SEQ ID NO:36),

**5.** The compound according to any one of claims 1 to 4, wherein in at least one, at least two, at least three or all peptide moieties P, at least two of the amino acid residues ($AA_1$), ($AA_5$), ($AA_7$) and ($AA_9$) comprise an amino acid residue with a positively charged amino acid side chain,
and/or
wherein in at least one, at least two, at least three or all peptide moieties P, amino acid residue ($AA_9$) comprises a positively charged amino acid side chain, in particular wherein ($AA_9$) is selected from a group consisting of: L-arginine, L-lysine and L-2-amino-4-guanidinobutyric acid,
and/or
wherein in at least one, at least two, at least three or all peptide moieties P, at least one of the amino acid residues ($AA_2$) and/or ($AA_5$) and at least one of the amino acid residues ($AA_7$) and/or ($AA_8$) comprises a positively charged amino acid side chain,
and/or
wherein the amino acid comprising the positively charged amino acid side chain in position ($AA_2$) and/or ($AA_5$) is selected from a group consisting of: L-arginine, D-arginine and L-2-amino-4-guanidinobutyric acid; and/or wherein the amino acid comprising the positively charged amino acid side chain in position ($AA_7$) and/or ($AA_8$) is selected from a group consisting of: L-arginine, L-lysine and L-ornithine.

**6.** The compound according to any one of claims 1 to 5, wherein in at least one, at least two, at least three or all peptide moieties P, between 2 and 5 amino acid residues comprise a hydrophobic and/or aromatic amino acid side chain,
and/or
wherein in at least one, at least two, at least three or all peptide moieties P, amino acid residue ($AA_3$) is L-phenylalanine and/or amino acid residue ($AA_6$) is L-isoleucine or D-isoleucine,
and/or
wherein at least one, at least two, at least three or all peptide moieties P comprise a sequence independently selected from a group consisting of:

RYFvRIOYR (SEQ ID NO:37);
RRFvYIYRR (SEQ ID NO:38);
RYFvRIYRR (SEQ ID NO:39);
RYFvrIOYR (SEQ ID NO:40);
RYFvRiOYR (SEQ ID NO:41);
RYFvriOYR (SEQ ID NO:42);
RRFvYIYKR (SEQ ID NO:43);
RRFqYIYKR (SEQ ID NO:44);
RYFvRIYKR (SEQ ID NO:45);
RYFvRIYRK (SEQ ID NO:46) ;
RRFaYIYKR (SEQ ID NO:47);
RRFpYIYKR (SEQ ID NO:48);
RYFvRIKYR (SEQ ID NO:49);
RYFqRIKYR (SEQ ID NO:50);
YFvRIOYR (SEQ ID NO:51);
(Agb)YFv(Agb)IKY(Agb) (SEQ ID NO:52);

YFvRIKYR (SEQ ID NO:53);
rYFvRIKYR (SEQ ID NO:54);
RYFvrIKYR (SEQ ID NO:55);
rrFvYIYRR (SEQ ID NO:56);
RSTqRYRVR (SEQ ID NO:57);
RYFvRIKYR (SEQ ID NO:58);
RYFvRIKAR (SEQ ID NO:59);
RAAvRAKYR (SEQ ID NO:60);
RAAvRIKYR (SEQ ID NO:61);
RSTqRYRVR (SEQ ID NO:62);
RSTqRYKVR (SEQ ID NO:63);
RGGgRGKGR (SEQ ID NO:64);
RHHhRHKHR (SEQ ID NO:65);
RWvRVKVR (SEQ ID NO:66);
RLLIRLKLR (SEQ ID NO:67);
RMMmRMKMR (SEQ ID NO:68);
RIIiRIKIR (SEQ ID NO:69); or
RYFVRiKYR (SEQ ID NO:70);

wherein capital letters represent L-amino acids and wherein lower-case letters represent D-amino acids.

**7.** The compound according to any one of claims 1 to 5, wherein at least one, at least two, at least three or all peptide moieties P comprise a positively charged amino acid in position ($AA_1$), ($AA_3$) and ($AA_9$), in particular wherein ($AA_1$) and/or ($AA_9$) are L-arginine and/or wherein ($AA_3$) is L-lysine,
and/or
wherein at least one, at least two, at least three or all peptide moieties P comprise an amino acid residue comprising a positively charged amino acid side chain in positions ($AA_5$) or ($AA_7$), in particular wherein ($AA_5$) or ($AA_7$) are L-arginine and/or L-lysine,
and/or
wherein at least one, at least two, at least three or all peptide moieties P comprise between two and five amino acid residues comprising a hydrophobic and/or aromatic amino acid side chain, in particular wherein the amino acid residues comprising the hydrophobic and/or aromatic side chains are located in positions ($AA_2$), ($AA_4$), ($AA_6$), ($AA_8$) and/or one of positions ($AA_5$) or ($AA_7$), and/or
wherein at least one, at least two, at least three or all peptide moieties P comprise a sequence independently selected from a group consisting of:

RYKvFIKYR (SEQ ID NO:71);
RYKvAIKYR (SEQ ID NO:72);
RYKvRIAYR (SEQ ID NO:73);
RYKvRIFYR (SEQ ID NO:74);
RYKvKFIYR (SEQ ID NO:75);
RYKvFIRYR (SEQ ID NO:76);
RYKvRFIYR (SEQ ID NO:77);
RMKiVMKFR (SEQ ID NO:78); or
RFKfFFKFR (SEQ ID NO:79);

wherein capital letters represent L-amino acids and wherein lower-case letters represent D-amino acids.

**8.** The compound according to any one of claims 1 to 5, wherein at least one, at least two, at least three or all peptide moieties P comprise an amino acid residue comprising a positively charged amino acid side chain in positions ($AA_1$), ($AA_3$), ($AA_5$) and ($AA_7$), in particular wherein ($AA_1$) and/or ($AA_5$) are L-arginine and/or wherein ($AA_3$) and/or ($AA_7$) are L-lysine,
and/or
wherein at least one, at least two, at least three or all peptide moieties P comprise between two and five amino acid residues comprising a hydrophobic and/or aromatic amino acid side chain, in particular wherein the amino acid residues comprising the hydrophobic and/or aromatic side chains are located in positions ($AA_2$), ($AA_4$) and ($AA_6$), and optionally (AA8) and/or ($AA_9$),
and/or

wherein in at least one, at least two, at least three or all peptide moieties P, amino acid residues ($AA_8$) and ($AA_9$) are both absent or are both amino acids comprising a positively charged amino acid side chain, a hydrophobic amino acid side chain and/or an aromatic amino acid side chain,
and/or
wherein at least one, at least two, at least three or all peptide moieties P comprise a sequence independently selected from a group consisting of:

RYKvRIK (SEQ ID NO:80);
RYKvRIKYF (SEQ ID NO:81);
RYKvRIKYA (SEQ ID NO:82);
RYKvRIKHH (SEQ ID NO:83);
RMKiRVK (SEQ ID NO:84);
RMKiRVKFM (SEQ ID NO:85);
RLKIRLKLL (SEQ ID NO:86); or
RYKvRIKYr (SEQ ID NO:87);

wherein capital letters represent L-amino acids and wherein lower-case letters represent D-amino acids.

**9.** The compound according to any one of claims 1 to 8, wherein at least one, at least two, at least three or all peptide moieties P comprise at least one D-amino acid, in particular wherein amino acid residue ($AA_4$) and/or ($AA_6$) is a D-amino acid,
and/or
wherein at least one, at least two, at least three or all peptide moieties P comprise not more than one amino acid residue comprising a negatively charged amino acid side chain, in particular wherein none of the amino acid residues in position ($AA_1$) to ($AA_3$) and ($AA_5$) to ($AA_9$) comprise an amino acid residue comprising a negatively charged amino acid side chain,
and/or
wherein at least one, at least two, at least three or all peptide moieties P are free of amino acid residues comprising a negatively charged amino acid side chain,
and/or
wherein at least one, at least two, at least three or all peptide moieties P comprise not more than one proline residue, in particular wherein none of the amino acid residues in position ($AA_1$) to ($AA_3$) and ($AA_5$) to ($AA_9$) comprise a proline residue,
and/or
wherein at least one, at least two, at least three or all peptide moieties P are free of proline residues,
and/or
wherein at least two, at least three or all peptide moieties P have identical amino acid sequences.

**10.** The compound according to any one of claims 1 to 9 further conjugated to a payload,
wherein preferably the payload is conjugated to the reactive moiety Z, preferably wherein the payload is a biologically active molecule (BAM) or an imaging agent,
preferably wherein the BAM is a mono- or polysaccharide, a cytotoxic agent, an antineoplastic agent, an anti-inflammatory agent, an anti-viral agent, an anti-bacterial agent or an agent for the treatment of protozoan infections.

**11.** A pharmaceutical composition comprising the payload conjugate according to claim 10 and a pharmaceutically acceptable carrier or excipient.

**12.** The payload conjugate according to claim 10 or the pharmaceutical composition according to claim 11 for use as a medicament, in particular for use in the treatment of cancer.

**13.** The payload conjugate or the pharmaceutical composition for use according to claim 12, wherein the payload conjugate comprises PUMA, SN-38, 6-thioguanine or a pro-apoptopic peptide.

**14.** The payload conjugate according to claim 10 or the pharmaceutical composition according to claim 11 for use in

a) the treatment of disorders involving leukocytes, wherein the payload conjugate specifically binds to chondroitin-4-sulfate,
or

b) the treatment of a heart disease or condition, a disease or condition of the ovary or testis, a disease or condition of the central nervous system, or a C6S accumulation disease, wherein the payload conjugate specifically binds to chondroitin-6-sulfate,

or

c) the treatment of a corneal disease or condition, a retina disease or condition, a disease or condition of the central nervous system, or a keratan sulfate accumulation disease, wherein the payload conjugate specifically binds to keratan sulfate.

**15.** The payload conjugate according to claim 10 or the pharmaceutical composition according to claim 11 for use in

a) diagnostics;

or

b) in the visualization of cells comprising chondroitin-4-sulfate, chondroitin-6-sulfate and/or keratan sulfate on their cell surface and/or tissues comprising cells comprising chondroitin-4-sulfate, chondroitin-6-sulfate and/or keratan sulfate on their cell surface;

or

c) diagnosis of diseases involving cells and/or tissues comprising proteoglycans on the surface;

or

d) diagnosis of diseases associated with elevated levels of proteoglycans on malignant cells, in particular wherein the proteoglycans are chondroitin-4-sulfate, chondroitin-6-sulfate and/or keratan sulfate.

**Fig.1**

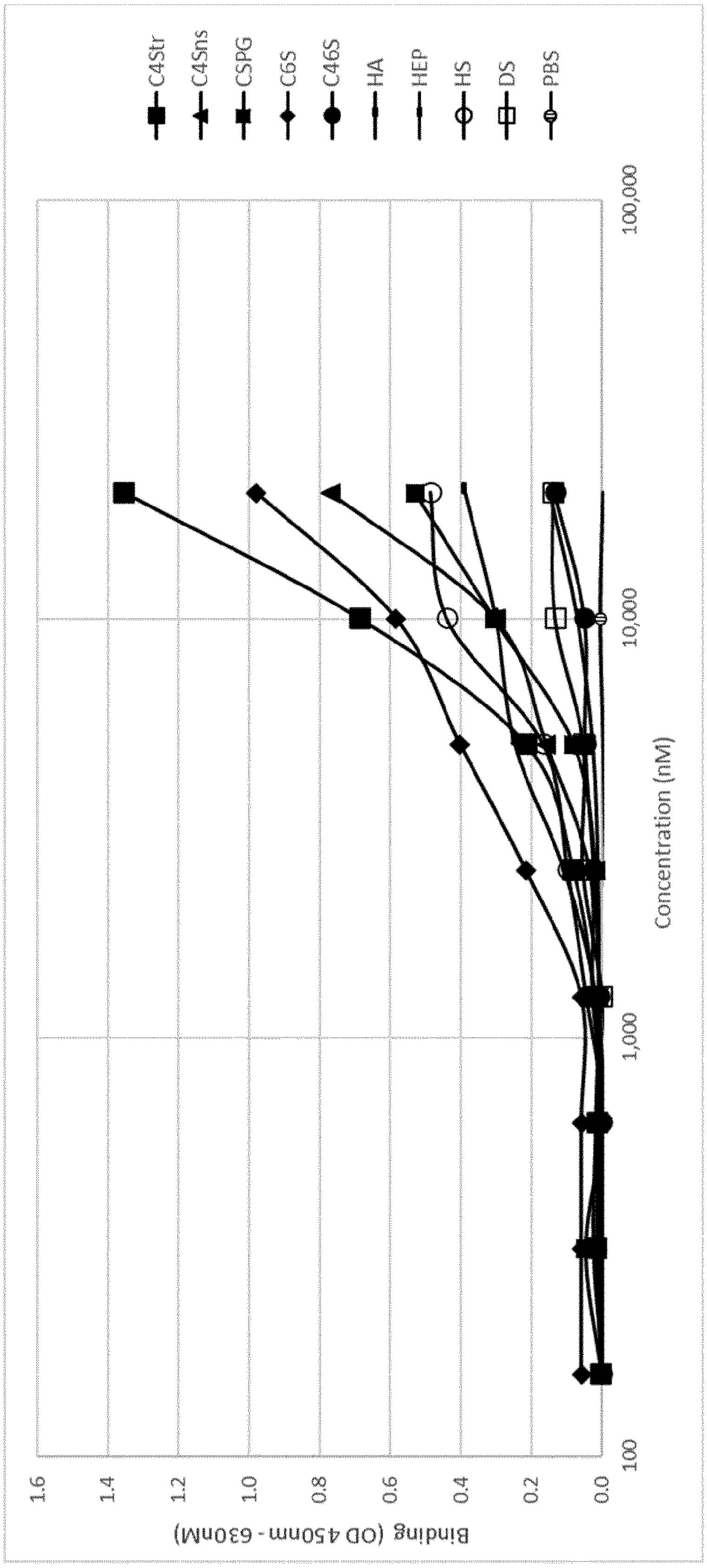
Binding (OD 450nm - 630nM)
0.0
0.2
0.4
0.6
0.8
1.0
1.2
1.4
1.6
100
1,000
10,000
100,000
Concentration (nM)
C4Str
C4Sns
CSPG
C6S
C46S
HA
HEP
HS
DS
PBS

**Fig.2**

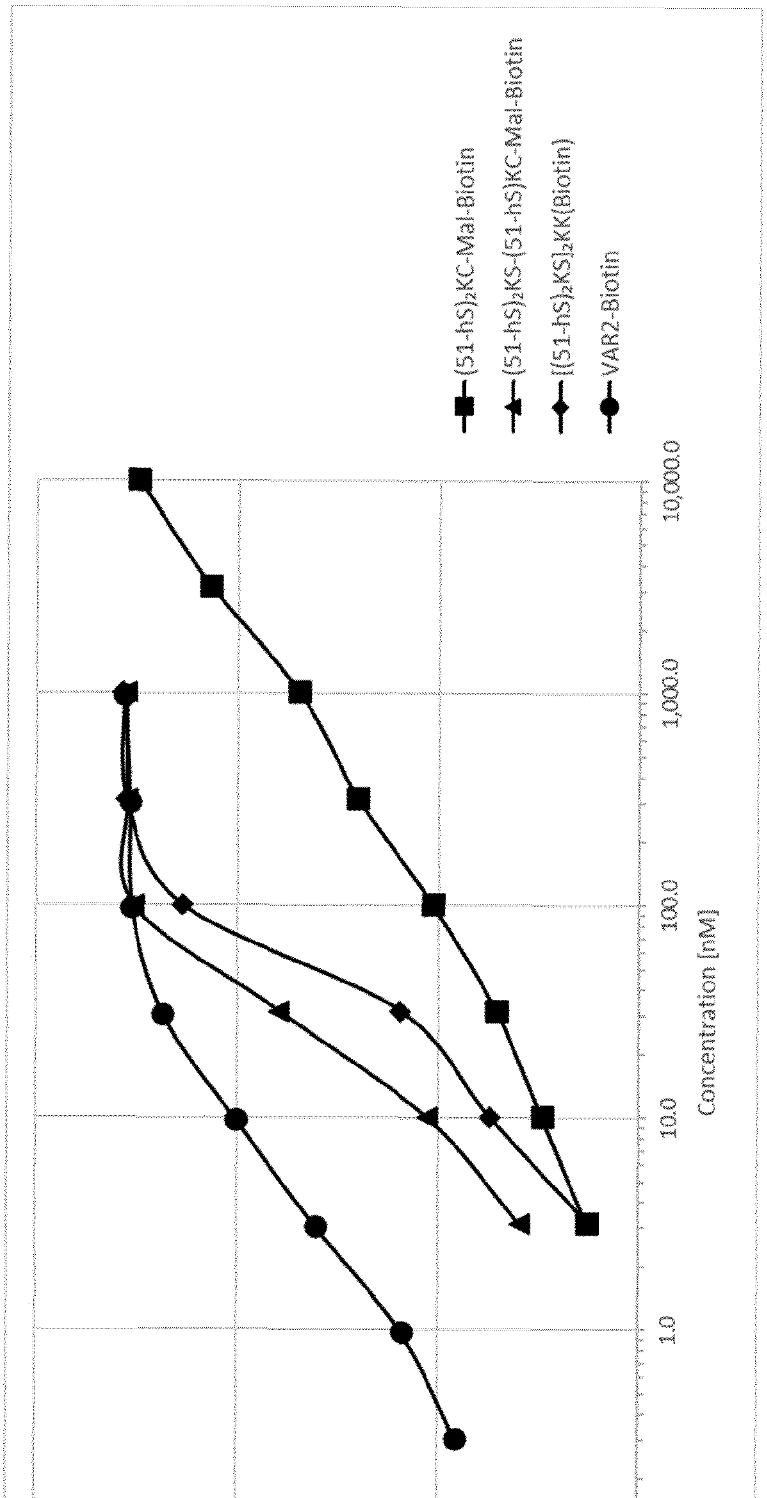
(51-hS)2KC-Mal-Biotin
(51-hS)2KS-(51-hS)KC-Mal-Biotin
[(51-hS)2KS]2KK(Biotin)
VAR2-Biotin
Binding (OD 450nm - 630nM)
10.0
1.0
0.1
0.0
Concentration [nM]
0.1
1.0
10.0
100.0
1,000.0
10,000.0

Fig.3A

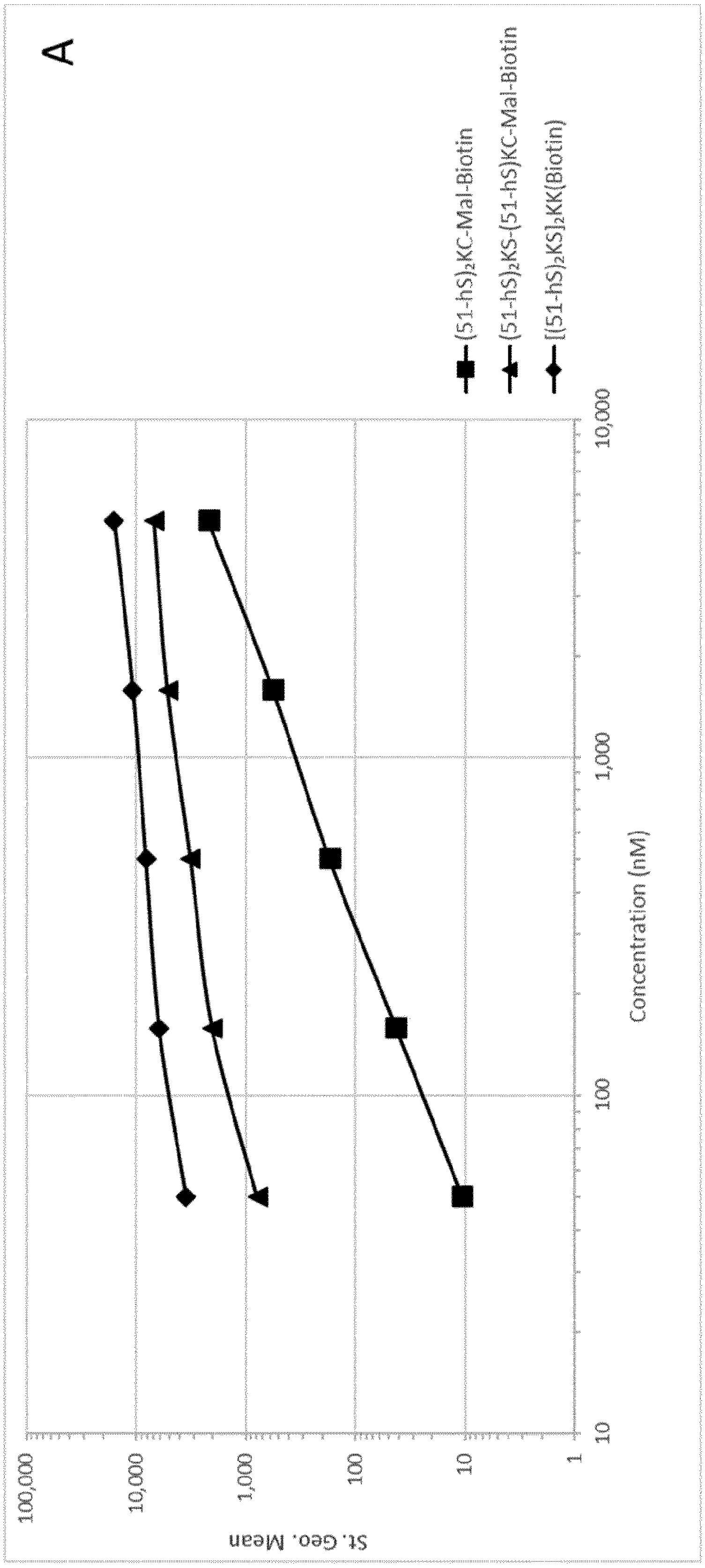
A
St. Geo. Mean
100,000
10,000
1,000
100
10
1
10
100
1,000
10,000
Concentration (nM)
(51-hS)$_2$KC-Mal-Biotin
(51-hS)$_2$KS-(51-hS)KC-Mal-Biotin
[(51-hS)$_2$KS]$_2$KK(Biotin)

**Fig.3B**

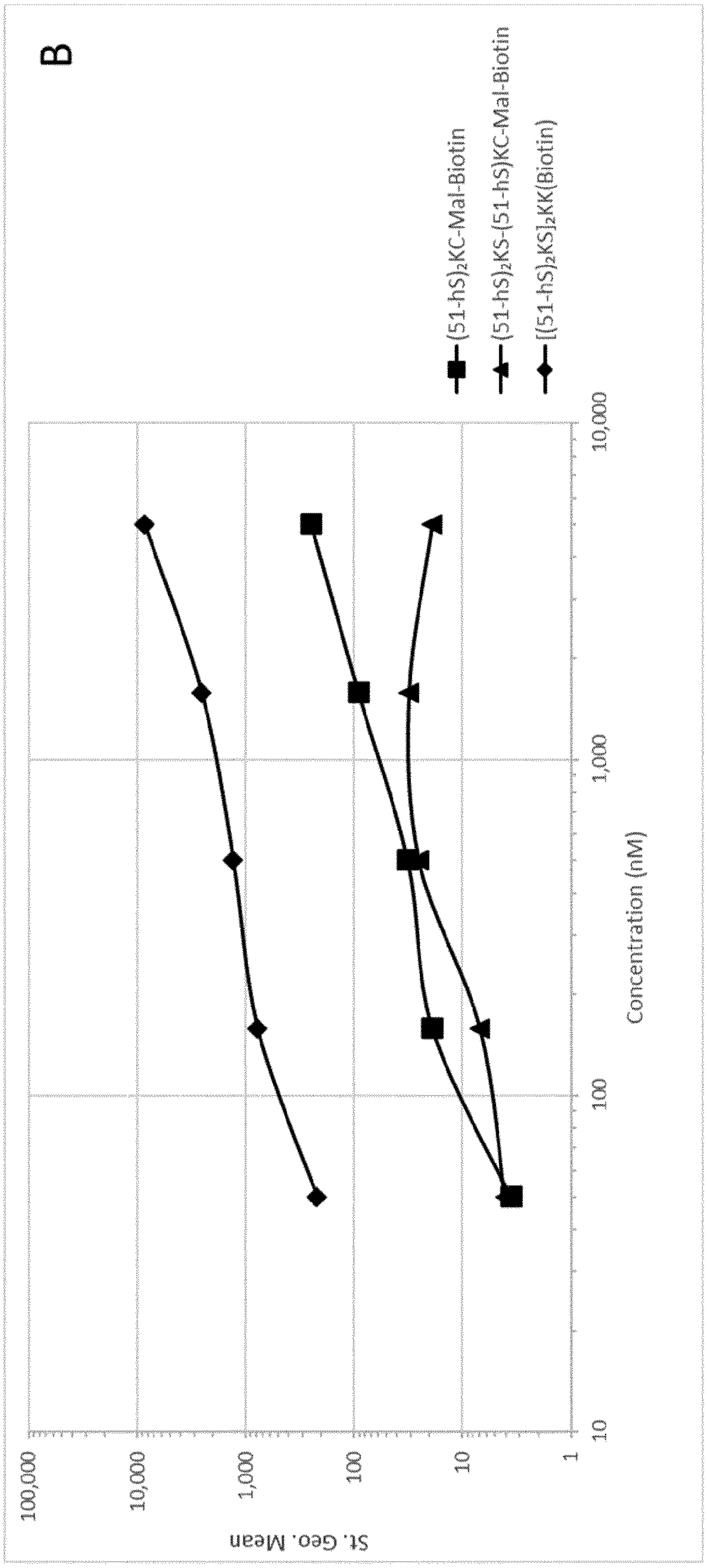
B
St. Geo. Mean
100,000
10,000
1,000
100
10
1
Concentration (nM)
10
100
1,000
10,000
(51-hS)$_2$KC-Mal-Biotin
(51-hS)$_2$KS-(51-hS)KC-Mal-Biotin
[(51-hS)$_2$KS]$_2$KK(Biotin)

Fig.4A

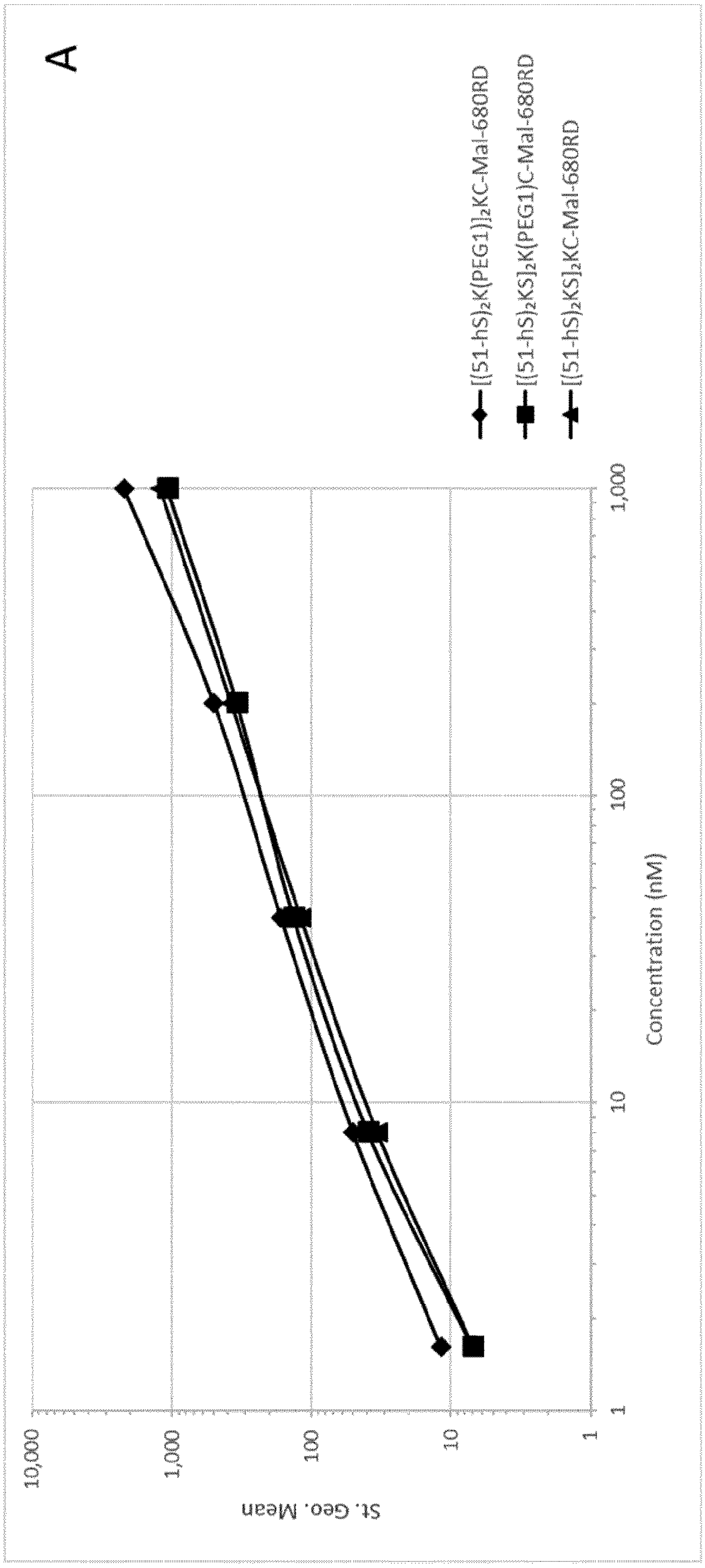
A
[(51-hS)2K(PEG1)]2KC-Mal-680RD
[(51-hS)2KS]2K(PEG1)C-Mal-680RD
[(51-hS)2KS]2KC-Mal-680RD
St. Geo. Mean
10,000
1,000
100
10
1
Concentration (nM)
1
10
100
1,000

**Fig.4B**

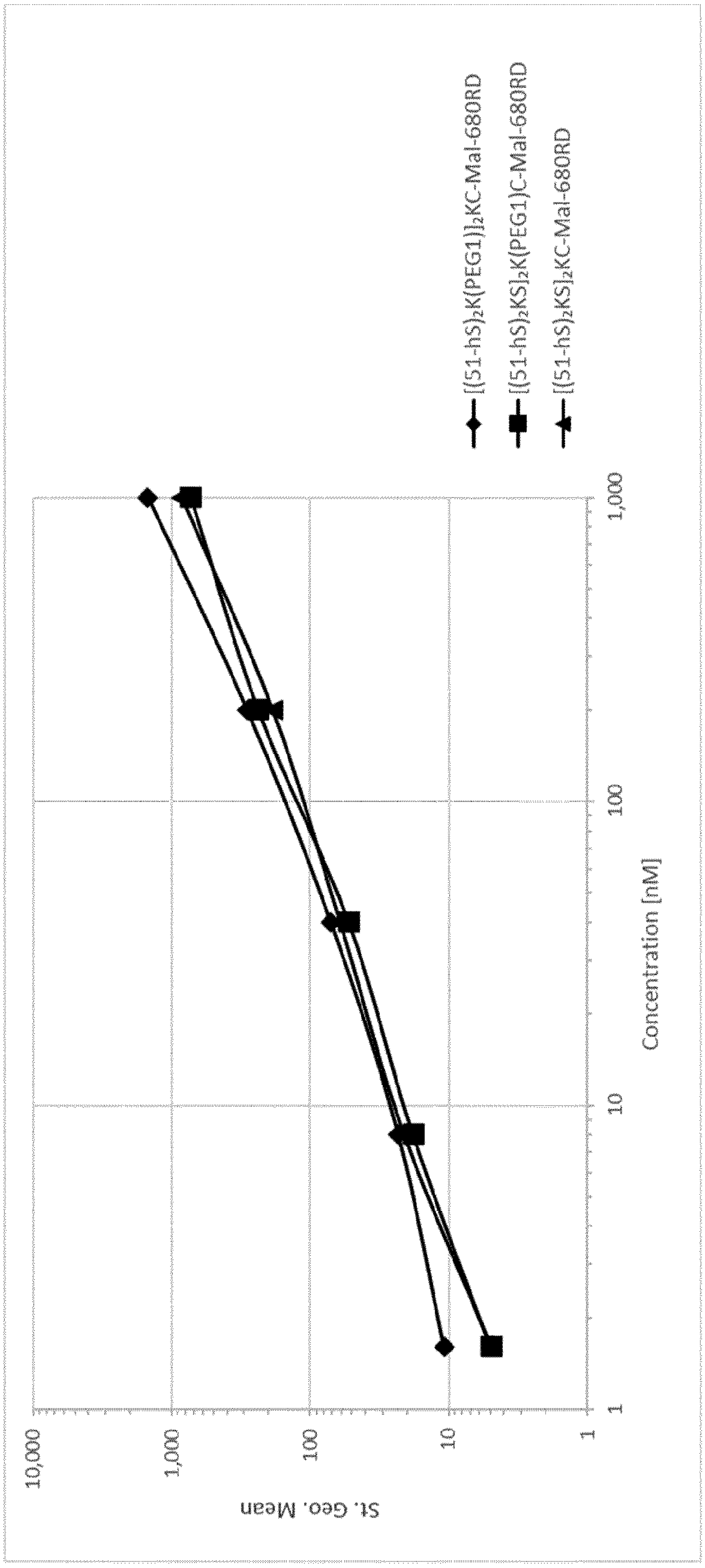
St. Geo. Mean
10,000
1,000
100
10
1
Concentration [nM]
1
10
100
1,000
[(51-hS)$_2$K(PEG1)]$_2$KC-Mal-680RD
[(51-hS)$_2$KS]$_2$K(PEG1)C-Mal-680RD
[(51-hS)$_2$KS]$_2$KC-Mal-680RD

Fig.5A

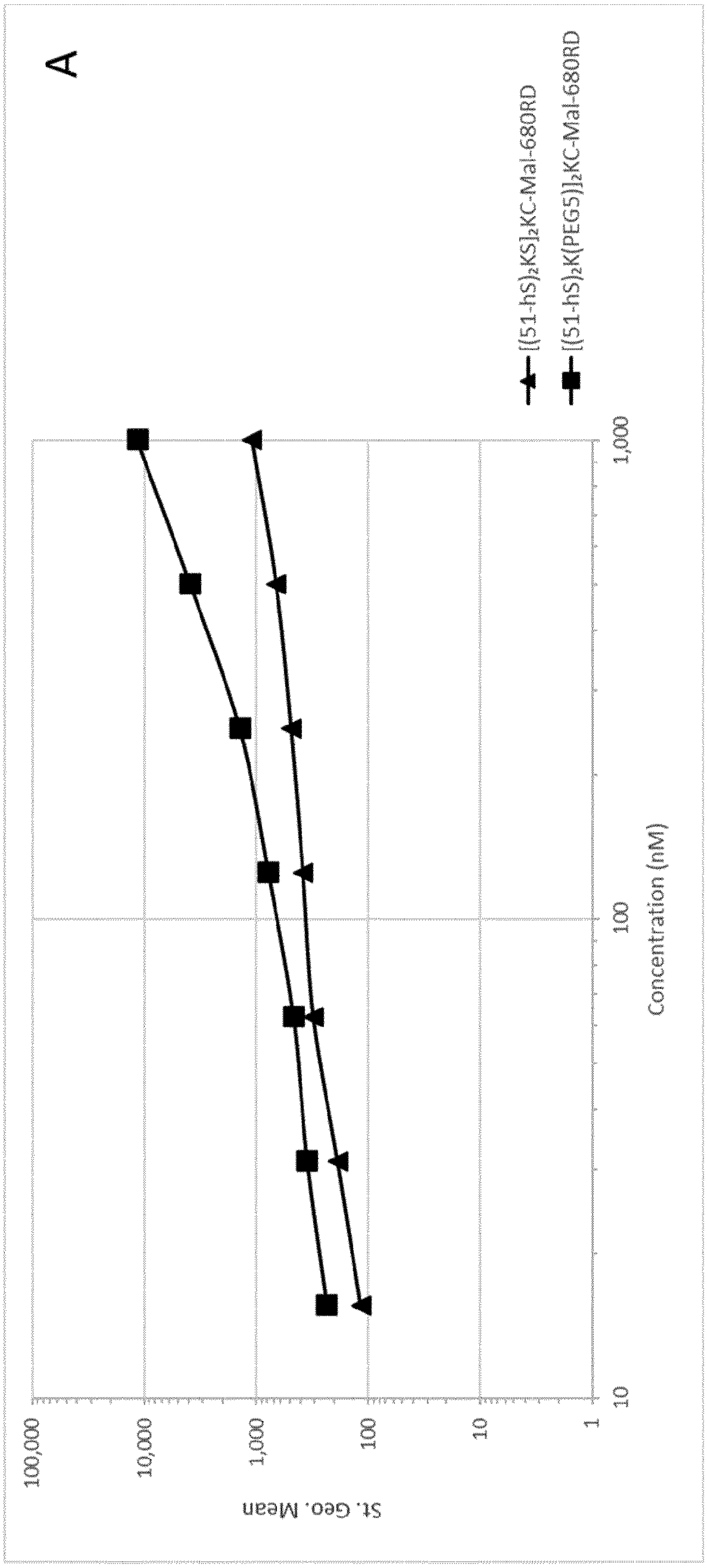
A
[(51-hS)$_2$KS]$_2$KC-Mal-680RD
[(51-hS)$_2$K(PEG5)]$_2$KC-Mal-680RD
St. Geo. Mean
100,000
10,000
1,000
100
10
1
10
100
1,000
Concentration (nM)

**Fig.5B**

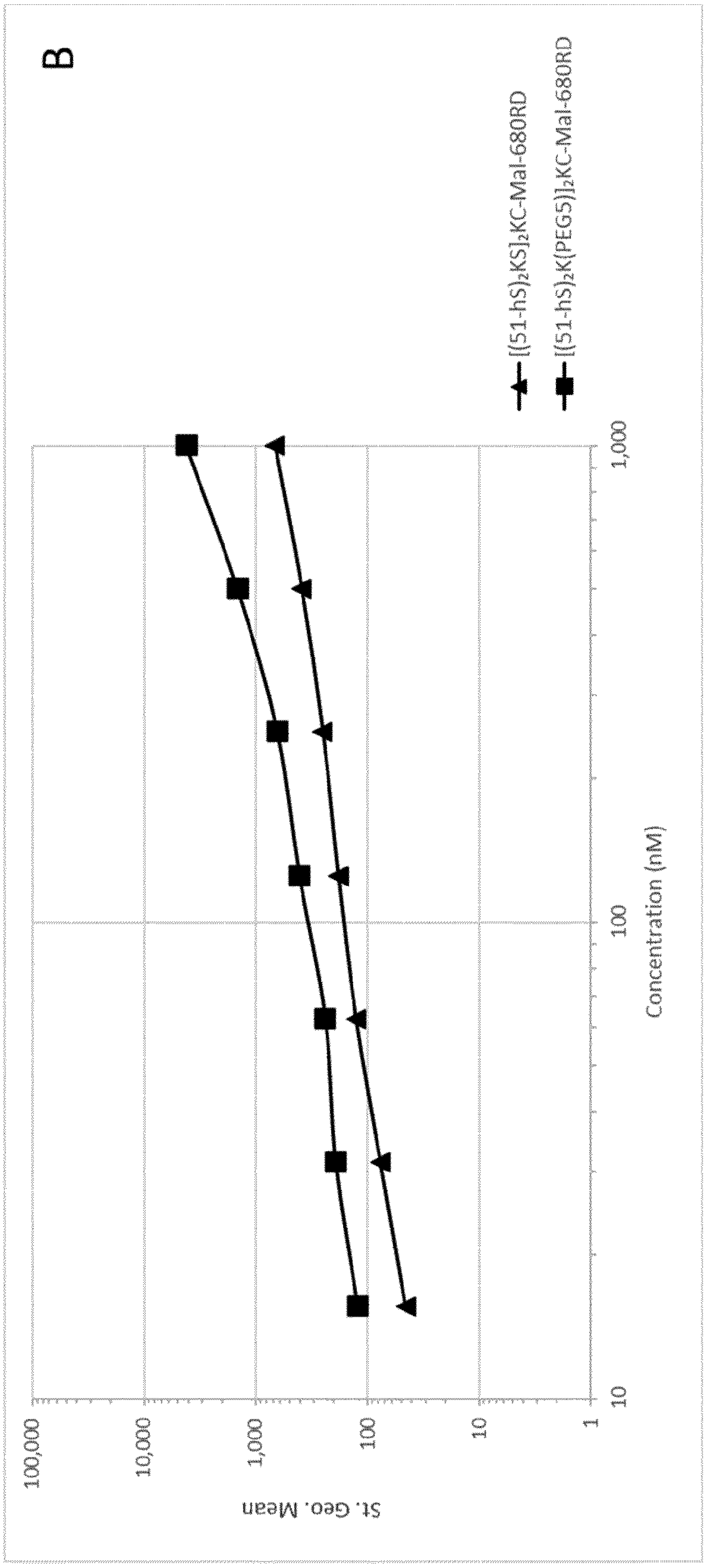
B
St. Geo. Mean
100,000
10,000
1,000
100
10
1
10
100
1,000
Concentration (nM)
[(51-hS)$_2$KS]$_2$KC-Mal-680RD
[(51-hS)$_2$K(PEG5)]$_2$KC-Mal-680RD

Fig.6A

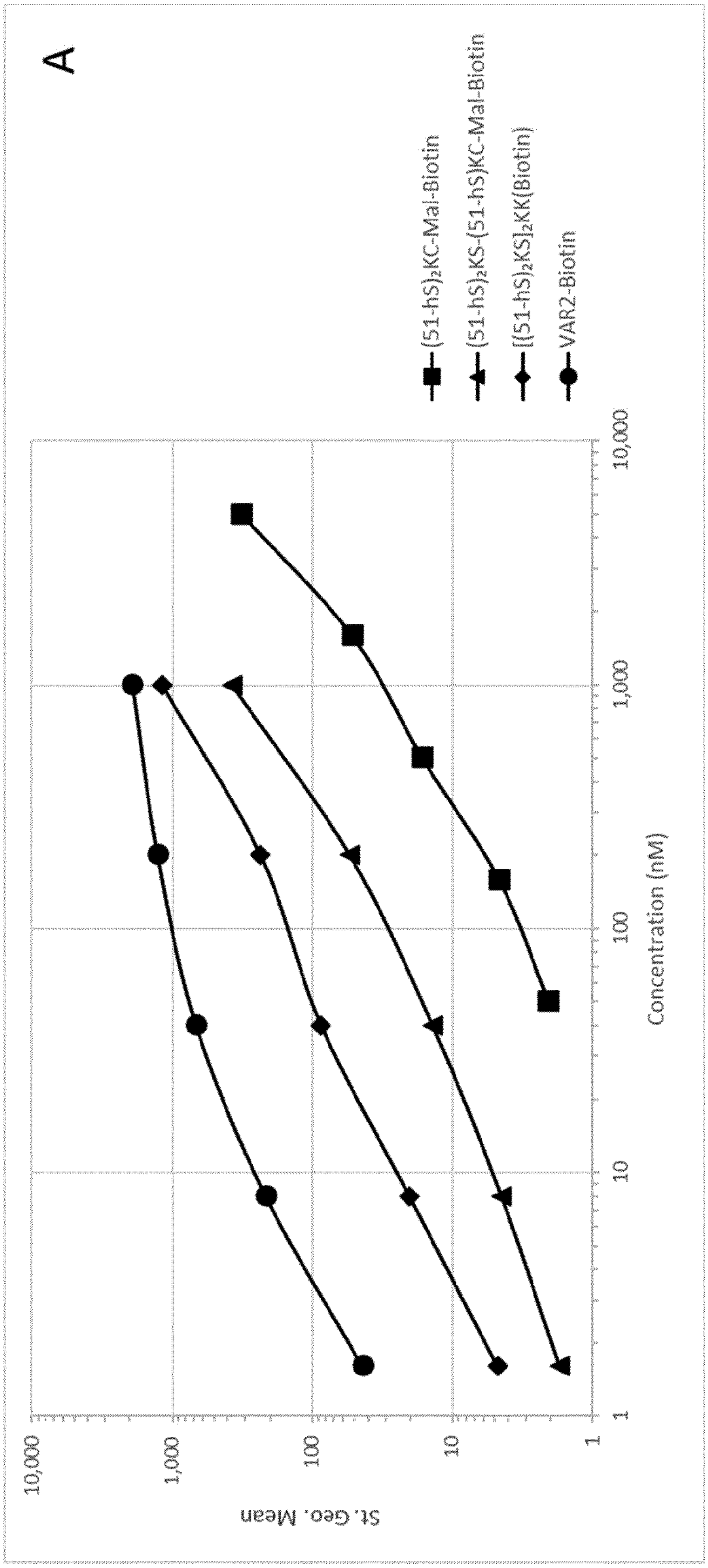
A
(51-hS)₂KC-Mal-Biotin
(51-hS)₂KS-(51-hS)KC-Mal-Biotin
[(51-hS)₂KS]₂KK(Biotin)
VAR2-Biotin
St.Geo. Mean
10,000
1,000
100
10
1
Concentration (nM)
1
10
100
1,000
10,000

**Fig.6B**

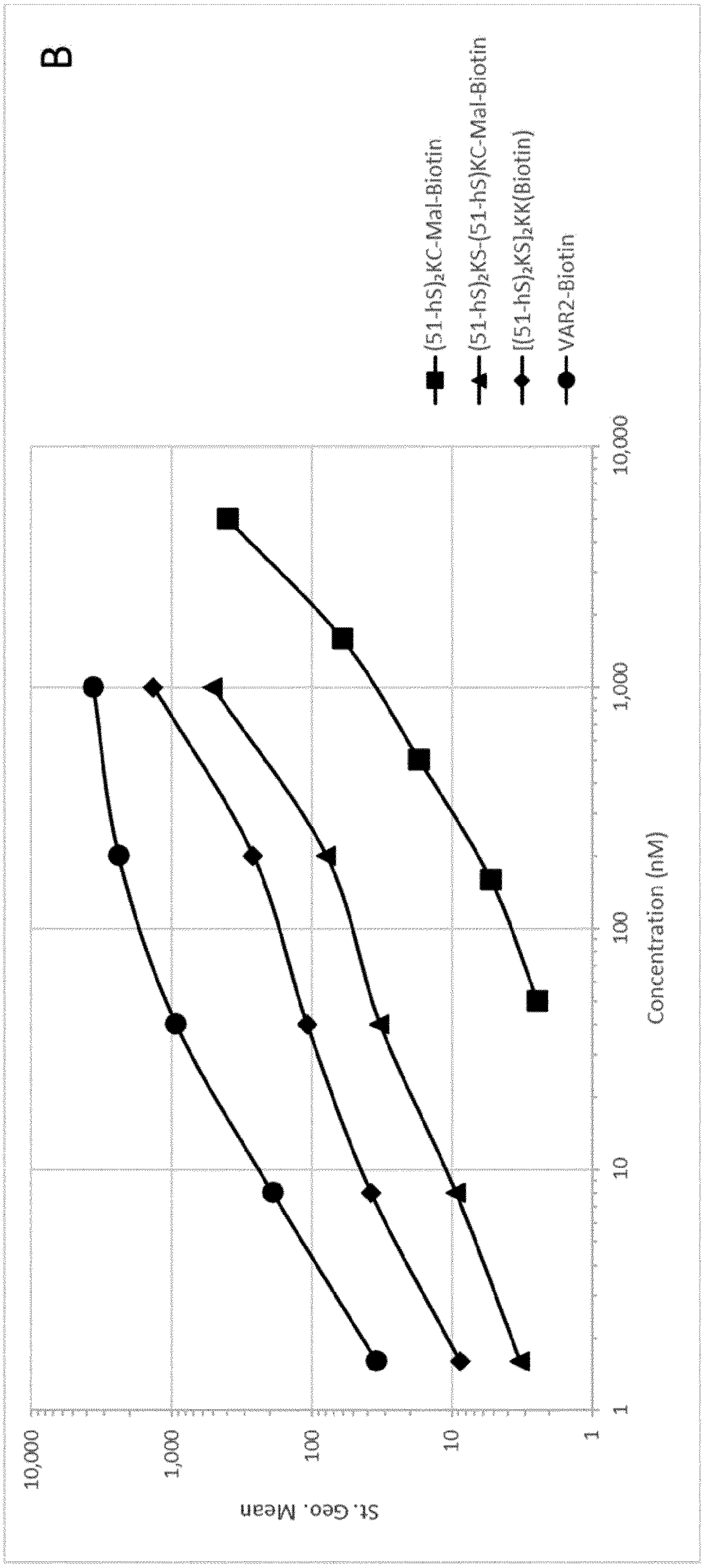
B
$(51\text{-hS})_2$KC-Mal-Biotin
$(51\text{-hS})_2$KS-(51-hS)KC-Mal-Biotin
$[(51\text{-hS})_2\text{KS}]_2$KK(Biotin)
VAR2-Biotin
St. Geo. Mean
10,000
1,000
100
10
1
Concentration (nM)
1
10
100
1,000
10,000

Fig.7

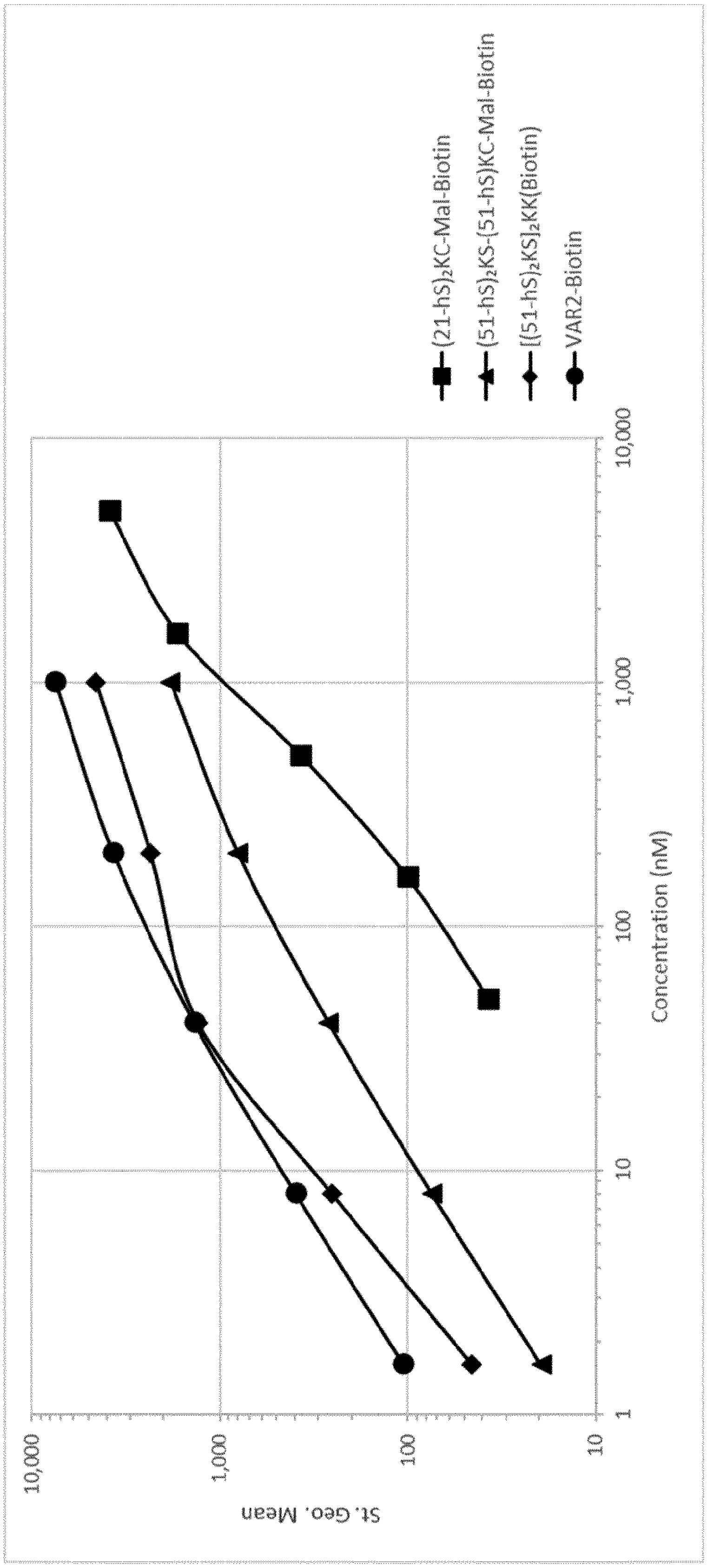
St. Geo. Mean
10,000
1,000
100
10
1
10
100
1,000
10,000
Concentration (nM)
(21-hS)$_2$KC-Mal-Biotin
(51-hS)$_2$KS-(51-hS)KC-Mal-Biotin
[(51-hS)$_2$KS]$_2$KK(Biotin)
VAR2-Biotin

**Fig. 8**

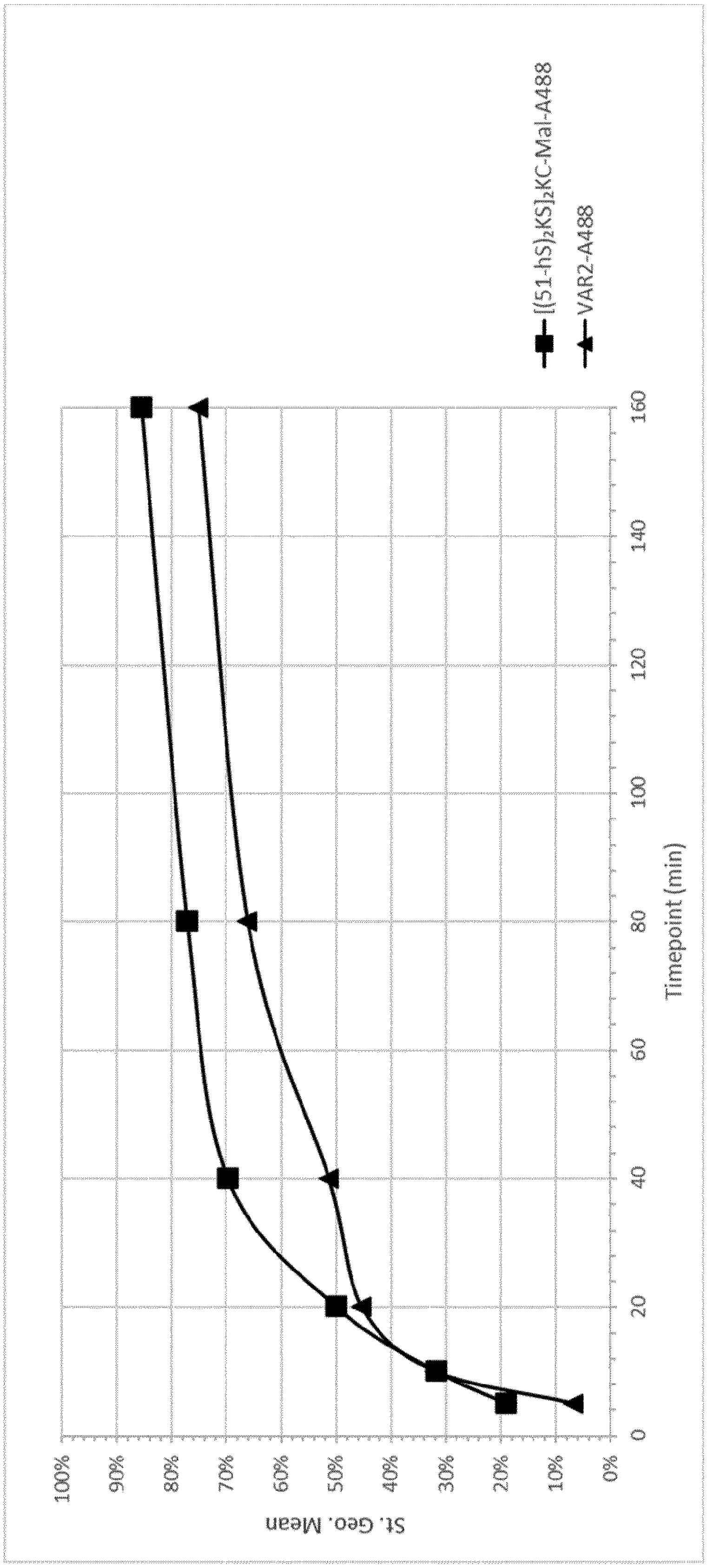
St. Geo. Mean
100%
90%
80%
70%
60%
50%
40%
30%
20%
10%
0%
0
20
40
60
80
100
120
140
160
Timepoint (min)
[(51-hS)$_2$KS]$_2$KC-Mal-A488
VAR2-A488

Fig.9A

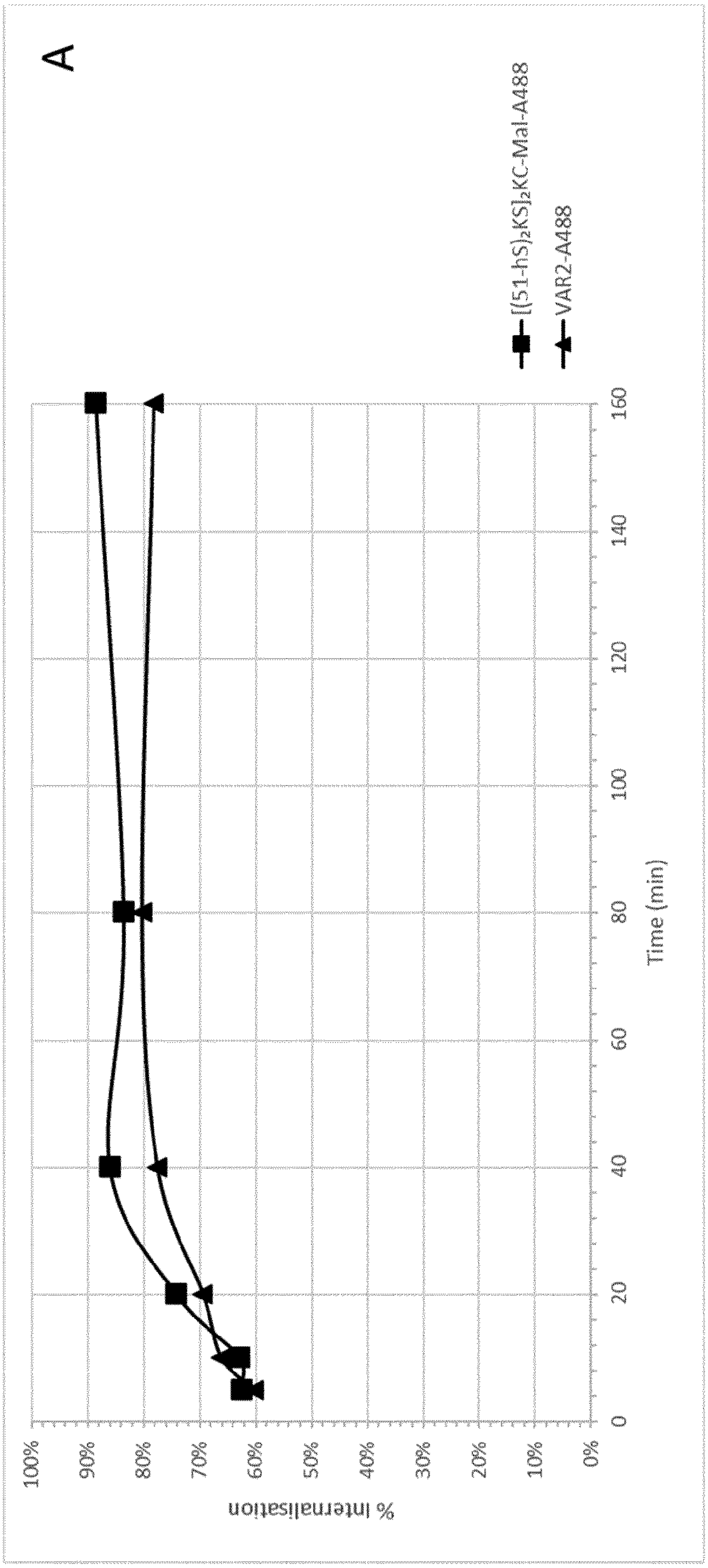
A
[(51-hS)$_2$KS]$_2$KC-Mal-A488
VAR2-A488
% Internalisation
100%
90%
80%
70%
60%
50%
40%
30%
20%
10%
0%
Time (min)
0
20
40
60
80
100
120
140
160

Fig.9B

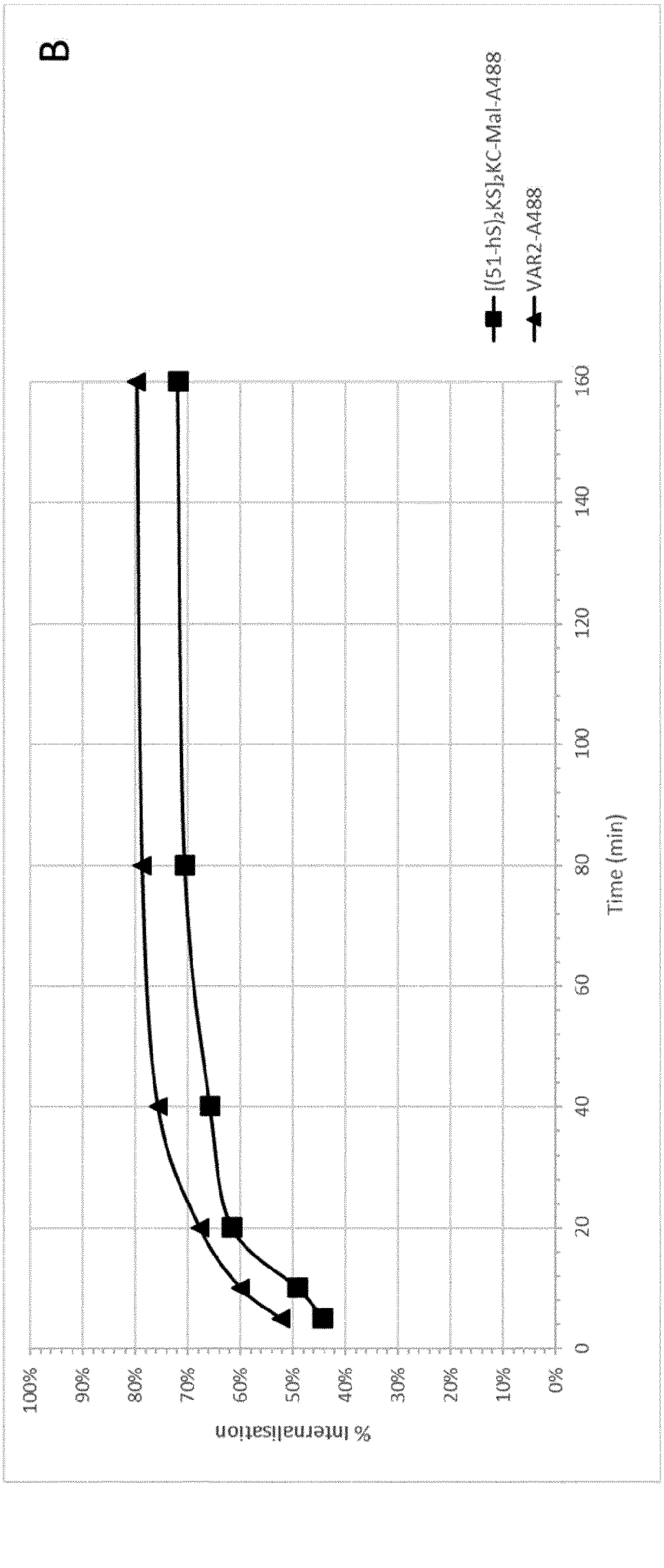
B
[(51-hS)2KS]2KC-Mal-A488
VAR2-A488
% Internalisation
100%
90%
80%
70%
60%
50%
40%
30%
20%
10%
0%
Time (min)
0
20
40
60
80
100
120
140
160

**Fig.10A**

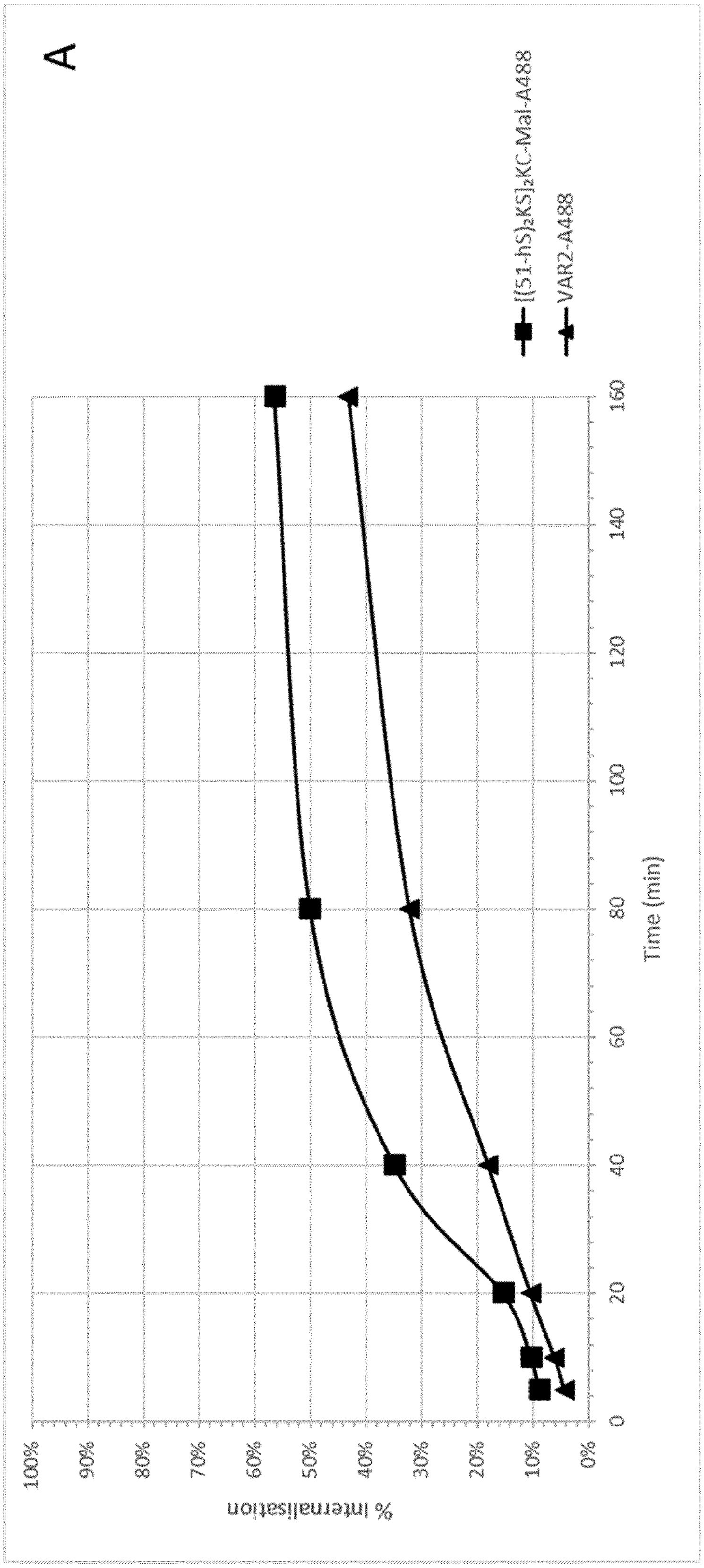
A
% internalisation
100%
90%
80%
70%
60%
50%
40%
30%
20%
10%
0%
0
20
40
60
80
100
120
140
160
Time (min)
[(51-hS)₂KS]₂KC-Mal-A488
VAR2-A488

Fig.10B

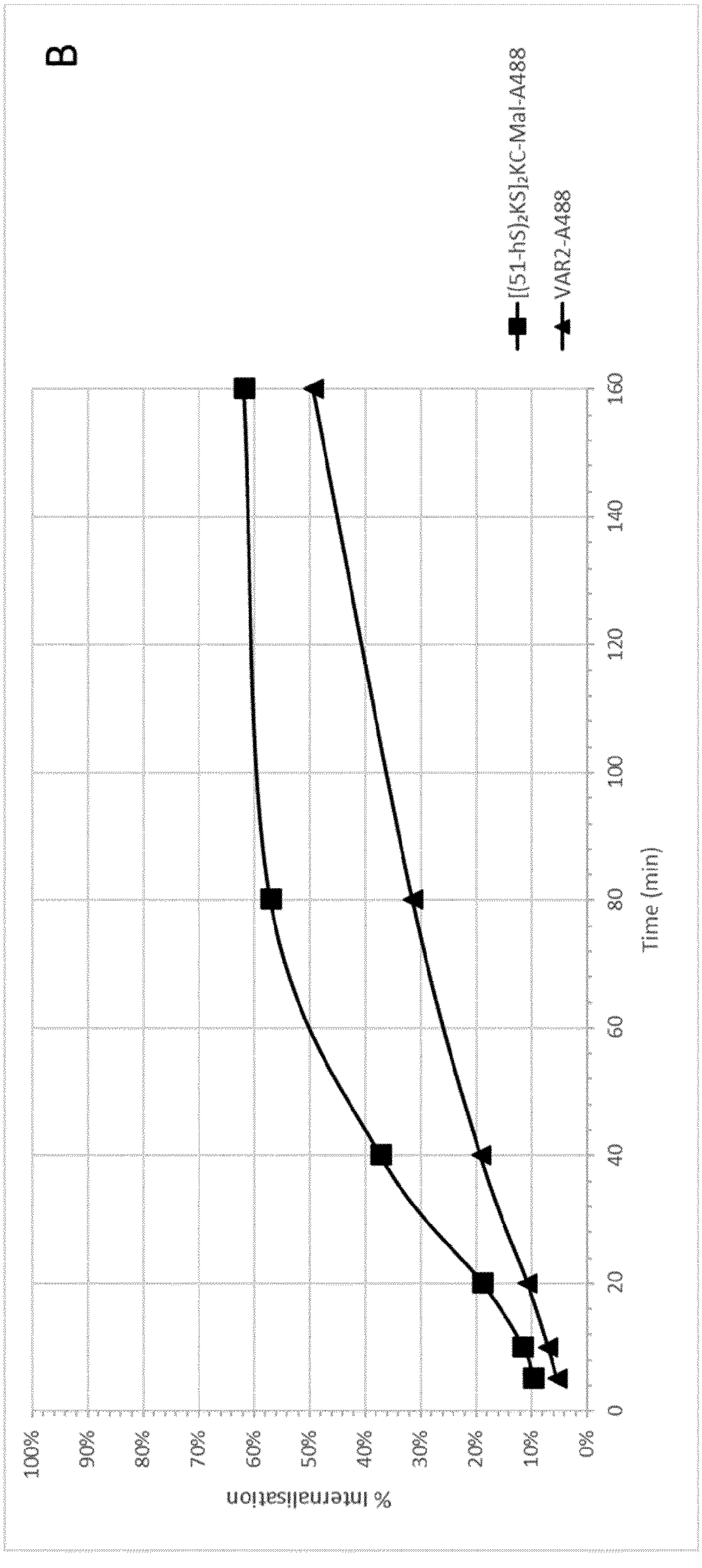
B
[(51-hS)2KS]2KC-Mal-A488
VAR2-A488
% internalisation
100%
90%
80%
70%
60%
50%
40%
30%
20%
10%
0%
Time (min)
0
20
40
60
80
100
120
140
160

Fig.11

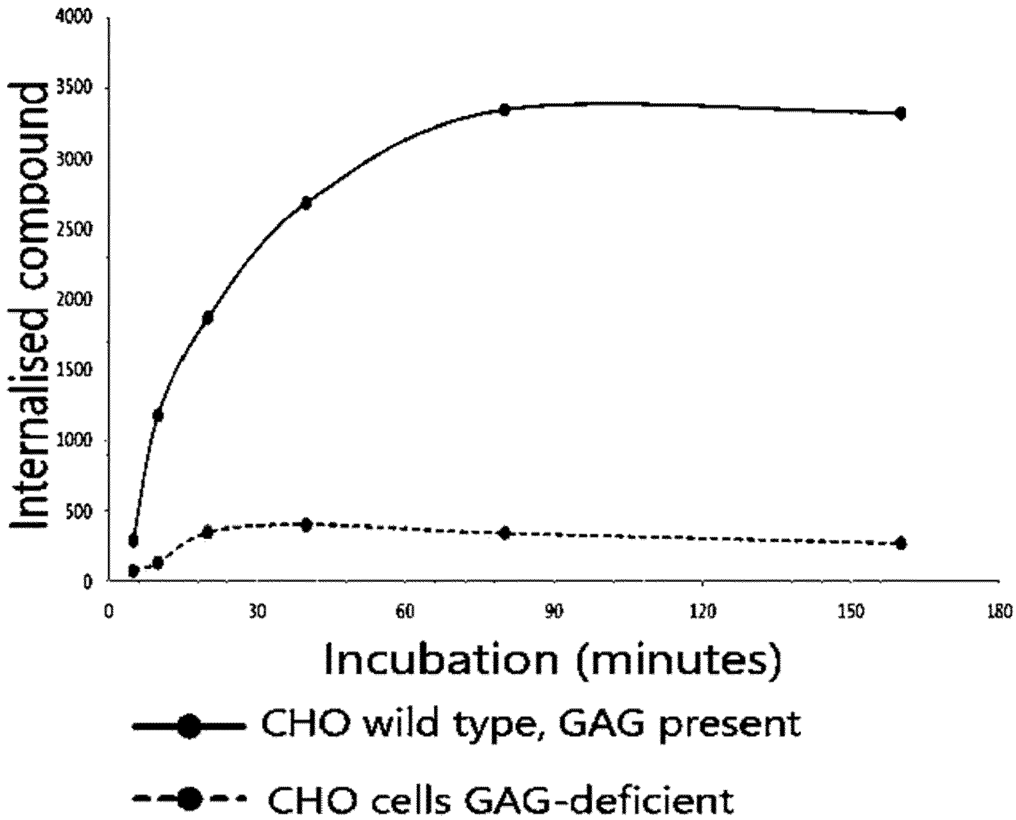
Internalised compound
4000
3500
3000
2500
2000
1500
1000
500
0
0
30
60
90
120
150
180
Incubation (minutes)
CHO wild type, GAG present
CHO cells GAG-deficient

Fig.12

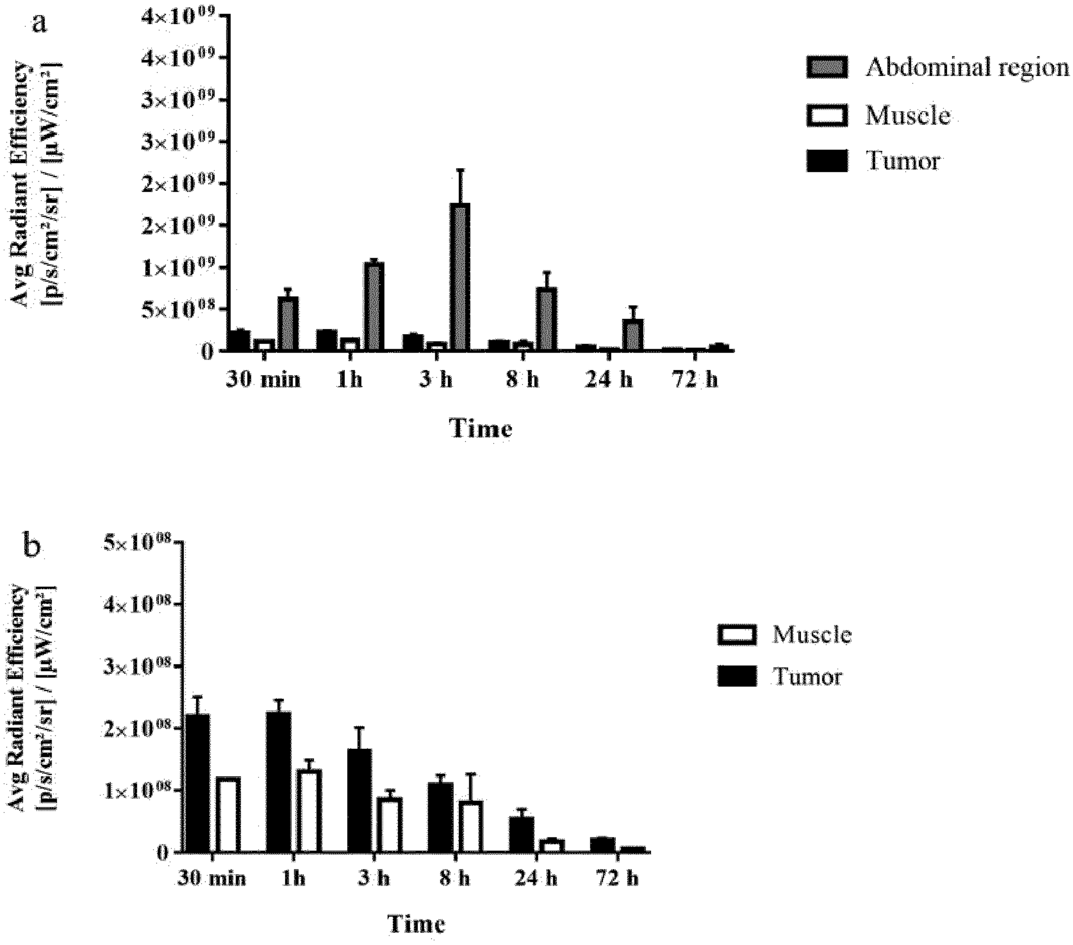
a
Avg Radiant Efficiency [p/s/cm²/sr] / [µW/cm²]
4×10⁰⁹
3×10⁰⁹
2×10⁰⁹
1×10⁰⁹
5×10⁰⁸
0
30 min
1h
3 h
8 h
24 h
72 h
Time
Abdominal region
Muscle
Tumor
b
Avg Radiant Efficiency [p/s/cm²/sr] / [µW/cm²]
5×10⁰⁸
4×10⁰⁸
3×10⁰⁸
2×10⁰⁸
1×10⁰⁸
0
30 min
1h
3 h
8 h
24 h
72 h
Time
Muscle
Tumor

Fig.13

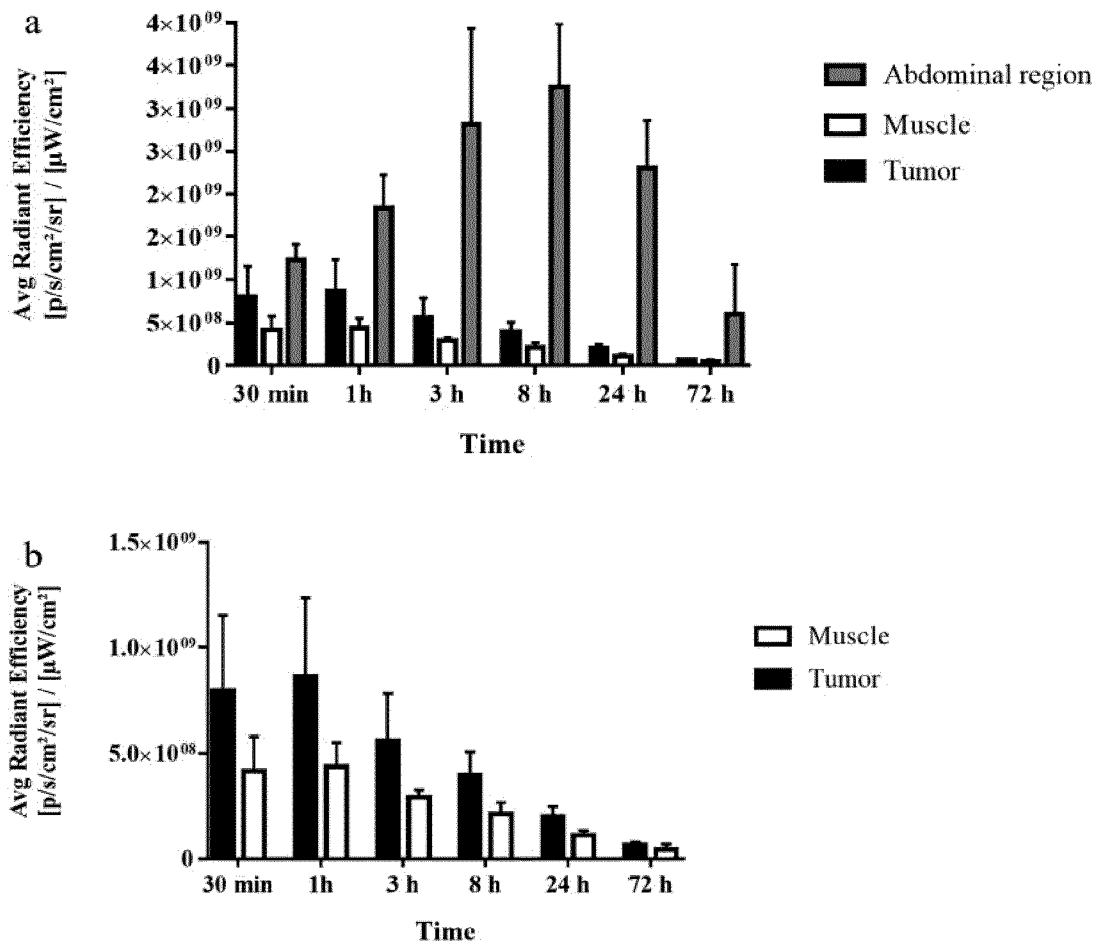
a
Avg Radiant Efficiency [p/s/cm²/sr] / [µW/cm²]
4×10^09
4×10^09
3×10^09
3×10^09
2×10^09
2×10^09
1×10^09
5×10^08
0
30 min
1h
3 h
8 h
24 h
72 h
Time
Abdominal region
Muscle
Tumor
b
Avg Radiant Efficiency [p/s/cm²/sr] / [µW/cm²]
1.5×10^09
1.0×10^09
5.0×10^08
0
30 min
1h
3 h
8 h
24 h
72 h
Time
Muscle
Tumor

**Fig.14**

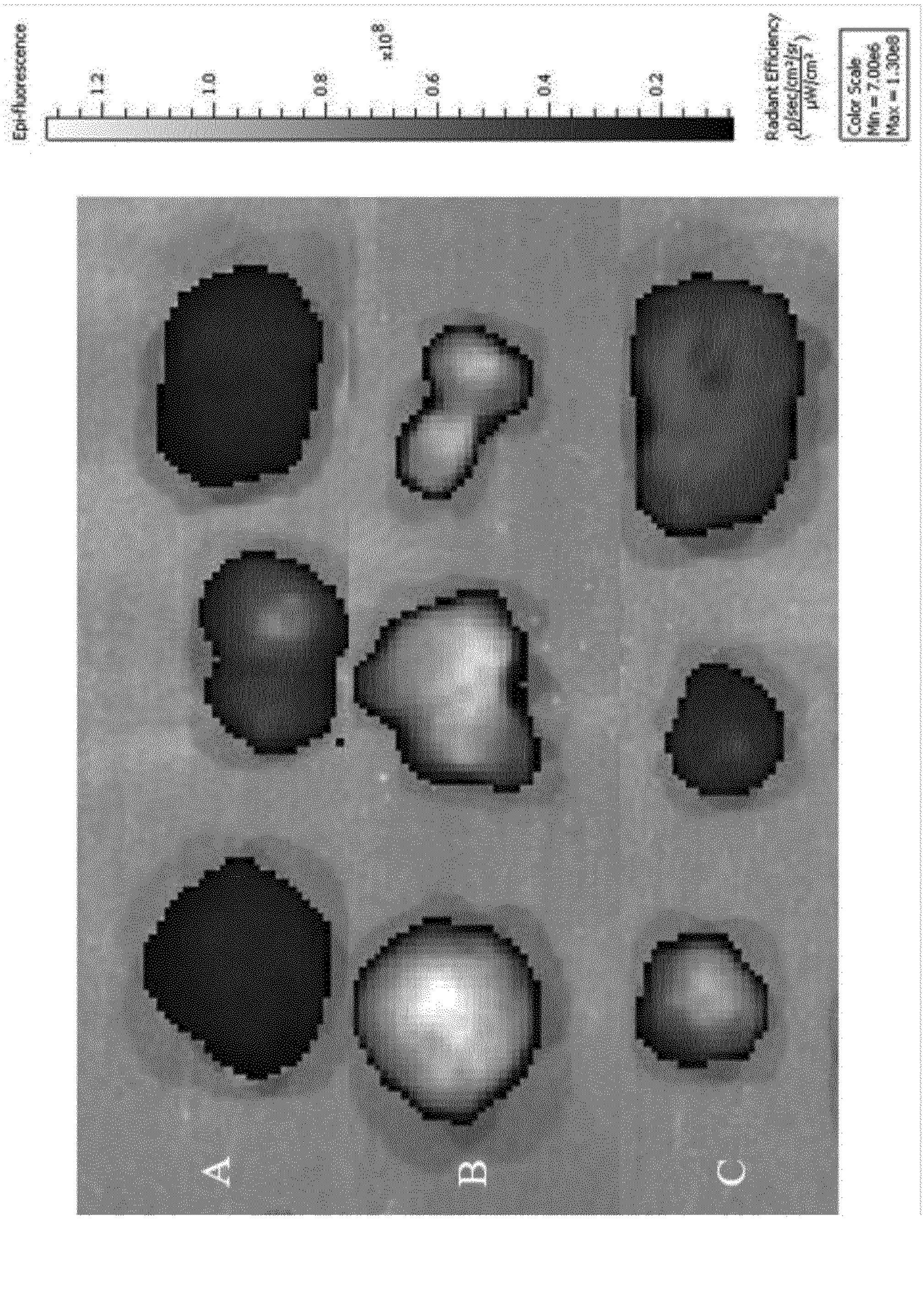
Epi-fluorescence
1.2
1.0
0.8
0.6
0.4
0.2
x10^8
Radiant Efficiency
p/sec/cm²/sr
µW/cm²
Color Scale
Min = 7.00e6
Max = 1.30e8
A
B
C

**Fig.15**

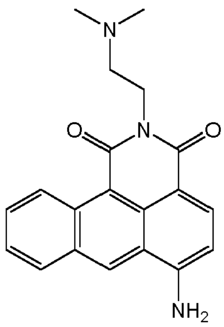

tPSA: 66.64
CLogP: 3.2556

**35**

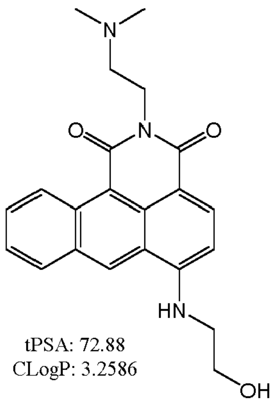

tPSA: 72.88
CLogP: 3.2586

**38a**

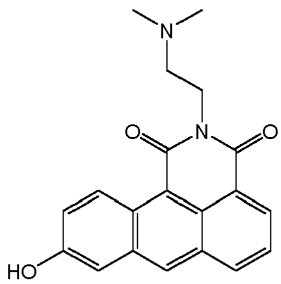

tPSA: 60.85
CLogP: 3.44075

**46**

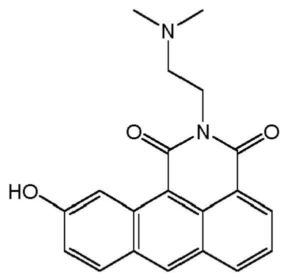

tPSA: 60.85
CLogP: 3.44075

**38b**

Europäisches Patentamt
European Patent Office
Office européen des brevets

# EUROPEAN SEARCH REPORT

Application Number
EP 20 21 5263

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2014/072411 A1 (PHI PHARMA SA [CH]) 15 May 2014 (2014-05-15)<br>* the whole document *<br>----- | 1-15 | INV.<br>A61K47/64<br>A61K49/00<br>A61P35/00 |
| X,D | WO 2015/162285 A1 (PHI PHARMA SA [CH]) 29 October 2015 (2015-10-29)<br>* the whole document *<br>----- | 1-15 | |
| X,D | WO 2015/162287 A1 (PHI PHARMA SA [CH]) 29 October 2015 (2015-10-29)<br>* the whole document *<br>----- | 1-15 | |
| | | | TECHNICAL FIELDS SEARCHED (IPC) |
| | | | A61K<br>A61P |

The present search report has been drawn up for all claims

1

| Place of search | Date of completion of the search | Examiner |
|---|---|---|
| The Hague | 31 May 2021 | Mooren, Nicolai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 21 5263

This annex lists the patent family members relating to the patent documents cited in the above-mentioned European search report. The members are as contained in the European Patent Office EDP file on
The European Patent Office is in no way liable for these particulars which are merely given for the purpose of information.

31-05-2021

| Patent document cited in search report | Publication date | Patent family member(s) | Publication date |
|---|---|---|---|
| WO 2014072411 A1 | 15-05-2014 | AU 2013343526 B2 | 12-09-2019 |
| | | CN 104903342 A | 09-09-2015 |
| | | EP 2917227 A1 | 16-09-2015 |
| | | HK 1209437 A1 | 01-04-2016 |
| | | IL 238428 A | 31-07-2019 |
| | | JP 6595340 B2 | 23-10-2019 |
| | | JP 2015535299 A | 10-12-2015 |
| | | JP 2019001814 A | 10-01-2019 |
| | | KR 20150082411 A | 15-07-2015 |
| | | MX 365599 B | 07-06-2019 |
| | | US 2015299253 A1 | 22-10-2015 |
| | | WO 2014072411 A1 | 15-05-2014 |
| WO 2015162285 A1 | 29-10-2015 | US 2017137465 A1 | 18-05-2017 |
| | | WO 2015162285 A1 | 29-10-2015 |
| WO 2015162287 A1 | 29-10-2015 | US 2017114094 A1 | 27-04-2017 |
| | | WO 2015162287 A1 | 29-10-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010072406 A
- WO 2010072228 A
- WO 2010072275 A
- WO 2014072411 A
- WO 2015162285 A
- WO 2015162287 A
- US 6214345 B
- EP 3130587 A
- WO 2010112471 A
- WO 2003083441 A
- WO 2015001045 A
- US 6747159 B, Caputo
- US 6448008 B, Caputo
- US 6136612 A, Delia Ciana
- US 4981977 A, Southwick
- US 5268486 A, Waggoner
- US 5569587 A, Waggoner
- US 5569766 A, Waggoner
- US 5486616 A, Waggoner
- US 5627027 A, Waggoner
- US 5808044 A, Brush
- US 5877310 A, Reddington
- US 6002003 A, Shen
- US 6004536 A, Leung
- US 6008373 A, Waggoner
- US 6043025 A, Minden
- US 6127134 A, Minden
- US 6130094 A, Waggoner
- US 6133445 A, Waggoner
- US 7445767 B, Licha
- US 6534041 B, Licha
- US 7547721 B, Miwa
- US 7488468 B, Miwa
- US 7473415 B, Kawakami
- WO 9617628 A
- EP 0796 I
- EP 1181940 B1
- EP 0988060 B1
- WO 9847538 A
- WO 0016810 A
- EP 1113822 B1
- WO 0143781 A
- EP 1237583 A1
- WO 03074091 A
- EP 1480683 B1
- WO 06072580 A
- EP 1833513 A1
- EP 1679082 A1
- WO 9740104 A
- WO 9951702 A
- WO 0121624 A
- EP 1065250 A1


### Non-patent literature cited in the description


- Remington's Pharmaceutical Sciences. Mack Publishing Company, 1990, 1445
- **KUBASKI et al.** Glycosaminoglycans detection methods: Applications of mass spectrometry. *Mol Genet Metab,* 2017, vol. 120 (1-2), 67-77
- **SAMI et al.** *J. Med. Chem.,* 1996, vol. 39 (8), 1609-1618
- **SAMI et al.** *J. Med. Chem.,* 1996, vol. 39 (25), 4978-4987
- **MAYR et al.** *Anti-Cancer Drugs,* 1999, vol. 10 (2), 163-170
- **MIN et al.** *Archives of Pharmacal Research,* 2018, vol. 41, 594-616
- **WEISSLEDE et al.** *Nature Biotech.,* 1999, vol. 17, 375-378
- **BREMER et al.** *Nature Med.,* 2001, vol. 7, 743-748
- **CAMPO et al.** *Photochem. Photobiol.,* 2007, vol. 83, 958-965
- **TYAGI et al.** *Nat. Biotechnol,* 2000, vol. 18, 1191-1196
- **TYAGI et al.** *Nat. Biotechnol.,* 1998, vol. 16, 49-53
- **ACHILEFU et al.** *Invest. Radiol,* 2000, vol. 35, 479-485
- **BECKER et al.** *Nature Biotech.,* 2001, vol. 19, 327-331
- **BUJAI et al.** *J. Biomed. Opt.,* 2001, vol. 6, 122-133
- **BALLOU et al.** *Biotechnol. Prog.,* 1997, vol. 13, 649-658
- **NERI et al.** *Nature Biotech,* 1997, vol. 15, 1271-1275
- **LACKOWICZ.** Principles of Fluorescence Spectroscopy. Kluwar Academic, 1999
- *Tetrahedron Letters,* 2000, vol. 41, 9185-88
- Remington: The Science and Practice of Pharmacy. Mack Publishing Company, 2005

- Pharmaceutical Formulation Development of Peptides and Proteins. Taylor & Francis, 2000
- Handbook of Pharmaceutical Excipients. Pharmaceutical Press, 2000